# EUROPEAN PATENT APPLICATION

(11) **EP 4 089 078 A1**
(43) Date of publication of application: **16.11.2022**
(21) Application number: 20912650.7
(22) Date of filing: 11.12.2020
(51) Int. Cl.: C07D 239/22, C07D 239/54, C07D 409/10, A01N 43/54

(54) **CARBOXYLIC ACID DERIVATIVE-SUBSTITUTED IMINO ARYL COMPOUND, PREPARATION METHOD THEREFOR, HERBICIDAL COMPOSITION AND USE THEREOF**

(30) Priority: 11.01.2020 CN 202010028477; 24.01.2020 CN 202010077193; 25.02.2020 CN 202010117877; 10.04.2020 CN 202010281666
(71) Applicant: Qingdao KingAgroot Chemical Compound Co., Ltd., Qingdao, Shandong 266000 (CN)
(72) Inventor: LIAN, Lei, Qingdao, Shandong 266000 (CN); HUA, Rongbao, Qingdao, Shandong 266000 (CN); PENG, Xuegang, Qingdao, Shandong 266000 (CN); ZHAO, De, Qingdao, Shandong 266000 (CN); CUI, Qi, Qingdao, Shandong 266000 (CN)
(74) Representative: Grünecker Patent- und Rechtsanwälte PartG mbB
(86) International application number: PCT/CN2020/135784
(87) International publication number: WO 2021/139482

(57) **Abstract**

The invention relates to the field of pesticide technology, and in particular a type of carboxylic acid derivative-substituted iminoaryl compound, preparation method, herbicidal composition and use thereof. The compound, as shown in general formula I: 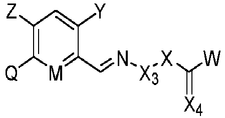 wherein, Q represents 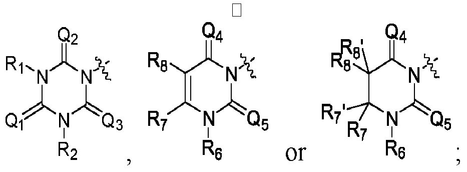 Y represents halogen, haloalkyl or cyano; Z represents halogen; M represents CH or N; W represents OX₅, SX₅ or N(X₅)₂; X represents -CX₁X₂-(alkyl)ₙ-, -alkyl-CX₁X₂-(alkyl)ₙ- or -(CH₂)ᵣ-; X₃ and X₄ each independently represent O, S, NH or N-alkyl, etc.. The compound has excellent herbicidal activity against gramineous weeds, broadleaf weeds, cyperaceae weeds and so on even at low application rates, and has high selectivity for crops.

## Description

### Technical Field

The invention relates to the field of pesticide technology, and in particular a type of carboxylic acid derivative-substituted iminoaryl compound, preparation method, herbicidal composition and use thereof.

### Technical background

Weed control is one of the most important links in the course of achieving high-efficiency agriculture. Various herbicides are available in the market, for example, patents WO00/50409 etc. disclose the use of a compound of general formula 1-aryl-4-thiotriazine as a herbicide, WO95/06641 discloses a compound of substituted 1-amino-3-phenyluracils with herbicidal activity, WO95/25725 discloses a compound of pyrimidinyl aryl ketone oximes with herbicidal and insecticidal activities. However, the herbicidal properties of these known compounds against harmful plants and their selectivities to crops are not completely satisfactory. And scientists still need to do continuously research and develop new herbicides with high efficacy, safety, economics and different modes of action due to problems such as the growing market, weed resistance, the service life and economics of pesticides as well as people's increasing concern on environment.

### Invention contents

The invention relates to the field of pesticide technology, and in particular a type of carboxylic acid derivative-substituted iminoaryl compound, preparation method, herbicidal composition and use thereof. The compound has excellent herbicidal activity against gramineous weeds, broadleaf weeds, cyperaceae weeds and so on even at low application rates, and has high selectivity for crops.

The technical solution adopted by the invention is as follows:
A carboxylic acid derivative-substituted iminoaryl compound, represented by general formula I':

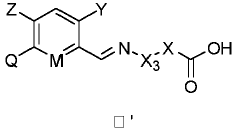

Wherein, the derivative refers to a derivative suitable for agricultural chemistry, which is used to describe the change of the carboxylic acid functional group of the present invention, and it refers to any ester, acylhydrazide, imidate, thioimidate, amidine, amide, orthoester, acyl cyanide, acyl halide, thioester, thionoester, dithiolester, nitrile or any other carboxylic acid derivative well known in the art.

To be specific, the carboxylic acid derivative-substituted iminoaryl compound, represented by general formula I:

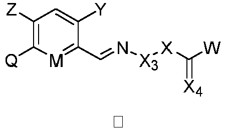

In the above general formulas I'and I,
Q represents 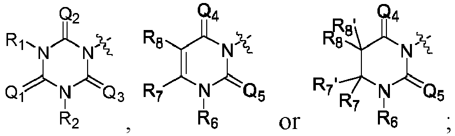
Y represents halogen, haloalkyl or cyano;
Z represents halogen;
M represents CH or N;
W represents OX₅, SX₅ or N(X₅)₂;
X represents -CX₁X₂-(alkyl)ₙ-, -alkyl-CX₁X₂-(alkyl)ₙ- or -(CH₂)ᵣ-;
X₁, X₂ each independently represent H, halogen, cyano, amino, nitro, formyl, cyanoalkyl, hydroxyalkyl, alkyl, alkenyl, alkynyl, cycloalkyl, cycloalkylalkyl, alkoxy, alkylthio, alkylamino, haloalkoxy, haloalkylthio, alkyl carbonyl, alkoxy carbonyl, alkoxyalkyl, haloalkoxyalkyl, alkylaminoalkyl, aryl, heterocyclyl, arylalkyl or heterocyclic alkyl, wherein, the "alkyl", "alkenyl" and "alkynyl" are each independently unsubstituted or substituted by halogen, the "cycloalkyl", "cycloalkylalkyl", "aryl", "heterocyclyl", "arylalkyl" and "heterocyclic alkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring; and X₁, X₂ are not hydrogen at the same time;
X₃, X₄ each independently represent O, S, NH or N-alkyl;
X₅ represents H, alkyl, alkenyl, alkynyl, cycloalkyl, cycloalkenyl, heterocyclyl, aryl, 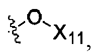 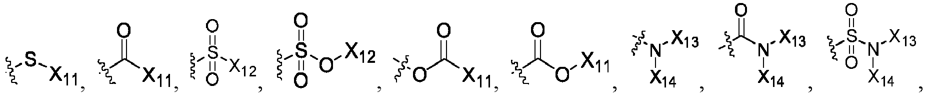 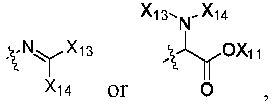 wherein, the "alkyl", "alkenyl" and "alkynyl" are each independently unsubstituted or substituted by at least one group selected from halogen, cyano, nitro, cycloalkyl, cycloalkenyl, heterocyclyl, aryl, 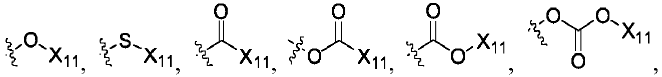 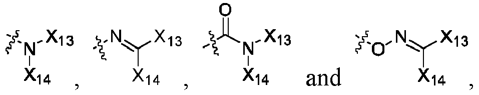 the "cycloalkyl", "cycloalkenyl", "heterocyclyl" and "aryl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
or N(X₅)₂ represents 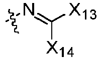 or unsubstituted or substituted heterocyclyl with nitrogen atom at 1-position;
Q₁, Q₂, Q₃, Q₄, Q₅ each independently represent O or S;
R₁, R₂ each independently represent H, cyano, alkyl, alkenyl, alkynyl, formyl alkyl, cyanoalkyl, amino, aminoalkyl, amino carbonyl, amino carbonylalkyl, aminosulfonyl, cycloalkyl, cycloalkylalkyl, cycloalkenyl, cycloalkenyl alkyl, heterocyclyl, heterocyclic alkyl, aryl, arylalkyl, R₄R₅N-(CO)-NR₃-, 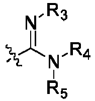 , R₃-S(O)ₘ-(alkyl)ₙ-, R₃-O-(alkyl)ₙ-, R₃-(CO)-(alkyl)ₙ-, R₃-O-(alkyl)ₙ-(CO)-, R₃-(CO)-O-(alkyl)ₙ-, R₃-S-(CO)-(alkyl)ₙ-, R₃-O-(CO)-alkyl- or R₃-O-(CO)-O-alkyl-, wherein,
the "alkyl", "alkenyl" and "alkynyl" are each independently unsubstituted or substituted by halogen,
the "amino", "aminoalkyl", "amino carbonyl", "amino carbonylalkyl" and "aminosulfonyl" are each independently unsubstituted or substituted by one or two groups selected from -R₁₁, -OR₁₁, -(CO)R₁₁, -(CO)OR₁₁, -alkyl-(CO)OR₁₁, -(SO₂)R₁₁, -(SO₂)OR₁₁, -alkyl-(SO₂)R₁₁, -(CO)N(R₁₂)₂ and -(SO₂)N(R₁₂)₂,
the "cycloalkyl", "cycloalkylalkyl", "cycloalkenyl", "cycloalkenyl alkyl", "heterocyclyl", "heterocyclic alkyl", "aryl" and "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
R₆ represents alkyl, alkenyl, alkynyl or cyano, wherein, the "alkyl", "alkenyl" and "alkynyl" are each independently unsubstituted or substituted by at least one group selected from halogen, alkoxy and alkoxy carbonyl;
R₇, R₇', R₈, R₈' each independently represent H, alkyl, halogen, haloalkyl, amino, hydroxyalkyl or alkoxy;
X₁₁ independently represents H, alkyl, alkenyl, alkynyl, haloalkyl, haloalkenyl, haloalkynyl, cycloalkyl, cycloalkylalkyl, cycloalkenyl, cycloalkenyl alkyl, heterocyclyl, heterocyclic alkyl, aryl, arylalkyl or 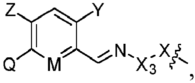 wherein, the "cycloalkyl", "cycloalkylalkyl", "cycloalkenyl", "cycloalkenyl alkyl", "heterocyclyl", "heterocyclic alkyl", "aryl" and "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
X₁₂ independently represents alkyl, alkenyl, alkynyl, haloalkyl, haloalkenyl, haloalkynyl, cycloalkyl, cycloalkylalkyl, cycloalkenyl, cycloalkenyl alkyl, heterocyclyl, heterocyclic alkyl, aryl or arylalkyl, wherein, the "cycloalkyl", "cycloalkylalkyl", "cycloalkenyl", "cycloalkenyl alkyl", "heterocyclyl", "heterocyclic alkyl", "aryl" and "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
X₁₃, X₁₄ each independently represent H, halogen, cyano, alkoxy, alkoxyalkyl, alkyl carbonyl, alkoxy carbonyl, alkylsulfonyl, alkyl, alkenyl, alkynyl, cycloalkyl, cycloalkylalkyl, cycloalkenyl, cycloalkenyl alkyl, aryl, arylalkyl, heterocyclyl or heterocyclic alkyl, or C, X₁₃, X₁₄, taken together, form unsubstituted or substituted cyclic structure, or N, X₁₃, X₁₄, taken together, form unsubstituted or substituted heterocyclyl with nitrogen atom at 1-position, wherein, the "alkyl", "alkenyl" and "alkynyl" are each independently unsubstituted or substituted by halogen, the "cycloalkyl", "cycloalkylalkyl", "cycloalkenyl", "cycloalkenyl alkyl", "aryl", "arylalkyl", "heterocyclyl" and "heterocyclic alkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
R₃, R₄, R₅ each independently represent H, alkyl, alkenyl, alkynyl, cycloalkyl, cycloalkylalkyl, cycloalkenyl, cycloalkenylalkyl, heterocyclyl, heterocyclic alkyl, aryl or arylalkyl, wherein, the "alkyl", "alkenyl" and "alkynyl" are each independently unsubstituted or substituted by halogen, the "cycloalkyl", "cycloalkylalkyl", "cycloalkenyl", "cycloalkenylalkyl", "heterocyclyl", "heterocyclic alkyl", "aryl" and "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
R₁₁ independently represents alkyl, alkenyl, alkynyl, cycloalkyl, cycloalkylalkyl, cycloalkenyl, cycloalkenylalkyl, phenyl, benzyl, wherein, the "alkyl", "alkenyl" and "alkynyl" are each independently unsubstituted or substituted by halogen, the "phenyl" and "benzyl" are each independently unsubstituted or substituted by at least one group selected from halogen, cyano, nitro, alkyl, haloalkyl, alkoxy carbonyl, alkylthio, alkylsulfonyl, alkoxy and haloalkoxy;
R₁₂ independently represents H, alkyl, alkenyl, alkynyl, alkoxy, alkylsulfonyl, cycloalkyl, cycloalkylalkyl, cycloalkenyl or cycloalkenylalkyl, or N(R₁₂)₂ in -(CO)N(R₁₂)₂ or -(SO₂)N(R₁₂)₂ each independently represents unsubstituted or substituted heterocyclyl with nitrogen atom at 1-position;
R₁₃ independently represents H, alkyl, haloalkyl, phenyl or phenyl substituted by at least one group selected from halogen, cyano, nitro, alkyl, haloalkyl, alkoxy carbonyl, alkylthio, alkylsulfonyl, alkoxy and haloalkoxy;
r represents an integer of 2 or more; m represents 0, 1 or 2; n independently represents 0 or 1.

Preferably, Y represents halogen, halo C1-C8 alkyl or cyano;
X represents -CX₁X₂-(C1-C8 alkyl)n-, -(C1-C8 alkyl)-CX₁X₂-(C1-C8 alkyl)n- or -(CH₂)ᵣ-;
X₁, X₂ each independently represent H, halogen, cyano, amino, nitro, formyl, cyano C1-C8 alkyl, hydroxy C1-C8 alkyl, C1-C8 alkyl, C2-C8 alkenyl, C2-C8 alkynyl, C3-C8 cycloalkyl, C3-C8 cycloalkyl C1-C8 alkyl, C1-C8 alkoxy, C1-C8 alkylthio, C1-C8 alkylamino, halo C1-C8 alkoxy, halo C1-C8 alkylthio, C1-C8 alkyl carbonyl, C1-C8 alkoxy carbonyl, C1-C8 alkoxy C1-C8 alkyl, halo C1-C8 alkoxy C1-C8 alkyl, C1-C8 alkylamino C1-C8 alkyl, aryl, heterocyclyl, aryl C1-C8 alkyl or heterocyclyl C1-C8 alkyl, wherein, the "C1-C8 alkyl", "C2-C8 alkenyl" and "C2-C8 alkynyl" are each independently unsubstituted or substituted by halogen, the "C3-C8 cycloalkyl", "C3-C8 cycloalkyl C1-C8 alkyl", "aryl", "heterocyclyl", "aryl C1-C8 alkyl" and "heterocyclyl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring; and X₁, X₂ are not hydrogen at the same time;
X₃, X₄ each independently represent O, S, NH or N-(C1-C8)alkyl;
X₅ represents H, C1-C8 alkyl, C2-C8 alkenyl, C2-C8 alkynyl, C3-C8 cycloalkyl, C3-C8 cycloalkenyl, heterocyclyl, aryl, 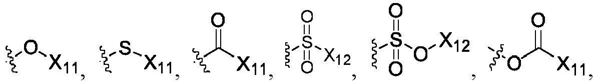 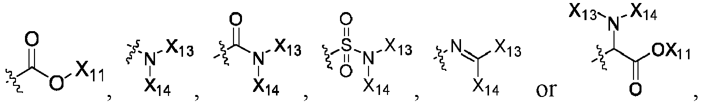 wherein, the "C1-C8 alkyl", "C2-C8 alkenyl" and "C2-C8 alkynyl" are each independently unsubstituted or substituted by at least one group selected from halogen, cyano, nitro, C3-C8 cycloalkyl, C3-C8 cycloalkenyl, heterocyclyl, aryl, 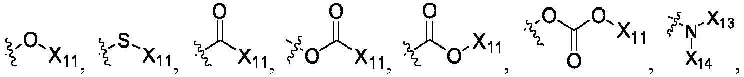 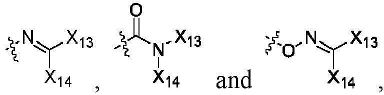 the "C3-C8 cycloalkyl", "C3-C8 cycloalkenyl", "heterocyclyl" and "aryl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
or N(X₅)₂ represents 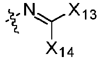 or heterocyclyl 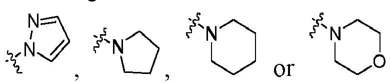 with nitrogen atom at 1-position that is unsubstituted or substituted by at least one group selected from oxo and C1-C8 alkyl;
R₁, R₂ each independently represent H, cyano, C1-C8 alkyl, C2-C8 alkenyl, C2-C8 alkynyl, formyl C1-C8 alkyl, cyano C1-C8 alkyl, amino, amino C1-C8 alkyl, amino carbonyl, amino carbonyl C1-C8 alkyl, aminosulfonyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl, aryl C1-C8 alkyl, R₄R₅N-(CO)-NR₃-, 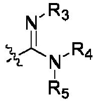 , R₃-S(O)ₘ-(C1-C8 alkyl)ₙ-, R₃-O-(C1-C8 alkyl)ₙ-, R₃-(CO)-(C1-C8 alkyl)n-, R₃-O-(C1-C8 alkyl)ₙ-(CO)-, R₃-(CO)-O-(C1-C8 alkyl)n-, R₃-S-(CO)-(C1-C8 alkyl)n-, R₃-O-(CO)-(C1-C8 alkyl)- or R₃-O-(CO)-O-(C1-C8 alkyl)-, wherein,
the "C1-C8 alkyl", "C2-C8 alkenyl" and "C2-C8 alkynyl" are each independently unsubstituted or substituted by halogen,
the "amino", "amino C1-C8 alkyl", "amino carbonyl", "amino carbonyl C1-C8 alkyl" and "aminosulfonyl" are each independently unsubstituted or substituted by one or two groups selected from -R₁₁, -OR₁₁, -(CO)R₁₁, -(CO)OR₁₁, -(C1-C8 alkyl)-(CO)OR₁₁, -(SO₂)R₁₁, -(SO₂)OR₁₁, -(C1-C8 alkyl)-(SO₂)R₁₁, -(CO)N(R₁₂)₂ and -(SO₂)N(R₁₂)₂,
the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" and "aryl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
R₆ represents C1-C8 alkyl, C2-C8 alkenyl, C2-C8 alkynyl or cyano, wherein, the "C1-C8 alkyl", "C2-C8 alkenyl" and "C2-C8 alkynyl" are each independently unsubstituted or substituted by at least one group selected from halogen, C1-C8 alkoxy and C1-C8 alkoxy carbonyl;
R₇, R₇', R₈, R₈' each independently represent H, C1-C8 alkyl, halogen, halo C1-C8 alkyl, amino, hydroxy C1-C8 alkyl or C1-C8 alkoxy;
X₁₁ independently represents H, C1-C8 alkyl, C2-C8 alkenyl, C2-C8 alkynyl, halo C1-C8 alkyl, halo C2-C8 alkenyl, halo C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl, aryl C1-C8 alkyl or 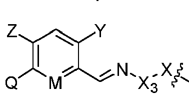 , wherein, the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" and "aryl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
X₁₂ independently represents C1-C8 alkyl, C2-C8 alkenyl, C2-C8 alkynyl, halo C1-C8 alkyl, halo C2-C8 alkenyl, halo C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl or aryl C1-C8 alkyl, wherein, the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" and "aryl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
X₁₃, X₁₄ each independently represent H, halogen, cyano, C1-C8 alkoxy, C1-C8 alkoxy C1-C8 alkyl, C1-C8 alkyl carbonyl, C1-C8 alkoxy carbonyl, C1-C8 alkylsulfonyl, C1-C8 alkyl, C2-C8 alkenyl, C2-C8 alkynyl, C3-C8 cycloalkyl, C3-C8 cycloalkylalkyl, C3-C8 cycloalkenyl, C3-C8 cycloalkenyl C1-C8 alkyl, aryl, aryl C1-C8 alkyl, heterocyclyl or heterocyclyl C1-C8 alkyl, or C, X₁₃, X₁₄, taken together, form 5~8 membered carbocyclyl or oxygen, sulfur or nitrogen-containing heterocyclyl, or N, X₁₃, X₁₄, taken together, form heterocyclyl with nitrogen atom at 1-position, wherein, the "C1-C8 alkyl", "C2-C8 alkenyl" and "C2-C8 alkynyl" are each independently unsubstituted or substituted by halogen, the "C3-C8 cycloalkyl", "C3-C8 cycloalkyl C1-C8 alkyl", "C3-C8 cycloalkenyl", "C3-C8 cycloalkenyl C1-C8 alkyl", "aryl", "aryl C1-C8 alkyl", "heterocyclyl" and "heterocyclyl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring, the "5~8 membered carbocyclyl or oxygen, sulfur or nitrogen-containing heterocyclyl" is unsubstituted or substituted by 1-4 groups selected from C1-C8 alkyl, C1-C8 alkoxy carbonyl and benzyl, or together with aryl or heterocyclyl forms a fused ring, the "heterocyclyl with nitrogen atom at 1-position"is unsubstituted or substituted by at least one group selected from oxo and C1-C8 alkyl;
R₃, R₄, R₅ each independently represent H, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl or aryl C1-C8 alkyl, wherein, the "C1-C8 alkyl", "C2-C8 alkenyl" and "C2-C8 alkynyl" are each independently unsubstituted or substituted by halogen, the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" and "aryl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
R₁₁ independently represents C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, phenyl, benzyl, wherein, the "C1-C8 alkyl", "C2-C8 alkenyl" and "C2-C8 alkynyl" are each independently unsubstituted or substituted by halogen, the "phenyl" and "benzyl" are each independently unsubstituted or substituted by at least one group selected from halogen, cyano, nitro, C1-C8 alkyl, halo C1-C8 alkyl, C1-C8 alkoxy carbonyl, C1-C8 alkylthio, C1-C8 alkylsulfonyl, C1-C8 alkoxy and halo C1-C8 alkoxy;
R₁₂ independently represents H, C1-C8 alkyl, C2-C8 alkenyl, C2-C8 alkynyl, C1-C8 alkoxy, C1-C8 alkylsulfonyl, C3-C8 cycloalkyl, C3-C8 cycloalkyl C1-C8 alkyl, C3-C8 cycloalkenyl or C3-C8 cycloalkenyl C1-C8 alkyl, or N(R₁₂)₂ in -(CO)N(R₁₂)₂ or -(SO₂)N(R₁₂)₂ independently represents heterocyclyl 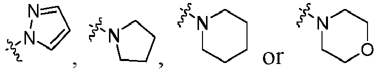 with nitrogen atom at 1-position that is unsubstituted or substituted by at least one group selected from oxo and C1-C8 alkyl;
R₁₃ independently represents H, C1-C8 alkyl, halo C1-C8 alkyl, phenyl or phenyl substituted by at least one group selected from halogen, cyano, nitro, C1-C8 alkyl, halo C1-C8 alkyl, C1-C8 alkoxy carbonyl, C1-C8 alkylthio, C1-C8 alkylsulfonyl, C1-C8 alkoxy and halo C1-C8 alkoxy;
r represents 2, 3, 4, 5 or 6.

More preferably, Y represents halogen, halo C1-C6 alkyl or cyano;
X represents -CX₁X₂-(C1-C6 alkyl)n-, -(C1-C6 alkyl)-CX₁X₂-(C1-C6 alkyl)n- or -(CH₂)ᵣ-;
X₁, X₂ each independently represent H, halogen, cyano, amino, nitro, formyl, cyano C1-C6 alkyl, hydroxy C1-C6 alkyl, C1-C6 alkyl, C2-C6 alkenyl, C2-C6 alkynyl, C3-C6 cycloalkyl, C3-C6 cycloalkyl C1-C6 alkyl, C1-C6 alkoxy, C1-C6 alkylthio, C1-C6 alkylamino, halo C1-C6 alkoxy, halo C1-C6 alkylthio, C1-C6 alkyl carbonyl, C1-C6 alkoxy carbonyl, C1-C6 alkoxy C1-C6 alkyl, halo C1-C6 alkoxy C1-C6 alkyl, C1-C6 alkylamino C1-C6 alkyl, aryl, heterocyclyl, aryl C1-C6 alkyl or heterocyclyl C1-C6 alkyl, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C6 alkyl", "aryl", "heterocyclyl", "aryl C1-C6 alkyl" and "heterocyclyl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring; and X₁, X₂ are not hydrogen at the same time;
X₃, X₄ each independently represent O, S, NH or N-(C1-C6)alkyl;
X₅ represents H, C1-C6 alkyl, C2-C6 alkenyl, C2-C6 alkynyl, C3-C6 cycloalkyl, C3-C6 cycloalkenyl, heterocyclyl, aryl, 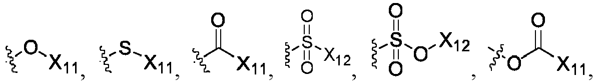 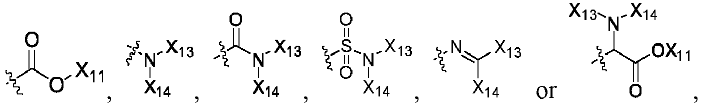 wherein, the "C1-C6 alkyl", "C2-C6 alkenyl" and "C2-C6 alkynyl" are each independently unsubstituted or substituted by at least one group selected from halogen, cyano, nitro, C3-C6 cycloalkyl, C3-C6 cycloalkenyl, heterocyclyl, aryl, 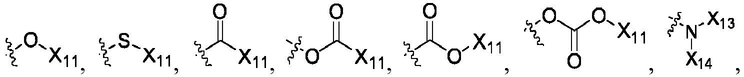 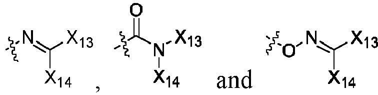 , the "C3-C6 cycloalkyl", "C3-C6 cycloalkenyl", "heterocyclyl" and "aryl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
or N(X₅)₂ represents 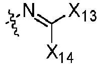 or heterocyclyl 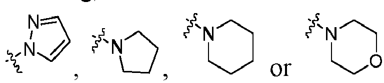 with nitrogen atom at 1-position that is unsubstituted or substituted by 1, 2 or 3 groups selected from oxo and C1-C6 alkyl;
R₁, R₂ each independently represent H, cyano, C1-C6 alkyl, C2-C6 alkenyl, C2-C6 alkynyl, formyl C1-C6 alkyl, cyano C1-C6 alkyl, amino, amino C1-C6 alkyl, amino carbonyl, amino carbonyl C1-C6 alkyl, aminosulfonyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl, aryl C1-C6 alkyl, R₄R₅N-(CO)-NR₃-, 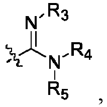 R₃-S(O)ₘ-(C1-C6 alkyl)ₙ-, R₃-O-(C1-C6 alkyl)ₙ-, R₃-(CO)-(C1-C6 alkyl)n-, R₃-O-(C1-C6 alkyl)ₙ-(CO)-, R₃-(CO)-O-(C1-C6 alkyl)n-, R₃-S-(CO)-(C1-C6 alkyl)n-, R₃-O-(CO)-(C1-C6 alkyl)- or R₃-O-(CO)-O-(C1-C6 alkyl)-, wherein,
the "C1-C6 alkyl", "C2-C6 alkenyl" and "C2-C6 alkynyl" are each independently unsubstituted or substituted by halogen,
the "amino", "amino C1-C6 alkyl", "amino carbonyl", "amino carbonyl C1-C6 alkyl" and "aminosulfonyl" are each independently unsubstituted or substituted by one or two groups selected from -R₁₁, -OR₁₁, -(CO)R₁₁, -(CO)OR₁₁, -(C1-C6 alkyl)-(CO)OR₁₁, -(SO₂)R₁₁, -(SO₂)OR₁₁, -(C1-C6 alkyl)-(SO₂)R₁₁, -(CO)N(R₁₂)₂ and -(SO₂)N(R₁₂)₂,
the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" and "aryl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
R₆ represents C1-C6 alkyl, C2-C6 alkenyl, C2-C6 alkynyl or cyano, wherein, the "C1-C6 alkyl", "C2-C6 alkenyl" and "C2-C6 alkynyl" are each independently unsubstituted or substituted by 1, 2 or 3 groups selected from halogen, C1-C6 alkoxy and C1-C6 alkoxy carbonyl;
R₇, R₇', R₈, R₈' each independently represent H, C1-C6 alkyl, halogen, halo C1-C6 alkyl, amino, hydroxy C1-C6 alkyl or C1-C6 alkoxy;
X₁₁ independently represents H, C1-C6 alkyl, C2-C6 alkenyl, C2-C6 alkynyl, halo C1-C6 alkyl, halo C2-C6 alkenyl, halo C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl, aryl C1-C6 alkyl or 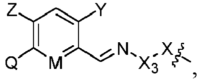 wherein, the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" and "aryl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃,
-(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
X₁₂ independently represents C1-C6 alkyl, C2-C6 alkenyl, C2-C6 alkynyl, halo C1-C6 alkyl, halo C2-C6 alkenyl, halo C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl or aryl C1-C6 alkyl, wherein, the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" and "aryl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
X₁₃, X₁₄ each independently represent H, halogen, cyano, C1-C6 alkoxy, C1-C6 alkoxy C1-C6 alkyl, C1-C6 alkyl carbonyl, C1-C6 alkoxy carbonyl, C1-C6 alkylsulfonyl, C1-C6 alkyl, C2-C6 alkenyl, C2-C6 alkynyl, C3-C6 cycloalkyl, C3-C6 cycloalkylalkyl, C3-C6 cycloalkenyl, C3-C6 cycloalkenyl C1-C6 alkyl, aryl, aryl C1-C6 alkyl, heterocyclyl or heterocyclyl C1-C6 alkyl, or C, X₁₃, X₁₄, taken together, form 5~8 membered carbocyclyl or oxygen, sulfur or nitrogen-containing heterocyclyl, or N, X₁₃, X₁₄, taken together, form heterocyclyl 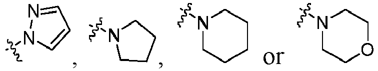 with nitrogen atom at 1-position, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C6 alkyl", "C3-C6 cycloalkenyl", "C3-C6 cycloalkenyl C1-C6 alkyl", "aryl", "aryl C1-C6 alkyl", "heterocyclyl" and "heterocyclyl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring, the "5~8 membered carbocyclyl or oxygen, sulfur or nitrogen-containing heterocyclyl" is unsubstituted or substituted by 1, 2 or 3 groups selected from C1-C6 alkyl, C1-C6 alkoxy carbonyl and benzyl, or together with aryl or heterocyclyl forms a fused ring, the 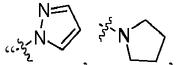 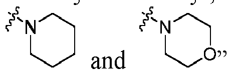 are unsubstituted or substituted by 1, 2 or 3 groups selected from oxo and C1-C6 alkyl;
R₃, R₄, R₅ each independently represent H, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl or aryl C1-C6 alkyl, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" and "aryl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
R₁₁ independently represents C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, phenyl, benzyl, wherein, the "C1-C6 alkyl", "C2-C6 alkenyl" and "C2-C6 alkynyl" are each independently unsubstituted or substituted by halogen, the "phenyl" and "benzyl"are each independently unsubstituted or substituted by 1, 2 or 3 groups selected from halogen, cyano, nitro, C1-C6 alkyl, halo C1-C6 alkyl, C1-C6 alkoxy carbonyl, C1-C6 alkylthio, C1-C6 alkylsulfonyl, C1-C6 alkoxy and halo C1-C6 alkoxy;
R₁₂ independently represents H, C1-C6 alkyl, C2-C6 alkenyl, C2-C6 alkynyl, C1-C6 alkoxy, C1-C6 alkylsulfonyl, C3-C6 cycloalkyl, C3-C6 cycloalkyl C1-C6 alkyl, C3-C6 cycloalkenyl or C3-C6 cycloalkenyl C1-C6 alkyl, or N(R₁₂)₂ in -(CO)N(R₁₂)₂ or -(SO₂)N(R₁₂)₂ independently represents heterocyclyl 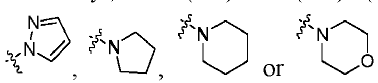 with nitrogen atom at 1-position that is unsubstituted or substituted by 1, 2 or 3 groups selected from oxo and C1-C6 alkyl;
R₁₃ independently represents H, C1-C6 alkyl, halo C1-C6 alkyl, phenyl or phenyl substituted by 1, 2 or 3 groups selected from halogen, cyano, nitro, C1-C6 alkyl, halo C1-C6 alkyl, C1-C6 alkoxy carbonyl, C1-C6 alkylthio, C1-C6 alkylsulfonyl, C1-C6 alkoxy and halo C1-C6 alkoxy.

Still more preferably, X represents -CX₁X₂-(C1-C3 alkyl)ₙ-, -(C1-C3 alkyl)-CX₁X₂-(C1-C3 alkyl)n- or -(CH₂)ᵣ-;
X₁, X₂ each independently represent H, halogen, cyano, amino, nitro, formyl, cyano C1-C3 alkyl, hydroxy C1-C3 alkyl, C1-C6 alkyl, C2-C6 alkenyl, C2-C6 alkynyl, C3-C6 cycloalkyl, C3-C6 cycloalkyl C1-C3 alkyl, C1-C6 alkoxy, C1-C6 alkylthio, C1-C6 alkylamino, halo C1-C6 alkoxy, halo C1-C6 alkylthio, C1-C6 alkyl carbonyl, C1-C6 alkoxy carbonyl, C1-C6 alkoxy C1-C3 alkyl, halo C1-C6 alkoxy C1-C3 alkyl, C1-C6 alkylamino C1-C3 alkyl, aryl, heterocyclyl, aryl C1-C3 alkyl or heterocyclyl C1-C3 alkyl, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C3 alkyl", "aryl", "heterocyclyl", "aryl C1-C3 alkyl" and "heterocyclyl C1-C3 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring; and X₁, X₂ are not hydrogen at the same time;
X₅ represents H, C1-C6 alkyl, C2-C6 alkenyl, C2-C6 alkynyl, C3-C6 cycloalkyl, C3-C6 cycloalkenyl, heterocyclyl, aryl, 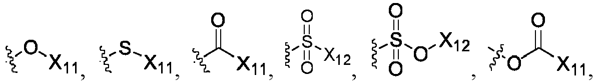 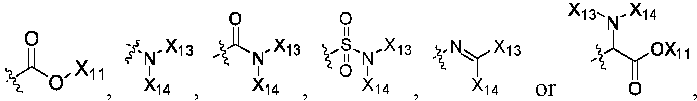 wherein, the "C1-C6 alkyl", "C2-C6 alkenyl" and "C2-C6 alkynyl" are each independently unsubstituted or substituted by 1, 2 or 3 groups selected from halogen, cyano, nitro, C3-C6 cycloalkyl, C3-C6 cycloalkenyl, heterocyclyl, aryl, 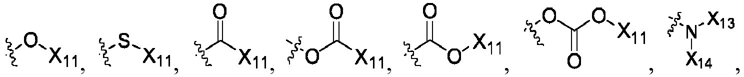 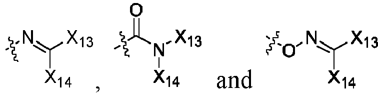 , the "C3-C6 cycloalkyl", "C3-C6 cycloalkenyl", "heterocyclyl" and "aryl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring;
or N(X₅)₂ represents 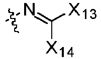 or heterocyclyl 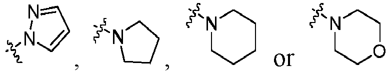 with nitrogen atom at 1-position that is unsubstituted or substituted by 1, 2 or 3 groups selected from oxo and C1-C6 alkyl;
X₁₁ independently represents H, C1-C6 alkyl, C2-C6 alkenyl, C2-C6 alkynyl, halo C1-C6 alkyl, halo C2-C6 alkenyl, halo C2-C6 alkynyl, C3-C6 cycloalkyl, C3-C6 cycloalkyl C1-C3 alkyl, C3-C6 cycloalkenyl, C3-C6 cycloalkenyl C1-C3 alkyl, heterocyclyl, heterocyclyl C1-C3 alkyl, aryl, aryl C1-C3 alkyl or 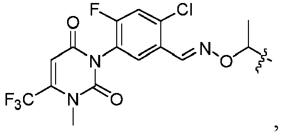 wherein, the "C3-C6 cycloalkyl", "C3-C6 cycloalkyl C1-C3 alkyl", "C3-C6 cycloalkenyl", "C3-C6 cycloalkenyl C1-C3 alkyl", "heterocyclyl", "heterocyclyl C1-C3 alkyl", "aryl" and "aryl C1-C3 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring;
X₁₂ independently represents C1-C6 alkyl, C2-C6 alkenyl, C2-C6 alkynyl, halo C1-C6 alkyl, halo C2-C6 alkenyl, halo C2-C6 alkynyl, C3-C6 cycloalkyl, C3-C6 cycloalkyl C1-C3 alkyl, C3-C6 cycloalkenyl, C3-C6 cycloalkenyl C1-C3 alkyl, heterocyclyl, heterocyclyl C1-C3 alkyl, aryl or aryl C1-C3 alkyl, wherein, the "C3-C6 cycloalkyl", "C3-C6 cycloalkyl C1-C3 alkyl", "C3-C6 cycloalkenyl", "C3-C6 cycloalkenyl C1-C3 alkyl", "heterocyclyl", "heterocyclyl C1-C3 alkyl", "aryl" and "aryl C1-C3 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring;
X₁₃, X₁₄ each independently represent H, halogen, cyano, C1-C6 alkoxy, C1-C6 alkoxy C1-C3 alkyl, C1-C6 alkyl carbonyl, C1-C6 alkoxy carbonyl, C1-C6 alkylsulfonyl, C1-C6 alkyl, C2-C6 alkenyl, C2-C6 alkynyl, C3-C6 cycloalkyl, C3-C6 cycloalkylalkyl, C3-C6 cycloalkenyl, C3-C6 cycloalkenyl C1-C3 alkyl, aryl, aryl C1-C3 alkyl, heterocyclyl or heterocyclyl C1-C3 alkyl, or C, X₁₃, X₁₄, taken together, form 5~8 membered saturated carbocyclyl, 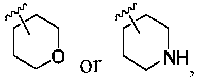 or N, X₁₃, X₁₄, taken together, form heterocyclyl 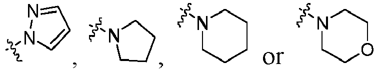 with nitrogen atom at 1-position, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C3 alkyl", "C3-C6 cycloalkenyl", "C3-C6 cycloalkenyl C1-C3 alkyl", "aryl", "aryl C1-C3 alkyl", "heterocyclyl" and "heterocyclyl C1-C3 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring, the "5~8 membered saturated carbocyclyl, 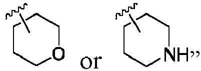 is unsubstituted or substituted by 1, 2 or 3 groups selected from C1-C6 alkyl, C1-C6 alkoxy carbonyl and benzyl, or together with phenyl or thienyl forms a fused ring, the 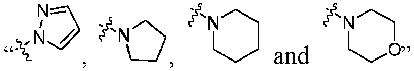 are unsubstituted or substituted by 1, 2 or 3 groups selected from oxo and C1-C6 alkyl.

Further more preferably, Q represents

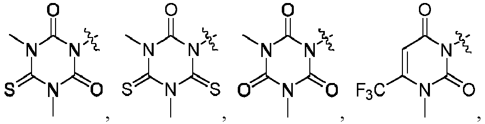

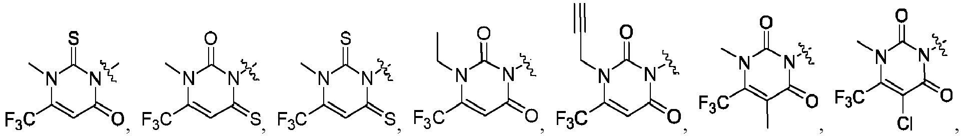

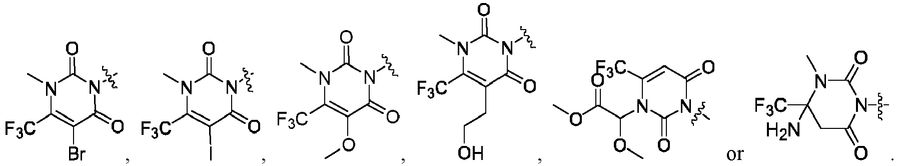

In the definition of the compound represented by the above Formula and all of the following structural formulas, the technical terms used, whether used alone or used in compound word, represent the following substituents: an alkyl having more than two carbon atoms may be linear or branched. For example, the alkyl in the compound word "-alkyl-(CO)OR₁₁" may be -CH₂-, -CH₂CH₂-, -CH(CH₃)-, -CH₂CH₂CH₂-, -C(CH₃)₂-, and the like; "-(CH₂)ᵣ-" may be -CH₂CH₂-, -CH₂CH₂CH₂-, and the like. The alkyl is, for example, C₁ alkyl: methyl; C₂ alkyl: ethyl; C₃ alkyl: propyl such as n-propyl or isopropyl; C₄ alkyl: butyl such as n-butyl, isobutyl, tert-butyl or 2-butyl; C₅ alkyl: pentyl such as n-pentyl; C₆ alkyl: hexyl such as n-hexyl, isohexyl and 1,3-dimethylbutyl. Similarly, the alkenyl is, for example, vinyl, allyl, 1-methylprop-2-en-1-yl, 2-methylprop-2-en-1-yl, but-2-en-1-yl, butyl-3-en-1-yl, 1-methylbut-3-en-1-yl and 1-methylbut-2-en-1-yl. The alkynyl is, for example, ethynyl, propargyl, but-2-yn-1-yl, but-3-yn-1-yl, 1-methylbut-3-yn-1-yl. The multiple bond(s) may be placed at any position of each unsaturated group. The cycloalkyl is a carbocyclic saturated ring system having, for example, three to six carbon atoms, such as cyclopropyl, cyclobutyl, cyclopentyl or cyclohexyl. Similarly, the cycloalkenyl is monocycloalkenyl having, for example, three to six carbon ring members, such as cyclopropenyl, cyclobutenyl, cyclopentenyl, and cyclohexenyl, wherein double bond can be at any position. Halogen is fluorine, chlorine, bromine or iodine.

Unless otherwise specified, the "aryl" of the present invention includes, but is not limited to, phenyl, naphthyl, 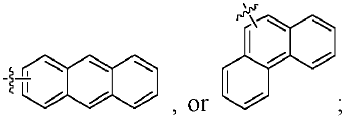 the "heterocyclyl" not only includes, but is not limited to, saturated or unsaturated non-aromatic cyclic group, 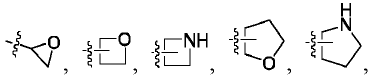 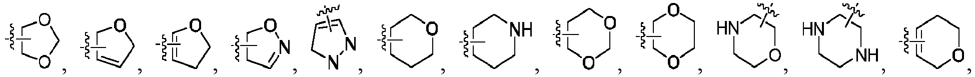 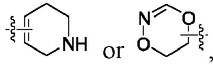 etc., but also includes, but is not limited to,"heteroaryl", which is an aromatic cyclic group having, for example, 3 to 6 ring atoms and optionally being fused with a benzo ring, and 1 to 4 (for example, 1, 2 , 3 or 4) heteroatoms of the ring are selected from the group consisting of oxygen, nitrogen and sulfur. For example,

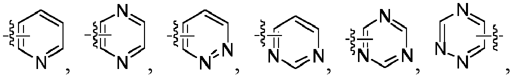

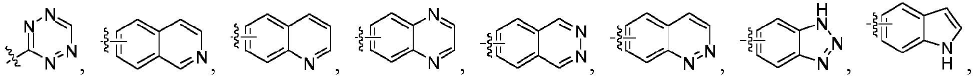

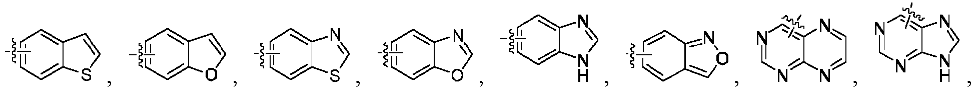

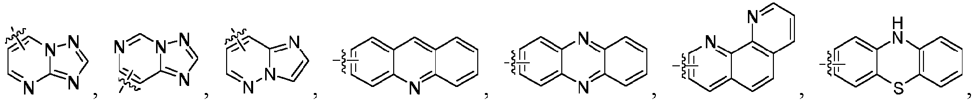

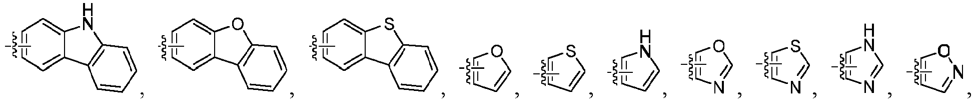

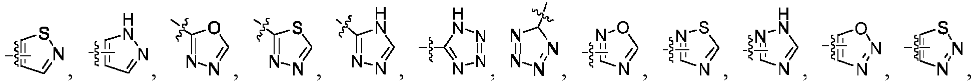

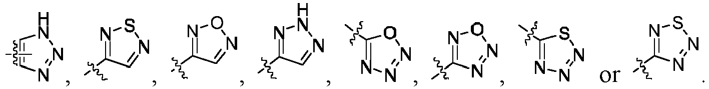

If a group is substituted by a group, which should be understood to mean that the group is substituted by one or more groups, which are same or different groups, selected from the mentioned groups. In addition, the same or different substitution characters contained in the same or different substituents are independently selected, and may be the same or different. This is also applicable to ring systems formed with different atoms and units. Meanwhile, the scope of the claims will exclude those compounds chemically unstable under standard conditions known to those skilled in the art.

In addition, unless specifically defined, the term occurring before or after multiple juxtaposed substituents (separated by "," or "or") in the present invention has a limiting effect on each of the substituents, such as the wording "unsubstituted or halogen-substituted" in the term "unsubstituted or halogen-substituted -OCH₂CH₂- or -OCH₂O-" has a limiting effect on each group "-OCH₂CH₂-" "-OCH₂O-" occurring thereafter; "alkylamino" refers to the amino group which is monosubstituted or disubstituted by alkyl, other substituted amino groups are similarly defined; a group (including heterocyclyl, aryl, etc.) without being specified a linking site may be attached at any site, including a C or N site; if it is substituted, the substituent may be substituted at any site as long as it comply with the valence bond theory. For example, if the heteroaryl 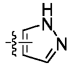 is substituted with one methyl, it can be 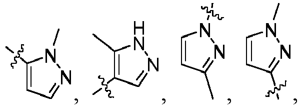 etc..

It should be pointed out that, when the carbon atom (C^{∗}) connected to X₁ and X₂ in the general formula is a chiral center (i.e., X₁ and X₂ are not the same), it is in R configuration or S configuration, preferably R configuration, and based on the content of stereoisomers having R and S configurations at this position, it has a stereochemical purity of 60-100% (R), preferably 70-100% (R), more preferably 80-100% (R), still more preferably 90-100% (R), still more preferably 95-100% (R). Wherein, "stereochemical purity" means the amount of the stated stereoisomer expressed as a percentage of the total amount of stereoisomers having the given chiral centre.

In the present invention the stereochemical configuration at the marked ^{∗} position of formula I is fixed as being predominantly (R) according to the Cahn-Ingold-Prelog system, however is the subject matter of the invention is also directed to all stereoisomers at other locants which are encompassed by formula I, and their mixtures. Such compounds of the formula I contain, e.g. one or more additional asymmetric carbon atoms or else double bonds which are not stated specifically in the formula I. It will be understood that the present invention embraces both the pure isomers and more or less enriched mixtures thereof, where the asymmetric carbon atom in marked ^{∗} position is in the R-configuration or, in mixtures, a compound or compounds of same chemical constitution have the R-configuration in marked ^{∗} position or are present in a ratio that compounds having the R-configuration are predominantly present (at least 60% R-configuration) whilst the other asymmetric carbon atom(s) may be present in racemic form or are more or less resolved too. Provided the condition for the stereochemical configuration at marked ^{∗} position is met, the possible stereoisomers which are defined by their specific spatial form, such as enantiomers, diastereomers, Z- and E-isomers, are all encompassed by formula I and can be obtained by customary methods from mixtures of the stereoisomers, or else be prepared by stereoselective reactions in combination with the use of stereochemically pure raw materials.

The invention also encompasses any keto and enol tautomer forms and mixtures and salts thereof, if respective functional groups are present.

Stereoisomers can be obtained by optical resolution from the mixture obtained in the preparation. The stereoisomers may also be prepared selectively by using stereoselective reactions and using optically active raw materials and/or auxiliaries.It is generally possible to use customary methods for optical resolutions (cf. Textbooks of Stereochemistry), for example following processes for separating mixtures into diastereomers, for example physical processes, such as crystallization, chromatographic processes, in particular column chromatography and high pressure liquid chromatography, distillation, if appropriate under reduced pressure, extraction and other processes, it is possible to separate the remaining mixtures of enantiomers, generally by chromatographic separation on chiral solid phases. Suitable for preparative amounts or use on an industrial scale are processes such as the crystallization of diastereomeric salts which can be obtained from the compounds (I) using optically active acids and, if appropriate, provided that acidic groups are present, using optically active bases.

A method for preparing the carboxylic acid derivative-substituted iminoaryl compound is provided, which comprises the following steps:
subjecting a compound represented by general formula II and a compound represented by general formula III' to an elimination reaction to obtain a compound represented by general formula I', with the chemical reaction equation shown as follows: 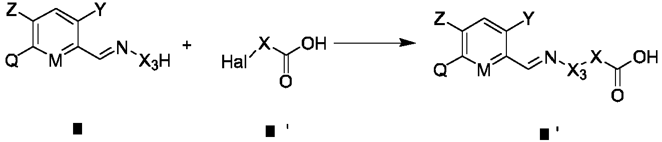
or, subjecting a compound represented by general formula II and a compound represented by general formula III to an elimination reaction to obtain a compound represented by general formula I, with the chemical reaction equation shown as follows: 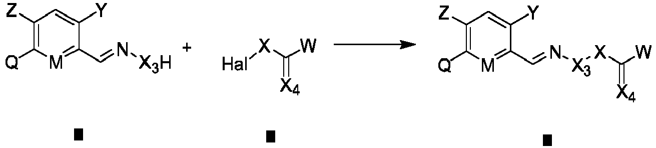
wherein, Hal represents halogen, other substituents Q, M, W, Y, Z, X, X₃ and X₄ are as defined above;
preferably, the reaction is carried out in the presence of a base and a solvent.

The base is at least one selected from inorganic bases (such as K₂CO₃, Na₂CO₃, Cs₂CO₃, NaHCO₃, KF, CsF, KOAc, AcONa, K₃PO₄, t-BuONa, EtONa, NaOH, KOH, NaOMe and the like) and organic bases (such as pyrazole, triethylamine, DIEA and the like).

The solvent is at least one selected from DMF, methanol, ethanol, acetonitrile, dichloroethane, DMSO, Dioxane, dichloromethane and ethyl acetate.

In addition, when Q represents 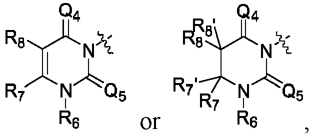 the target product can also be prepared by firstly preparing the intermediate product 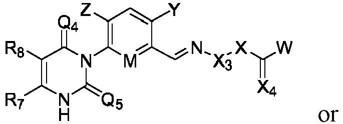 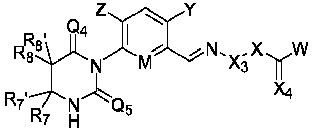 through the above method, and then performing conventional substitution reaction with R₆-Hal (Hal represents halogen, R₆ represents alkyl, alkenyl, alkynyl, or haloalkyl).

The present invention also provides a herbicidal composition, comprising (i) at least one of the carboxylic acid derivative-substituted iminoaryl compounds in a herbicidally effective amount; (component A); preferably, further comprising (ii) one or more other herbicides (component B) in a herbicidally effective amount and/or safeners; more preferably, further comprising (iii) a formulation auxiliary accepted in agricultural chemistry.

In a specific embodiment, the other herbicide is one or more selected from the following compounds and acids, salts and esters thereof:
(1) HPPD inhibitor selected from: topramezone(CAS NO.: 210631-68-8), isoxaflutole(CAS NO.: 141112-29-0), tembotrione(CAS NO.: 335104-84-2), tefuryltrione(CAS NO.: 473278-76-1), shuangzuocaotong(CAS NO.: 1622908-18-2), huanbifucaotong(CAS NO.: 1855929-45-1), sanzuohuangcaotong(CAS NO.: 1911613-97-2), benzuofucaotong(CAS NO.: 1992017-55-6), and 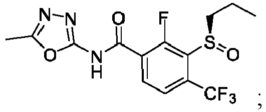
(2) PDS inhibitor selected from: flurtamone (CAS NO.: 96525-23-4), diflufenican (CAS NO.: 83164-33-4), and picolinafen (CAS NO.: 137641-05-5);
(3) DOXP inhibitor selected from: clomazone (CAS NO.: 81777-89-1), and bixlozone (CAS NO.: 81777-95-9);
(4) ALS inhibitor selected from: tribenuron-methyl(CAS NO.: 101200-48-0), thifensulfuron methyl(CAS NO.: 79277-27-3), pyrazosulfuron-ethyl(CAS NO.: 93697-74-6), thiencarbazone-methyl(CAS NO.: 317815-83-1), halosulfuron methyl(CAS NO.: 100784-20-1), rimsulfuron(CAS NO.: 122931-48-0), nicosulfuron(CAS NO.: 111991-09-4), and imazamox(CAS NO.: 114311-32-9);
(5) ACCase inhibitor selected from: clethodim (CAS NO.: 99129-21-2), sethoxydim (CAS NO.: 74051-80-2), and quizalofop-P-methyl (CAS NO.: 100646-51-3);
(6) PPO inhibitor selected from: oxyfluorfen(CAS NO.: 42874-03-3), oxadiazon(CAS NO.: 19666-30-9), oxadiargyl(CAS NO.: 39807-15-3), sulfentrazone(CAS NO.: 122836-35-5), pyraclonil(CAS NO.: 158353-15-2), flumioxazin(CAS NO.: 103361-09-7), saflufenacil(CAS NO.: 372137-35-4), carfentrazone-ethyl(CAS NO.: 128639-02-1), and trifludimoxazin(CAS NO.: 1258836-72-4);
(7) PSII inhibitor selected from: metribuzin(CAS NO.: 21087-64-9), terbuthylazine(CAS NO.: 5915-41-3), amicarbazone(CAS NO.: 129909-90-6), chlorotoluron(CAS NO.: 15545-48-9), isoproturon(CAS NO.: 34123-59-6), bromacil(CAS NO.: 314-40-9), propanil(CAS NO.: 709-98-8), desmedipham(CAS NO.: 13684-56-5), phenmedipham(CAS NO.: 13684-63-4), bentazone(CAS NO.: 25057-89-0), and bromoxynil(CAS NO.: 1689-84-5);
(8) inhibitor of microtubule assembly selected from: butralin (CAS NO.: 33629-47-9), and pendimethalin (CAS NO.: 40487-42-1);
(9) VLCFA inhibitor selected from: butachlor (CAS NO.: 23184-66-9), pretilachlor (CAS NO.: 51218-49-6), mefenacet (CAS NO.: 73250-68-7), s-metolachlor (CAS NO.: 87392-12-9), flufenacet (CAS NO.: 142459-58-3), pyroxasulfone (CAS NO.: 447399-55-5), and anilofos (CAS NO.: 764249-01-0);
(10) lipid synthesis (non-acetyl-CoA carboxylase) inhibitor: prosulfocarb (CAS NO.: 52888-80-9);
(11) Synthetic hormone inhibitor selected from: 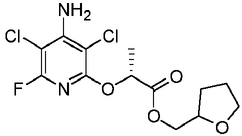 , fluroxypyr(CAS NO.: 69377-81-7), florpyrauxifen benzyl(CAS NO.: 1390661-72-9), halauxifen-methyl(CAS NO.: 943831-98-9), triclopyr(CAS NO.: 55335-06-3), clopyralid(CAS NO.: 1702-17-6), picloram(CAS NO.: 1918-02-1), aminopyralid(CAS NO.: 150114-71-9), dicamba(CAS NO.: 1918-00-9), 2-methyl-4-chlorophenoxyacetic acid(CAS NO.: 94-74-6), and 2,4-dichlorophenoxy acetic acid(CAS NO.: 94-75-7);
(12) EPSPS inhibitor: glyphosate (CAS NO.: 1071-83-6);
(13) GS inhibitor selected from: glufosinate ammonium (CAS NO.: 77182-82-2), and glufosinate-P-ammonium (CAS NO.: 35597-44-5);
(14) PSI inhibitor selected from: paraquat dichloride (CAS NO.: 1910-42-5), and diquat dibromide monohydrate (CAS NO.: 2764-72-9);
(15) Cellulose synthesis inhibitor selected from: triaziflam (CAS NO.: 131475-57-5), and indaziflam (CAS NO.: 950782-86-2);
(16) others: cinmethylin (CAS NO.: 87818-31-3).

In the context of the present specification, if an abbreviation of a generic name of an active compound is used, it includes in each case all customary derivatives, such as esters and salts, as well as isomers, in particular optical isomers, especially one or more commercially available forms. If the generic name denotes an ester or a salt, it also includes in each case all other conventional derivatives, such as other esters and salts, free acids and neutral compounds, as well as isomers, in particular optical isomers, especially one or more commercially available forms. The chemical name given to a compound means at least one compound encompassed by the generic name, and generally the preferred compound. In the case of sulfonamides such as sulfonylurea, salts also include salts formed by the exchange of cations with hydrogen atoms in the sulfonamide group.

Wherein, the active ingredient A to the active ingredient B in the herbicidal composition is in a weight ratio of 1:1000~1000:1, 1:800~800:1 or 1:600~600:1, preferably 1:500~500:1, 1:400~400:1 or 1:300~300:1, more preferably 1:200~200:1, 1:100~100:1 or 1:80~80:1, further preferably 1:50~50:1, 1:30~30:1, 1:20~20:1, 1:10~10:1, 1:5~1:1 or 1:1~5:1. In some embodiments, the active ingredients A and B together account for 1-95%, preferably 10-80%, of the total weight of the herbicidal composition.

The safener is selected from one or more of isoxadifen-ethyl (CAS: 163520-33-0), cyprosulfamide (CAS: 221667-31-8), mefenpyr-diethyl (CAS: 135590-91-9), cloquintocet-mexyl (CAS: 99607-70-2), gibberellic acid (CAS: 77-06-5), furilazole (CAS: 121776-33-8), and metcamifen (CAS: 129531-12-0).

The preparation adjuvant comprises, for example, a carrier and/or a surfactant.

The term "carrier" herein refers to an organic or inorganic, natural or synthetic substance, which facilitates the application of the active ingredients. In general, the carrier is inert and must be agriculturally acceptable, especially is acceptable to a plant to be treated. The carrier may be a solid, such as clay, a natural or synthetic silicate, silica, a resin, a wax, a solid fertilizer and so on; or a liquid such as water, an alcohol, a ketone, a petroleum fraction, an aromatic or paraffinic hydrocarbon, a chlorohydrocarbon, liquefied gas and so on.

The surfactant, which may be ionic or non-ionic, can include an emulsifier, a dispersant or a wetting agent. Examples which may be mentioned are a salt of polyacrylic acid, a salt of lignosulfonic acid, a salt of phenolsulfonic acid or of naphthalenesulfonic acid, a polymer of ethylene oxide with an aliphatic alcohol or with an aliphatic acid or with an aliphatic amine or with a substituted phenol (in particular, an alkylphenol or an arylphenol), a sulfosuccinate, a taurine derivative (especially an alkyl taurate) and a phosphoric ester of an alcohol or of a polyhydroxyethylated phenol, an alkyl sulfonate, an alkylaryl sulfonate, an alkyl sulfate, a laurylether sulfate, a fatty alcohol sulfate, a sulfated hexadecanol, heptadecanol and octadecanol and a sulfated fatty alcohol polyglycol ether, and further include a condensate of naphthalene or naphthalenesulfonic acid with phenol and formaldehyde, polyoxyethylene octylphenyl ether, ethoxylated isooctylphenol, octylphenol or nonylphenol, a polyethylene glycol alkylphenyl ether, a polyethylene glycol tributylphenyl ether, a polyethylene glycol tristearylphenyl ether, a alkylaryl polyether alcohol, an alcohol and fatty alcohol/ethylene oxide condensate, ethoxylated castor oil, a polyoxyethylene alkyl ether, an ethoxylated polyoxypropylene, a lauryl alcohol polyglycol ether acetal, a sorbitol ester, a lignin sulfite waste liquid, a protein, a denatured protein, a polysaccharide (e.g., methylcellulose), a hydrophobic modified starch, a polyvinyl alcohol, a polycarboxylate, a polyalkoxylate, a polyvinylamine, a polyvinylpyrrolidone, and a copolymer thereof. At least one surfactant may be required to facilitate dispersion of the active ingredient in water and proper application thereof to a plant.

The composition can also comprise various other components, such as a protective colloid, an adhesive, a thickener, a thixotropic agent, a penetrant, a stabilizer, a chelating agent, a dye, a colorant or a polymer.

The composition of the present invention may be diluted prior to use or used directly by users. The compositon can be prepared through a conventional processing method, that is, the active ingredient(s) is mixed with a liquid solvent or a solid carrier, and then one or more of the surfactants such as a dispersant, a stabilizer, a wetting agent, an adhesive, or a defoaming agent, etc. are added.

The herbicidal composition may be in a form of a formulation which is selected from: a dispersible oil suspension, a water suspension, a suspoemulsion, a wettable powder, an emulsifiable concentrate, a water-dispersible granule (a dry suspension), an aqueous emulsion and a microemulsion.

In short, the composition of the present invention can be mixed with solid and liquid additives conventionally used in formulations of the prior art. As the external conditions change, the amount of active ingredients used is also different. The external conditions are, for example, temperature, humidity, the nature of the herbicide used, etc. It can have a large variation range, for example between 0.001 and 1.0 kg/ha, or more active substances, but preferably between 0.005 and 750 g/ha, especially between 0.005 and 500 g/ha.

A method for controlling an undesirable plant is provided, which comprises applying at least one of the carboxylic acid derivative-substituted iminoaryl compounds or the herbicidal composition in a herbicidally effective amount on a plant or in its area or to soil or water to control the emergence or growth of undesirable plant.

Use of at least one of the carboxylic acid derivative-substituted iminoaryl compounds or the herbicidal composition for controlling a undesirable plant; preferably, the carboxylic acid derivative-substituted iminoaryl compound is used to control a weed in a useful crop, the useful crop is a genetically modified crop or a crop treated by genome editing technique.

The compounds of the formula I according to the invention have an outstanding herbicidal activity against a broad spectrum of economically important monocotyledonous and dicotyledonous harmful plants (undesirable plants). The active compounds also act efficiently on perennial weeds which produce shoots from rhizomes, root stocks or other perennial organs and which are difficult to control. In this context, it is generally immaterial whether the substances are applied pre-sowing, pre-emergence or post-emergence. Specifically, examples may be mentioned of some representatives of the monocotyledonous and dicotyledonous weed flora which can be controlled by the compounds according to the invention, without these being a restriction to certain species. Examples of weed species on which the active compounds act efficiently are, from amongst the monocotyledons, Avena, Lolium, Alopecurus, Phalaris, Echinochloa, Digitaria, Setaria and also Cyperus species from the annual sector and from amongst the perennial species Agropyron, Cynodon, Imperata and Sorghum, and also perennial Cyperus species.

In the case of the dicotyledonous weed species, the spectrum of action extends to species such as, for example, Galium, Viola, Veronica, Lamium, Stellaria, Amaranthus, Sinapis, Ipomoea, Sida, Matricaria and Abutilon from amongst the annuals, and Convolvulus, Cirsium, Rumex and Artemisia in the case of the perennial weeds. The active compounds according to the invention also effect outstanding control of harmful plants which occur under the specific conditions of rice growing such as, for example, Echinochloa, Sagittaria, Alisma, Eleocharis, Scirpus and Cyperus. If the compounds according to the invention are applied to the soil surface prior to germination, then the weed seedlings are either prevented completely from emerging, or the weeds grow until they have reached the cotyledon stage but then their growth stops, and, eventually, after three to four weeks have elapsed, they die completely. If the compounds according to the invention are applied to the soil surface prior to germination, then the weed seedlings are either prevented completely from emerging, or the weeds grow until they have reached the cotyledon stage but then their growth stops, and, eventually, after three to four weeks have elapsed, they die completely. In particular, the compounds according to the invention exhibit excellent activity against Apera spica venti, Chenopodium album, Lamium purpureum, Polygonum convulvulus, Stellaria media, Veronica hederifolia, Veronica persica, Viola tricolor and against Amaranthus, Galium and Kochia species.

The undesirable plants also include herbicide-resistant or tolerant weed species.

Although the compounds according to the invention have an excellent herbicidal activity against monocotyledonous and dicotyledonous weeds, crop plants of economically important crops such as, for example, wheat, barley, rye, rice, corn, sugarbeet, cotton and soya, are not damaged at all, or only to a negligible extent. In particular, they have excellent compatibility in cereals, such as wheat, barley and corn, in particular wheat. For these reasons, the present compounds are highly suitable for selectively controlling undesirable plant growth in plantings for agricultural use or in plantings of ornamentals.

Owing to their herbicidal properties, these active compounds can also be employed for controlling harmful plants in crops of known or still to be developed genetically engineered plants. The transgenic plants generally have particularly advantageous properties, for example resistance to certain pesticides, in particular certain herbicides, resistance to plant diseases or causative organisms of plant diseases, such as certain insects or microorganisms such as fungi, bacteria or viruses. Other particular properties relate, for example, to the quantity, quality, storage-stability, composition and to specific ingredients of the harvested product. Thus, transgenic plants having an increased starch content or a modified quality of the starch or those having a different fatty acid composition of the harvested produce are known.

The use of the compounds of the formula I according to the invention or their salts in economically important transgenic crops of useful and ornamental plants, for example of cereal, such as wheat, barley, rye, oats, millet, rice, maniok and corn, or else in crops of sugarbeet, cotton, soya, rapeseed, potato, tomato, pea and other vegetable species is preferred. The compounds of the formula I can preferably be used as herbicides in crops of useful plants which are resistant or which have been made resistant by genetic engineering toward the phytotoxic effects of the herbicides.

Conventional ways for preparing novel plants which have modified properties compared to known plants comprise, for example, traditional breeding methods and the generation of mutants. Alternatively, novel plants having modified properties can be generated with the aid of genetic engineering methods (see, for example, EP-A 0 221 044, EP-A 0 131 624). For example, there have been described several cases of:
- genetically engineered changes in crop plants in order to modify the starch synthesized in the plants (for example WO 92/11376, WO 92/14827, WO 91/19806),
- transgenic crop plants which are resistant to certain herbicides of the glufosinate- (cf., for example, EP-A 0 242 236, EP-A 0 242 246) or glyphosate-type (WO 92/00377), or of the sulfonylurea-type (EP-A 0 257 993, U.S. Pat. No. 5,013,659A),
- transgenic crop plants, for example cotton, having the ability to produce Bacillus thuringiensis toxins (Bt toxins) which impart resistance to certain pests to the plants (EP-A 0 142 924, EP-A 0 193 259),
- transgenic crop plants having a modified fatty acid composition (WO 91/13972).

Numerous molecular biological techniques which allow the preparation of novel transgenic plants having modified properties are known in principle; see, for example, Sambrook et al., 1989, Molecular Cloning, A Laboratory Manual, 2nd ed. Cold Spring Harbor Laboratory Press, Cold Spring Harbor, N.Y; or Winnacker "Gene und Klone" [Genes and Clones], VCH Weinheim, 2nd edition 1996, or Christou, "Trends in Plant Science" 1 (1996) 423-431). In order to carry out such genetic engineering manipulations, it is possible to introduce nucleic acid molecules into plasmids which allow a mutagenesis or a change in the sequence to occur by recombination of DNA sequences. Using the abovementioned standard processes it is possible, for example, to exchange bases, to remove partial sequences or to add natural or synthetic sequences. To link the DNA fragments with each other, it is possible to attach adaptors or linkers to the fragments.

Plant cells having a reduced activity of a gene product can be prepared, for example, by expressing at least one appropriate antisense-RNA, a sense-RNA to achieve a cosuppression effect, or by expressing at least one appropriately constructed ribozyme which specifically cleaves transcripts of the above-mentioned gene product.

To this end it is possible to employ both DNA molecules which comprise the entire coding sequence of a gene product including any flanking sequences that may be present, and DNA molecules which comprise only parts of the coding sequence, it being necessary for these parts to be long enough to cause an antisense effect in the cells. It is also possible to use DNA sequences which have a high degree of homology to the coding sequences of a gene product but which are not entirely identical.

When expressing nucleic acid molecules in plants, the synthesized protein can be localized in any desired compartment of the plant cells. However, to achieve localization in a certain compartment, it is, for example, possible to link the coding region with DNA sequences which ensure localization in a certain compartment. Such sequences are known to the person skilled in the art (see, for example, Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

The transgenic plant cells can be regenerated to whole plants using known techniques. The transgenic plants can in principle be plants of any desired plant species, i.e. both monocotyledonous and dicotyledonous plants. In this manner, it is possible to obtain transgenic plants which have modified properties by overexpression, suppression or inhibition of homologous (=natural) genes or gene sequences or by expression of heterologous (=foreign) genes or gene sequences.

When using the active compounds according to the invention in transgenic crops, in addition to the effects against harmful plants which can be observed in other crops, there are frequently effects which are specific for the application in the respective transgenic crop, for example a modified or specifically broadened spectrum of weeds which can be controlled, modified application rates which can be used for the application, preferably good combinability with the herbicides to which the transgenic crops are resistant, and an effect on the growth and the yield of the transgenic crop plants. The invention therefore also provides for the use of the compounds according to the invention as herbicides for controlling harmful plants in transgenic crop plants.

In addition, the substances according to the invention have outstanding growth-regulating properties in crop plants. They engage in the plant metabolism in a regulating manner and can this be employed for the targeted control of plant constituents and for facilitating harvesting, for example by provoking desiccation and stunted growth. Furthermore, they are also suitable for generally regulating and inhibiting undesirable vegetative growth, without destroying the plants in the process. Inhibition of vegetative growth plays an important role in many monocotyledon and dicotyledon crops because lodging can be reduced hereby, or prevented completely.

The compounds according to the invention can be applied in the customary formulations in the form of wettable powders, emulsifiable concentrates, sprayable solutions, dusts or granules. The invention therefore also provides herbicidal compositions comprising compounds of the formula I. The compounds of the formula I can be formulated in various ways depending on the prevailing biological and/or chemico-physical parameters. Examples of suitable formulation options are: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), oil dispersions (OD), oil- or water-based dispersions, oil-miscible solutions, dusts (DP), capsule suspensions (CS), seed-dressing compositions, granules for broadcasting and soil application, granules (GR) in the form of microgranules, spray granules, coating granules and adsorption granules, water-dispersible granules (WG), water-soluble granules (SG), ULV formulations, microcapsules and waxes. These individual formulation types are known in principle and are described, for example, in Winnacker-Küchler, "Chemische Technologie" [Chemical Technology], Volume 7, C. Hauser Verlag Munich, 4th. Edition 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

The necessary formulation auxiliaries, such as inert materials, surfactants, solvents and other additives, are likewise known and are described, for example, in Watkins, "Handbook of Insecticide Dust Diluents and Carriers", 2nd Ed., Darland Books, Caldwell N.J., H. v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflüchenaktive Äthylenoxidaddkte" [Surface-active ethylene oxide adducts], Wiss. Verlagagesell. Stuttgart 1976; Winnacker-Küchler, "Chemische Technologie" [Chemical Technology], Volume 7, C. Hauser Verlag Munich, 4th Edition 1986.

Wettable powders are preparations which are uniformly dispersible in water and which contain, in addition to the active compound and as well as a diluent or inert substance, surfactants of ionic and/or nonionic type (wetting agents, dispersants), for example polyethoxylated alkyl phenols, polyethoxylated fatty alcohols, polyethoxylated fatty amines, fatty alcohol polyglycol ethersulfates, alkanesulfonates, alkylbenzenesulfonates, sodium ligninsulfonate, sodium 2,2'-dinaphthylmethane-6,6'-disulfonate, sodium dibutyinaphthalenesulfona-te or else sodium oleoylmethyltaurinate. To prepare the wettable powders, the herbicidally active compounds are finely ground, for example in customary apparatus such as hammer mills, fan mills and air-jet mills, and are mixed simultaneously or subsequently with the formulation auxiliaries.

Emulsifiable concentrates are prepared by dissolving the active compound in an organic solvent, for example butanol, cyclohexanone, dimethylformamide, xylene or else relatively high-boiling aromatic compounds or hydrocarbons or mixtures of the solvents, with the addition of one or more surfactants of ionic and/or nonionic type (emulsifiers). Examples of emulsifiers which can be used are calcium alkylarylsulfonates, such as Ca dodecylbenzenesulfonate, or nonionic emulsifiers, such as fatty acid polyglycol esters, alkylaryl polyglycol ethers, fatty alcohol polyglycol ethers, propylene oxide-ethylene oxide condensation products, alkyl polyethers, sorbitan esters, for example sorbitan fatty acid esters or polyoxyethylene sorbitan esters, for example polyoxyethylene sorbitan fatty acid esters.

Dusts are obtained by grinding the active compound with finely divided solid substances, for example talc, natural clays, such as kaolin, bentonite and pyrophyllite, or diatomaceous earth. Suspension concentrates can be water- or oil-based. They can be prepared, for example, by wet milling using commercially customary bead mills, with or without the addition of surfactants as already mentioned above, for example, in the case of the other formulation types.

Emulsions, for example oil-in-water emulsions (EW), can be prepared for example by means of stirrers, colloid mills and/or static mixers using aqueous organic solvents and, if desired, surfactants as already mentioned above, for example, in the case of the other formulation types.

Granules can be prepared either by spraying the active compound onto adsorptive, granulated inert material or by applying active-compound concentrates to the surface of carriers such as sand, kaolinites or granulated inert material, by means of adhesive binders, for example polyvinyl alcohol, sodium polyacrylate or else mineral oils. Suitable active compounds can also be granulated in the manner which is customary for the preparation of fertilizer granules, if desired as a mixture with fertilizers. Water-dispersible granules are generally prepared by the customary processes, such as spray-drying, fluidized-bed granulation, disk granulation, mixing using high-speed mixers, and extrusion without solid inert material.

For the preparation of disk, fluidized-bed, extruder and spray granules, see for example processes in "Spray-Drying Handbook" 3rd ed. 1979, G Goodwin Ltd., London; J. E. Browning, "Agglomeration", Chemical and Engineering 1967, pages 147 ff.; "Perry's Chemical Engineer's Handbook", 5th Ed., McGraw-Hill, New York 1973, pp. 8-57. For further details on the formulation of crop protection products, see for example G C. Klingman, "Weed Control as a Science", John Wiley and Sons Inc., New York, 1961, pages 81-96 and J. D. Freyer, S. A. Evans, "Weed Control Handbook", 5th Ed., Blackwell Scientific Publications, Oxford, 1968, pages 101-103.

The agrochemical formulations generally contain from 0.1 to 99% by weight, in particular from 0.1 to 95% by weight, of active compound of the formula I. In wettable powders the concentration of active compound is, for example, from about 10 to 99% by weight, the remainder to 100% by weight consisting of customary formulation constituents. In emulsifiable concentrates the concentration of active compound can be from about 1 to 90%, preferably from 5 to 80%, by weight. Formulations in the form of dusts contain from 1 to 30% by weight of active compound, preferably most commonly from 5 to 20% by weight of active compound, while sprayable solutions contain from about 0.05 to 80%, preferably from 2 to 50%, by weight of active compound. In the case of water-dispersible granules the content of active compound depends partly on whether the active compound is in liquid or solid form and on the granulation auxiliaries, fillers, etc. that are used. In water-dispersible granules the content of active compound, for example, is between 1 and 95% by weight, preferably between 10 and 80% by weight.

In addition, the formulations of active compound may comprise the tackifiers, wetting agents, dispersants, emulsifiers, penetrants, preservatives, antifreeze agents, solvents, fillers, carriers, colorants, antifoams, evaporation inhibitors and pH and viscosity regulators which are customary in each case.

Based on these formulations it is also possible to produce combinations with other pesticidally active substances, for example insecticides, acaricides, herbicides and fungicides, and also with safeners, fertilizers and/or growth regulators, for example in the form of a ready-mix or tank mix.

Suitable active compounds which can be combined with the active compounds according to the invention in mixed formulations or in a tank mix are, for example, known active compounds as described in for example World Herbicide New Product Technology Handbook, China Agricultural Science and Farming Techniques Press, 2010.9 and in the literature cited therein. For example the following active compounds may be mentioned as herbicides which can be combined with the compounds of the formula I (note: the compounds are either named by the "common name" in accordance with the International Organization for Standardization (ISO) or by the chemical names, if appropriate together with a customary code number): acetochlor, butachlor, alachlor, propisochlor, metolachlor, s-metolachlor, pretilachlor, propachlor, ethachlor, napropamide, R-left handed napropamide, propanil, mefenacet, diphenamid, diflufenican, ethaprochlor, beflubutamid, bromobutide, dimethenamid, dimethenamid-P, etobenzanid, flufenacet, thenylchlor, metazachlor, isoxaben, flamprop-M-methyl, flamprop-M-propyl, allidochlor, pethoxamid, chloranocryl, cyprazine, mefluidide, monalide, delachlor, prynachlor, terbuchlor, xylachlor, dimethachlor, cisanilide, trimexachlor, clomeprop, propyzamide, pentanochlor, carbetamide, benzoylprop-ethyl, cyprazole, butenachlor, tebutam, benzipram, mogrton, dichlofluanid, naproanilide, diethatyl-ethyl, naptalam, flufenacet, EL-177, benzadox, chlorthiamid, chlorophthalimide, isocarbamide, picolinafen, atrazine, simazine, prometryn, cyanatryn, simetryn, ametryn, propazine, dipropetryn, SSH-108, terbutryn, terbuthylazine, triaziflam, cyprazine, proglinazine, trietazine, prometon, simetone, aziprotryne, desmetryn, dimethametryn, procyazine, mesoprazine, sebuthylazine, secbumeton, terbumeton, methoprotryne, cyanatryn, ipazine, chlorazine, atraton, pendimethalin, eglinazine, cyanuric acid, indaziflam, chlorsulfuron, metsulfuron-methyl, bensulfuron methyl, chlorimuron-ethyl, tribenuron-methyl, thifensulfuron-methyl, pyrazosulfuron-ethyl, mesosulfuron, iodosulfuron-methyl sodium, foramsulfuron, cinosulfuron, triasulfuron, sulfometuron methyl, nicosulfuron, ethametsulfuron-methyl, amidosulfuron, ethoxysulfuron, cyclosulfamuron, rimsulfuron, azimsulfuron, flazasulfuron, monosulfuron, monosulfuron-ester, flucarbazone-sodium, flupyrsulfuron-methyl, halosulfuron-methyl, oxasulfuron, imazosulfuron, primisulfuron, propoxycarbazone, prosulfuron, sulfosulfuron, trifloxysulfuron, triflusulfuron-methyl, tritosulfuron, sodium metsulfuron methyl, flucetosulfuron, HNPC-C, orthosulfamuron, propyrisulfuron, metazosulfuron, acifluorfen, fomesafen, lactofen, fluoroglycofen, oxyfluorfen, chlornitrofen, aclonifen, ethoxyfen-ethyl, bifenox, nitrofluorfen, chlomethoxyfen, fluorodifen, fluoronitrofen, furyloxyfen, nitrofen, TOPE, DMNP, PPG1013, AKH-7088, halosafen, chlortoluron, isoproturon, linuron, diuron, dymron, fluometuron, benzthiazuron, methabenzthiazuron, cumyluron, ethidimuron, isouron, tebuthiuron, buturon, chlorbromuron, methyldymron, phenobenzuron, SK-85, metobromuron, metoxuron, afesin, monuron, siduron, fenuron, fluothiuron, neburon, chloroxuron, noruron, isonoruron, 3-cyclooctyl-1, thiazfluron, tebuthiuron, difenoxuron, parafluron, methylamine tribunil, karbutilate, trimeturon, dimefuron, monisouron, anisuron, methiuron, chloreturon, tetrafluron, phenmedipham, phenmedipham-ethyl, desmedipham, asulam, terbucarb, barban, propham, chlorpropham, rowmate, swep, chlorbufam, carboxazole, chlorprocarb, fenasulam, BCPC, CPPC, carbasulam, butylate, benthiocarb, vernolate, molinate, triallate, dimepiperate, esprocarb, pyributicarb, cycloate, avadex, EPTC, ethiolate, orbencarb, pebulate, prosulfocarb, tiocarbazil, CDEC, dimexano, isopolinate, methiobencarb, 2,4-D butyl ester, MCPA-Na, 2,4-D isooctyl ester, MCPA isooctyl ester, 2,4-D sodium salt, 2,4-D dimethyla mine salt, MCPA-thioethyl, MCPA, 2,4-D propionic acid, high 2,4-D propionic acid salt, 2,4-D butyric acid, MCPA propionic acid, MCPA propionic acid salt, MCPA butyric acid, 2,4,5-D, 2,4,5-D propionic acid, 2,4,5-D butyric acid, MCPA amine salt, dicamba, erbon, chlorfenac, saison, TBA, chloramben, methoxy-TBA, diclofop-methyl, fluazifop-butyl, fluazifop-p-butyl, haloxyfop-methyl, haloxyfop-P, quizalofop-ethyl, quizalofop-p-ethyl, fenoxaprop-ethy, fenoxaprop-p-ethyl, propaquizafop, cyhalofop-butyl, metamifop, clodinafop-propargyl, fenthiaprop-ethyl, chloroazifop-propynyl, poppenate-methyl, trifopsime, isoxapyrifop, paraquat, diquat, oryzalin, ethalfluralin, isopropalin, nitralin, profluralin, prodinamine, benfluralin, fluchloraline, dinitramina, dipropalin, chlornidine, methalpropalin, dinoprop, glyphosate, anilofos, glufosinate ammonium, amiprophos-methyl, sulphosate, piperophos, bialaphos-sodium, bensulide, butamifos, phocarb, 2,4-DEP, H-9201, zytron, imazapyr, imazethapyr, imazaquin, imazamox, imazamox ammonium salt, imazapic, imazamethabenz-methyl, fluroxypyr, fluroxypyr isooctyl ester, clopyralid, picloram, trichlopyr, dithiopyr, haloxydine, 3,5,6-trichloro-2-pyridinol, thiazopyr, fluridone, aminopyralid, diflufenzopyr, triclopyr-butotyl, Cliodinate, sethoxydim, clethodim, cycloxydim, alloxydim, clefoxydim, butroxydim, tralkoxydim, tepraloxydim, buthidazole, metribuzin, hexazinone, metamitron, ethiozin, ametridione, amibuzin, bromoxynil, bromoxynil octanoate, ioxynil octanoate, ioxynil, dichlobenil, diphenatrile, pyraclonil, chloroxynil, iodobonil, flumetsulam, florasulam, penoxsulam, metosulam, cloransulam-methyl, diclosulam, pyroxsulam, benfuresate, bispyribac-sodium, pyribenzoxim, pyriftalid, pyriminobac-methyl, pyrithiobac-sodium, benzobicylon, mesotrione, sulcotrione, tembotrione, tefuryltrione, bicyclopyrone, ketodpiradox, isoxaflutole, clomazone, fenoxasulfone, methiozolin, fluazolate, pyraflufen-ethyl, pyrazolynate, difenzoquat, pyrazoxyfen, benzofenap, nipyraclofen, pyrasulfotole, topramezone, pyroxasulfone, cafenstrole, flupoxam, aminotriazole, amicarbazone, azafenidin, carfentrazone-ethyl, sulfentrazone, bencarbazone, benzfendizone, butafenacil, bromacil, isocil, lenacil, terbacil, flupropacil, cinidon-ethyl, flumiclorac-pentyl, flumioxazin, propyzamide, MK-129, flumezin, pentachlorophenol, dinoseb, dinoterb, dinoterb acetate, dinosam, DNOC, chloronitrophene, medinoterb acetate, dinofenate, oxadiargyl, oxadiazon, pentoxazone, Flufenacet, fluthiacet-methyl, fentrazamide, flufenpyr-ethyl, pyrazon, brompyrazon, metflurazon, kusakira, dimidazon, oxapyrazon, norflurazon, pyridafol, quinclorac, quinmerac, bentazone, pyridate, oxaziclomefone, benazolin, clomazone, cinmethylin, ZJ0702, pyribambenz-propyl, indanofan, sodium chlorate, dalapon, trichloroacetic acid, monochloroacetic acid, hexachloroacetone, flupropanate, cyperquat, bromofenoxim, epronaz, methazole, flurtamone, benfuresate, ethofumesate, tioclorim, chlorthal, fluorochloridone, tavron, acrolein, bentranil, tridiphane, chlorfenpropmethyl, thidiarizonaimin, phenisopham, busoxinone, methoxyphenone, saflufenacil, clacyfos, chloropon, alorac, diethamquat, etnipromid, iprymidam, ipfencarbazone, thiencarbazone-methyl, pyrimisulfan, chlorflurazole, tripropindan, sulglycapin, prosulfalin, cambendichlor, aminocyclopyrachlor, rodethanil, benoxacor, fenclorim, flurazole, fenchlorazole-ethyl, cloquintocet-mexyl, oxabetrinil, MG/91, cyometrinil, DKA-24, mefenpyr-diethyl, furilazole, fluxofenim, isoxadifen-ethyl, dichlormid, halauxifen-methyl, DOW florpyrauxifen, UBH-509, D489, LS 82-556, KPP-300, NC-324, NC-330, KH-218, DPX-N8189, SC-0744, DOWCO535, DK-8910, V-53482, PP-600, MBH-001, KIH-9201, ET-751, KIH-6127 and KIH-2023.

For use, the formulations which are present in commercially available form are, if appropriate, diluted in the customary manner, for example using water in the case of wettable powders, emulsifiable concentrates, dispersions and water-dispersible granules. Products in the form of dusts, granules for soil application or broadcasting and sprayable solutions are usually not further diluted with other inert substances prior to use. The application rate of the compounds of the formula I required varies with the external conditions, such as temperature, humidity, the nature of the herbicide used and the like. It can vary within wide limits, for example between 0.001 and 1.0 kg/ha or more of active substance, but it is preferably between 0.005 and 750 g/ha, especially between 0.005 and 250g/ha.

### Specific Mode for Carrying out the Invention

The following embodiments are used to illustrate the present invention in detail and should not be taken as any limit to the present invention. The scope of the invention would be explained through the Claims.

In view of economics and variety of a compound, we preferably synthesized several compounds, part of which are listed in the following Table 1 and Table A. The structure and information of a certain compound are shown in Table 1 and Table A. The compounds in Table 1 and Table A are listed for further explication of the present invention, other than any limit therefor. The subject of the present invention should not be interpreted by those skilled in the art as being limited to the following compounds.

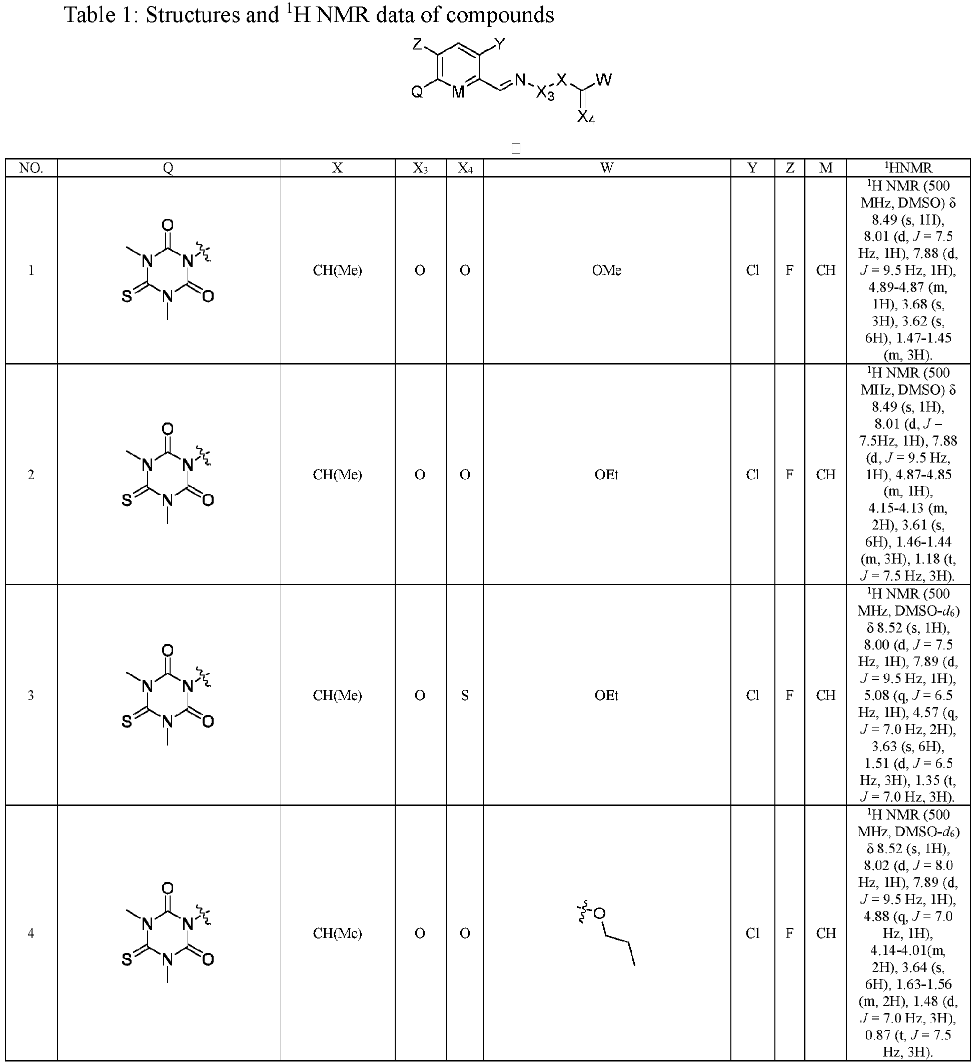

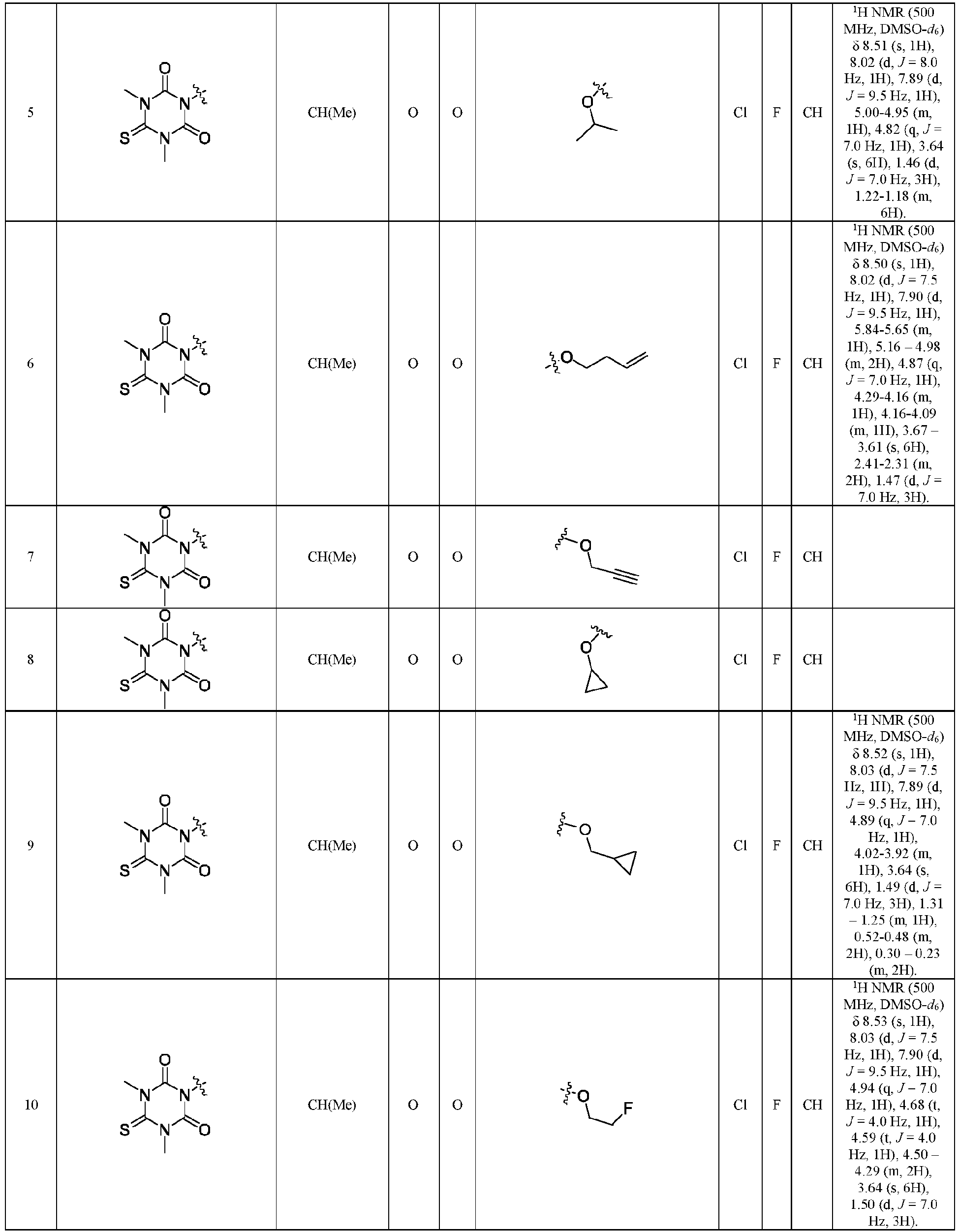

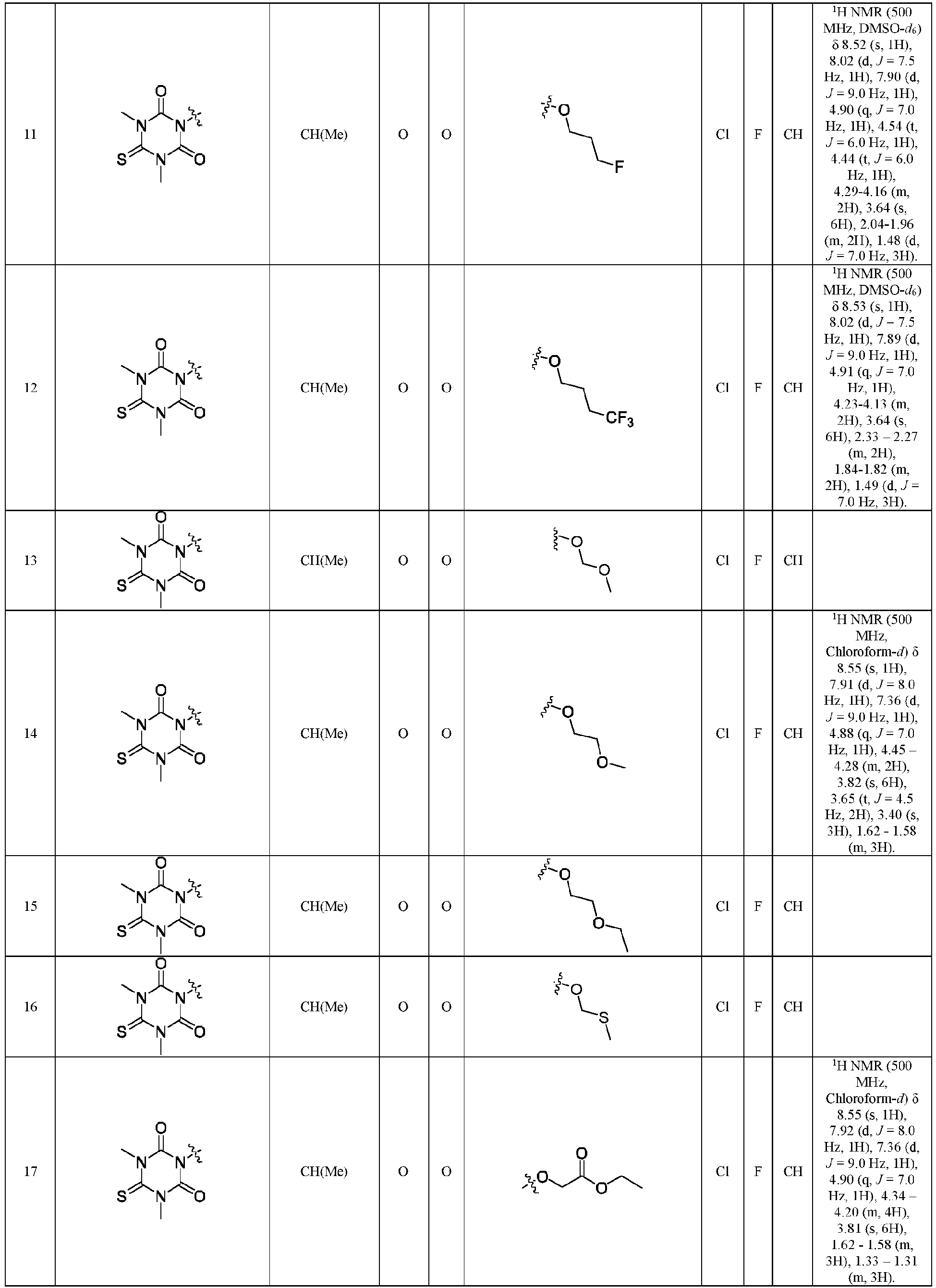

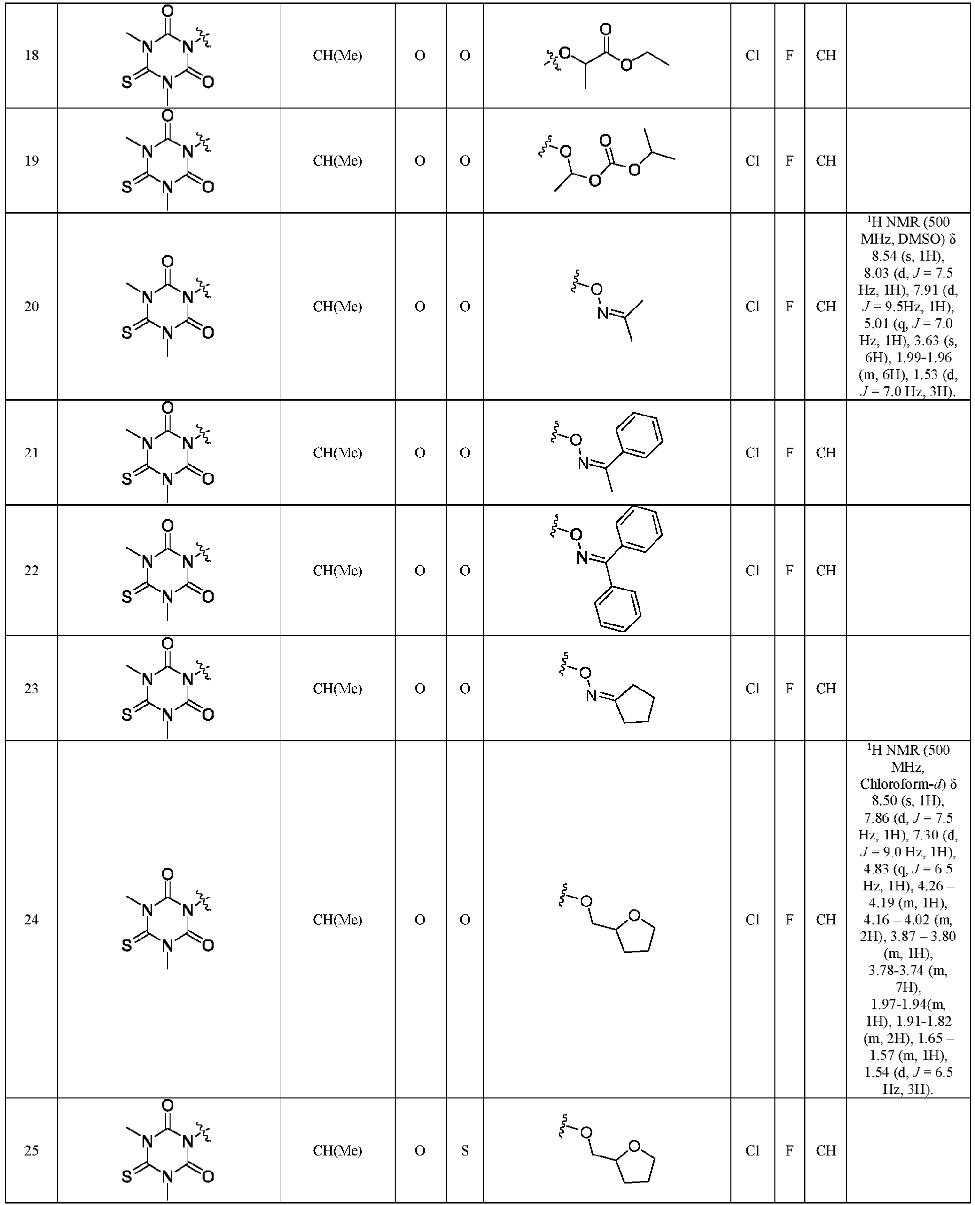

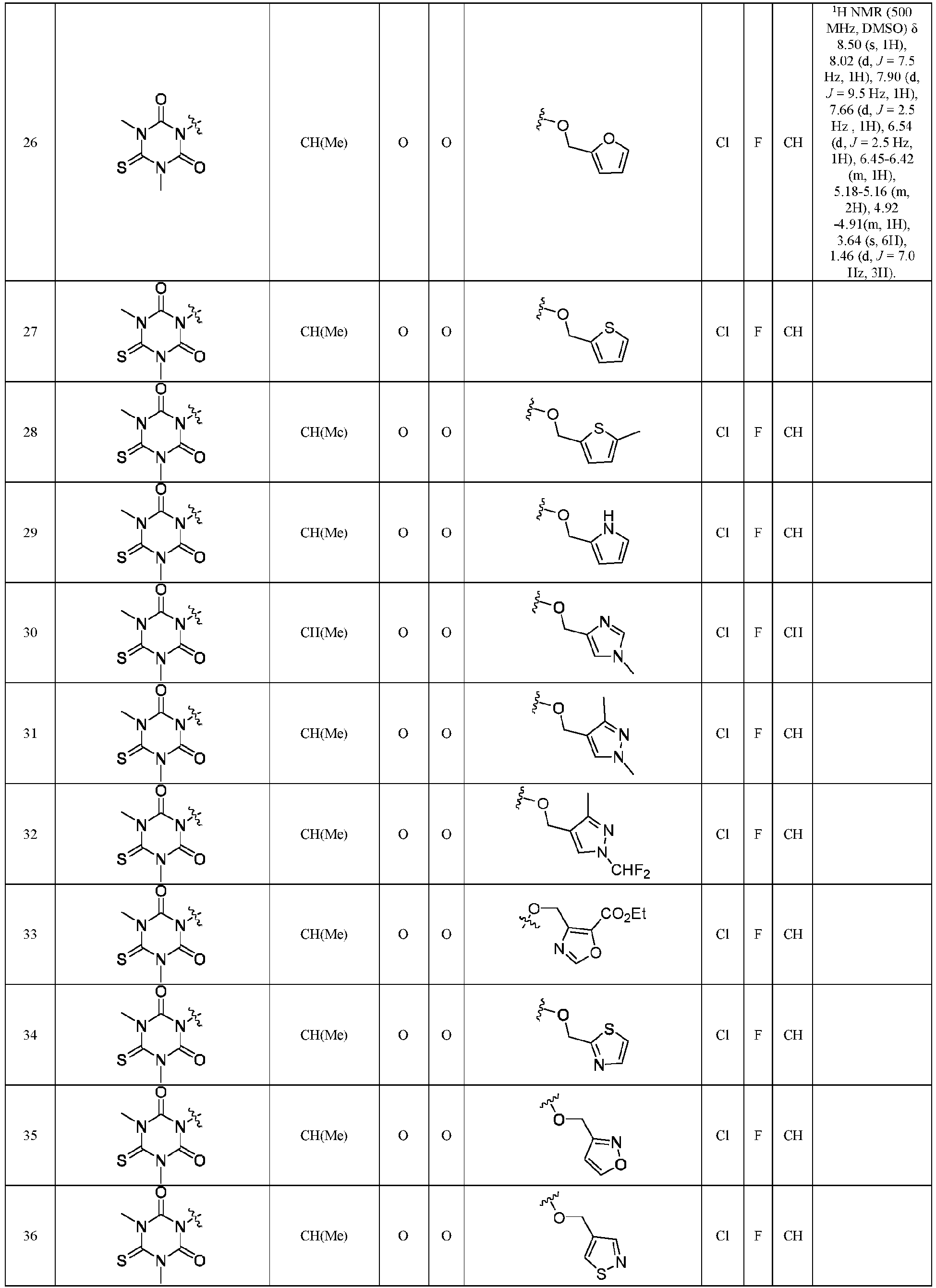

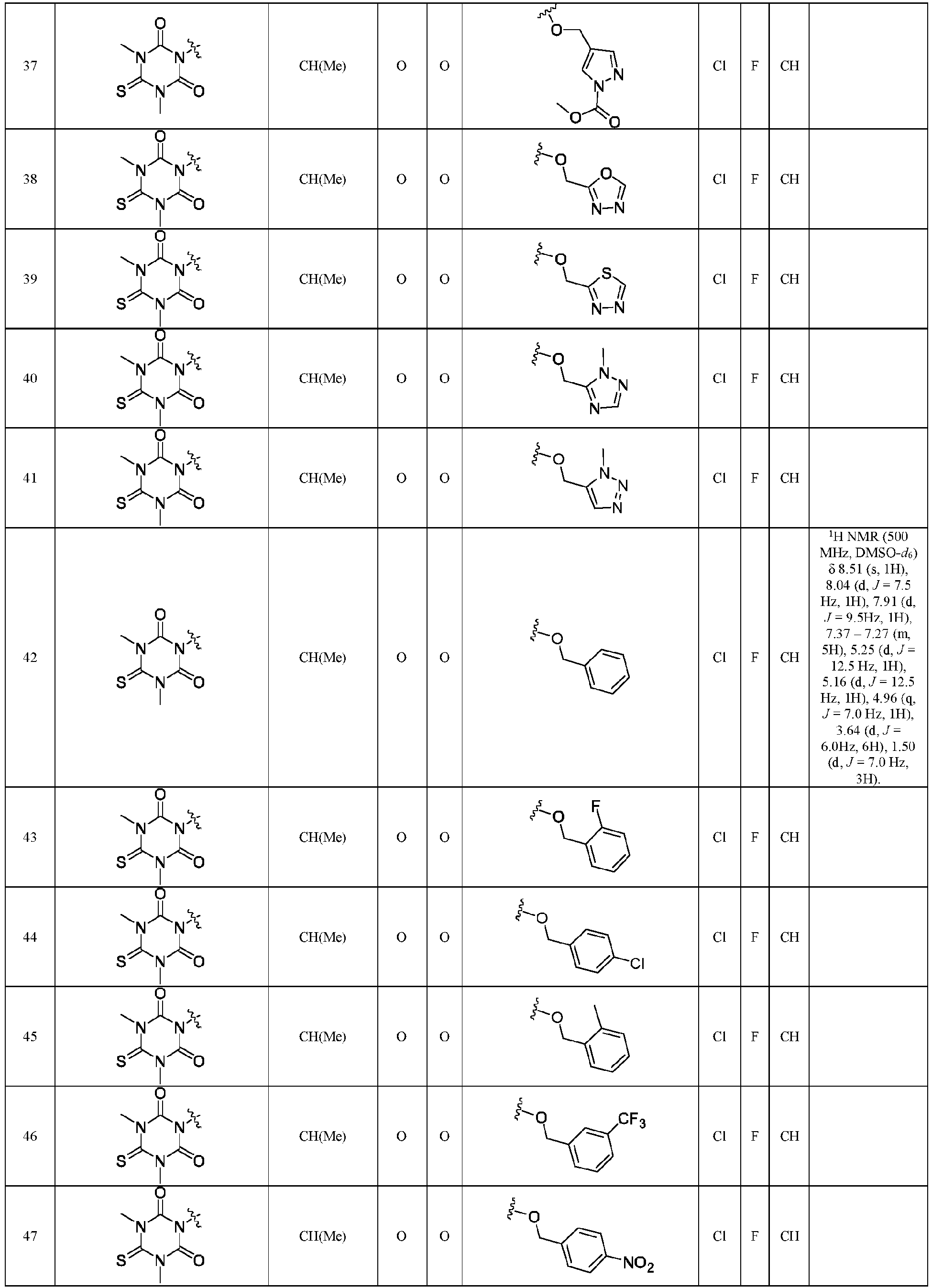

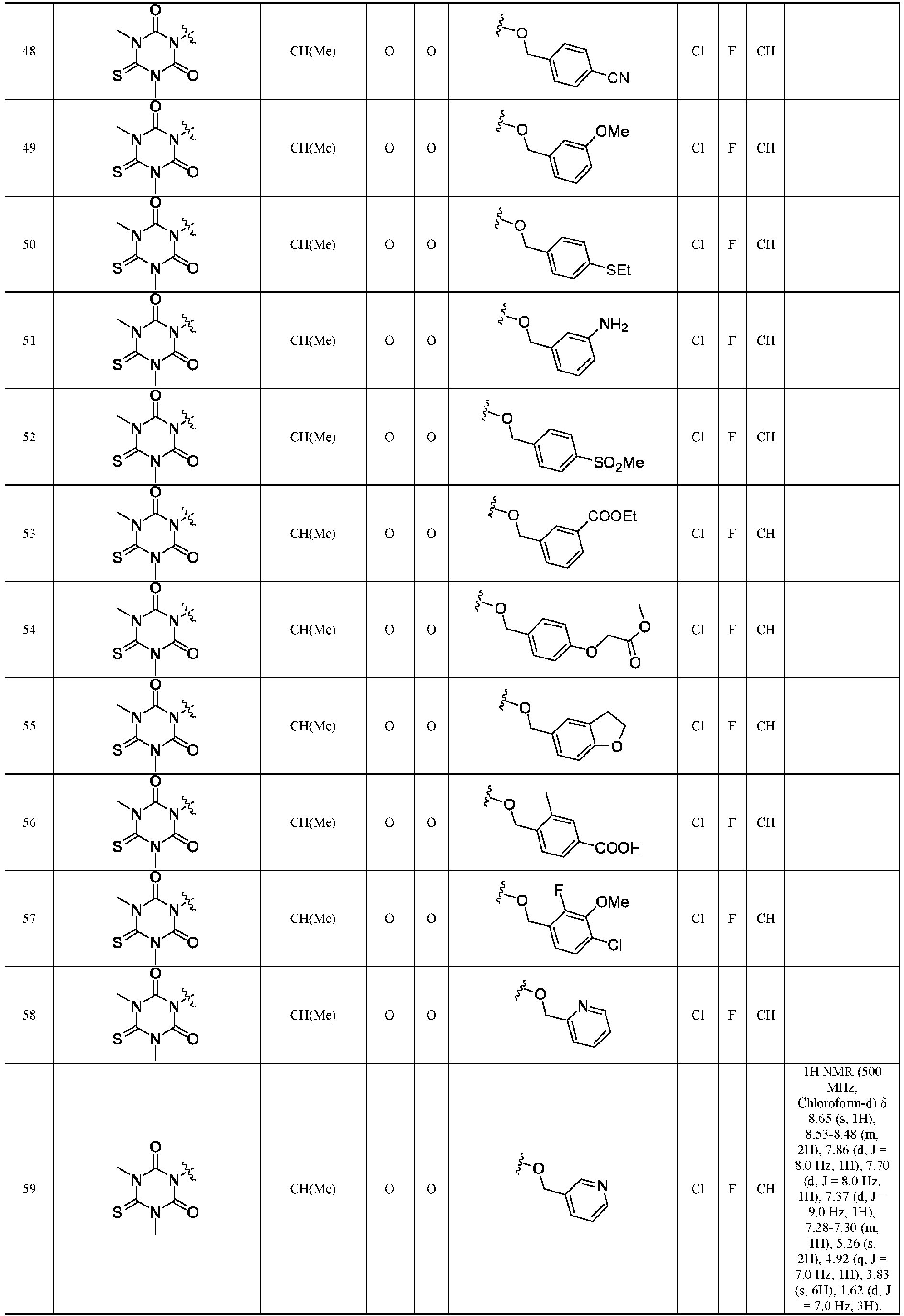

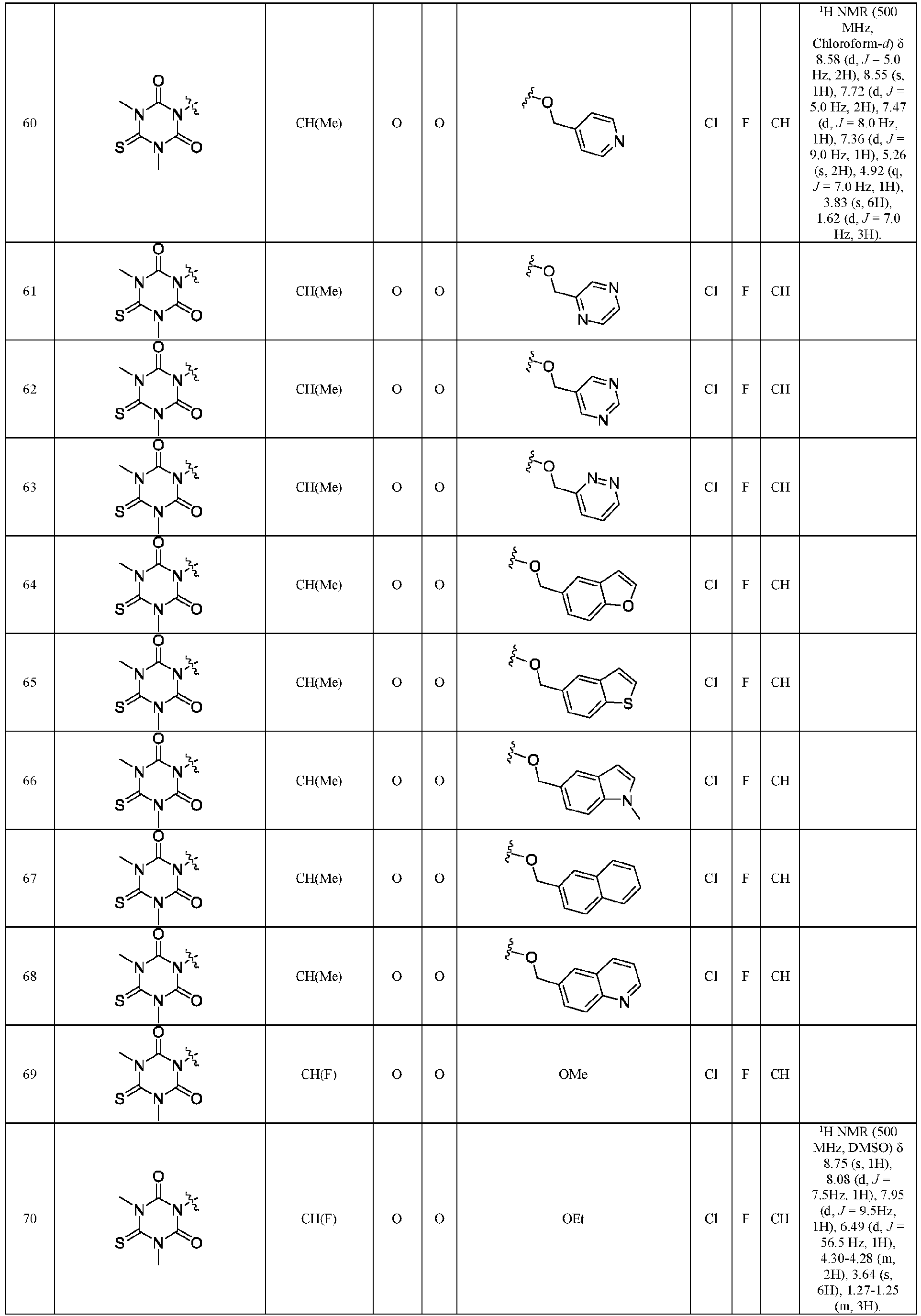

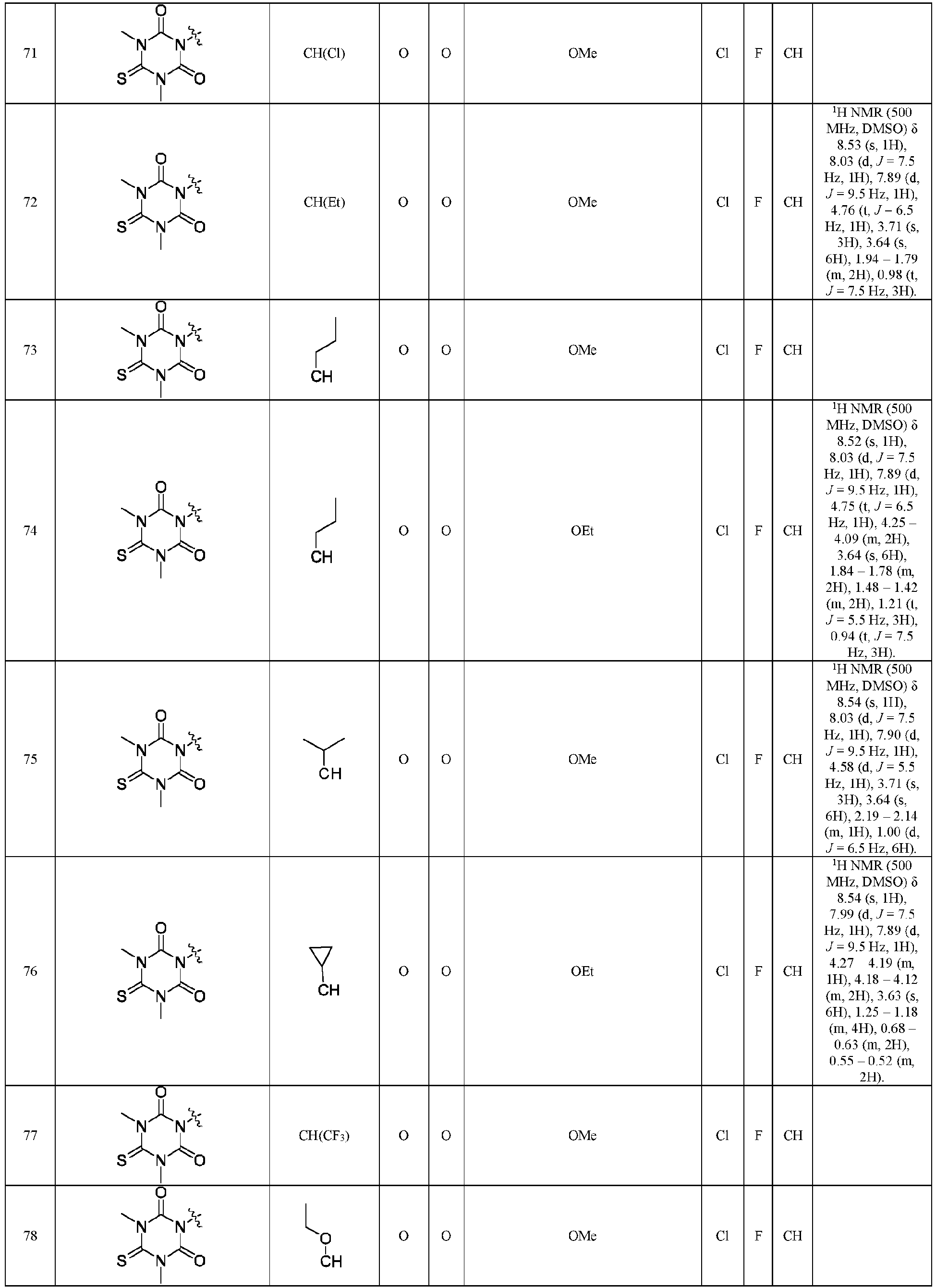

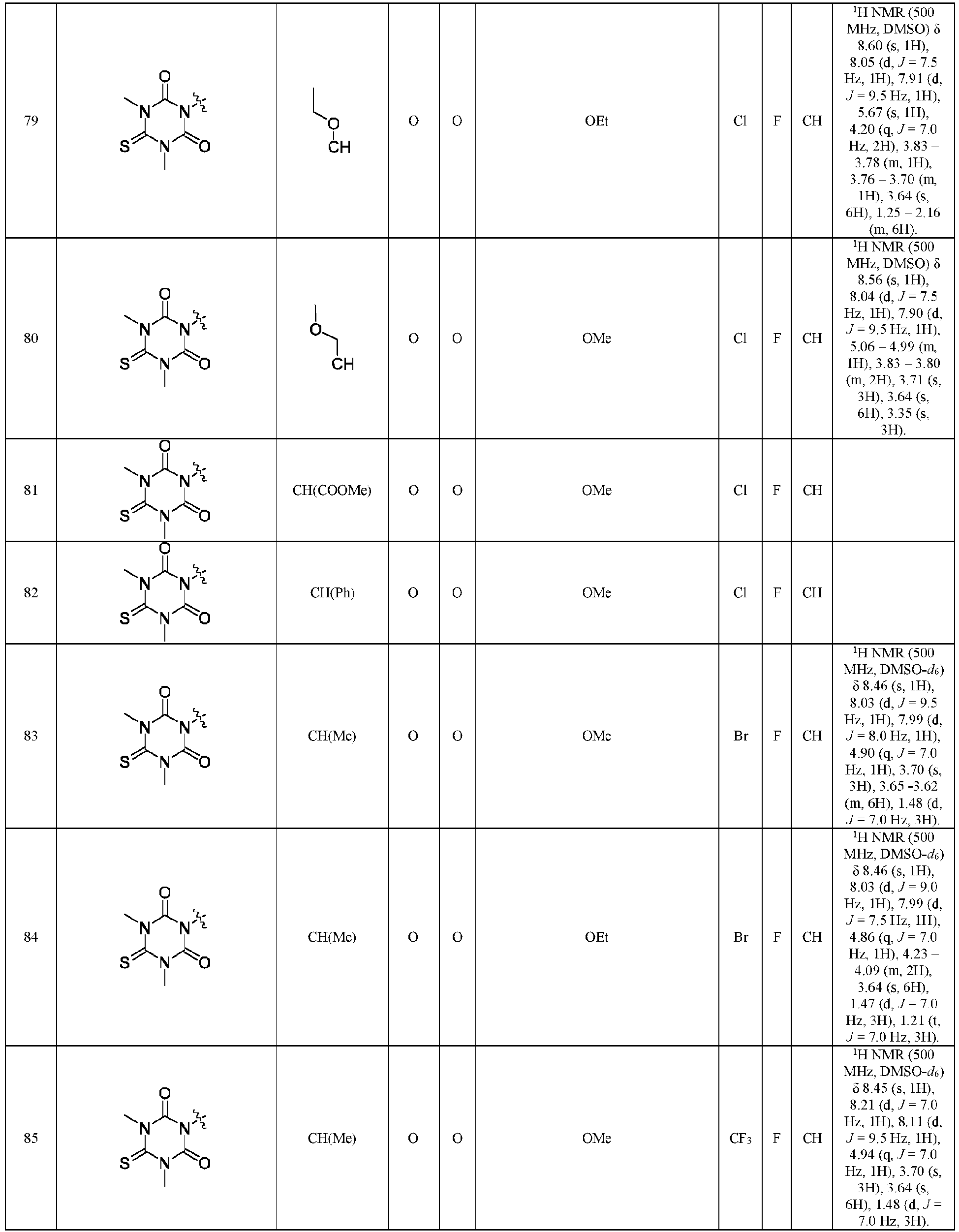

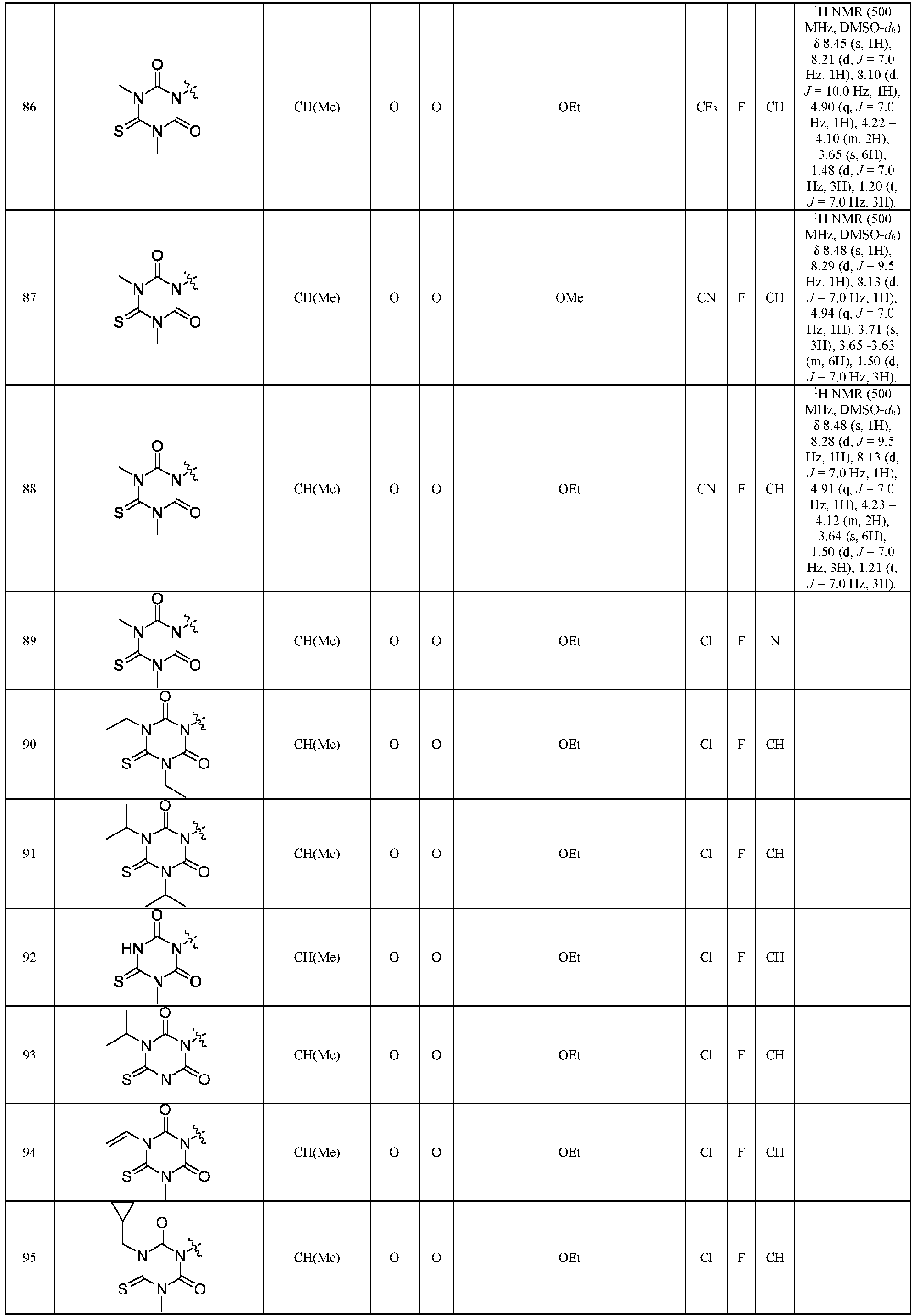

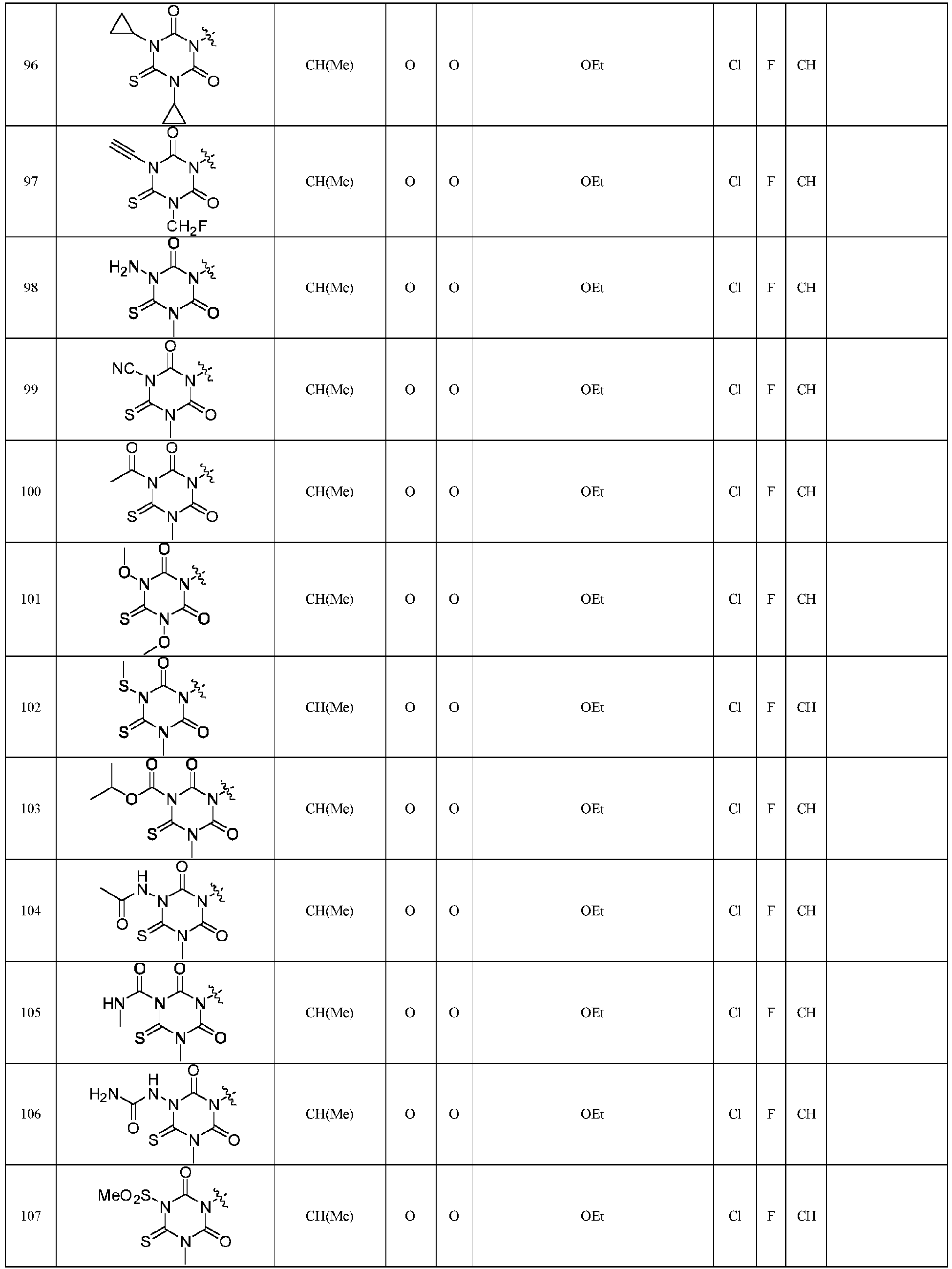

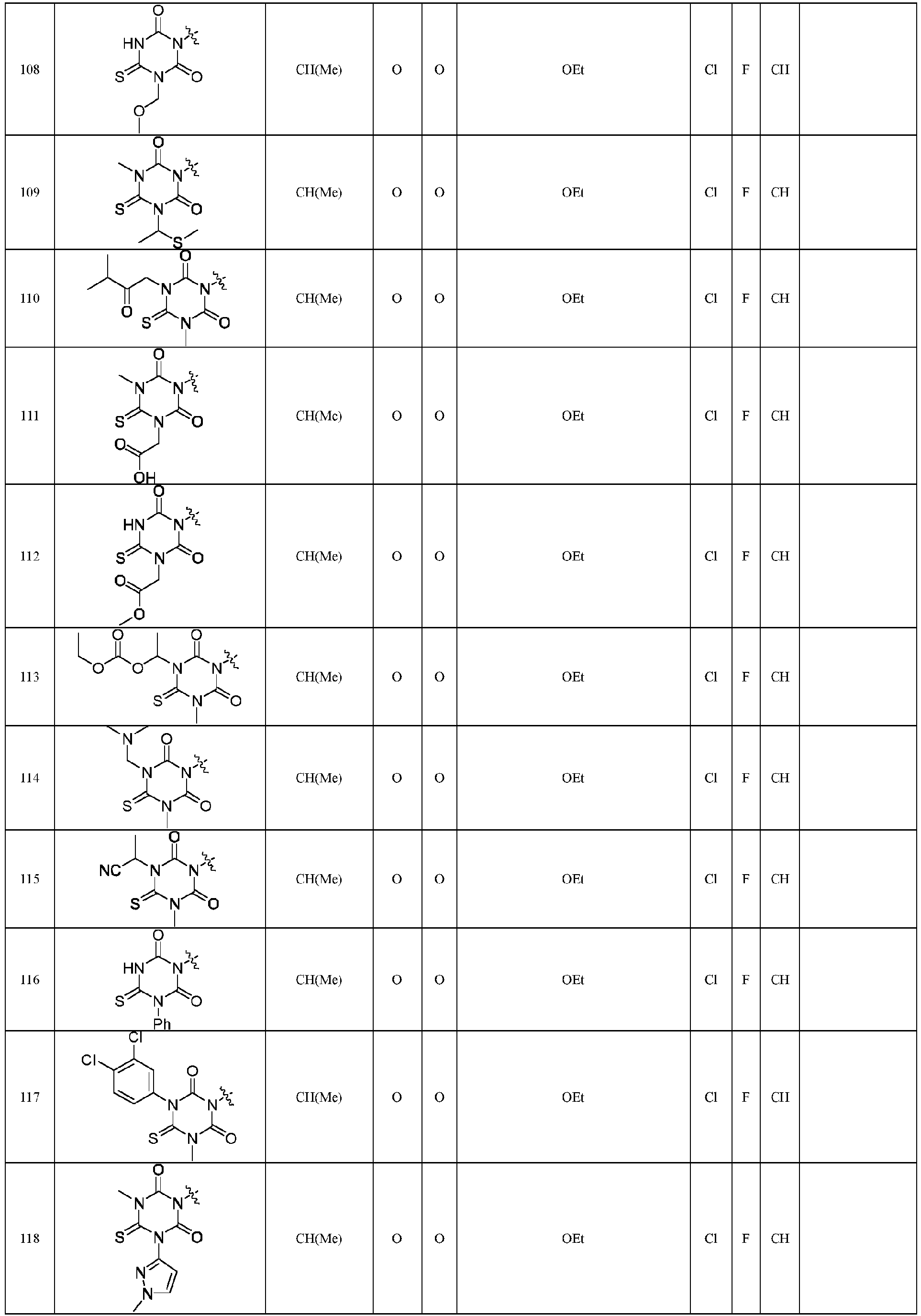

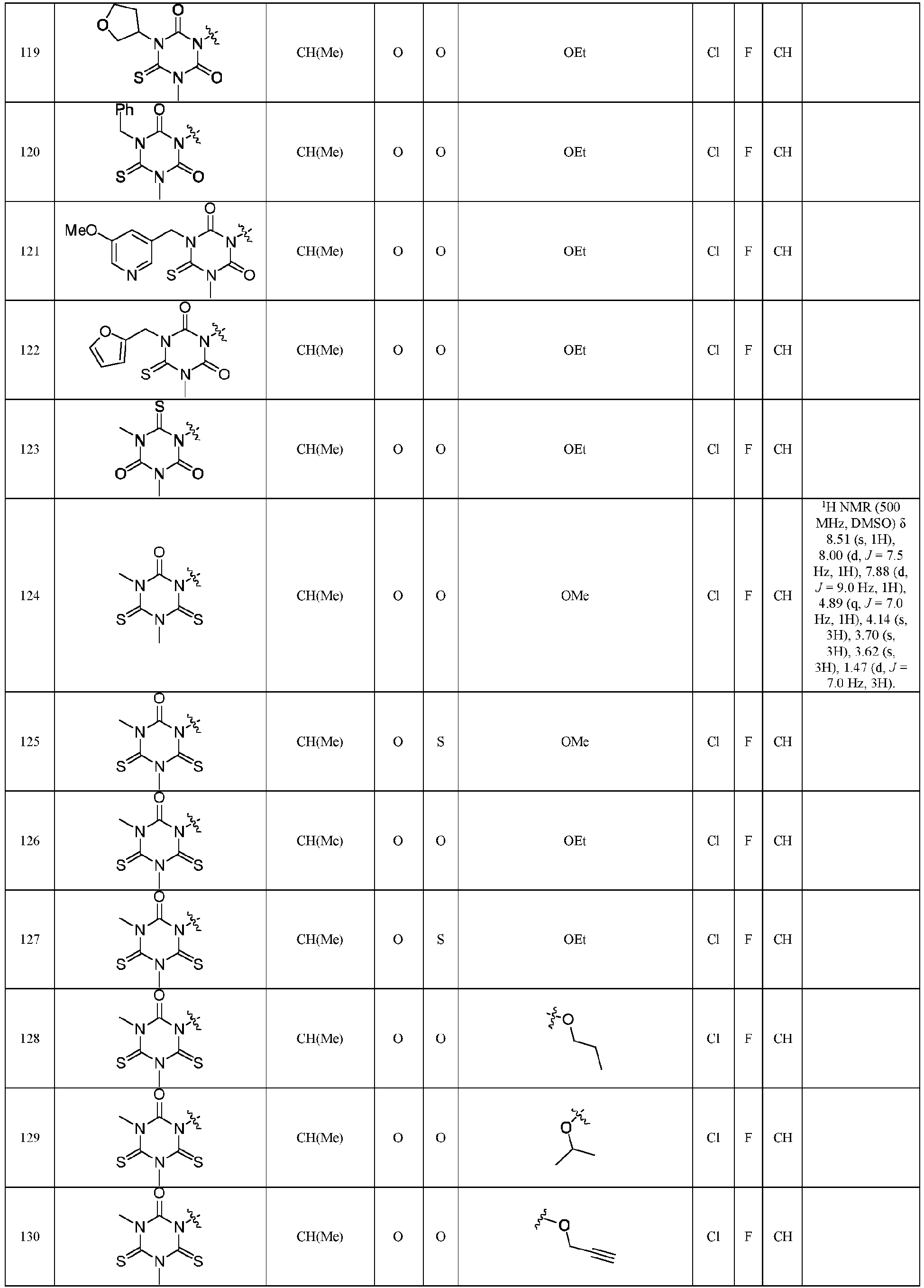

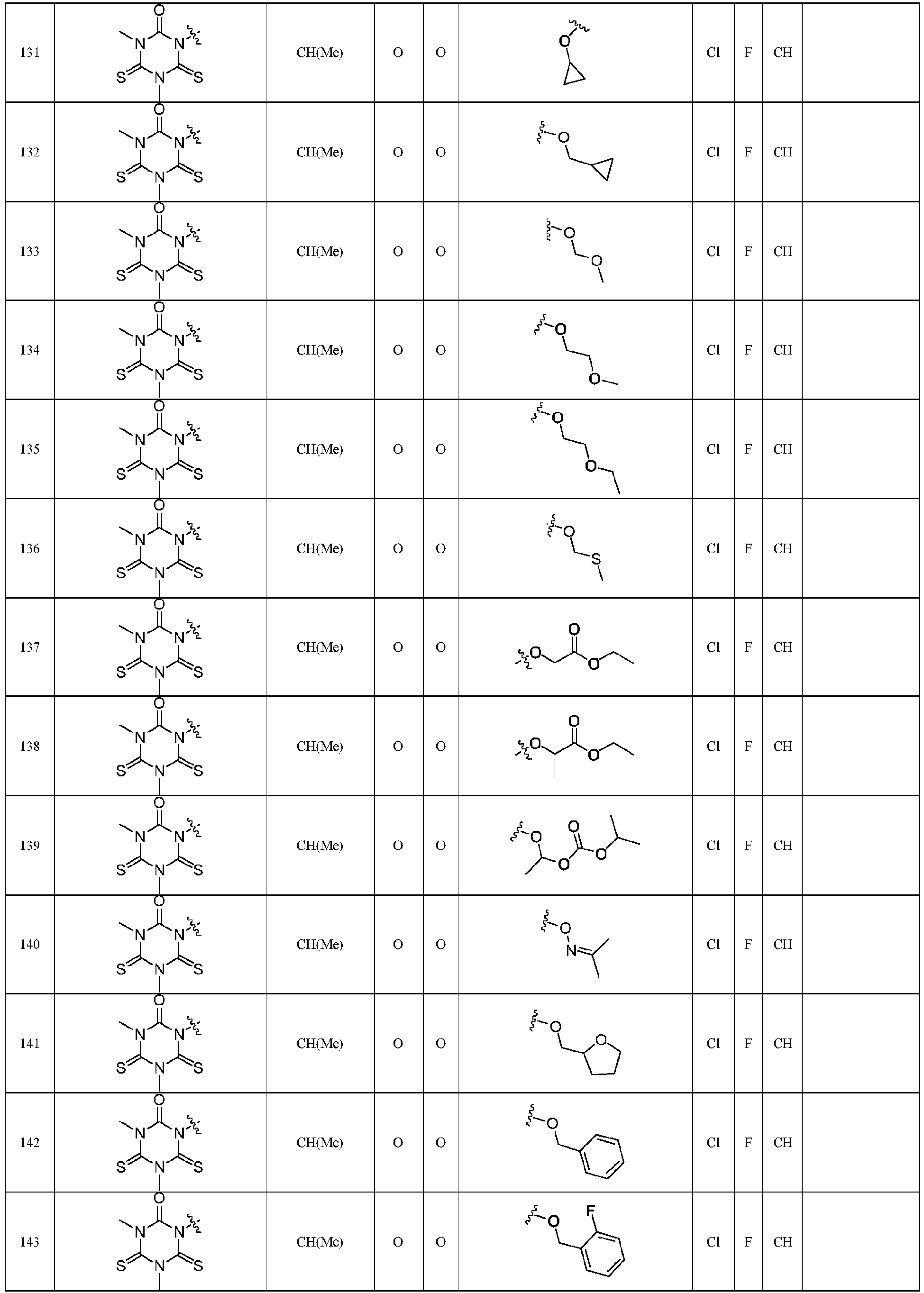

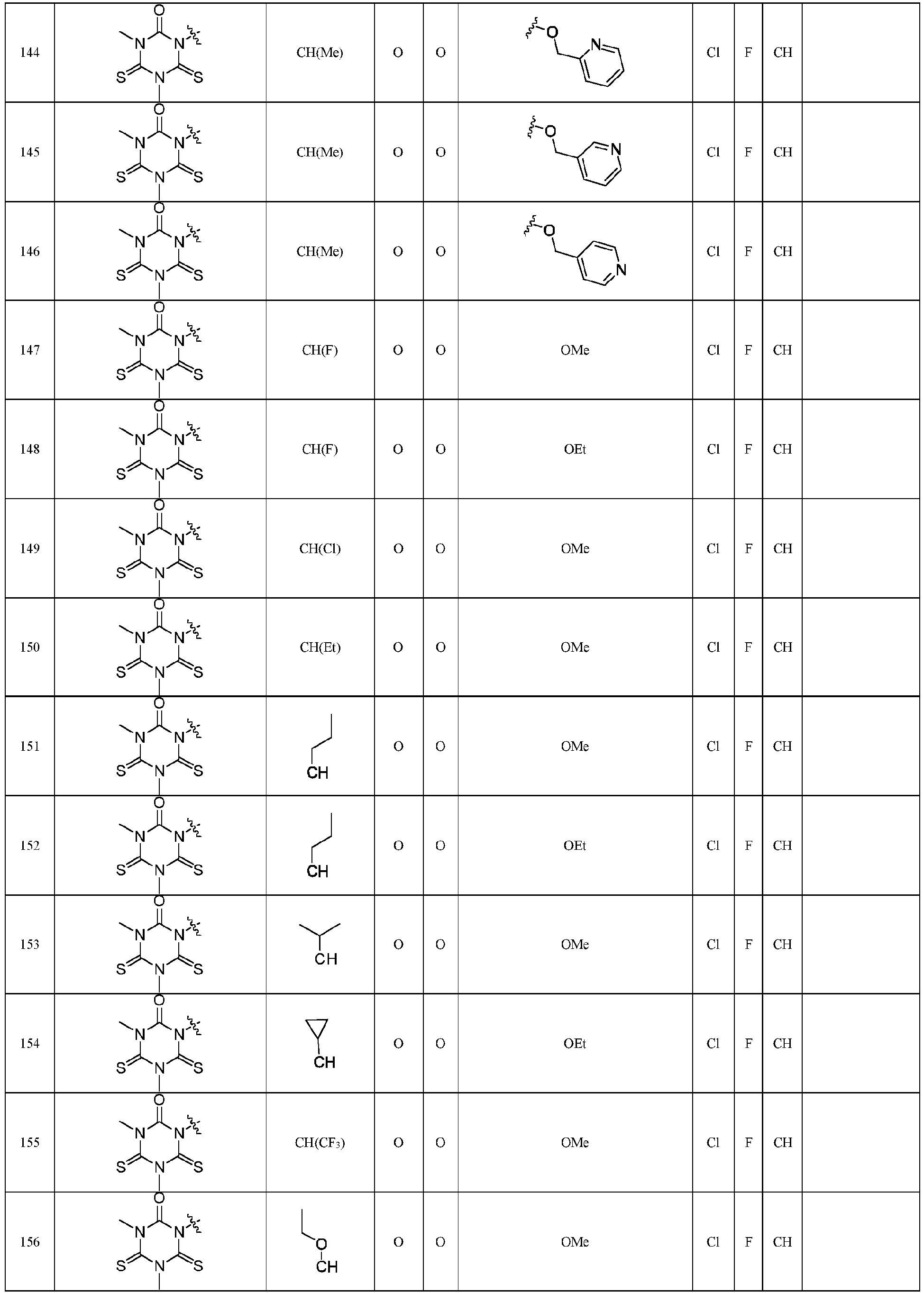

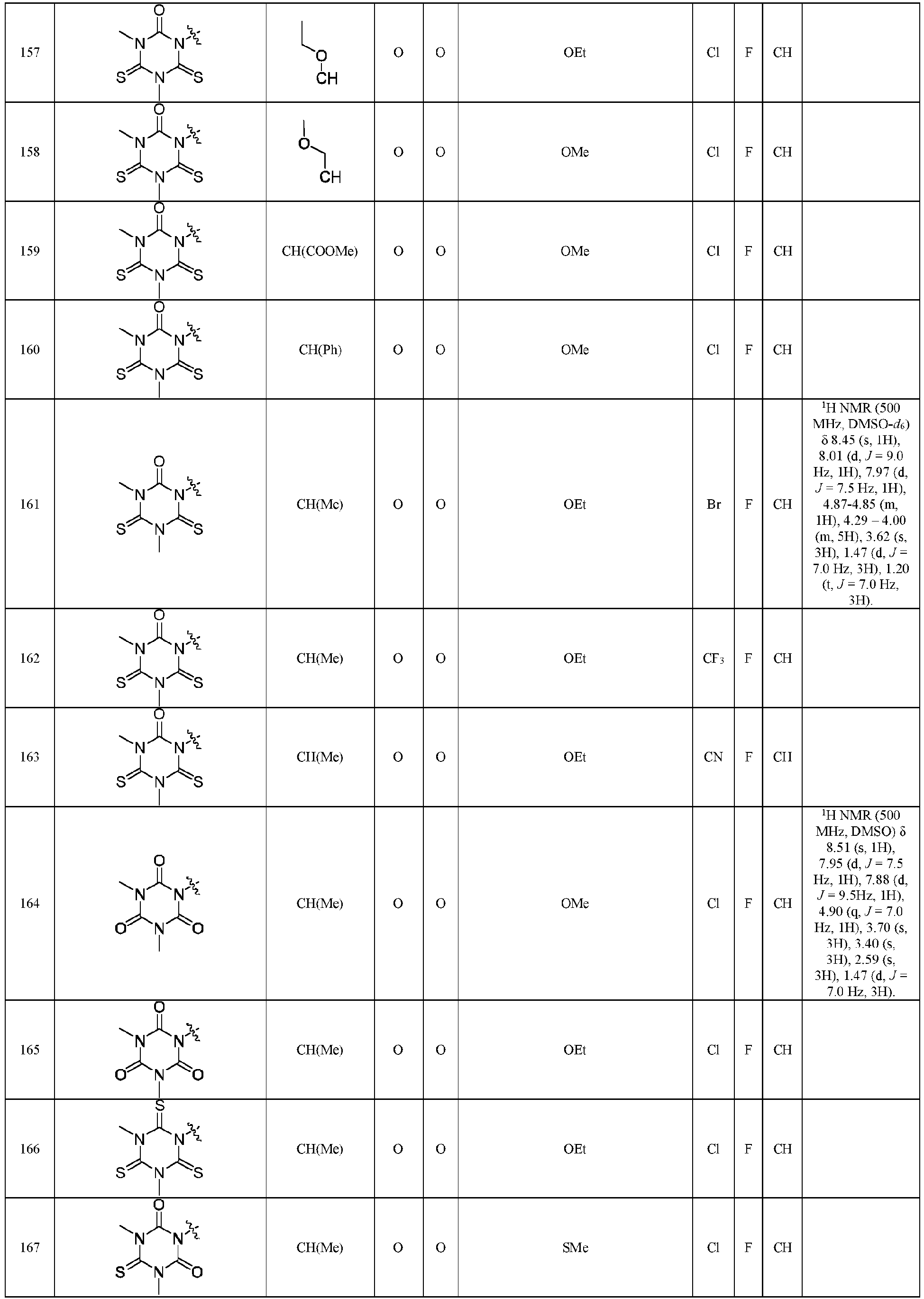

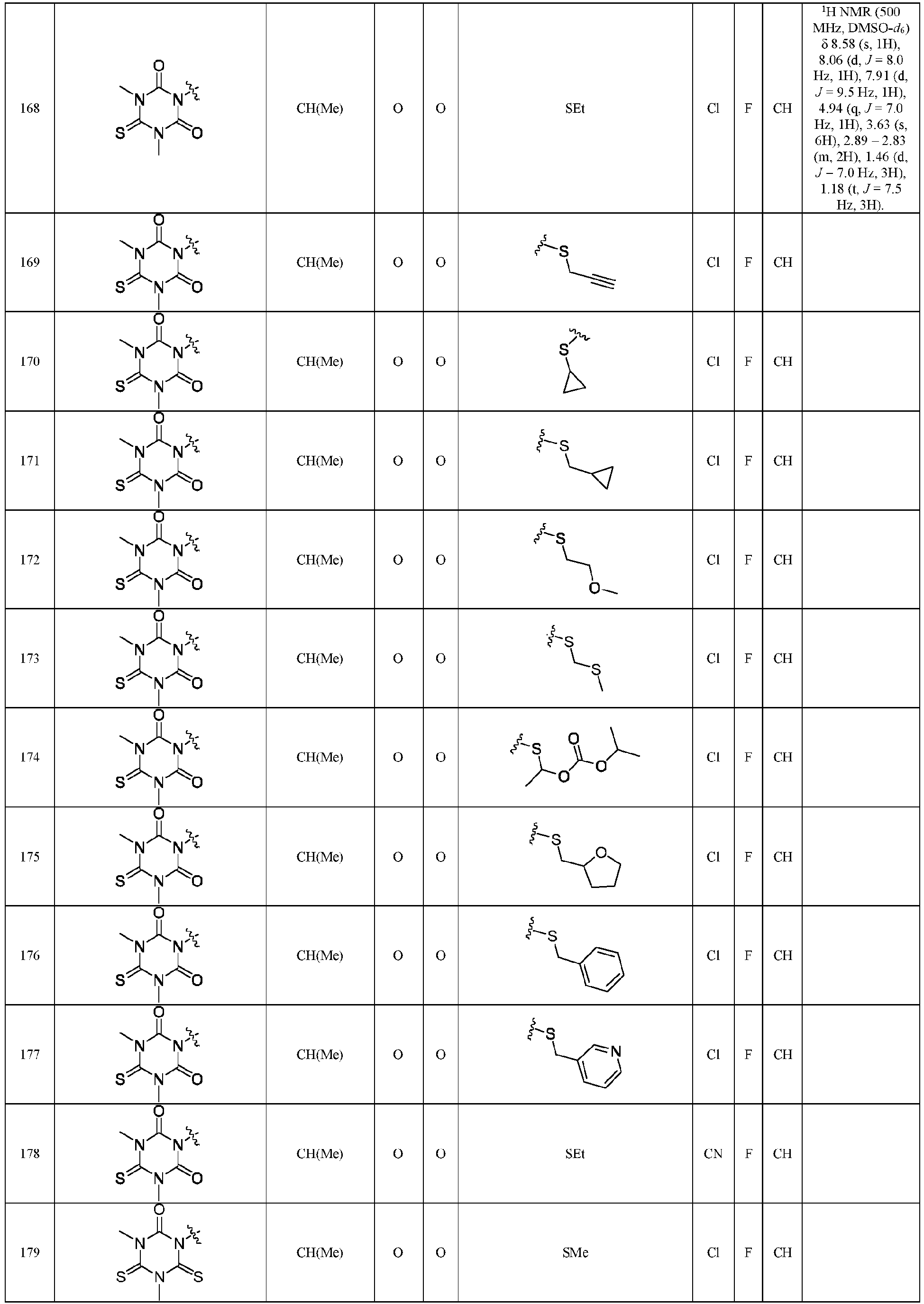

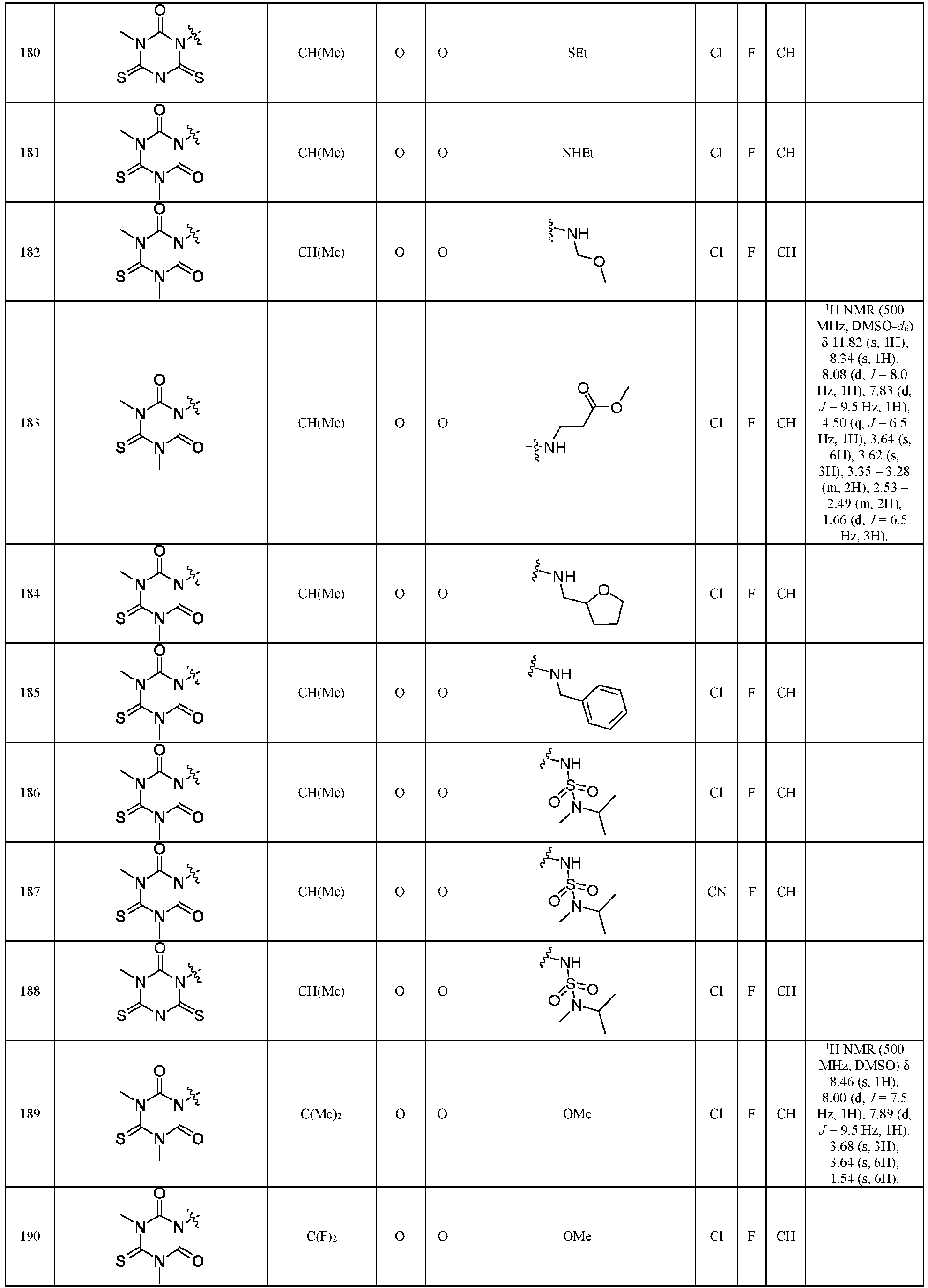

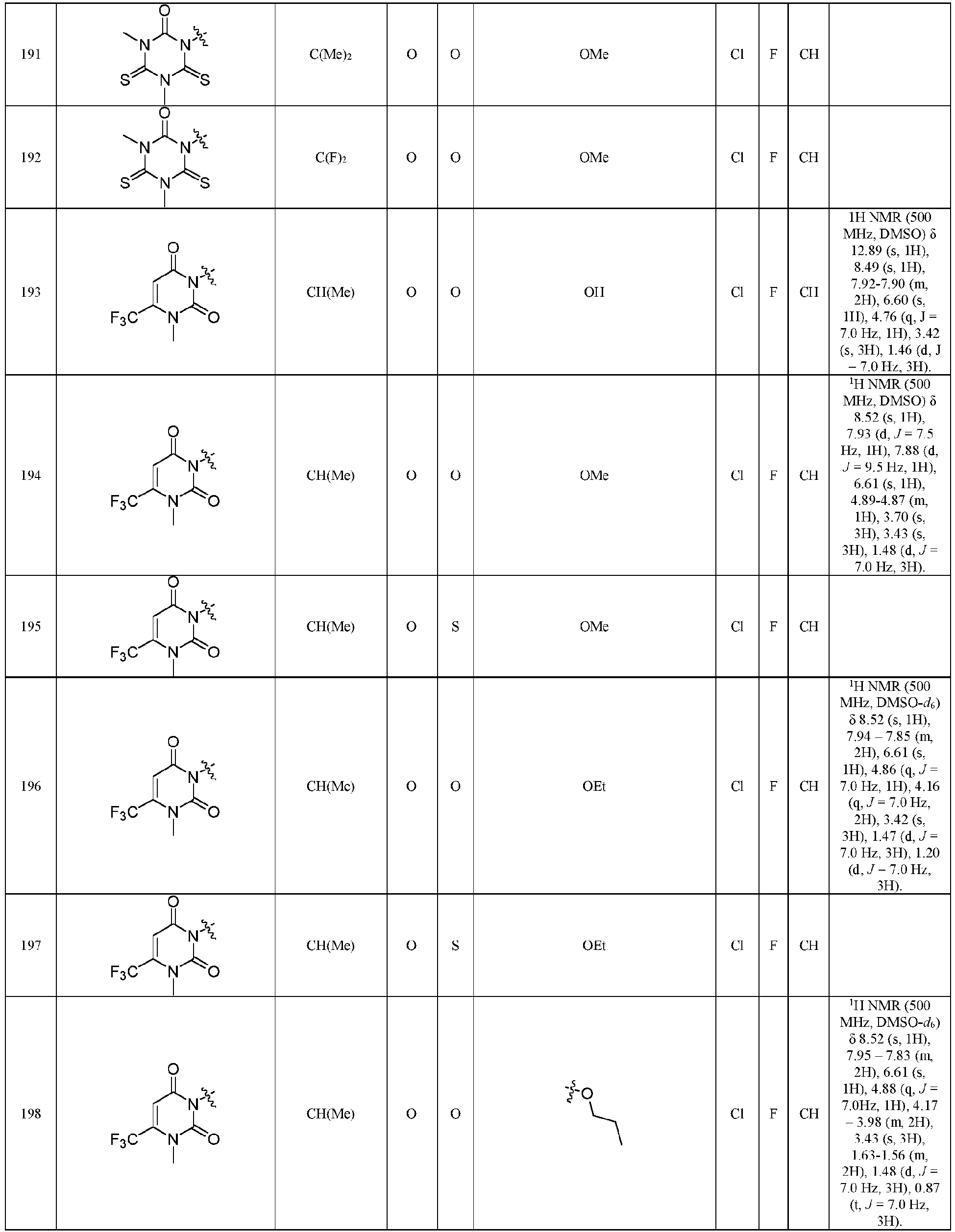

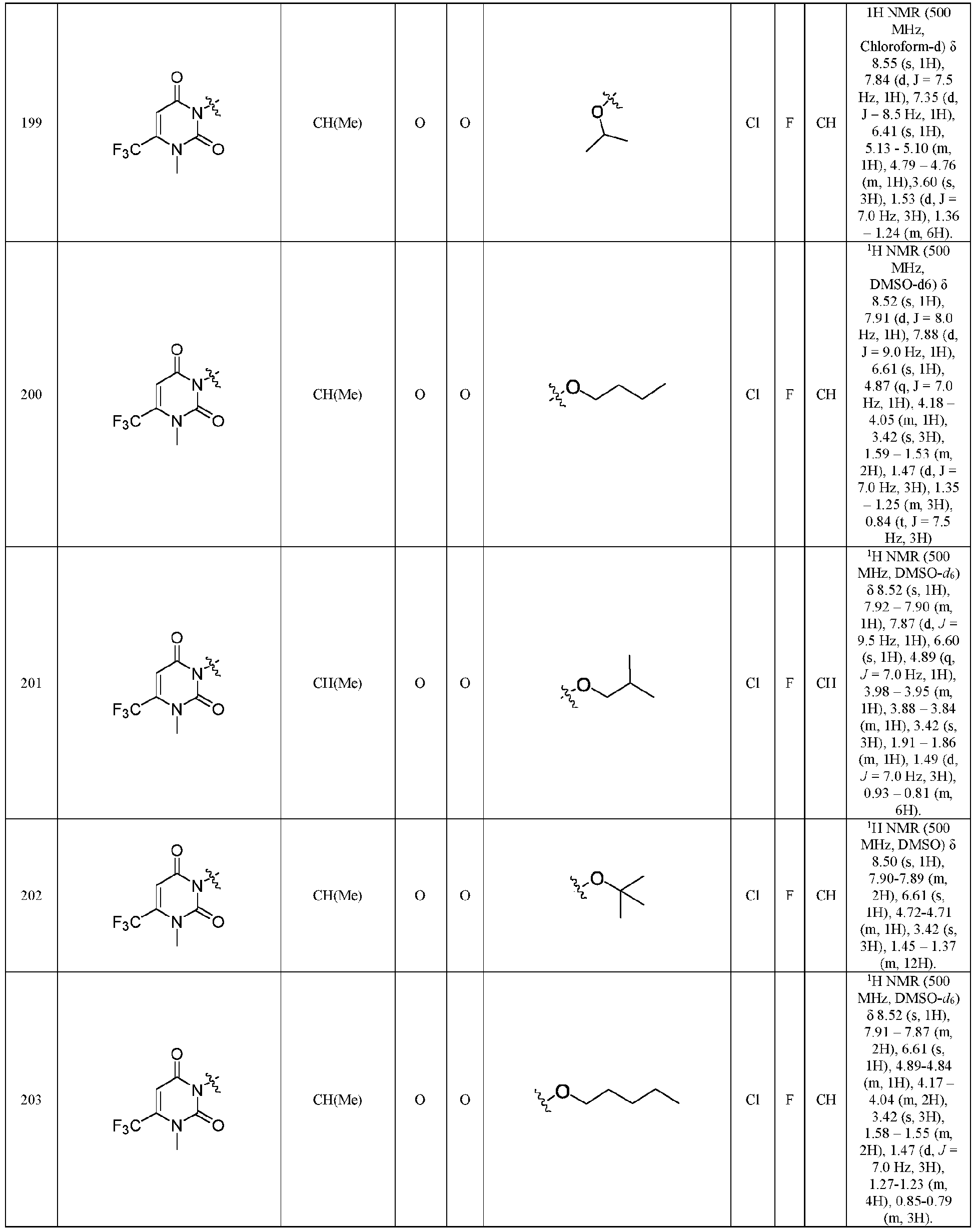

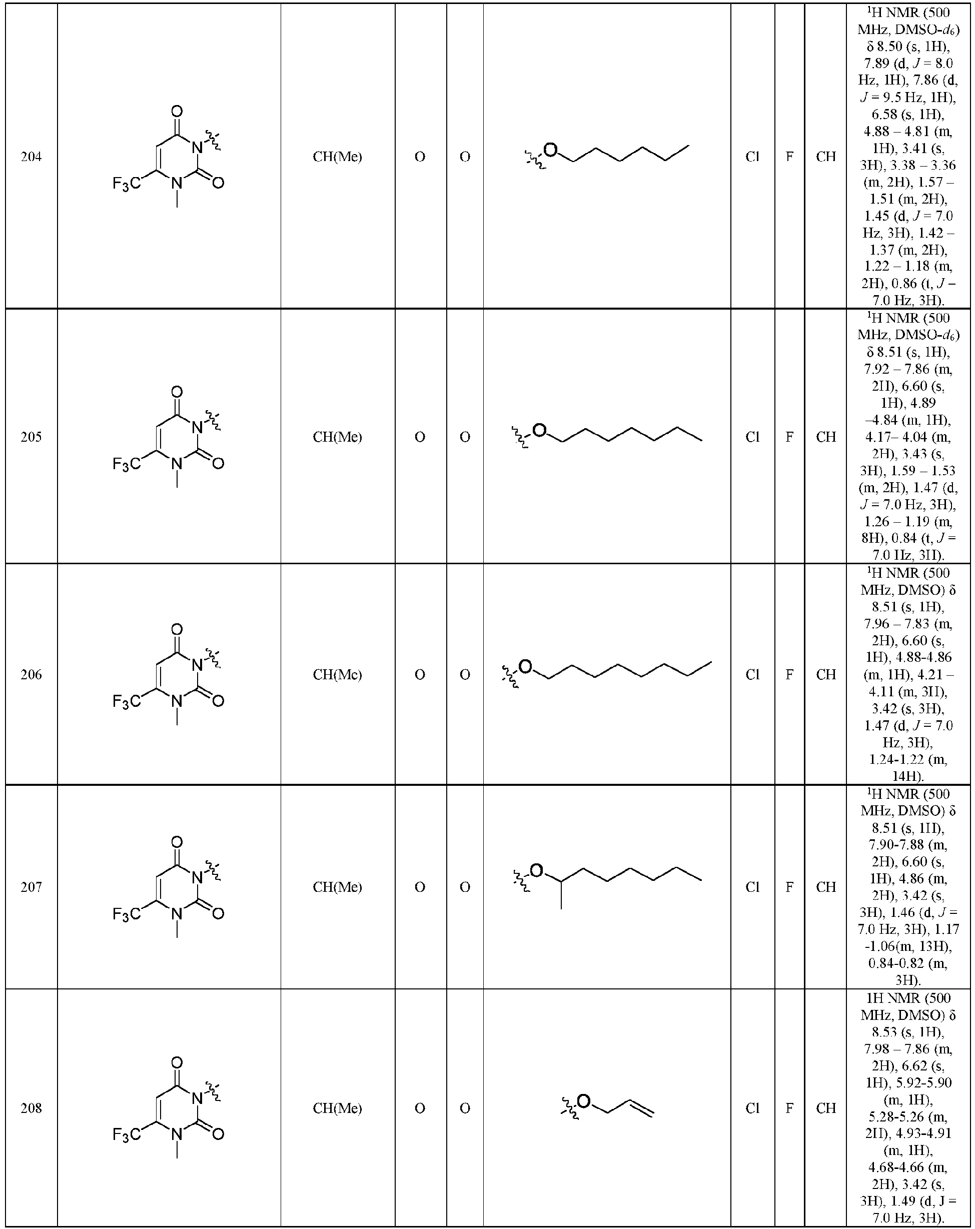

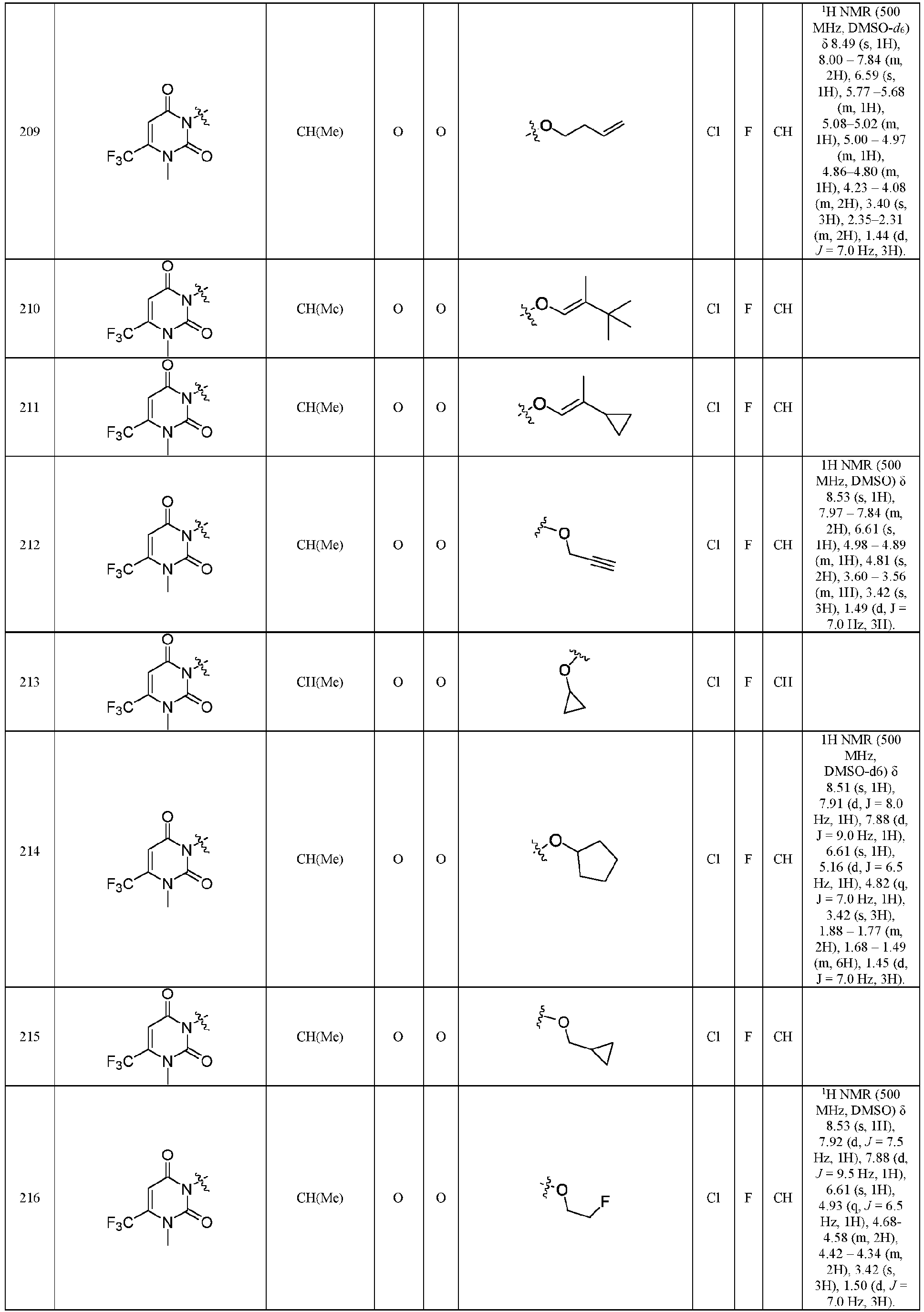

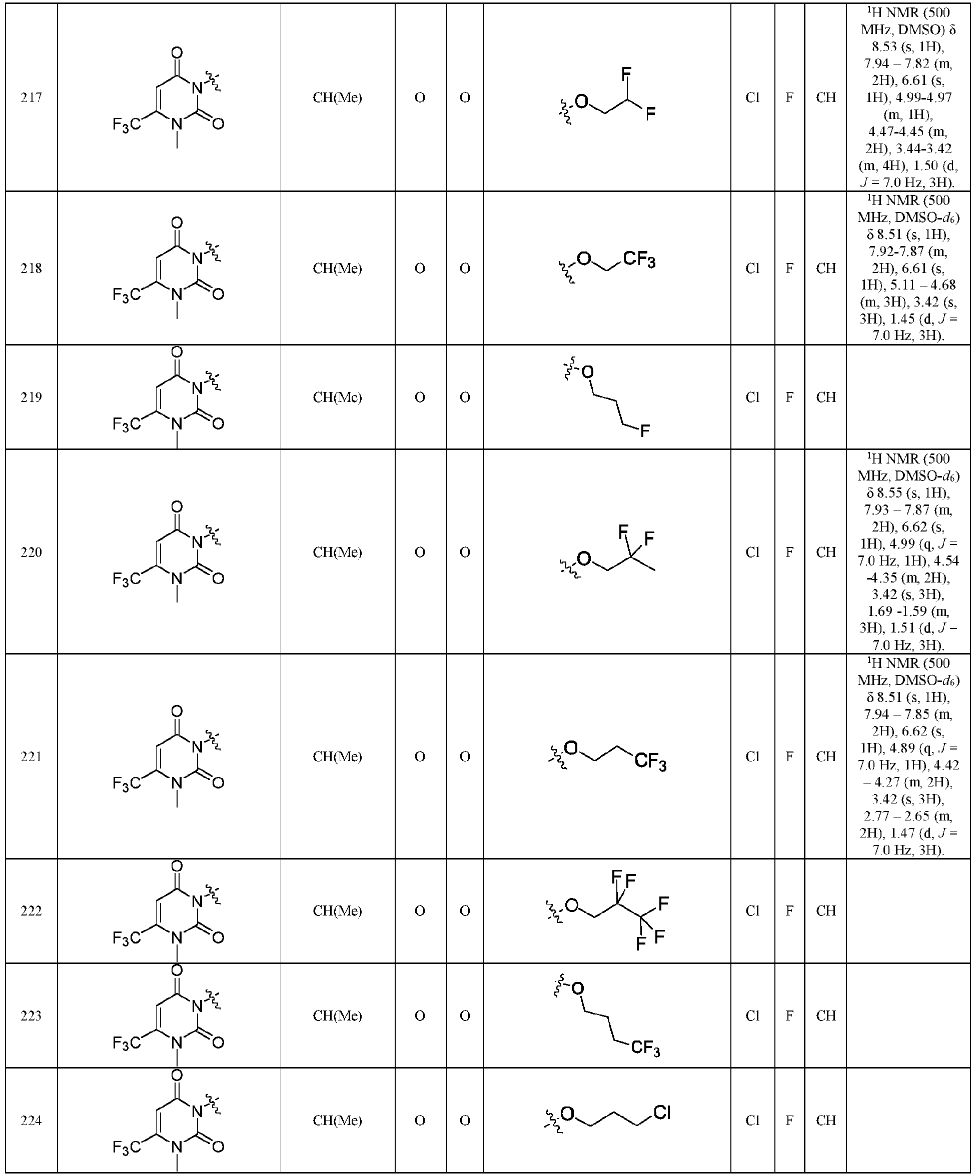

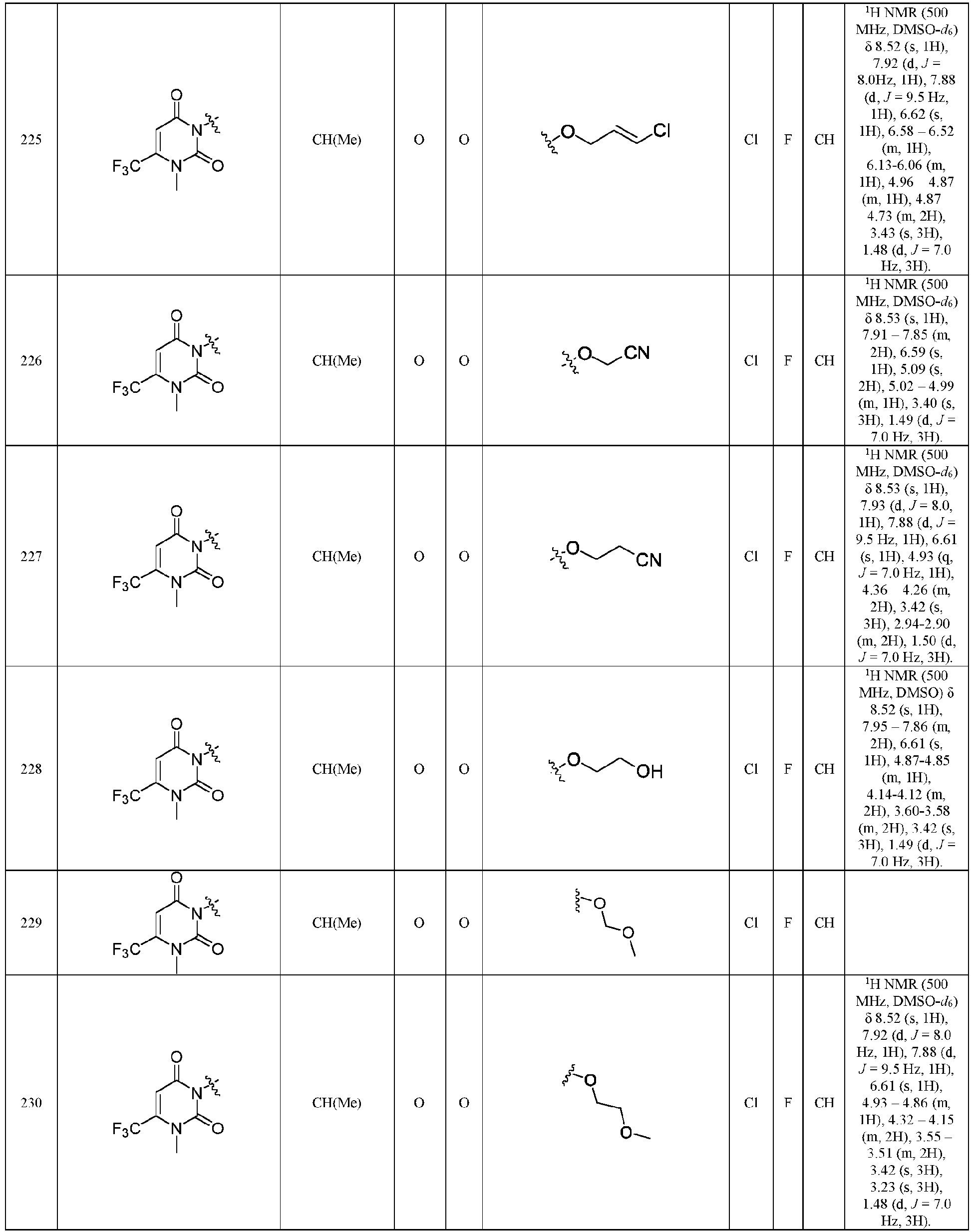

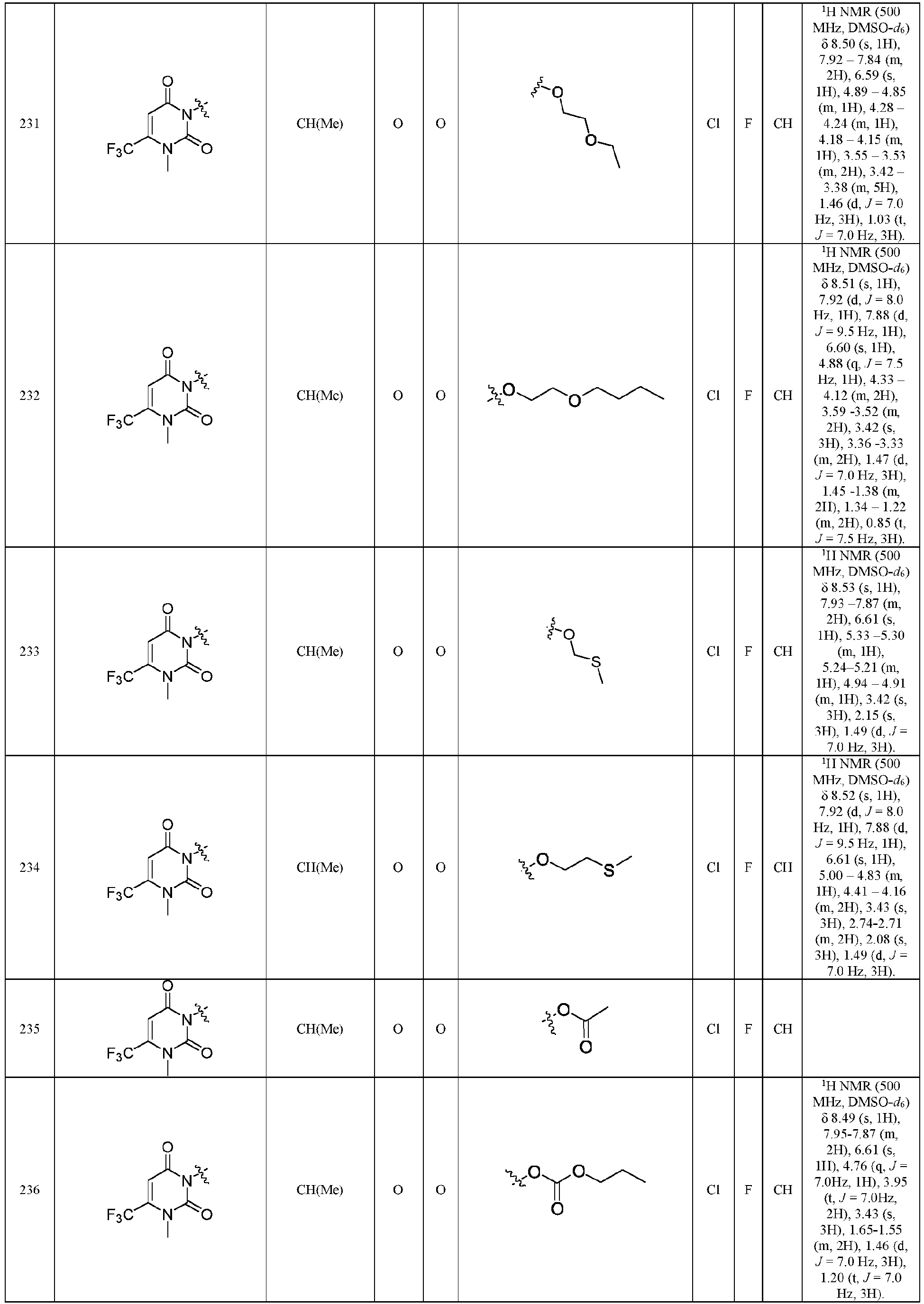

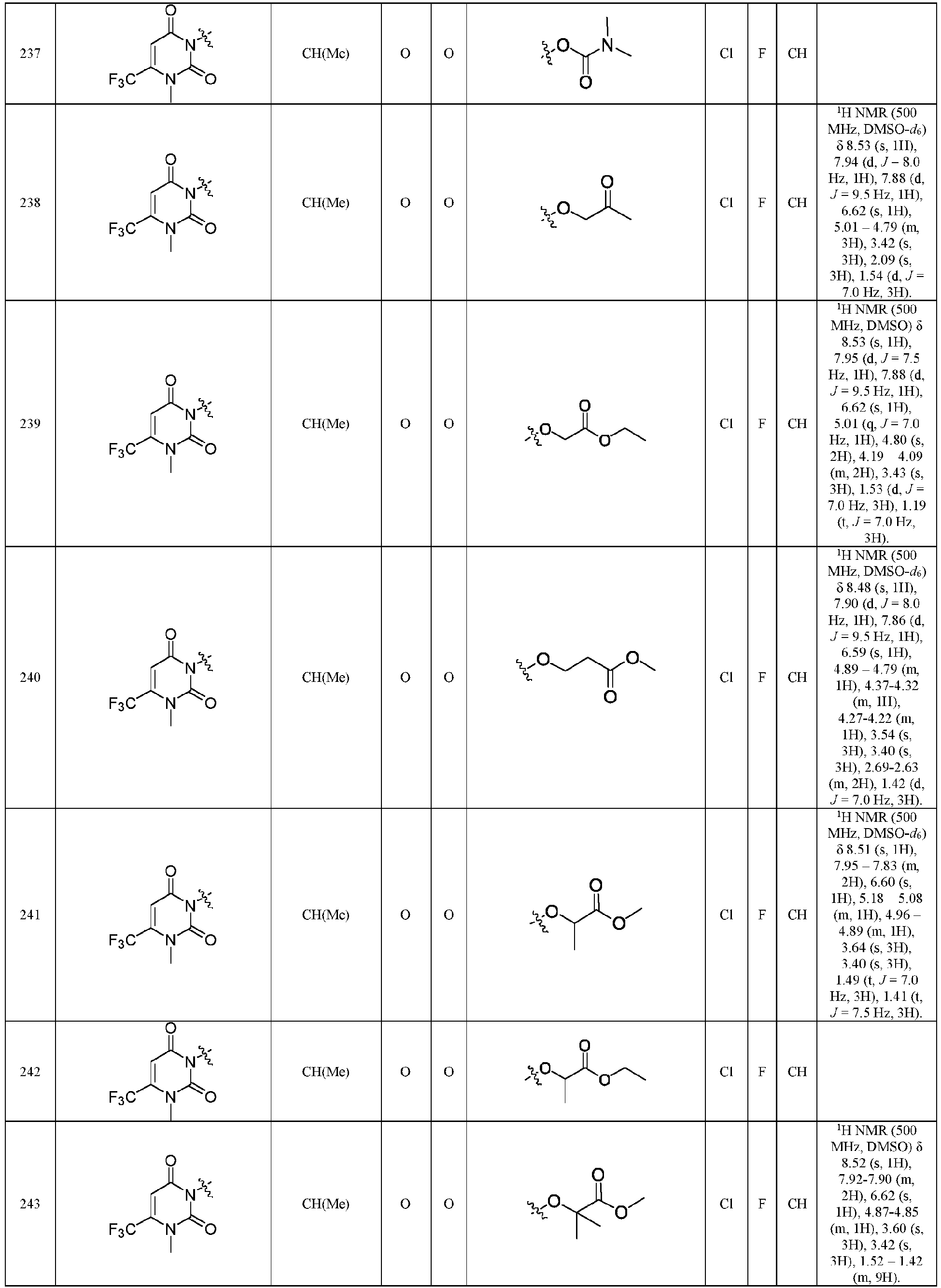

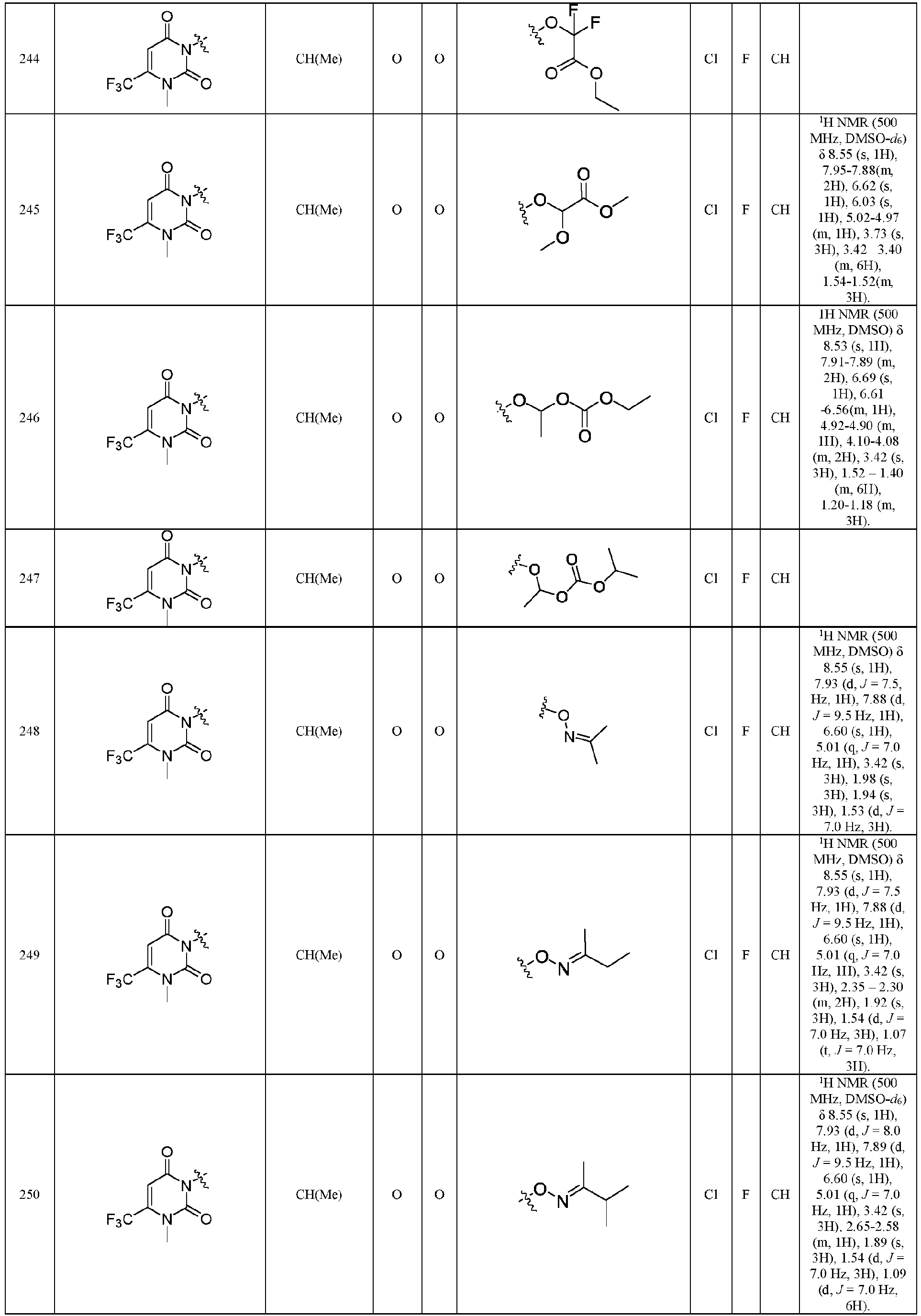

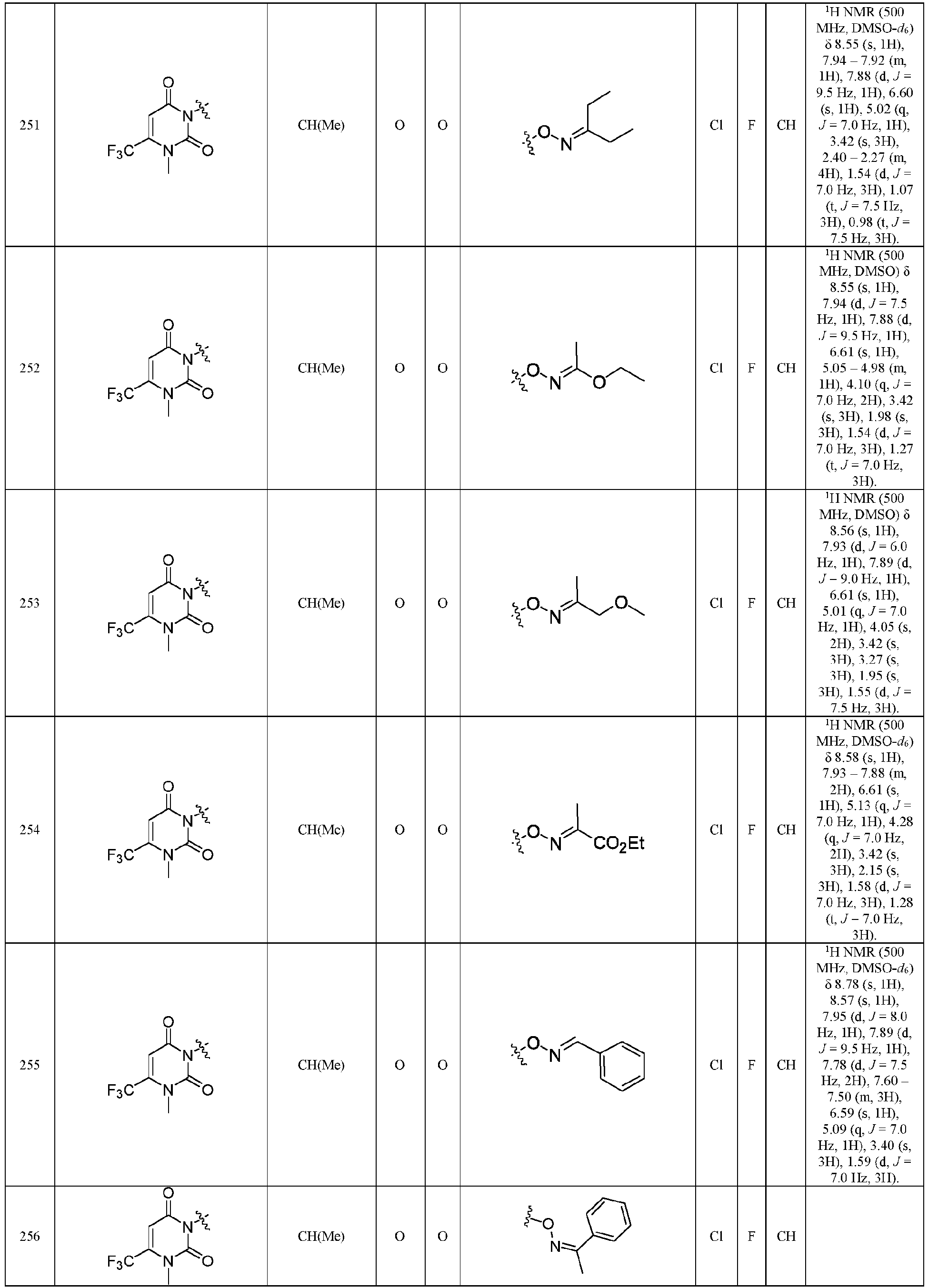

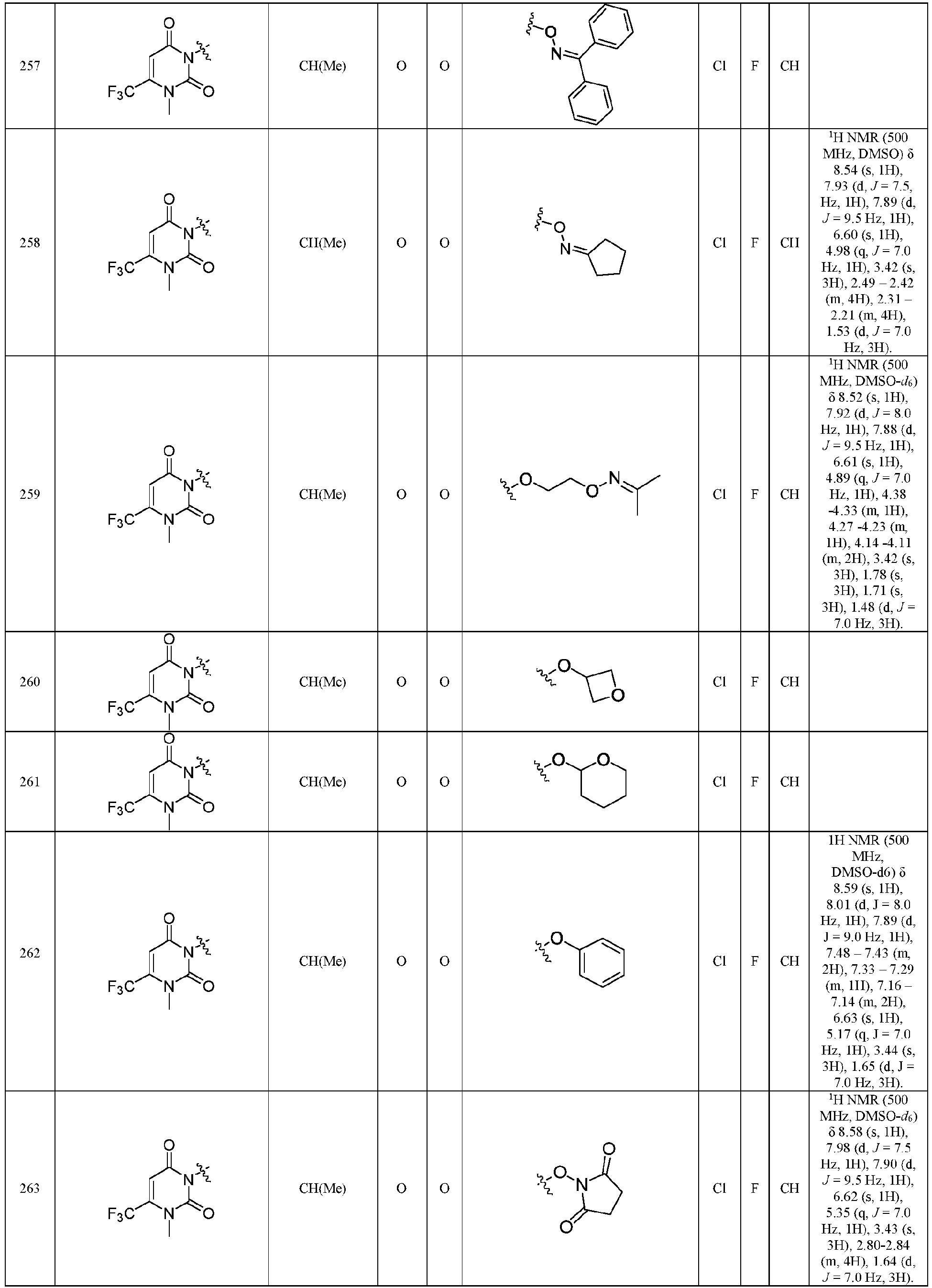

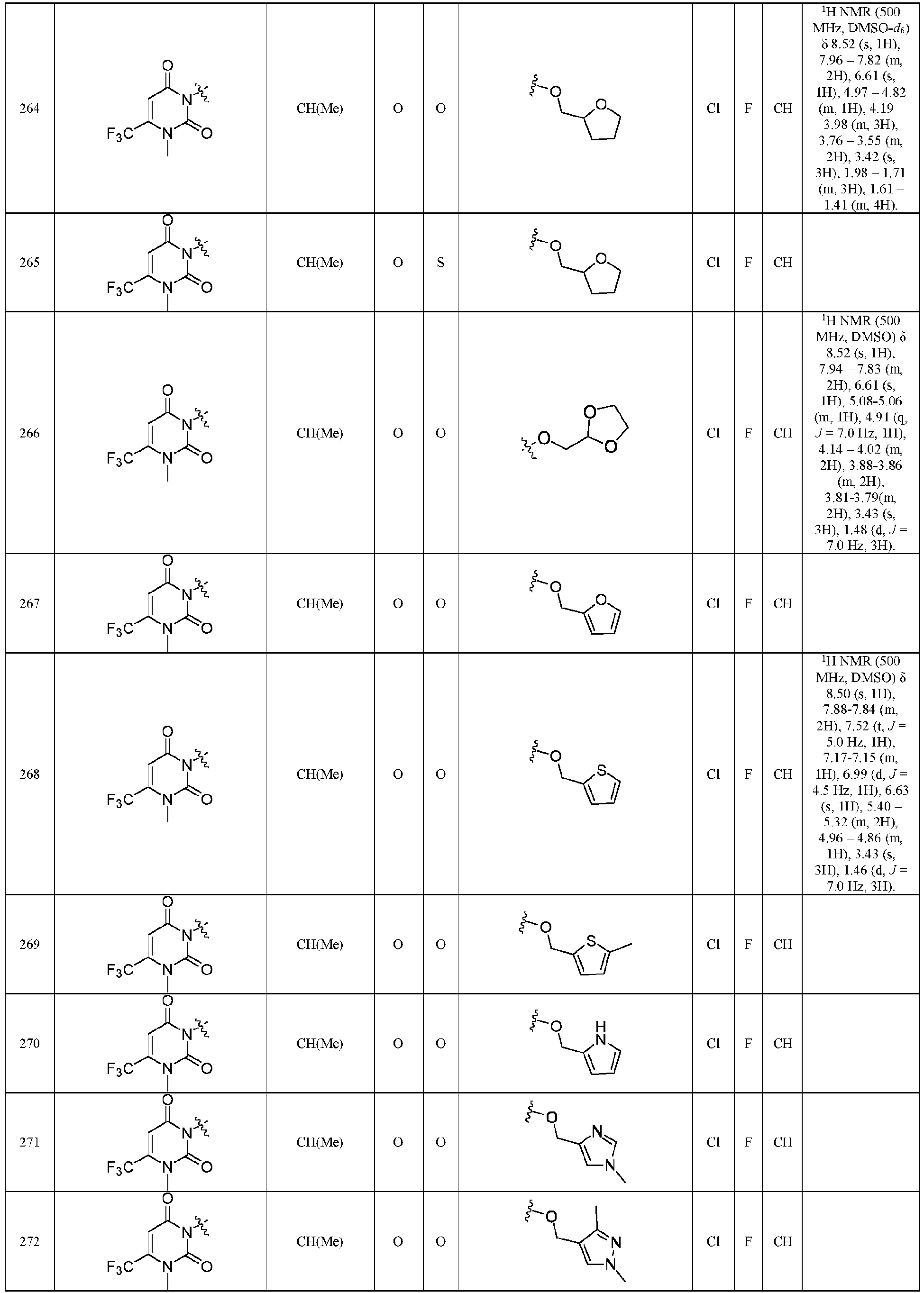

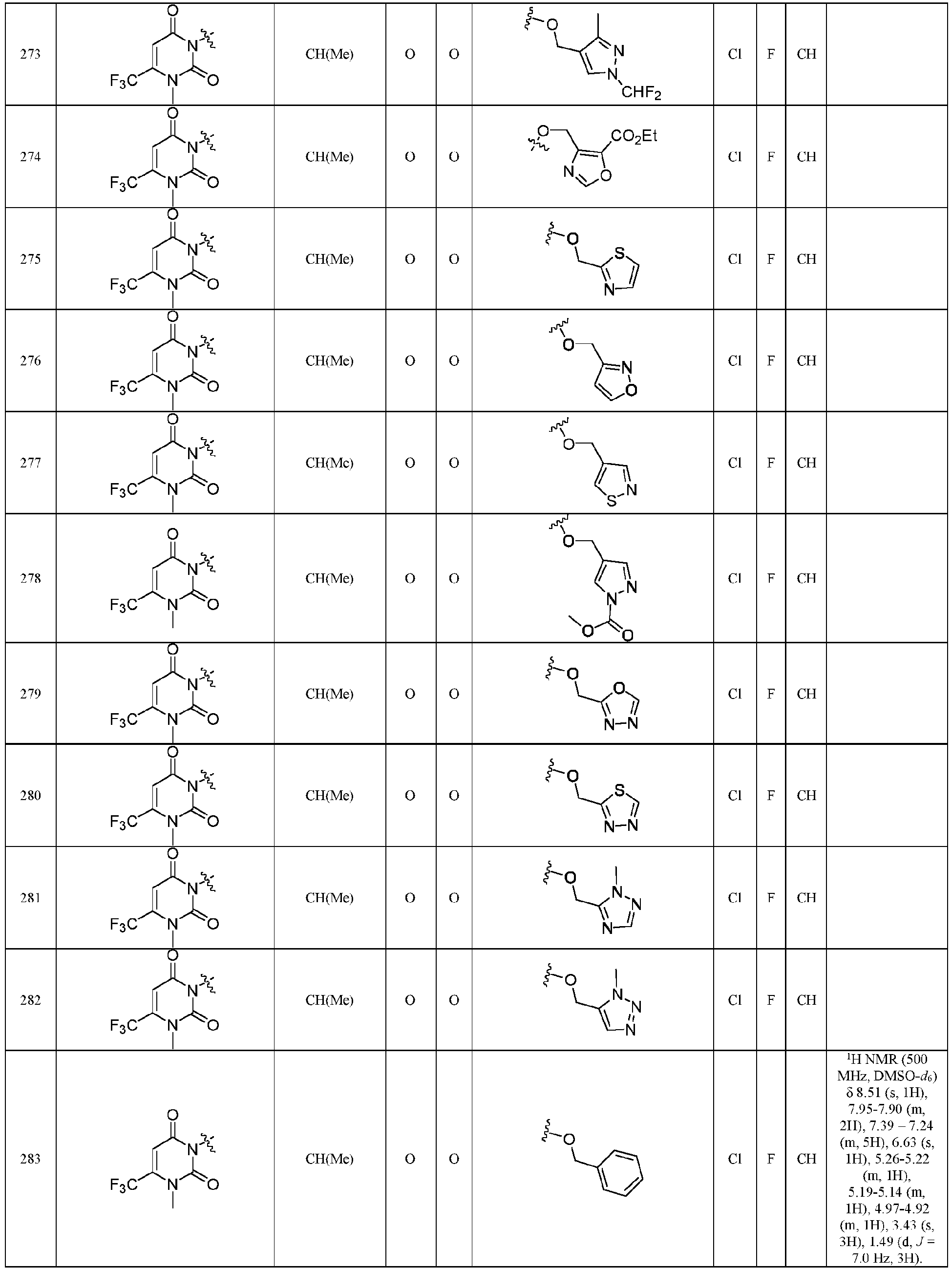

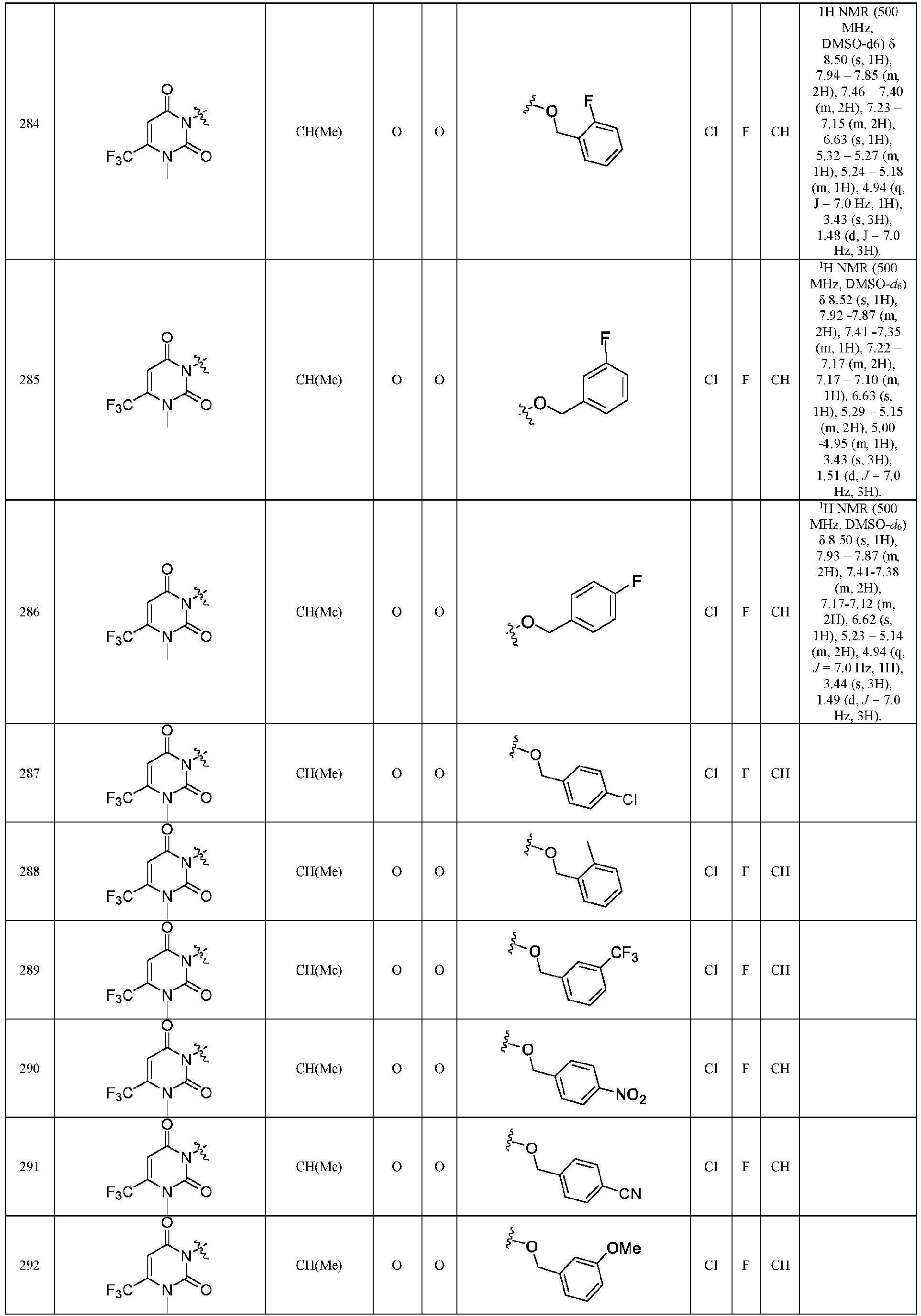

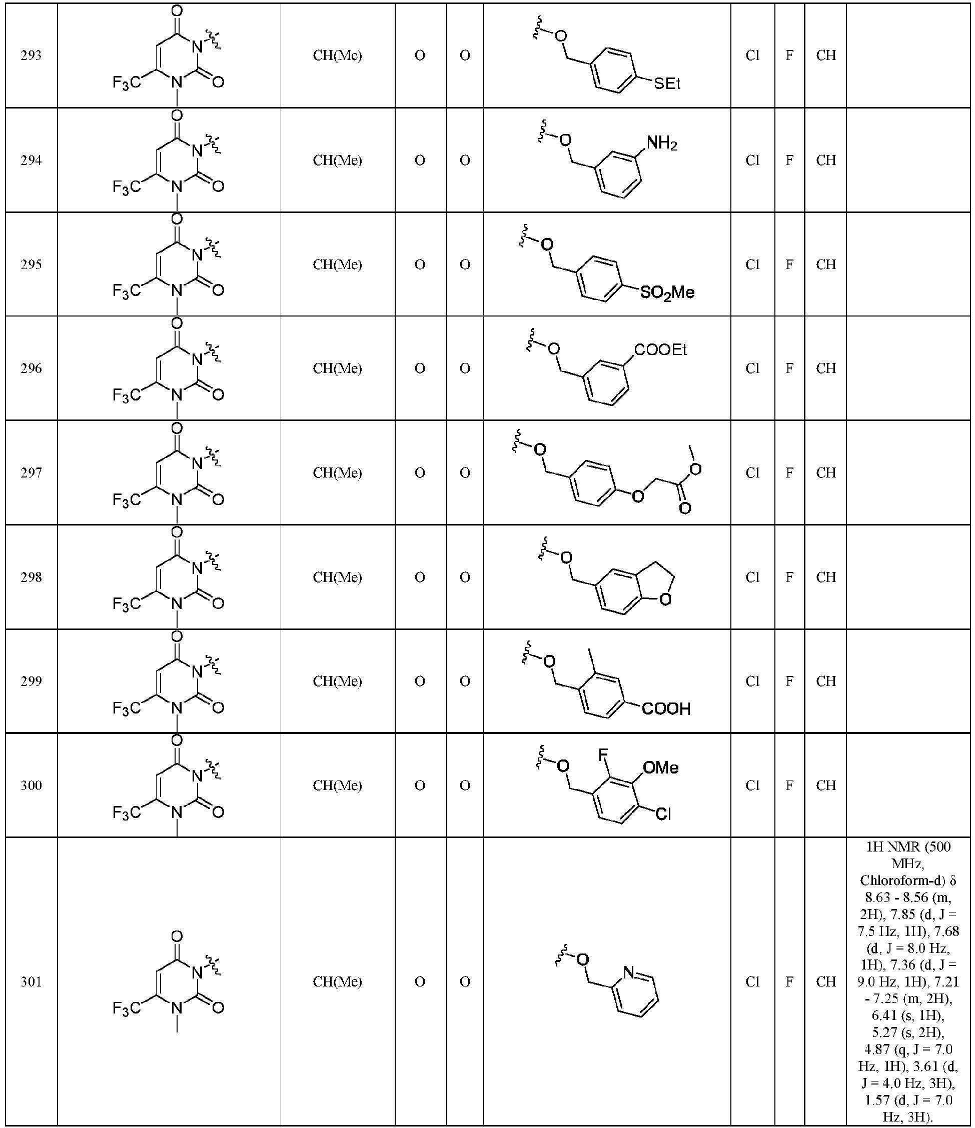

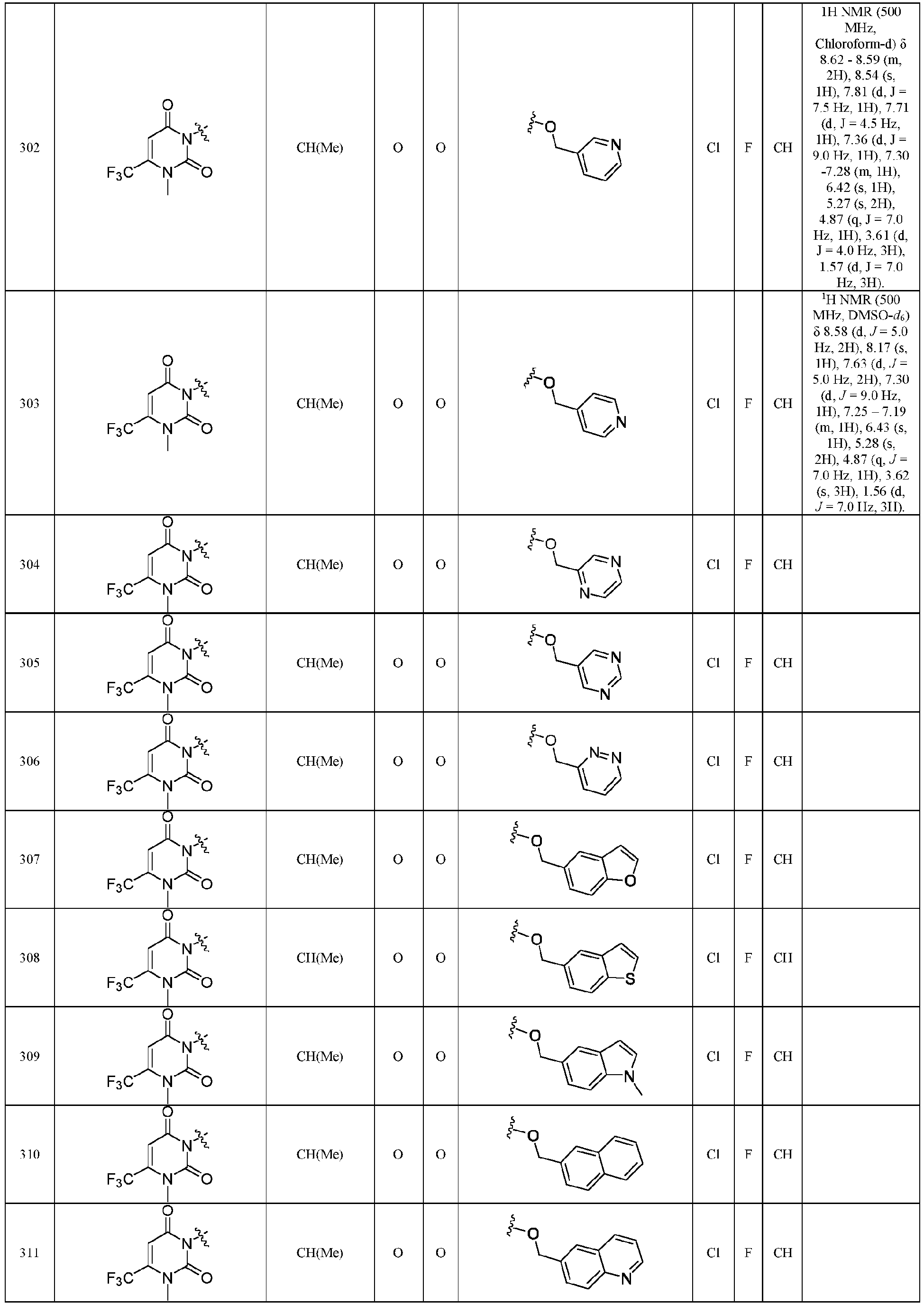

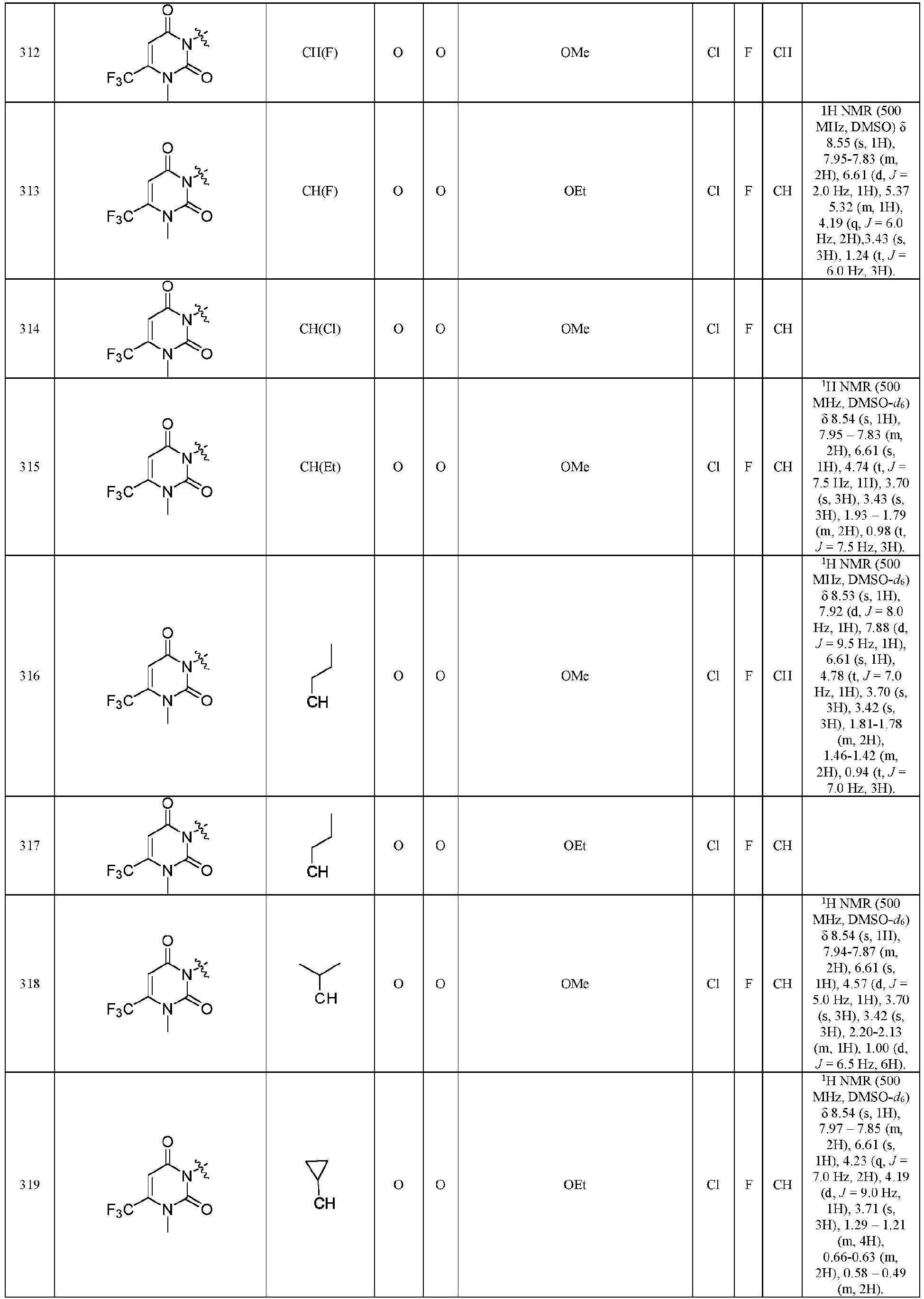

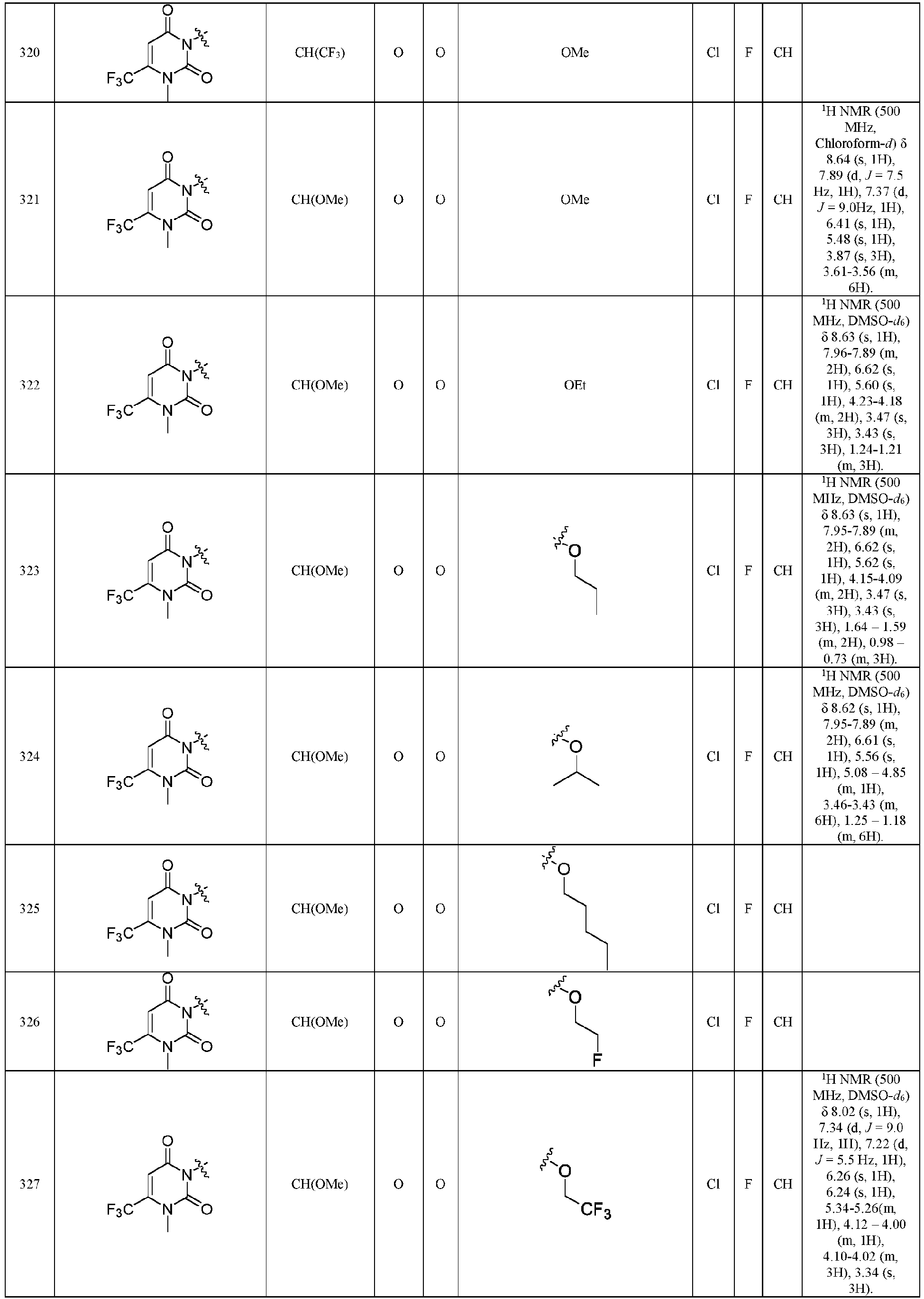

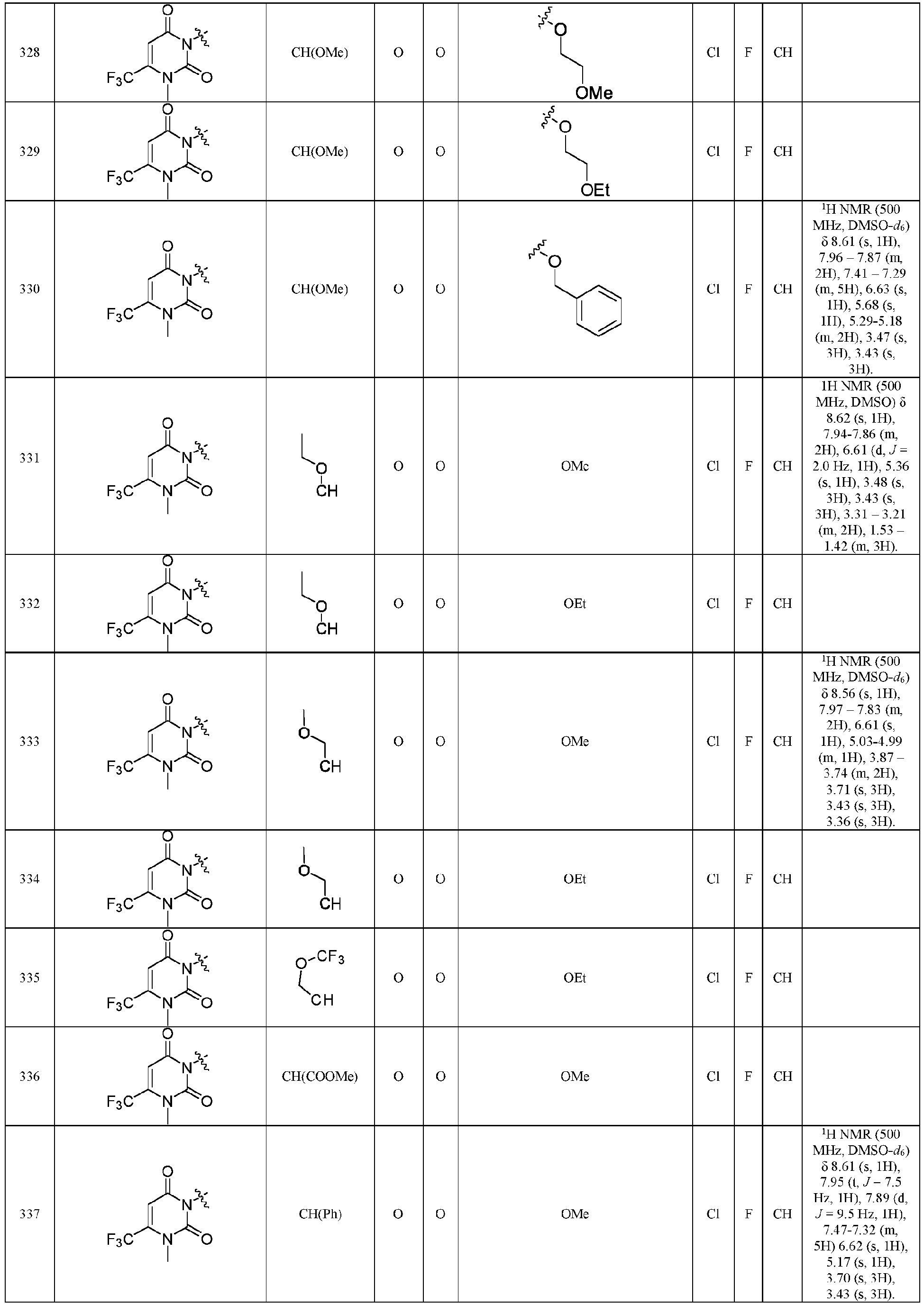

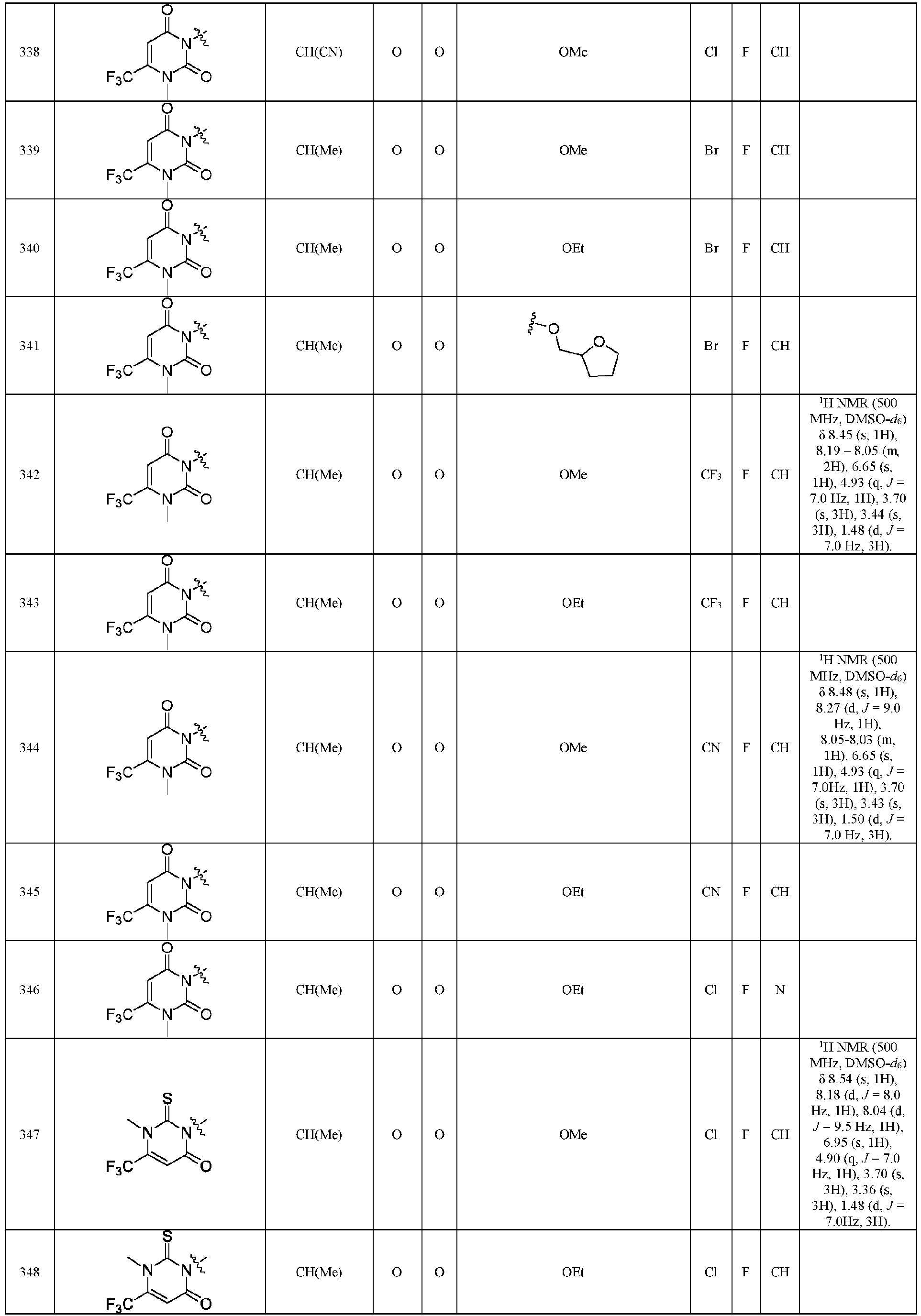

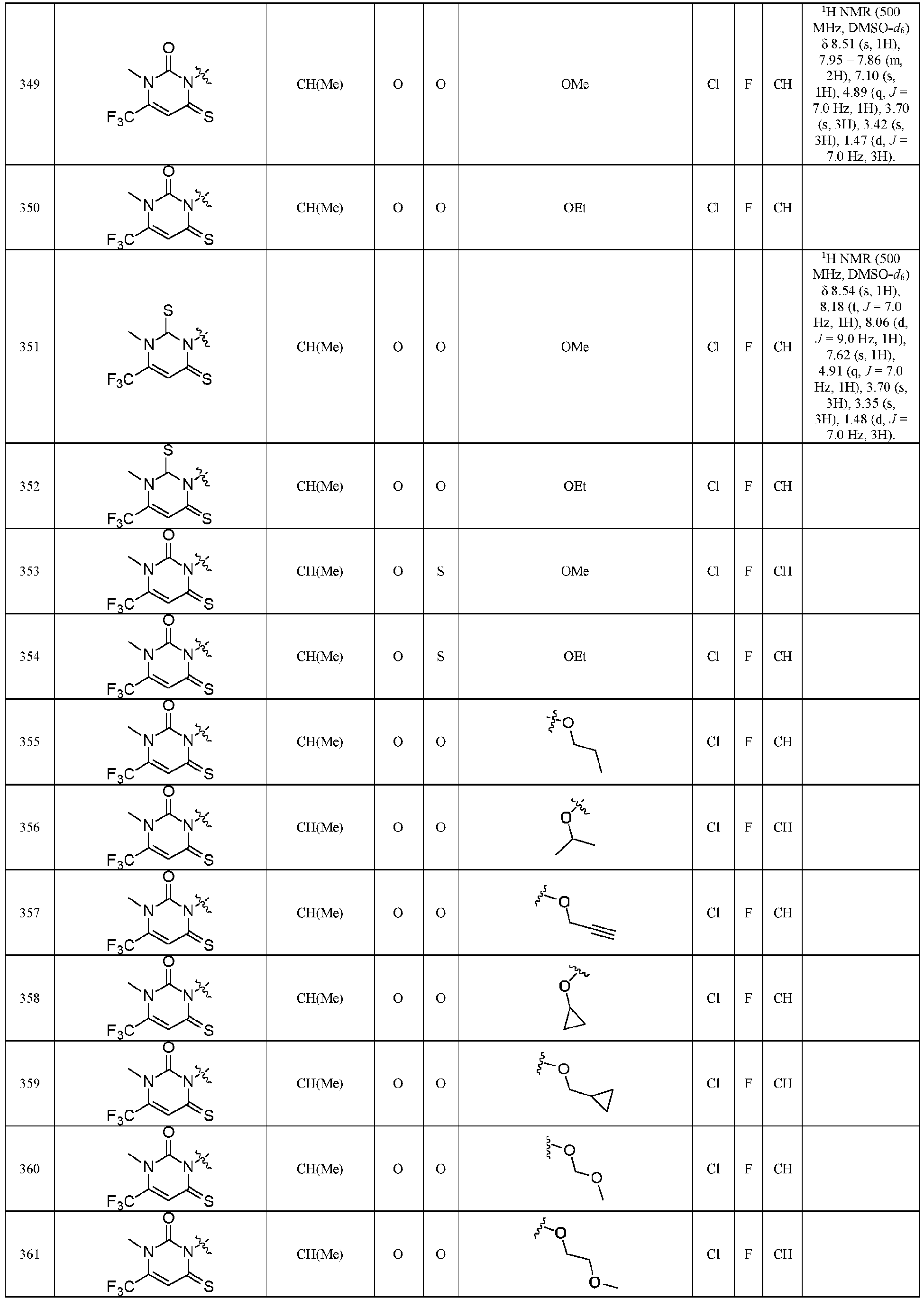

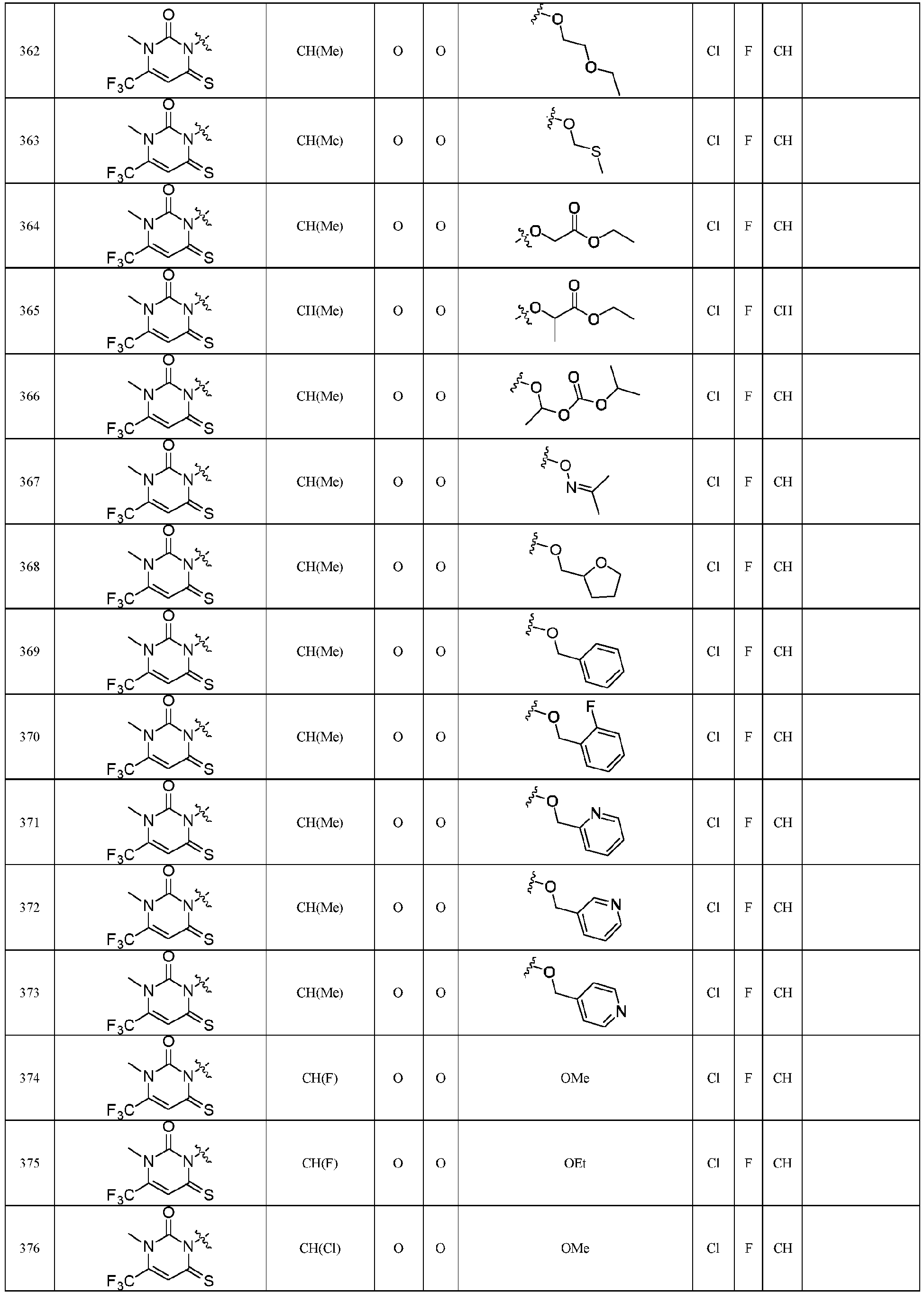

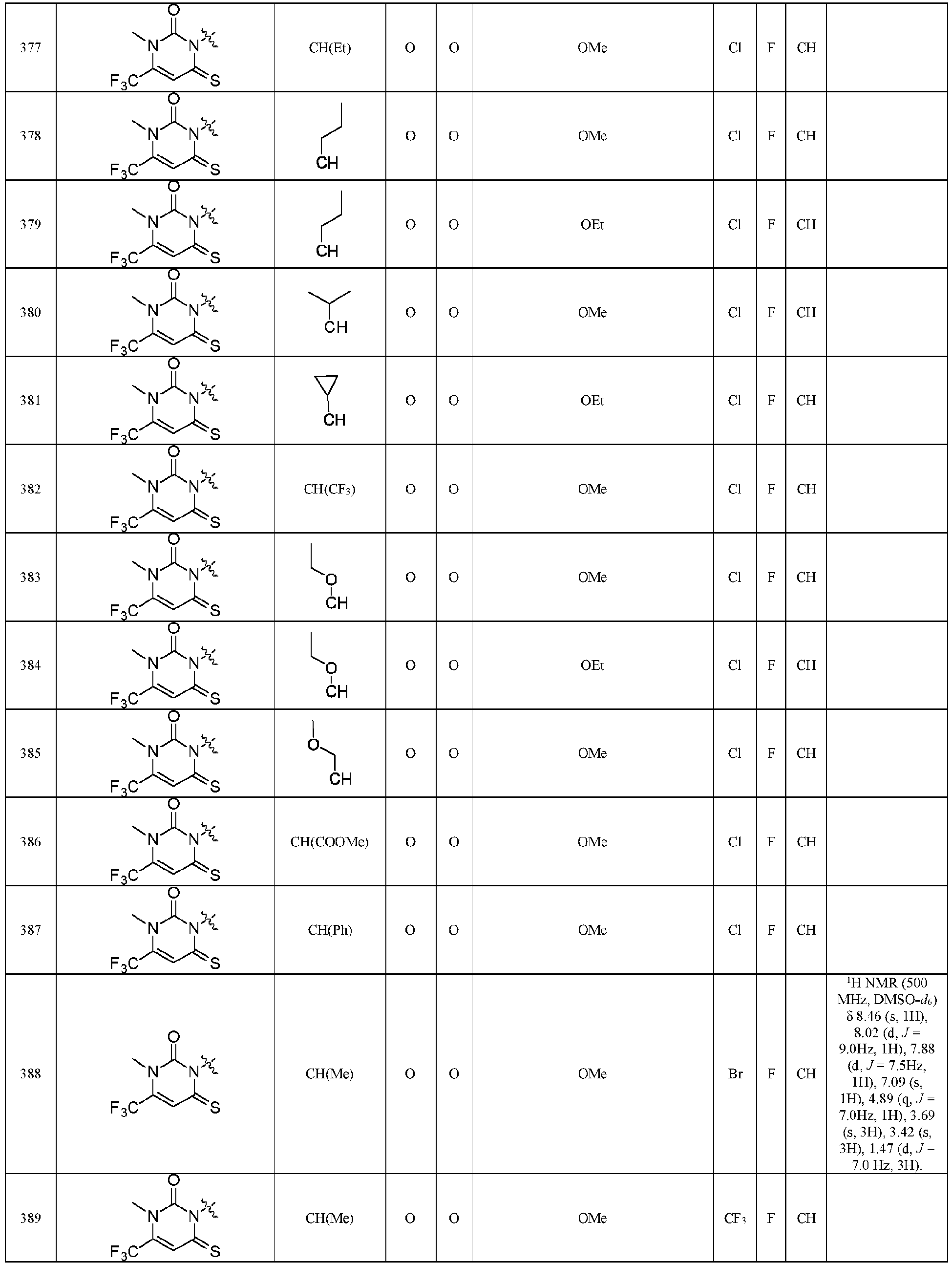

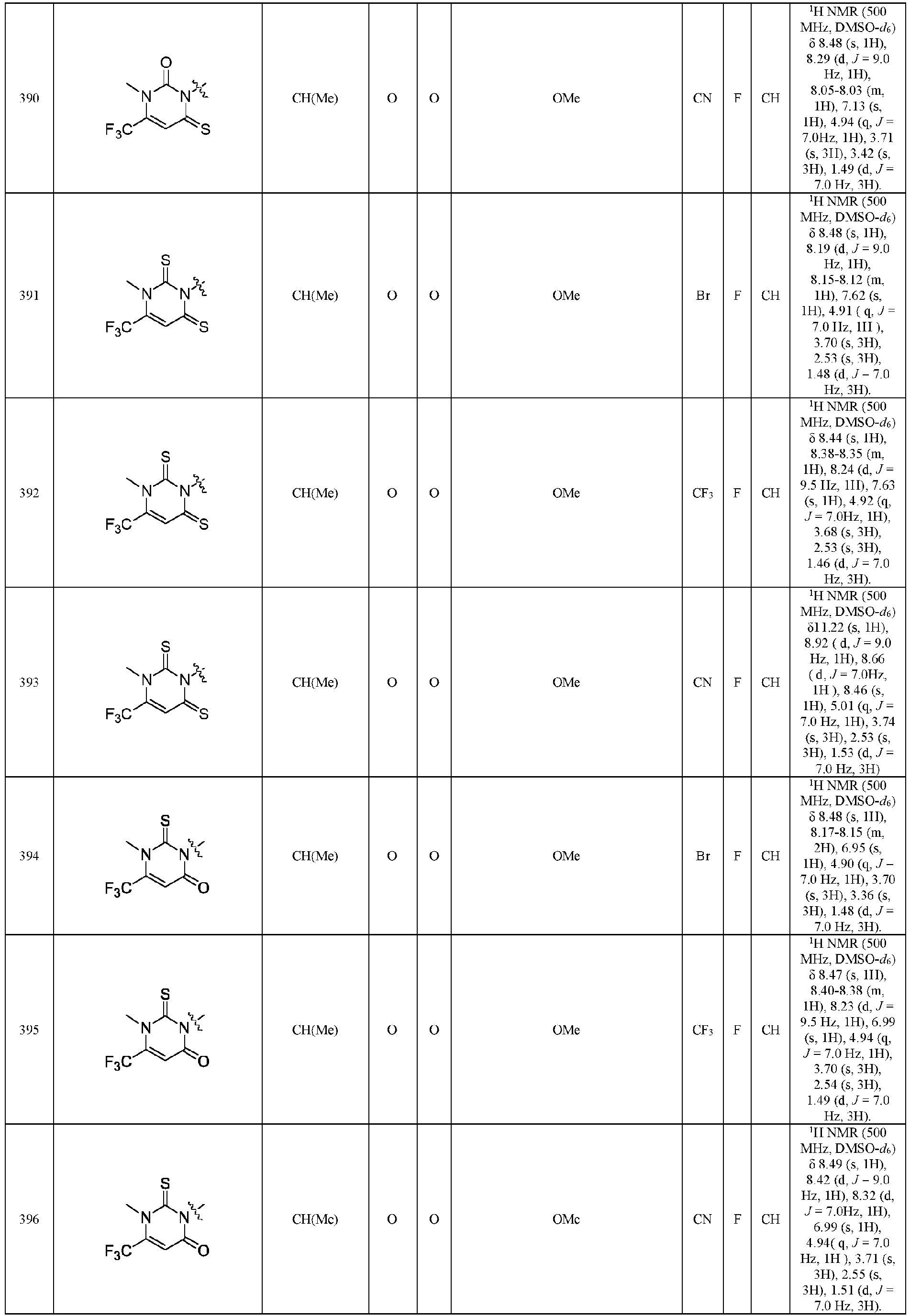

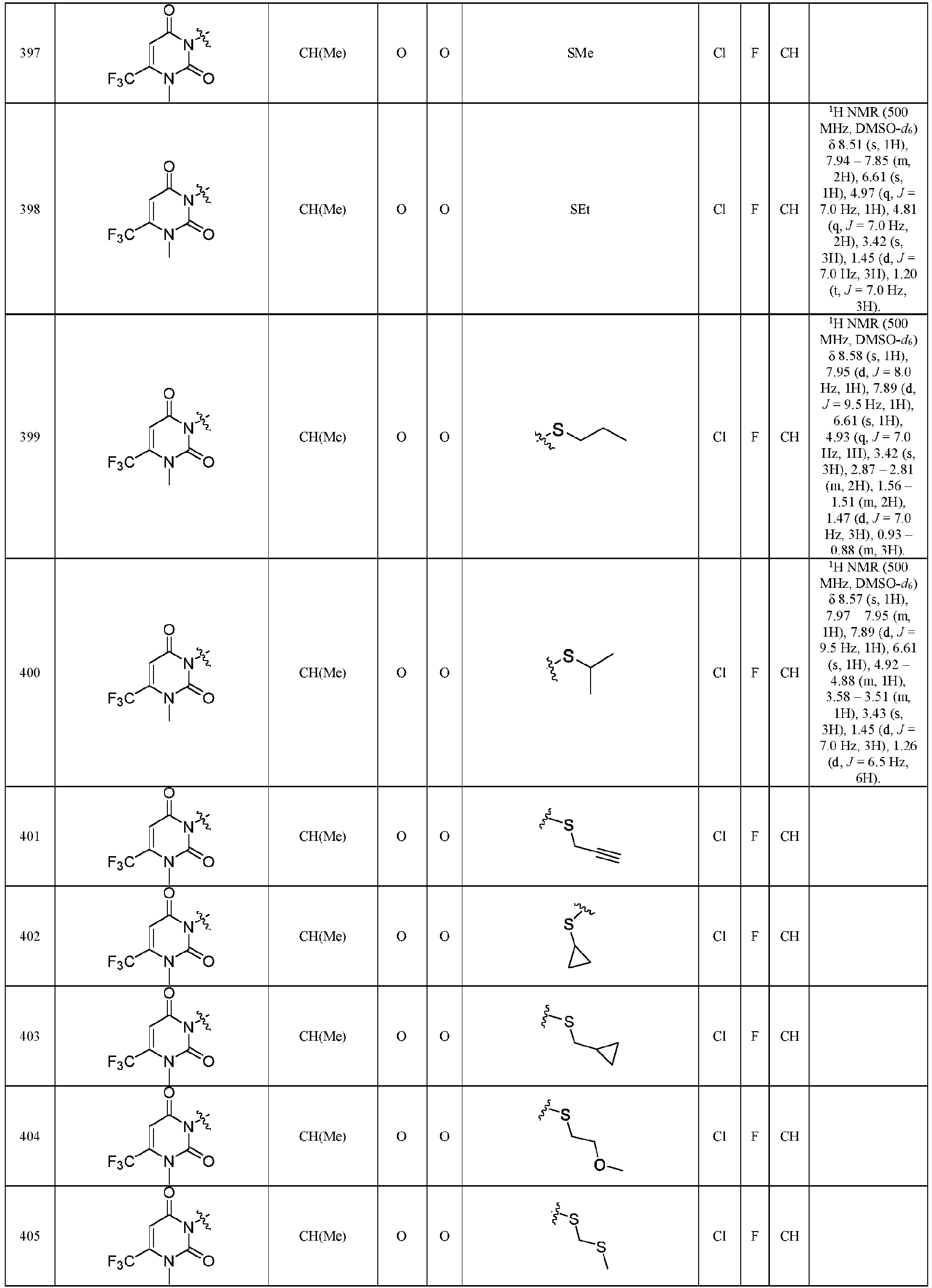

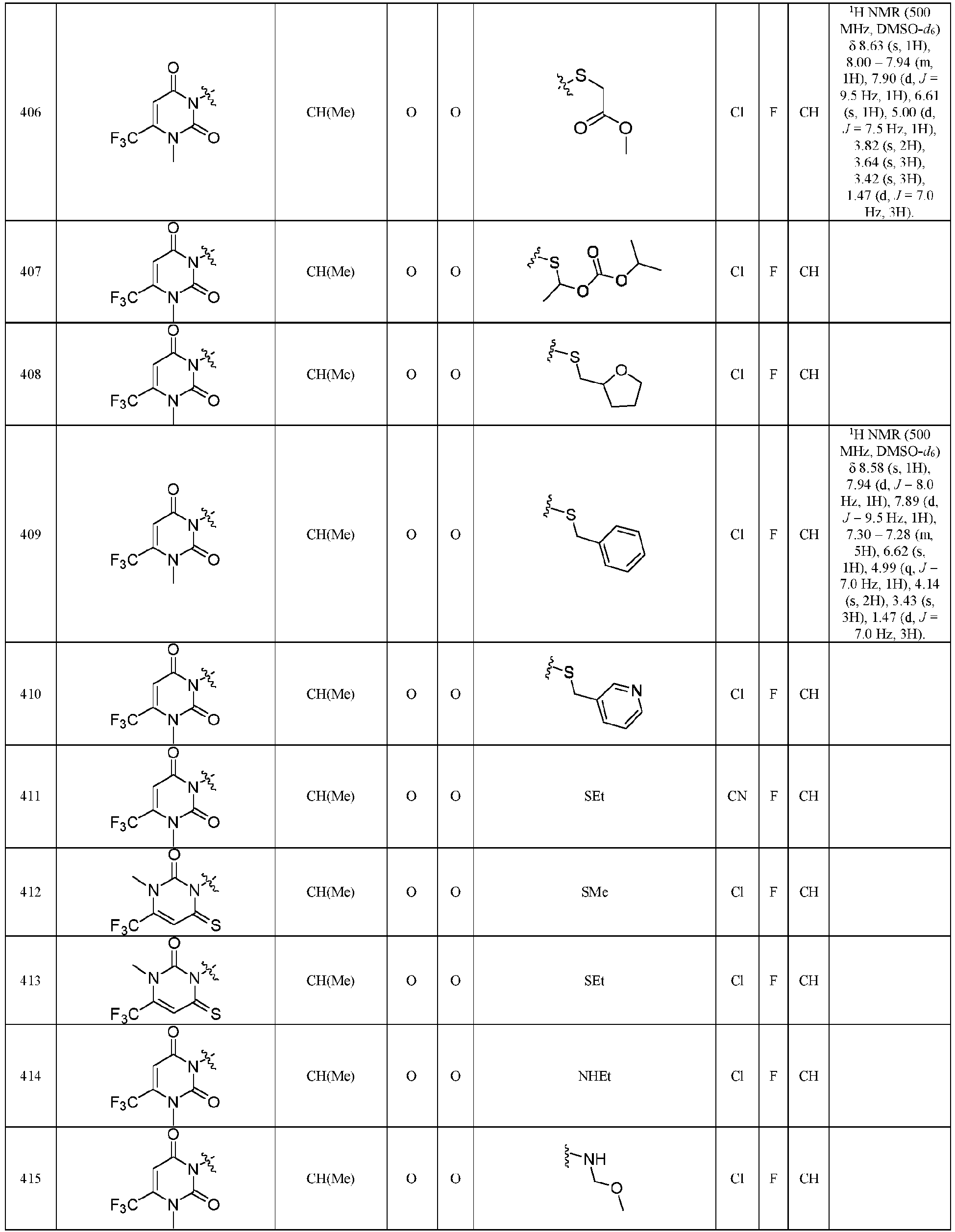

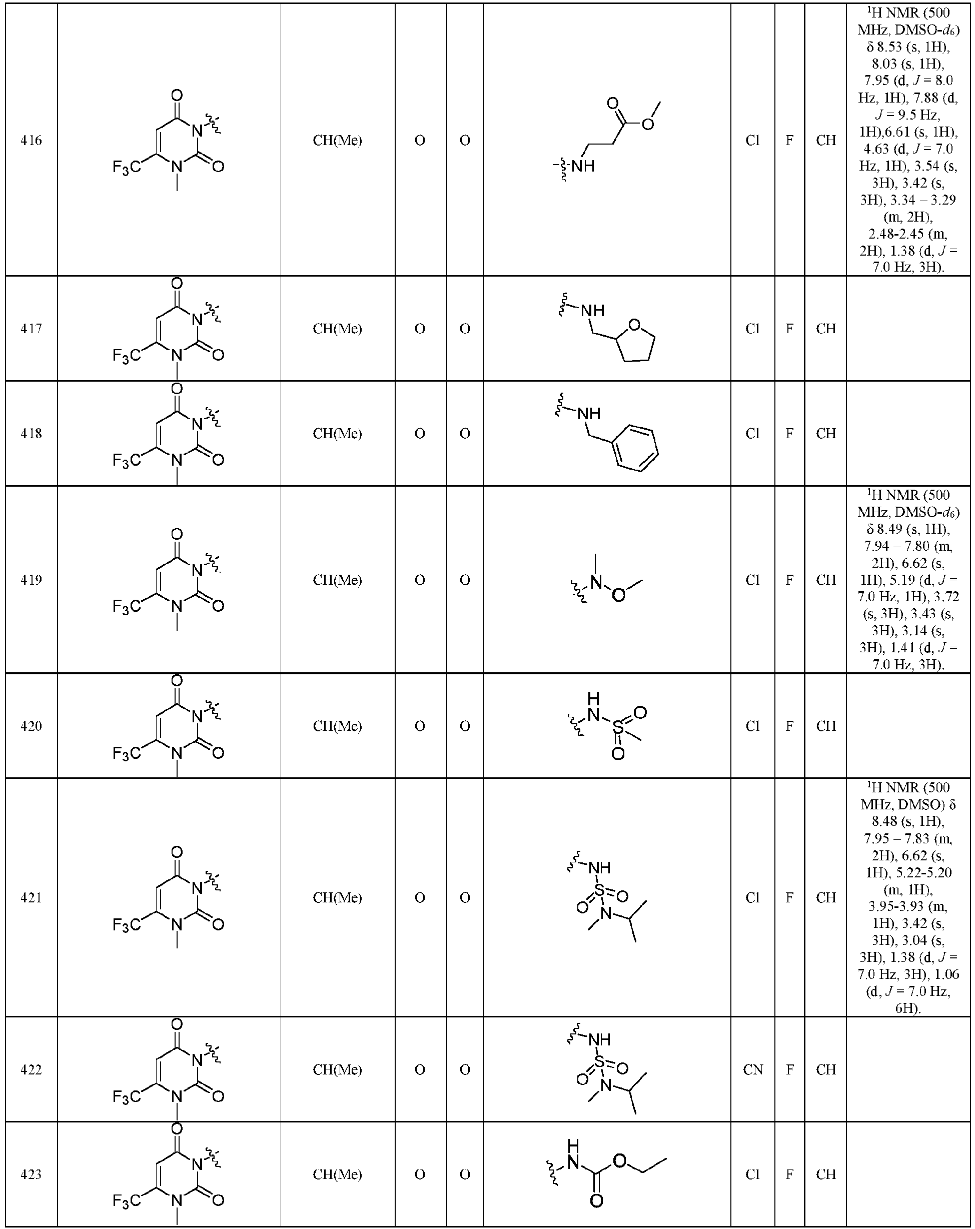

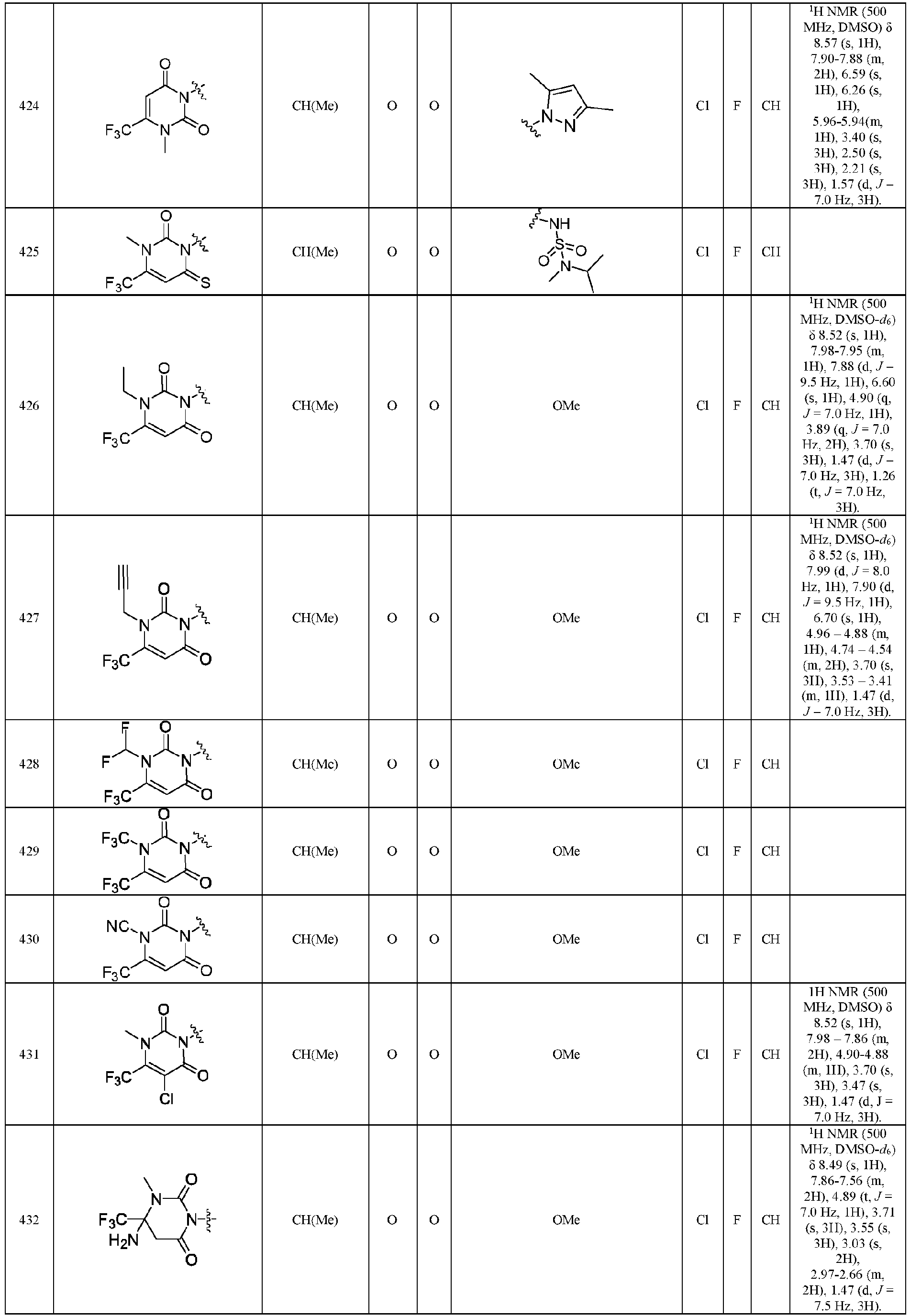

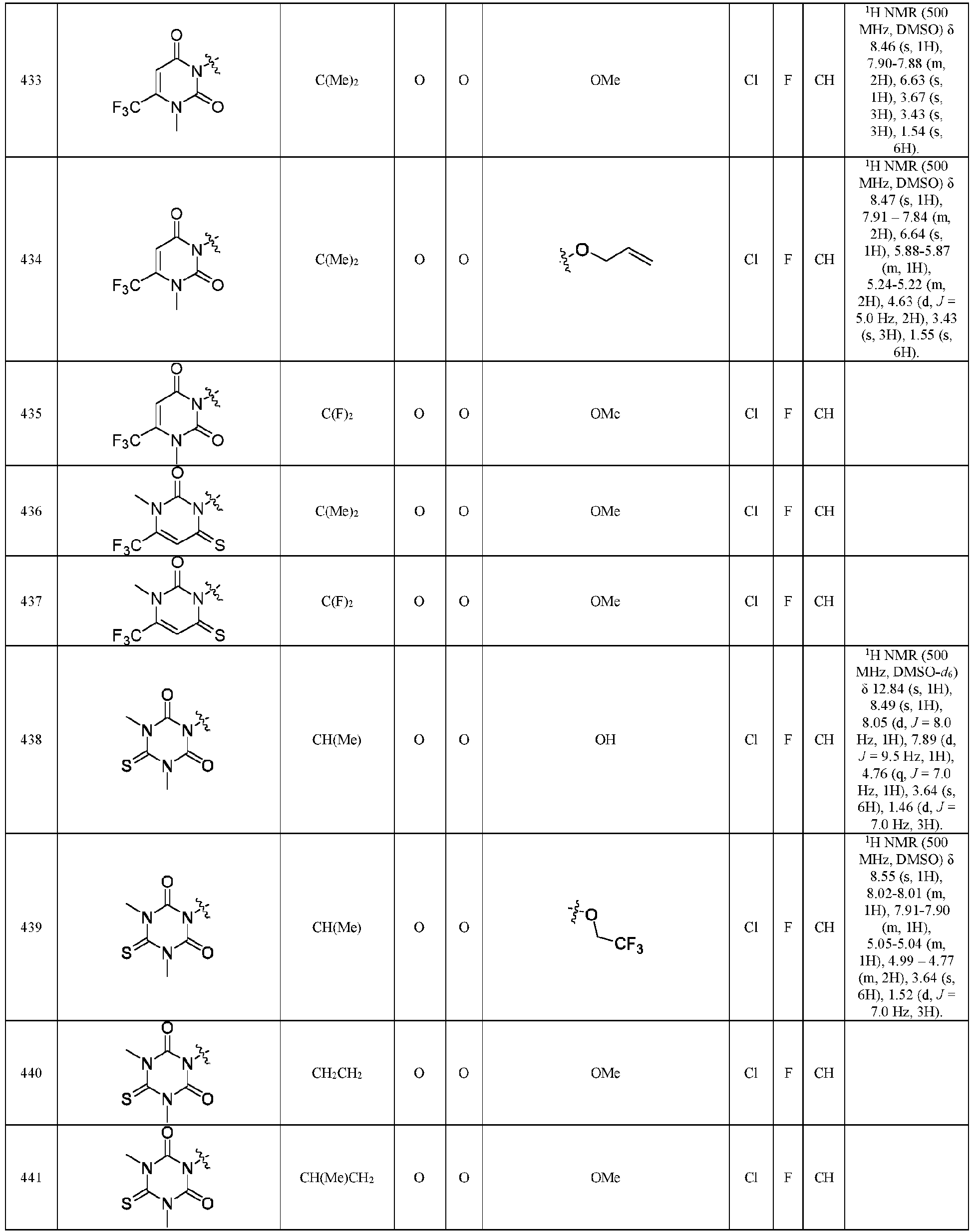

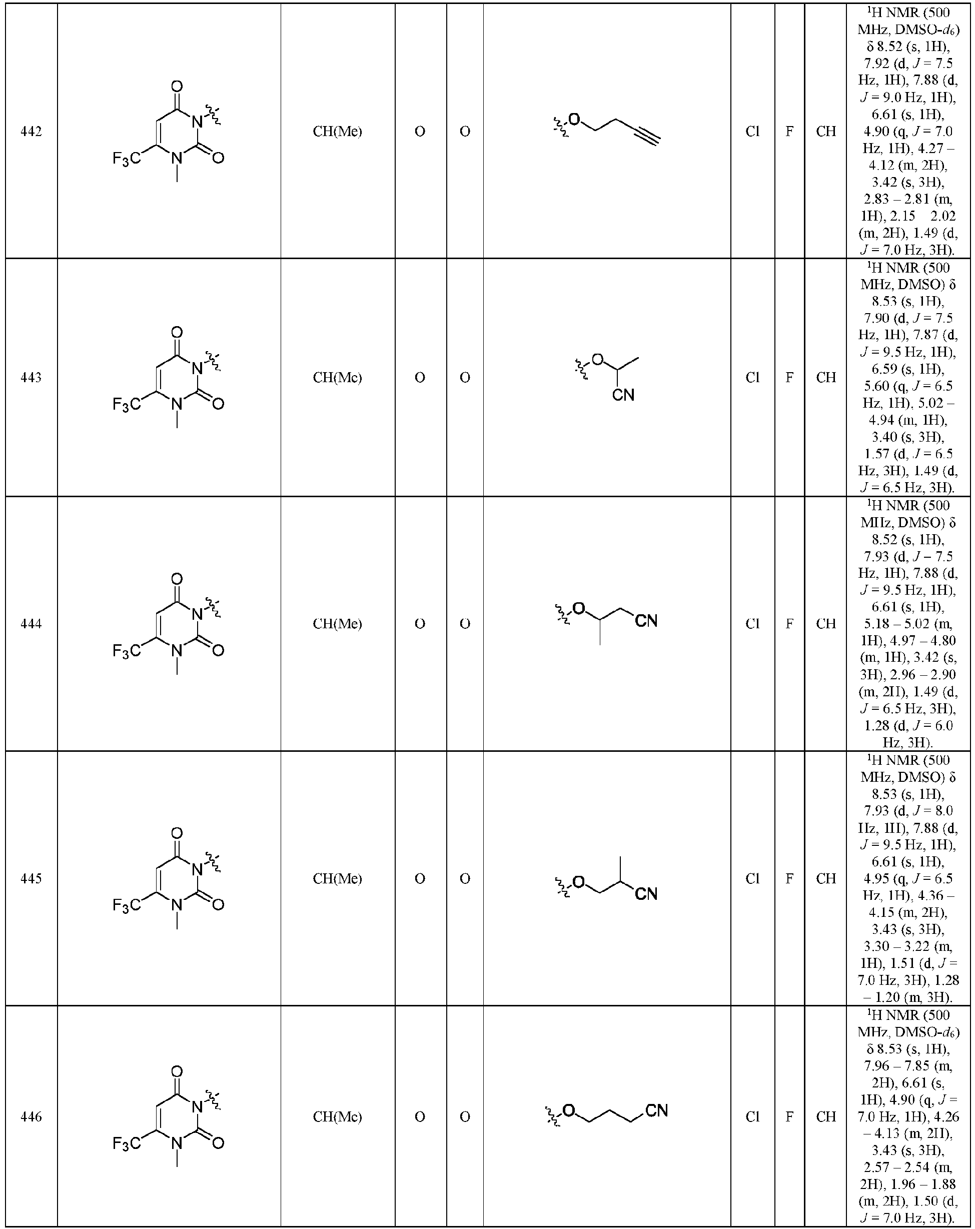

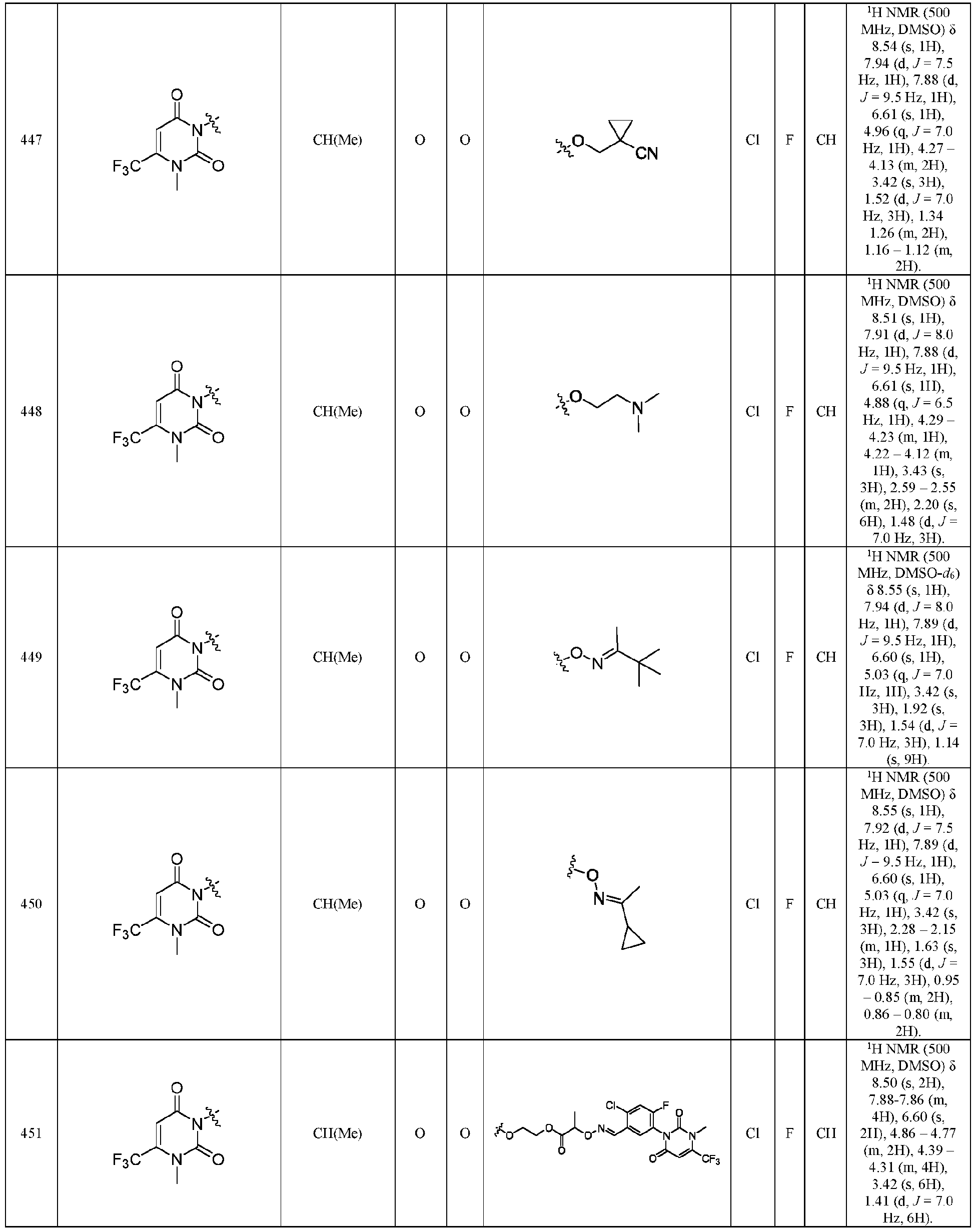

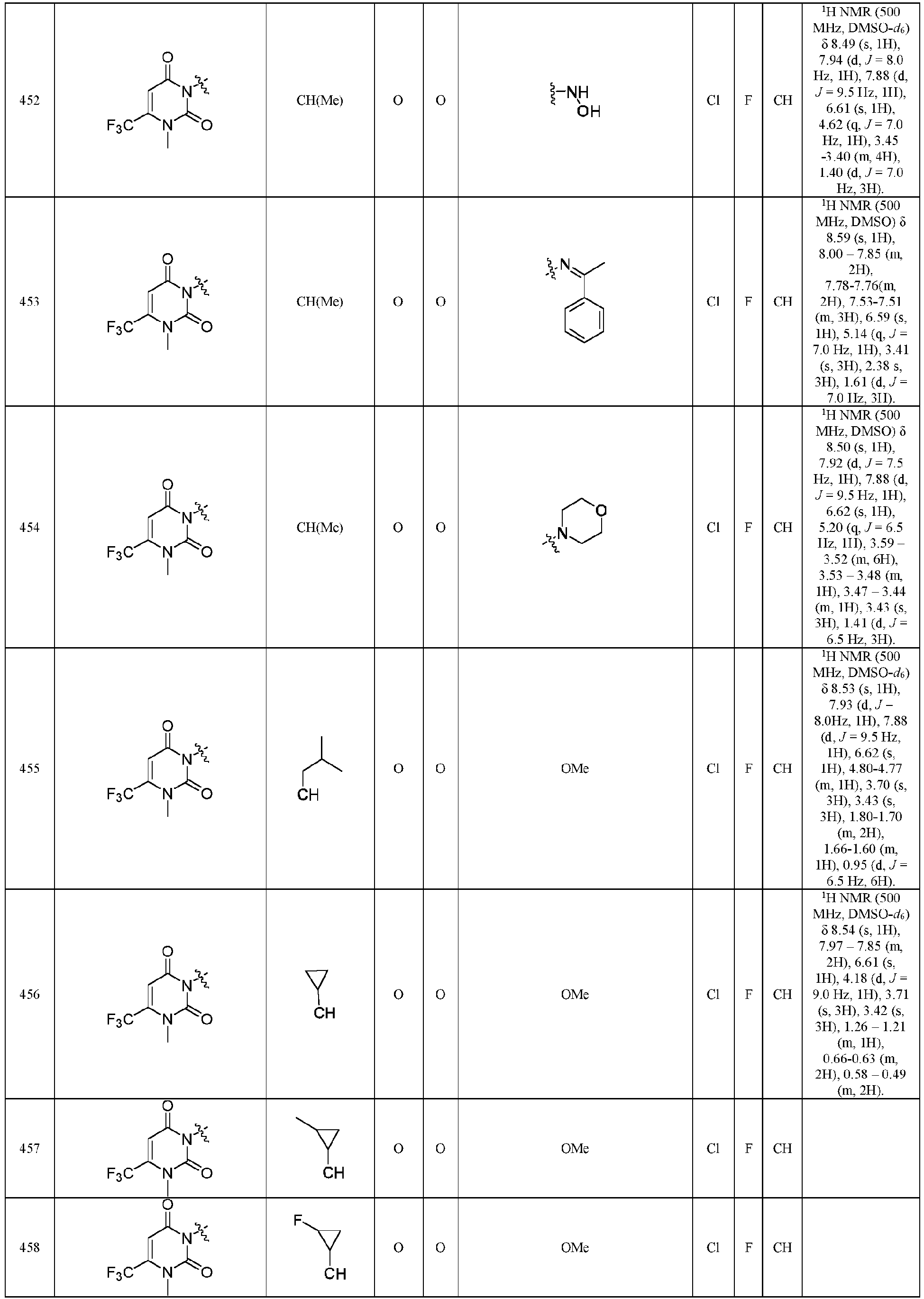

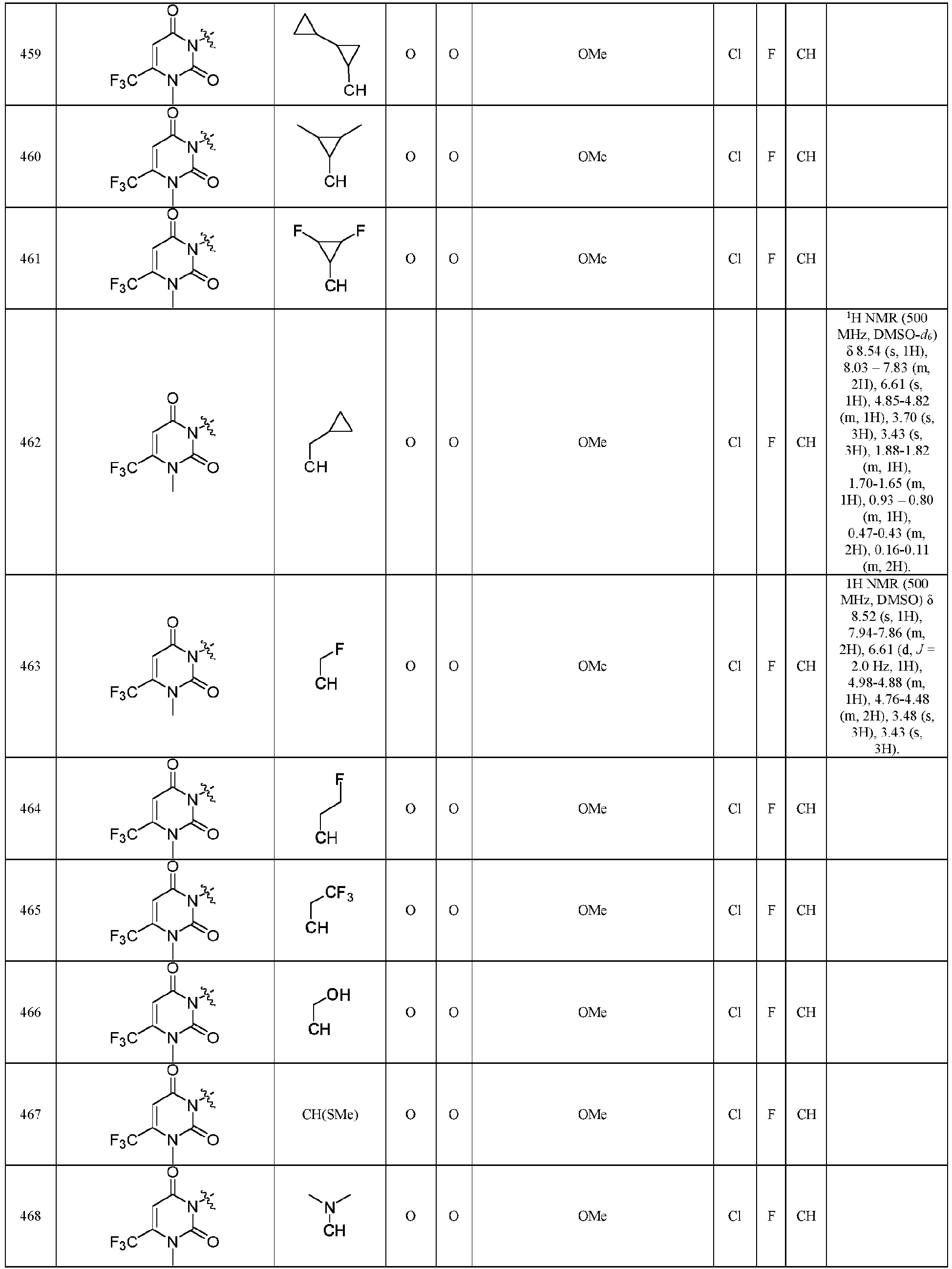

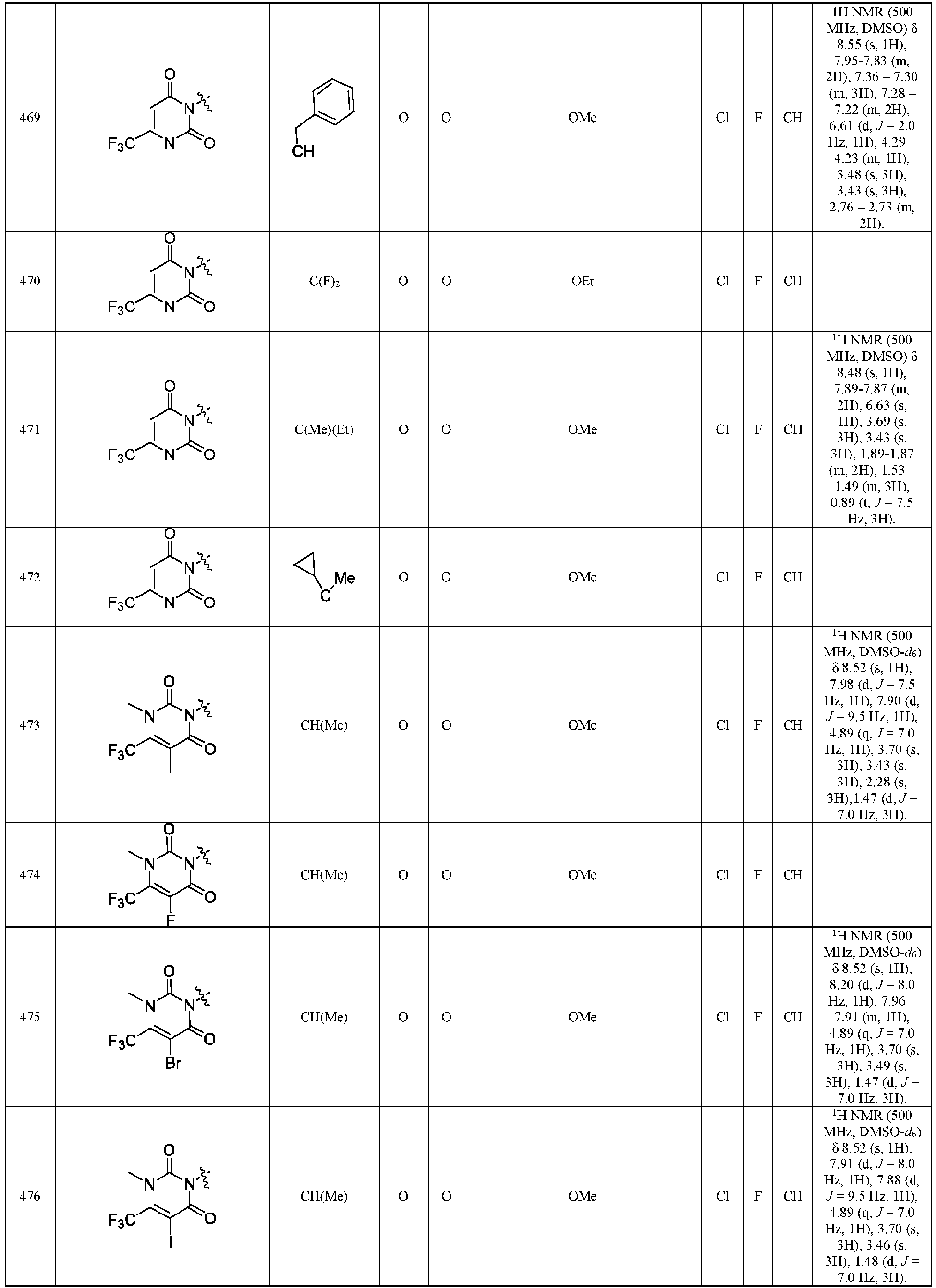

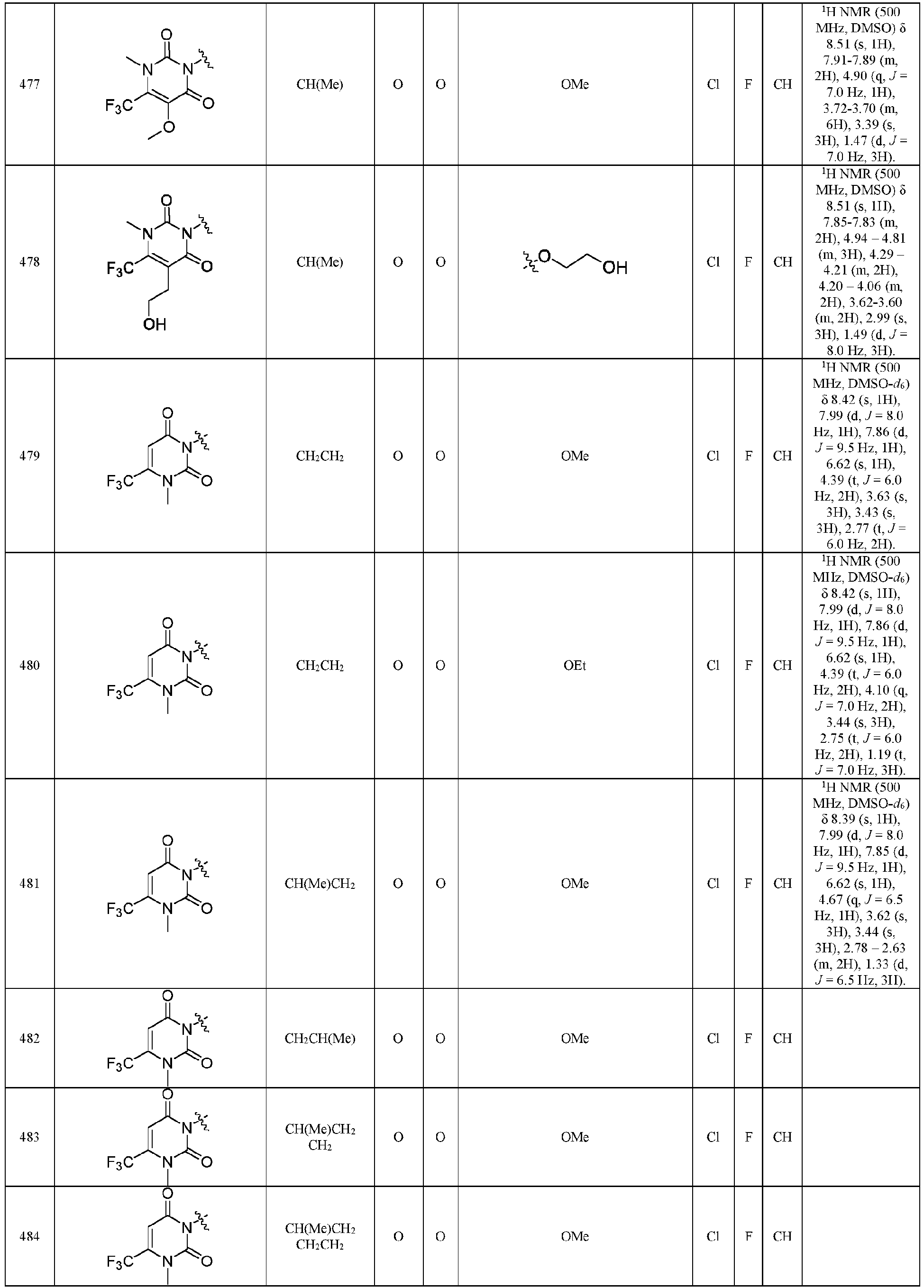

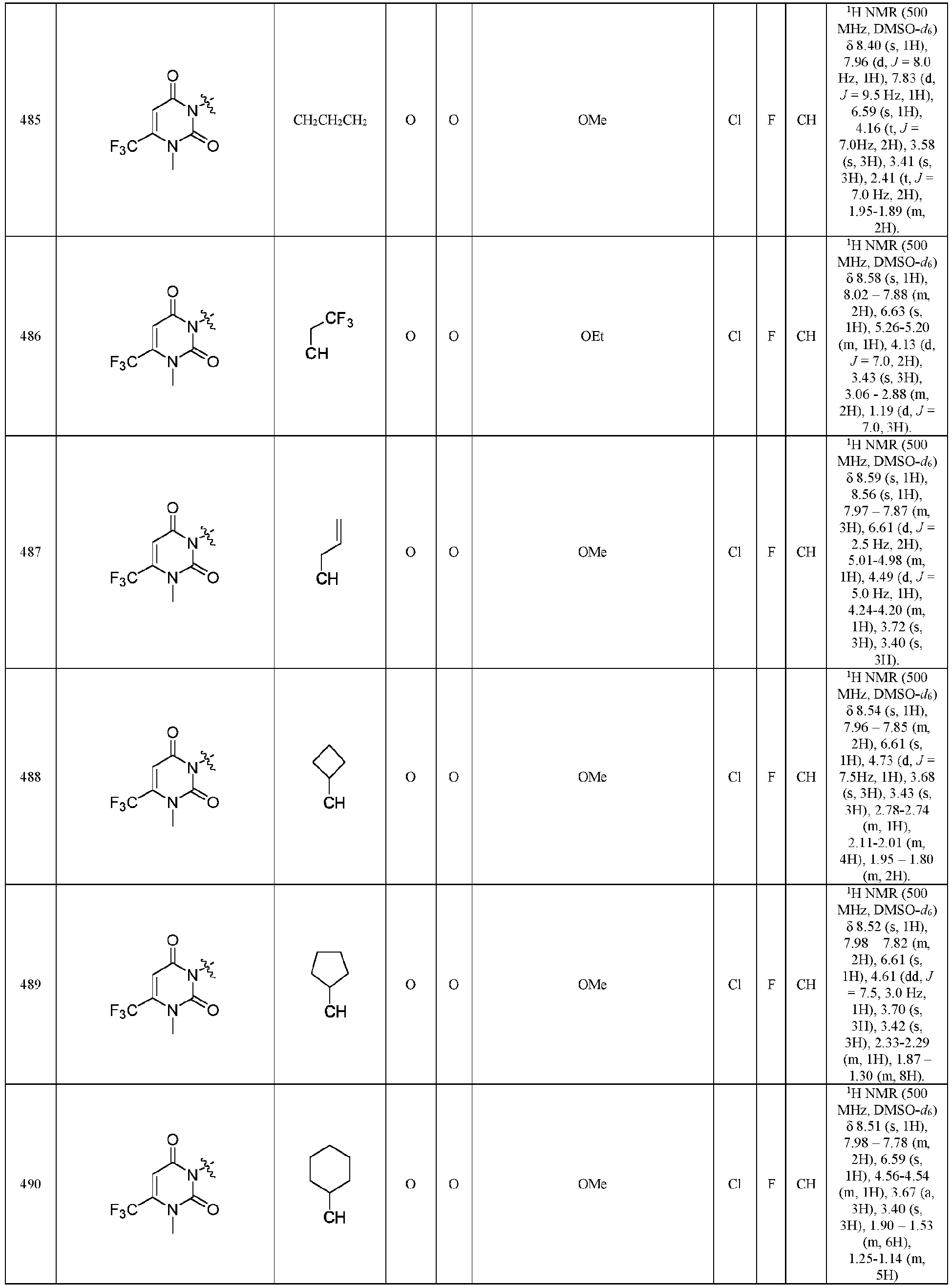

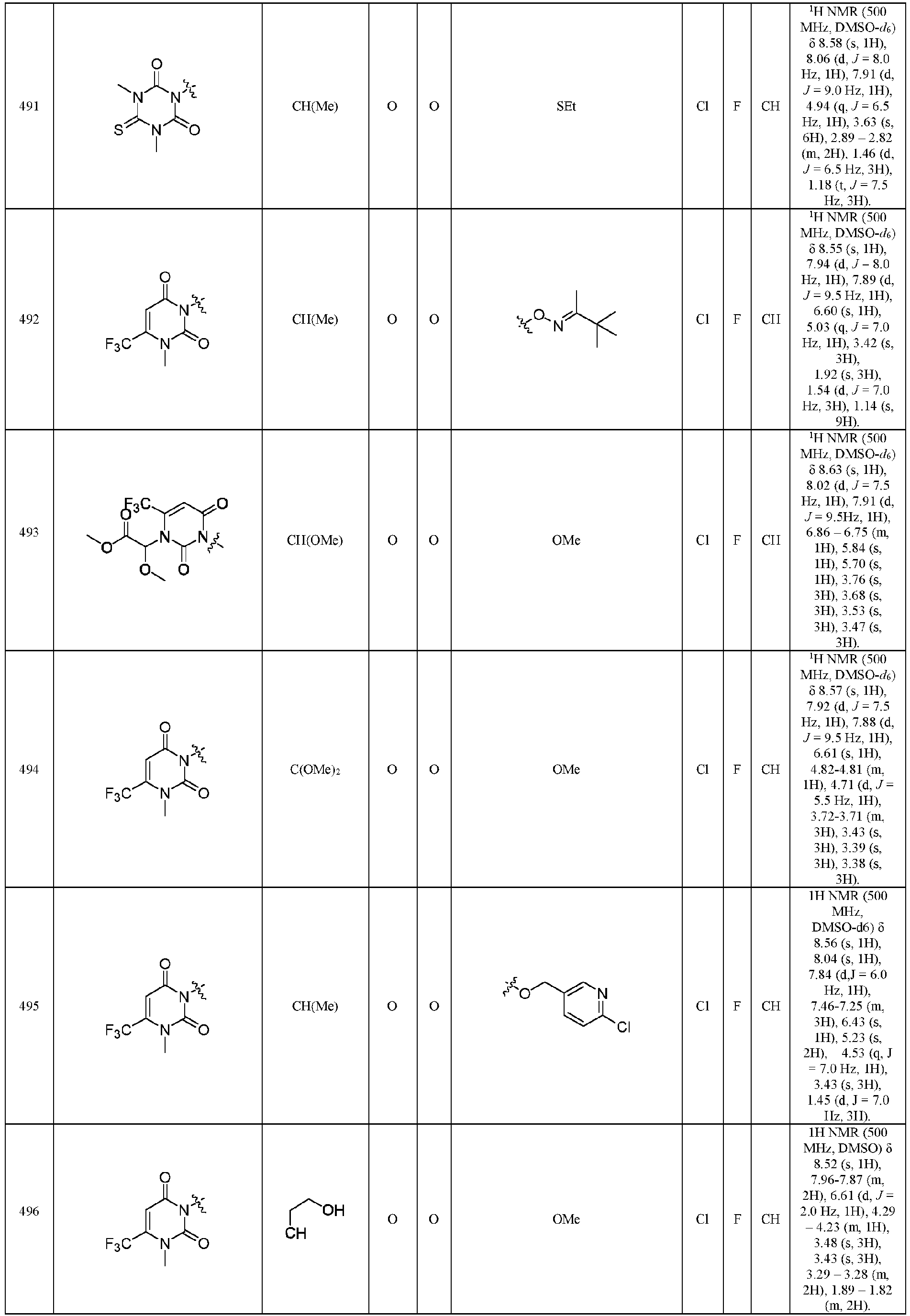

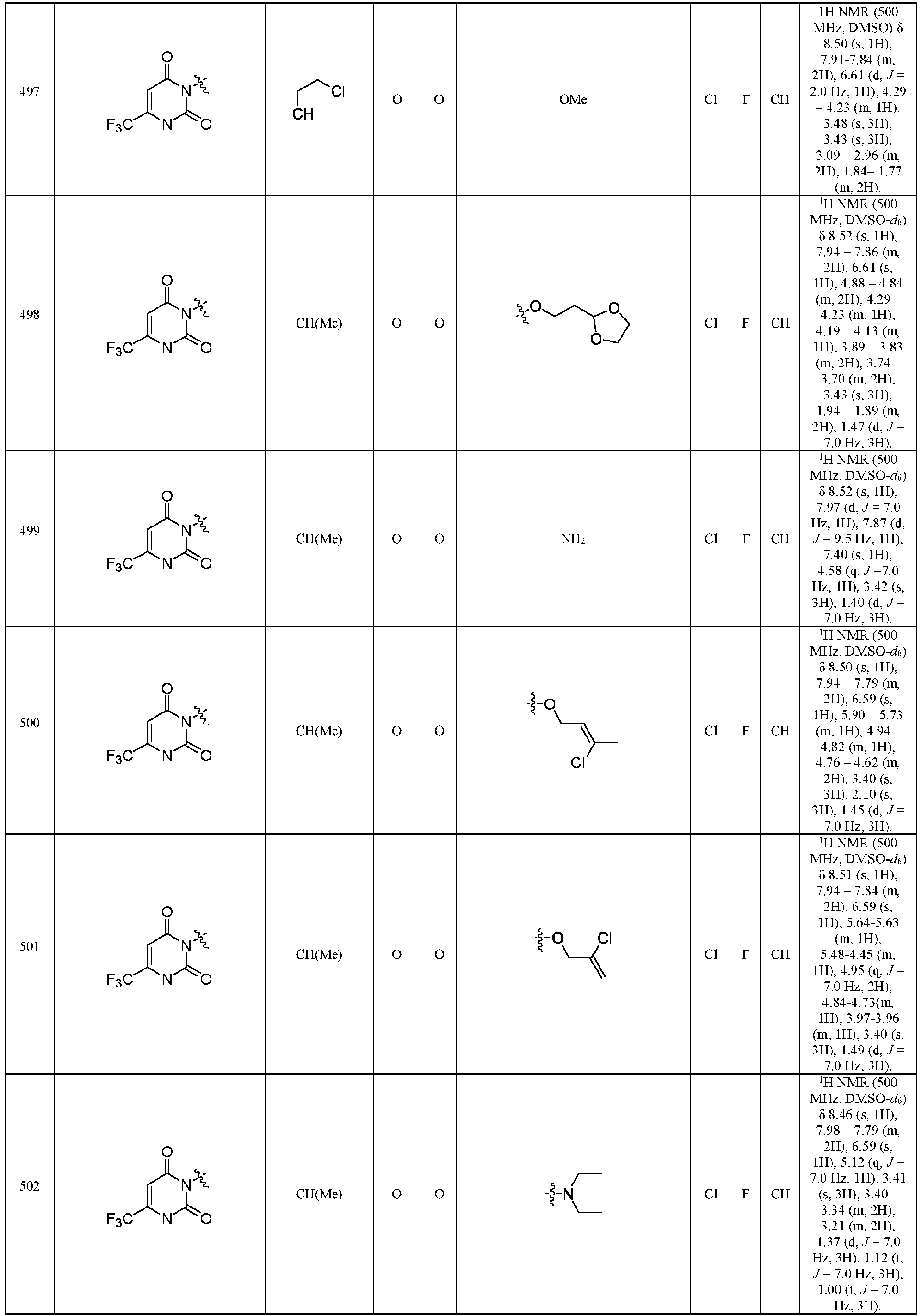

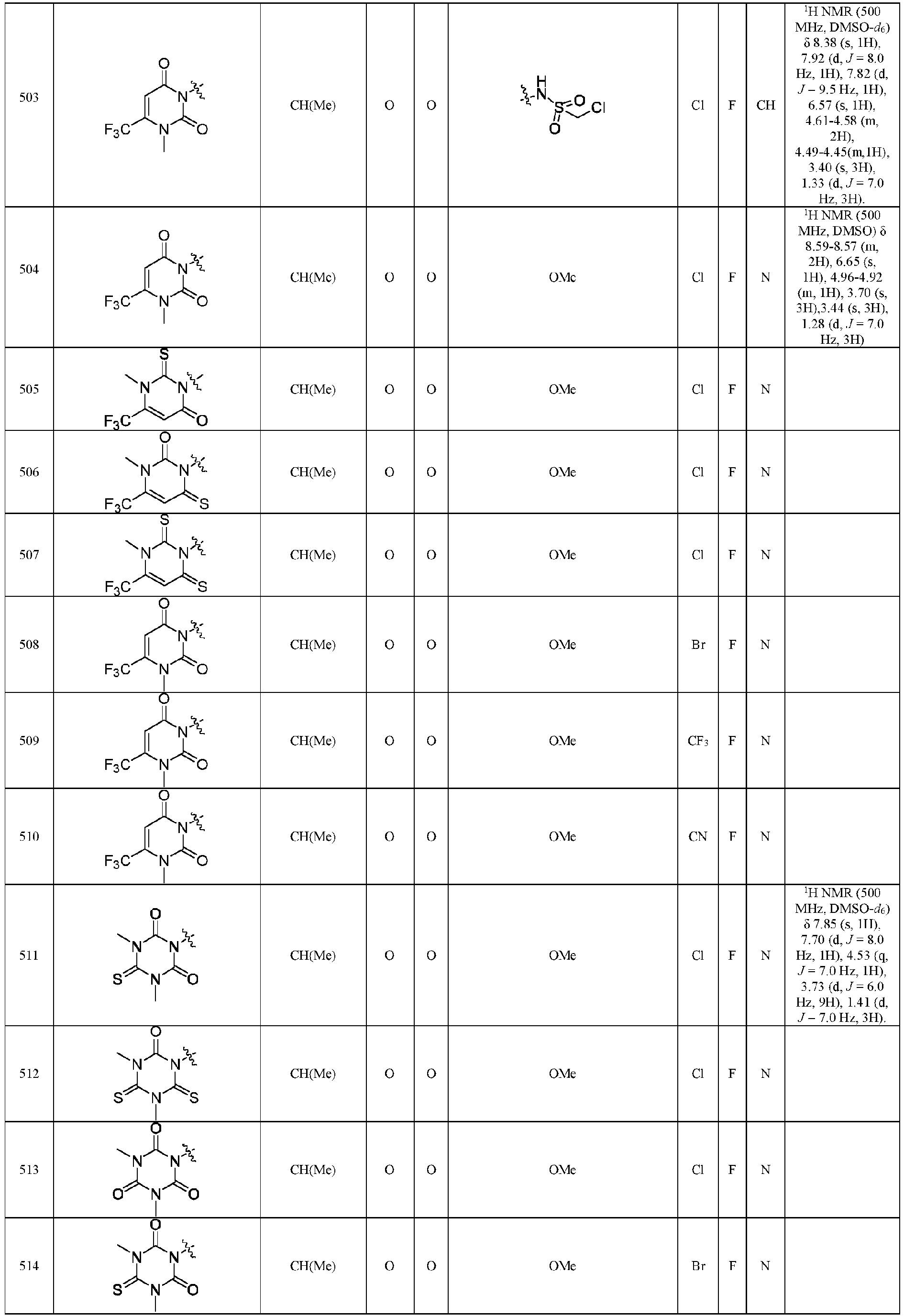

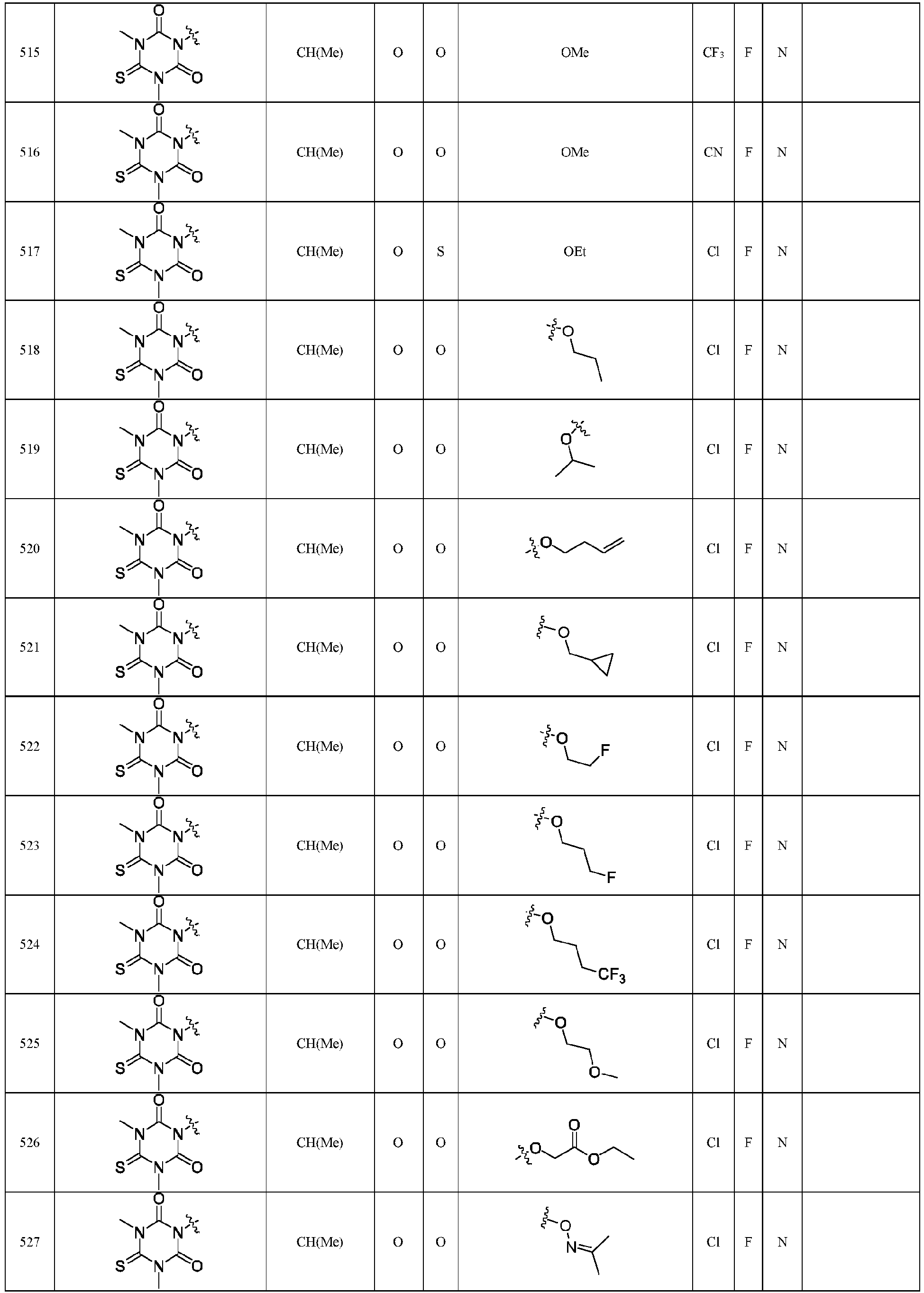

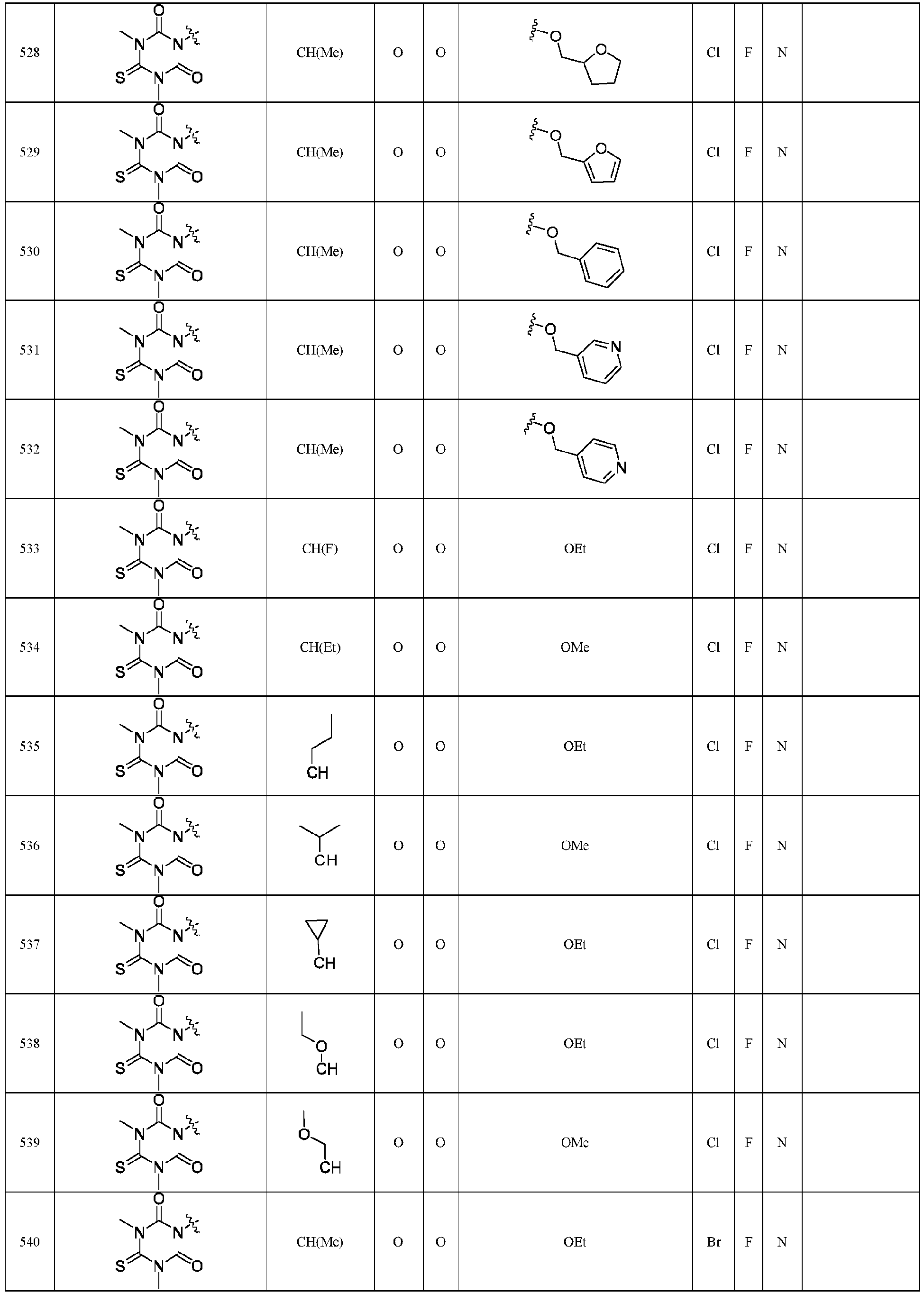

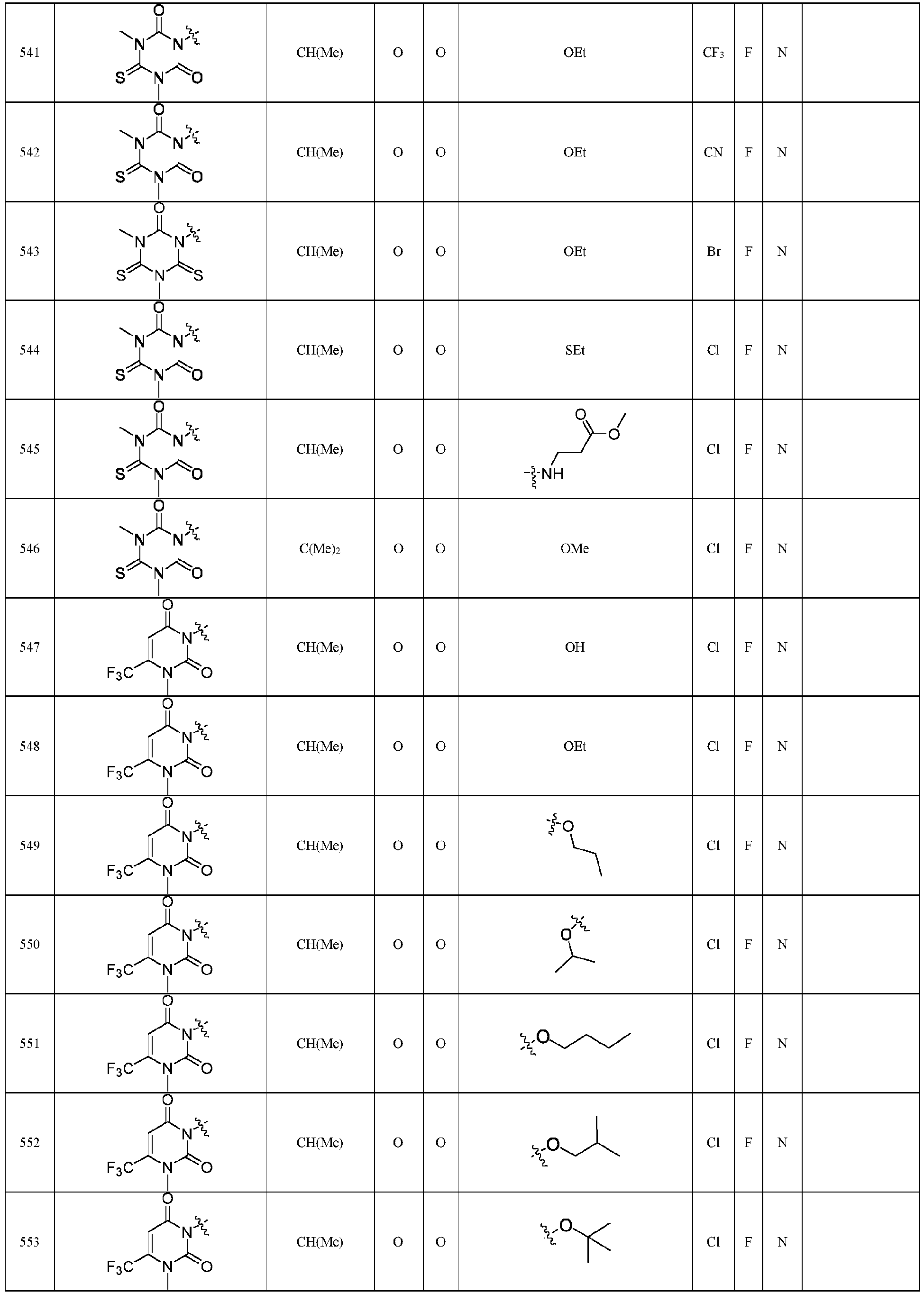

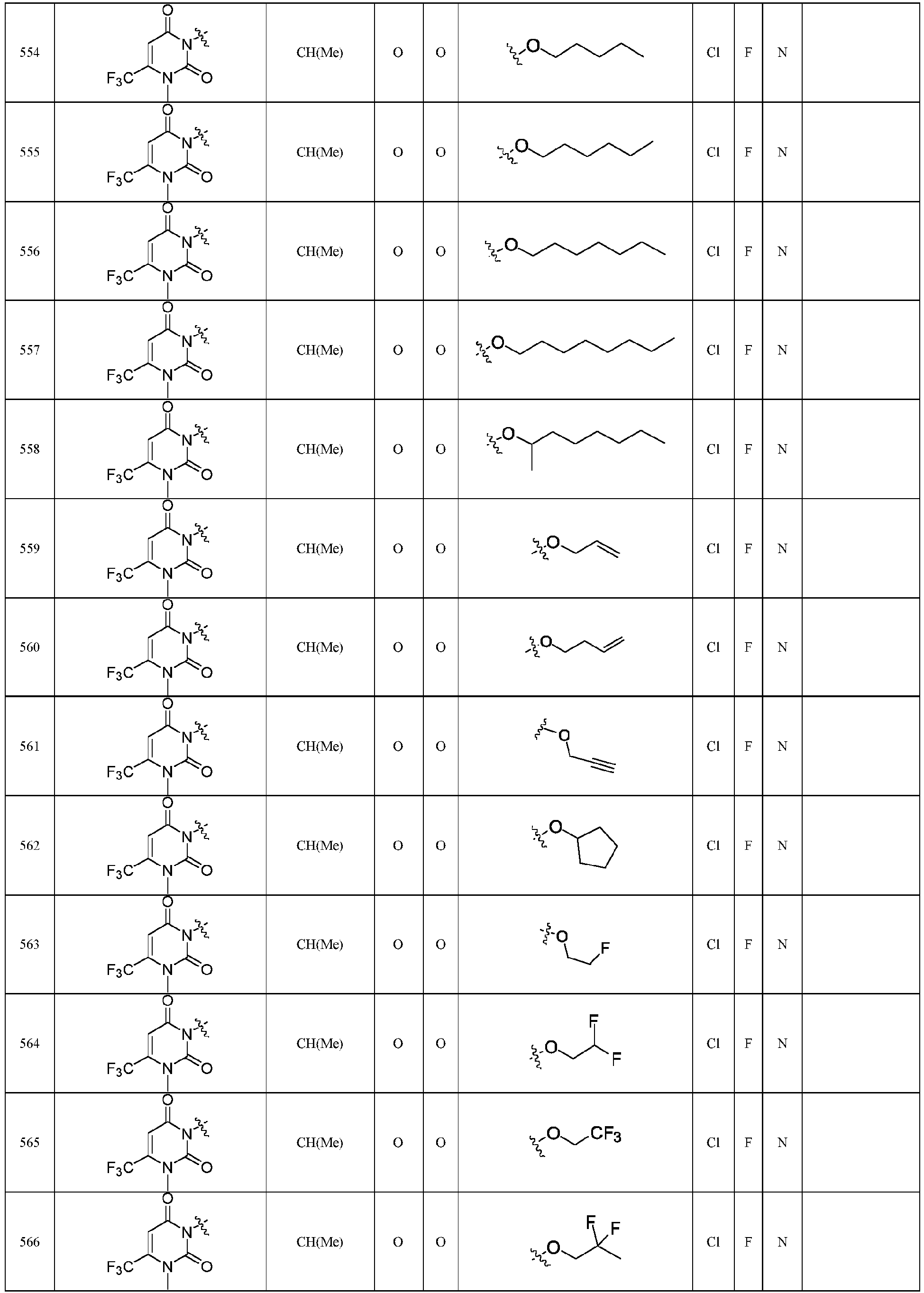

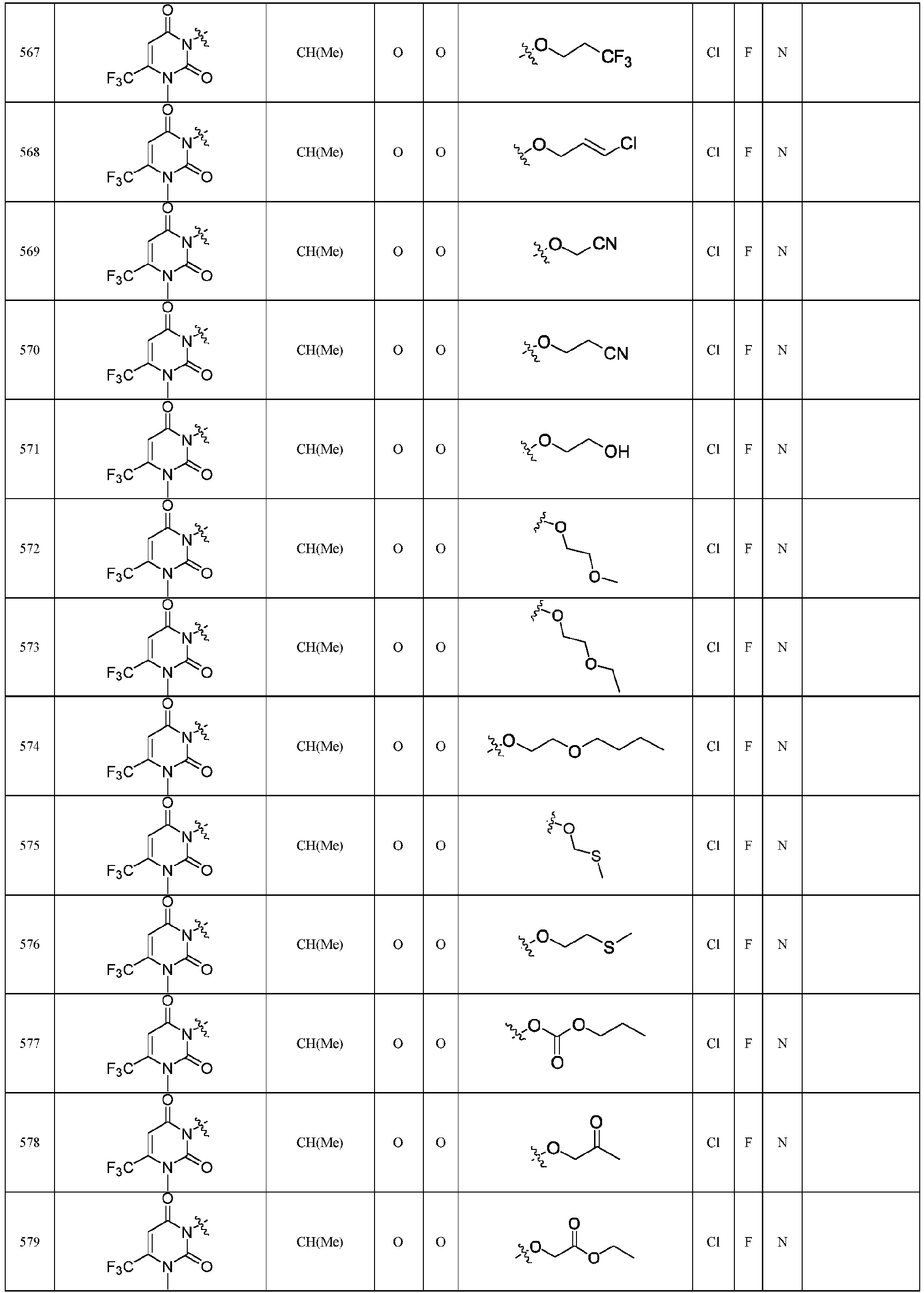

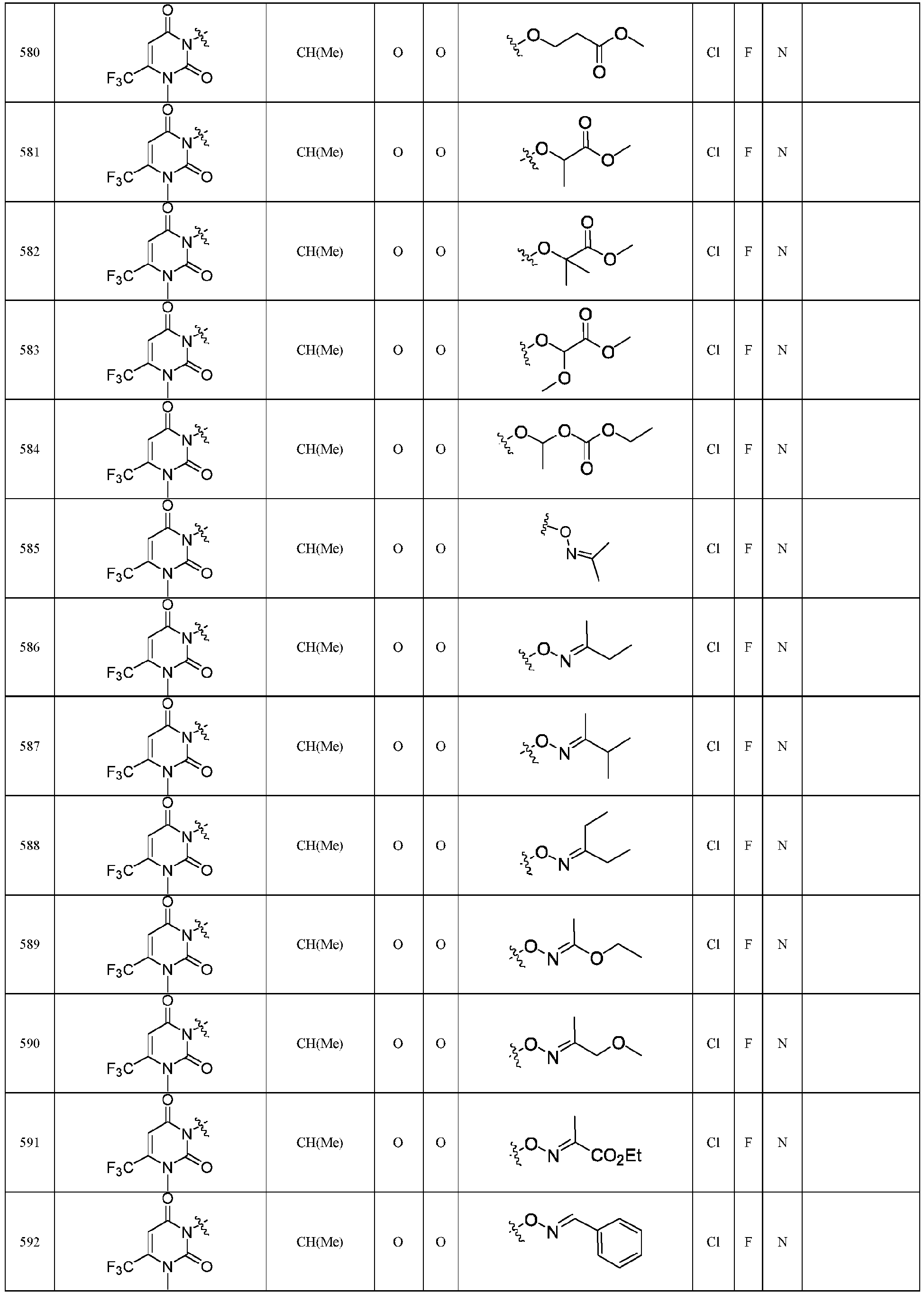

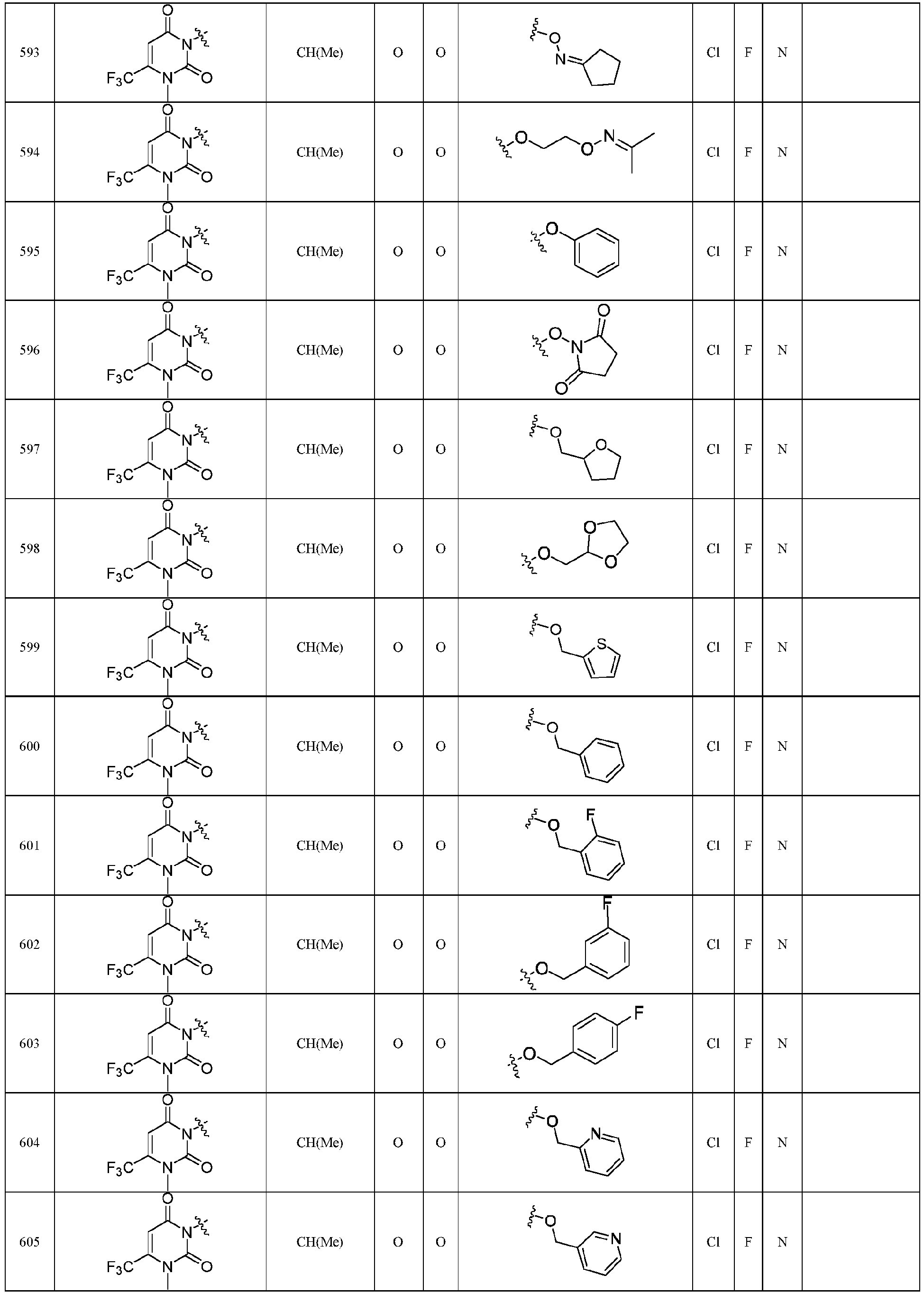

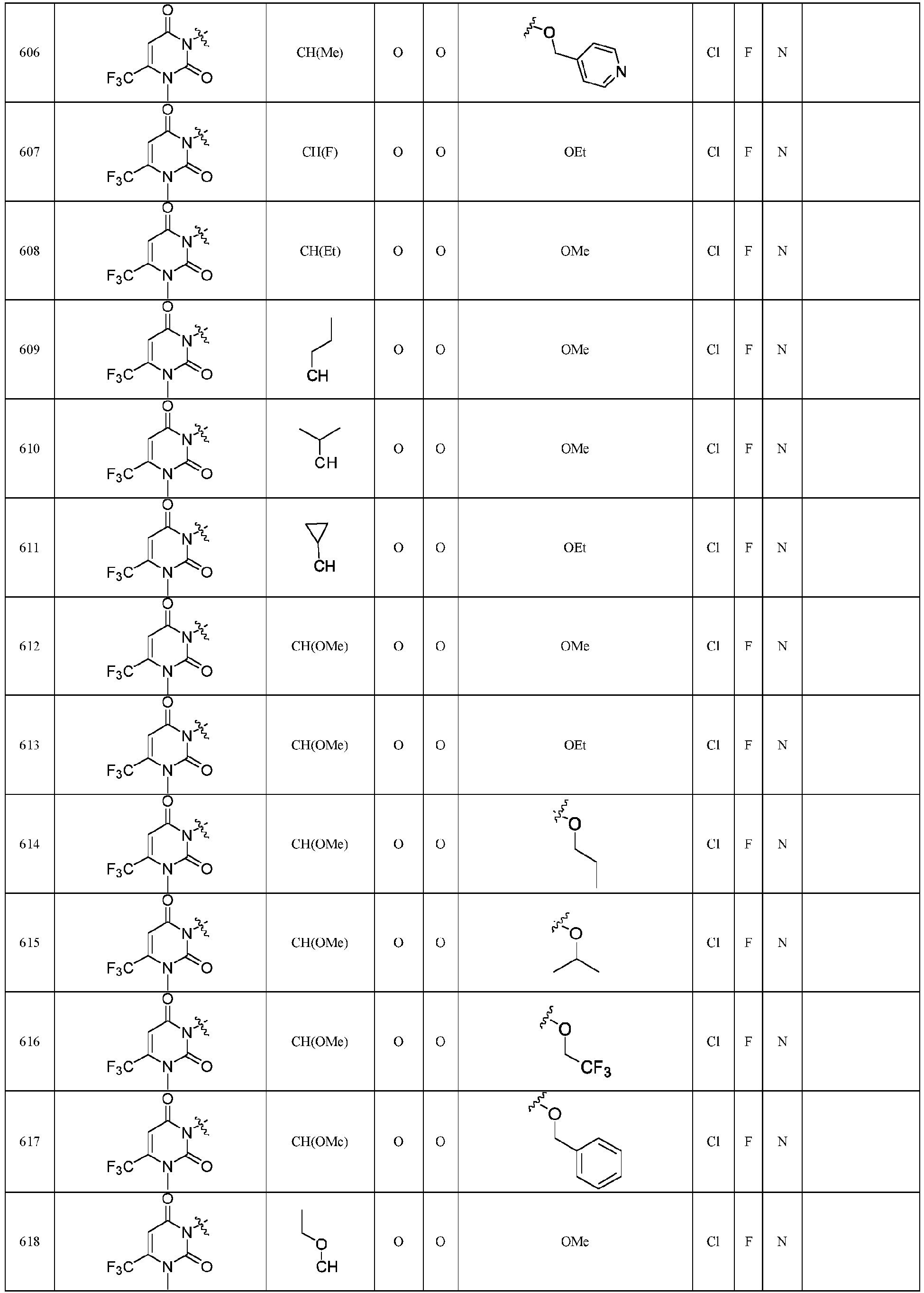

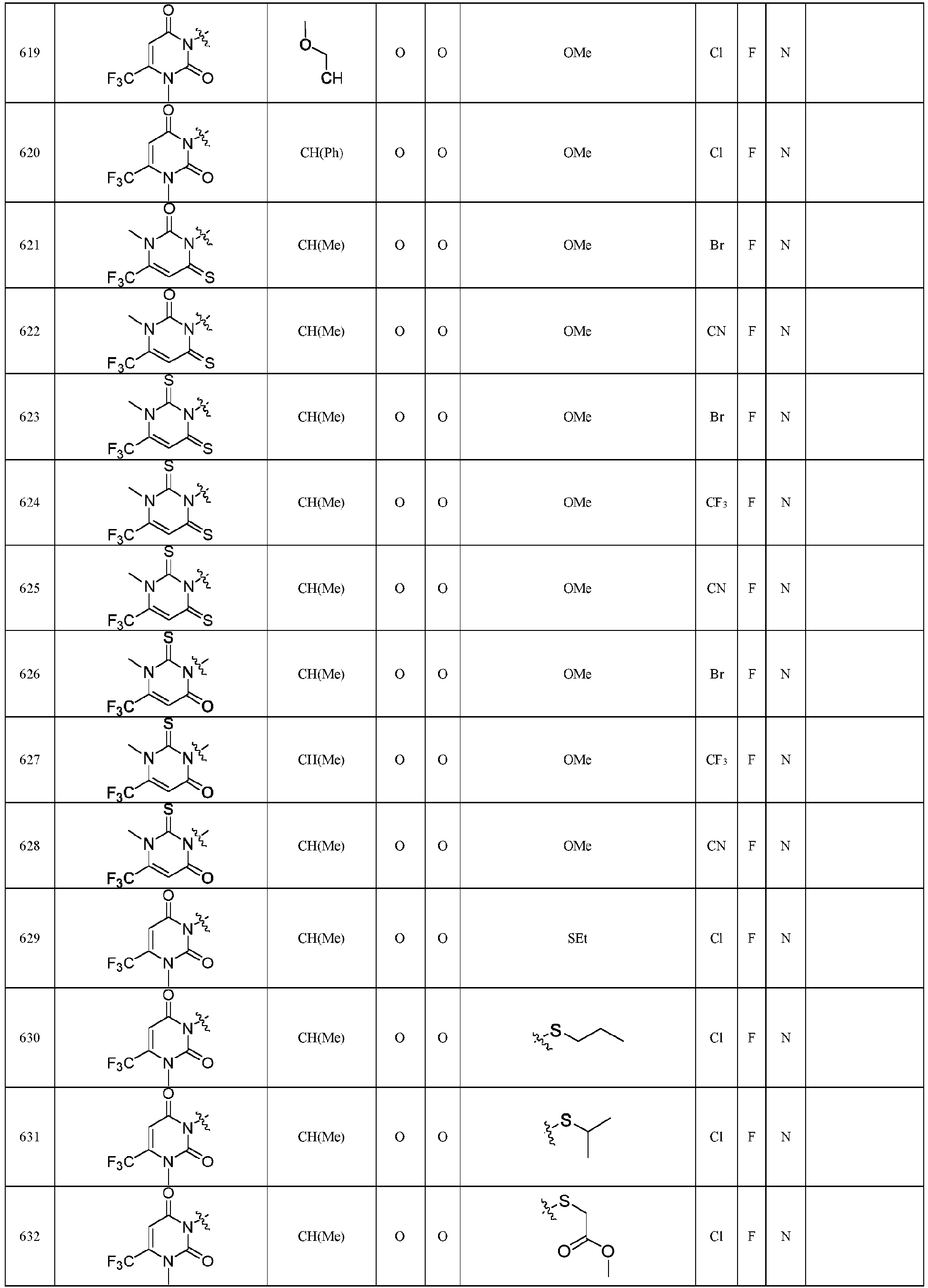

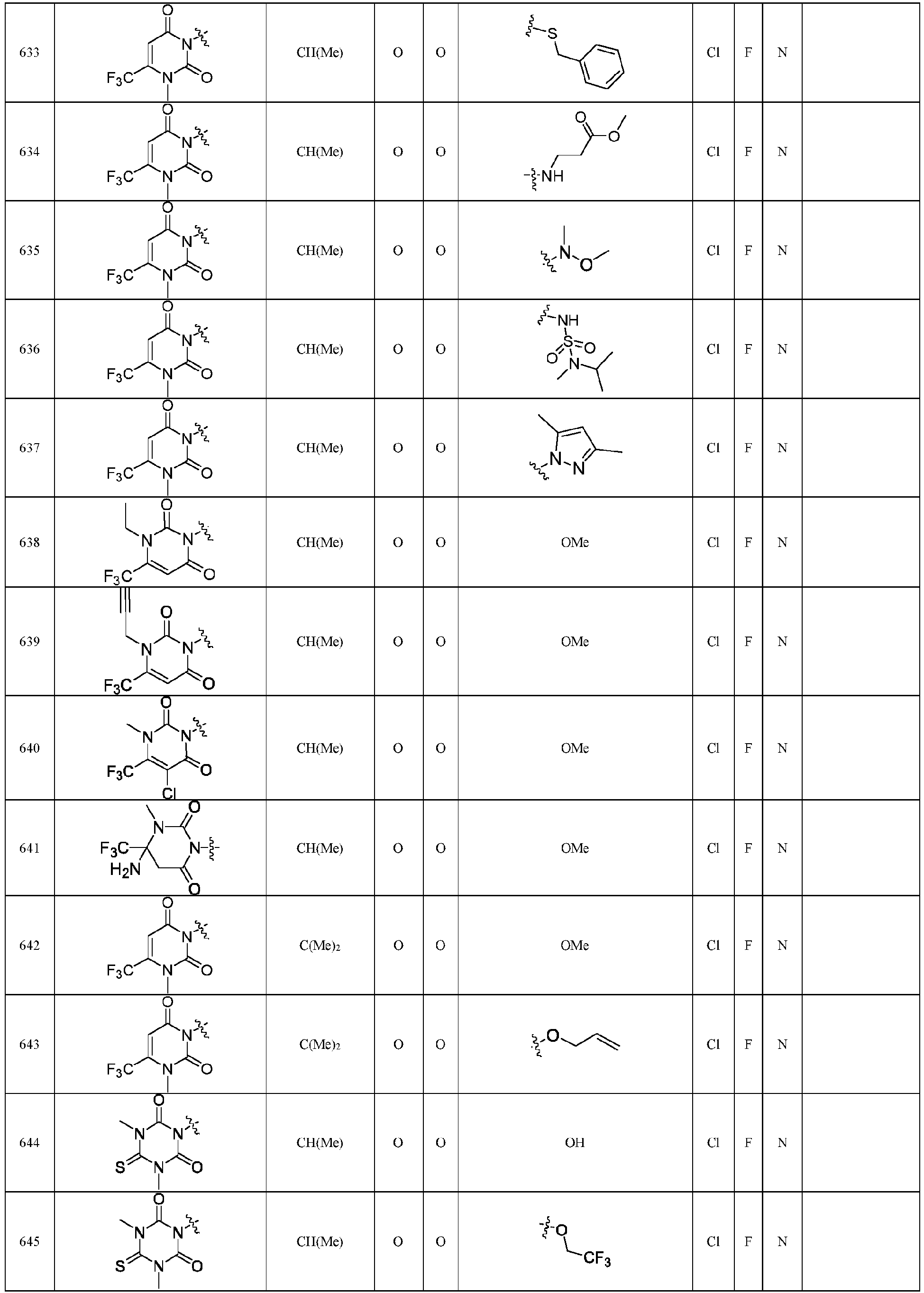

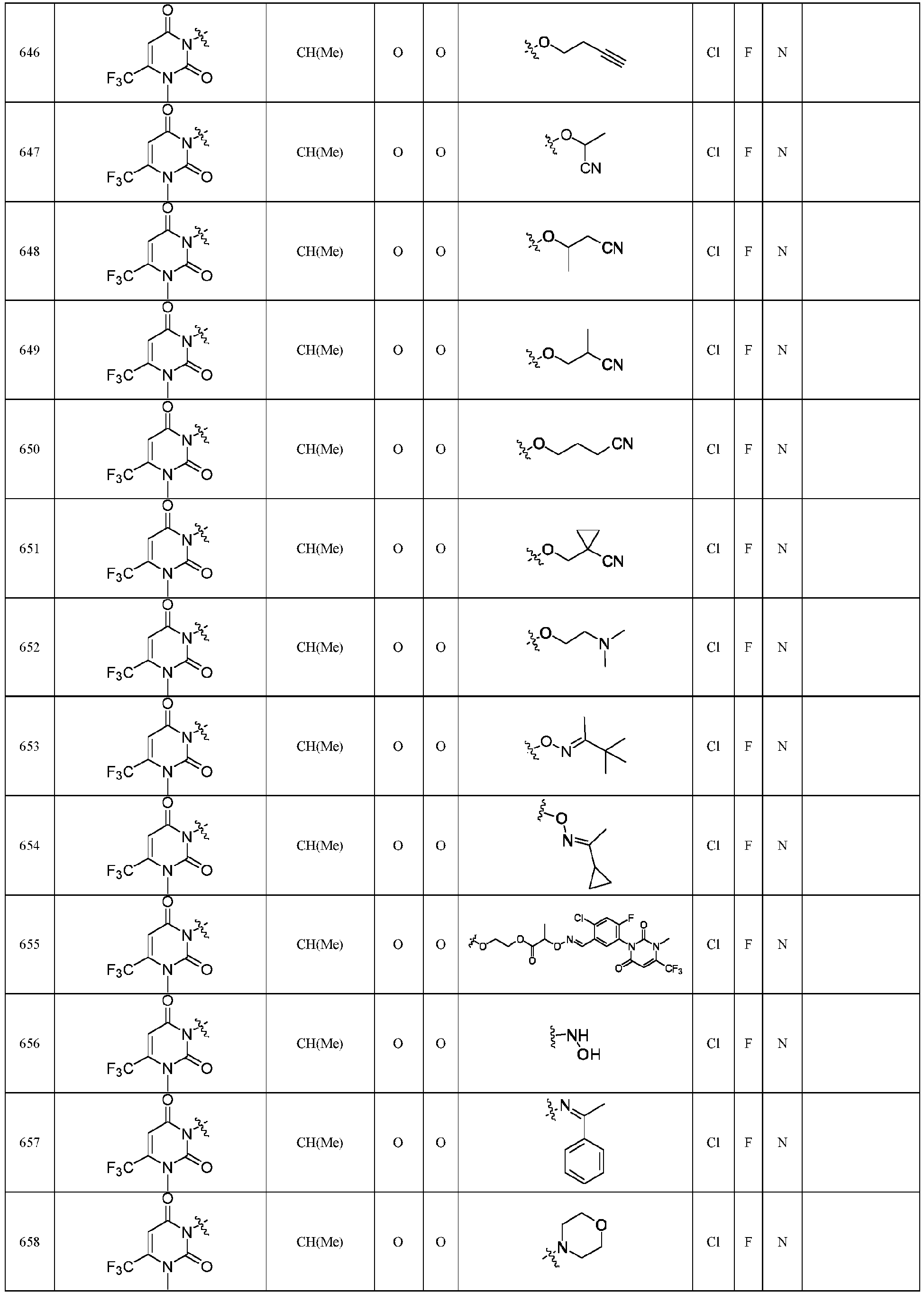

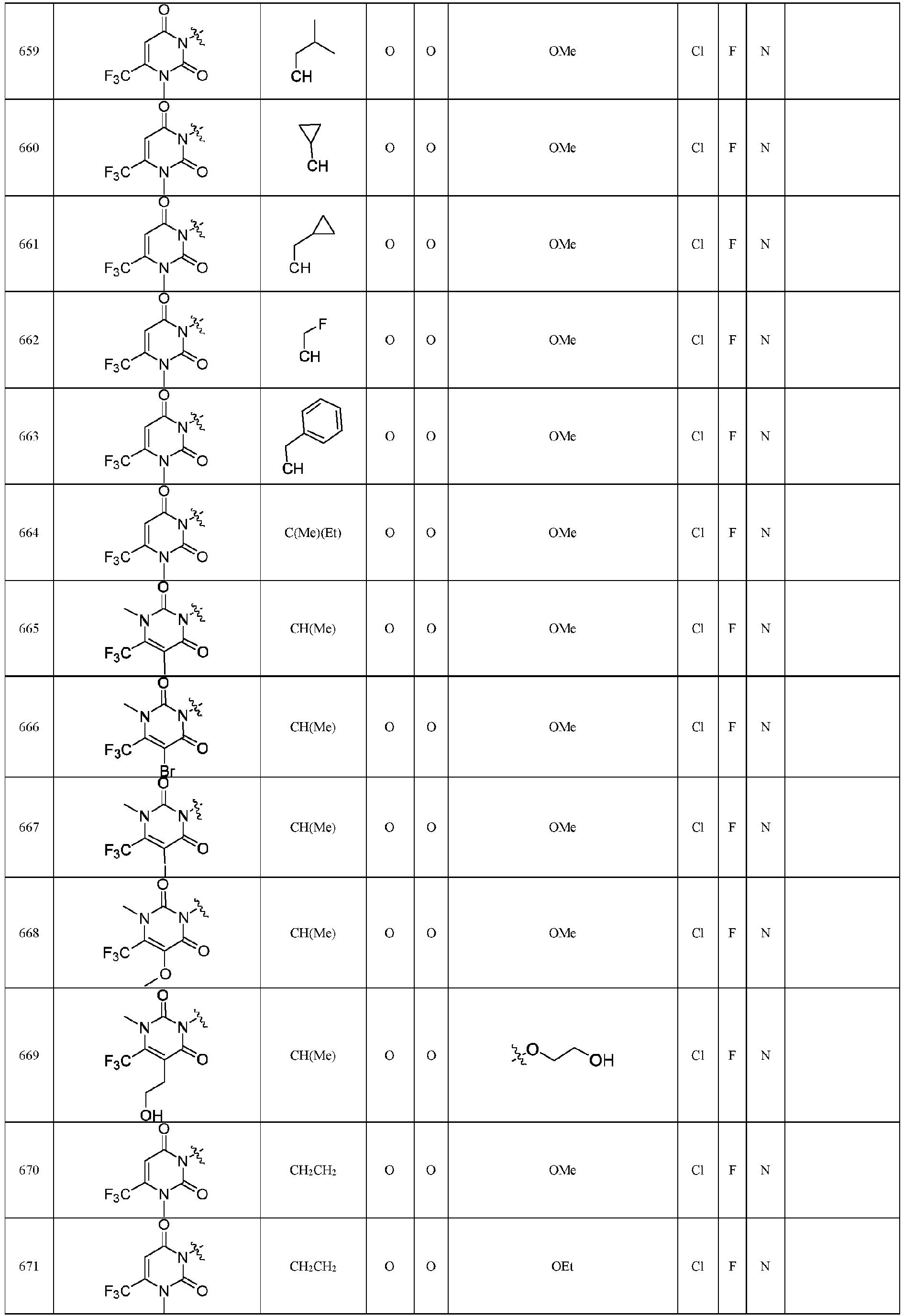

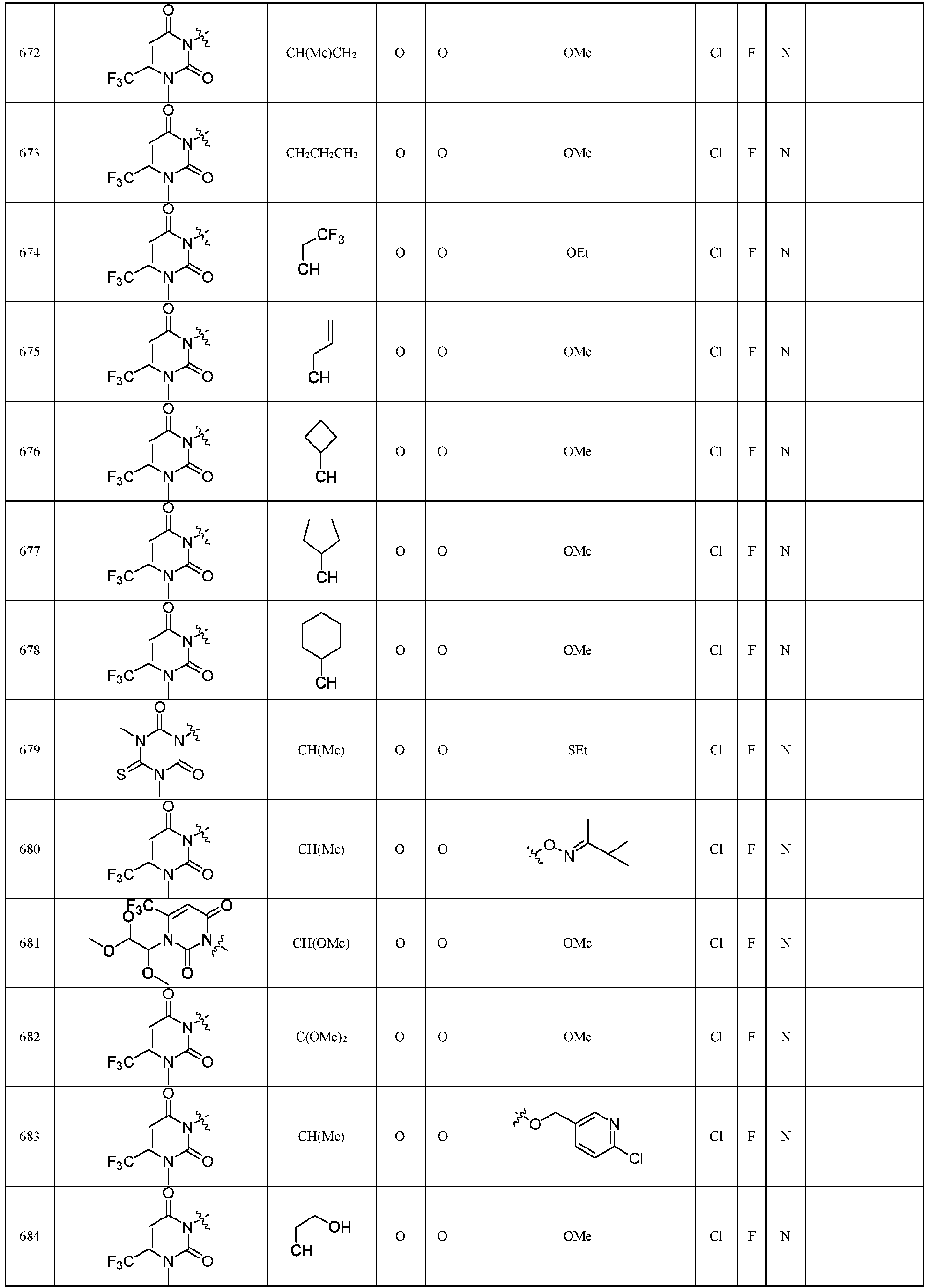

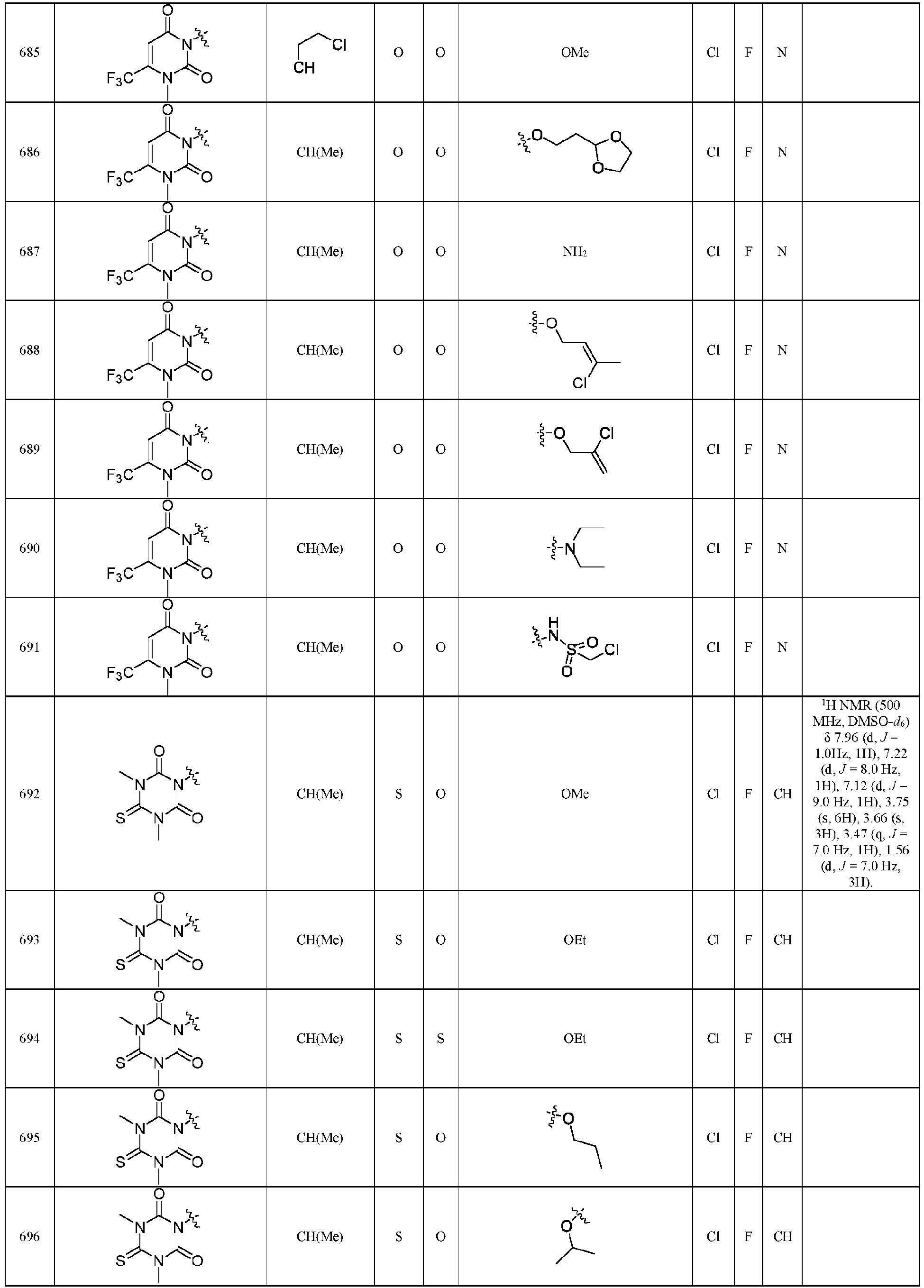

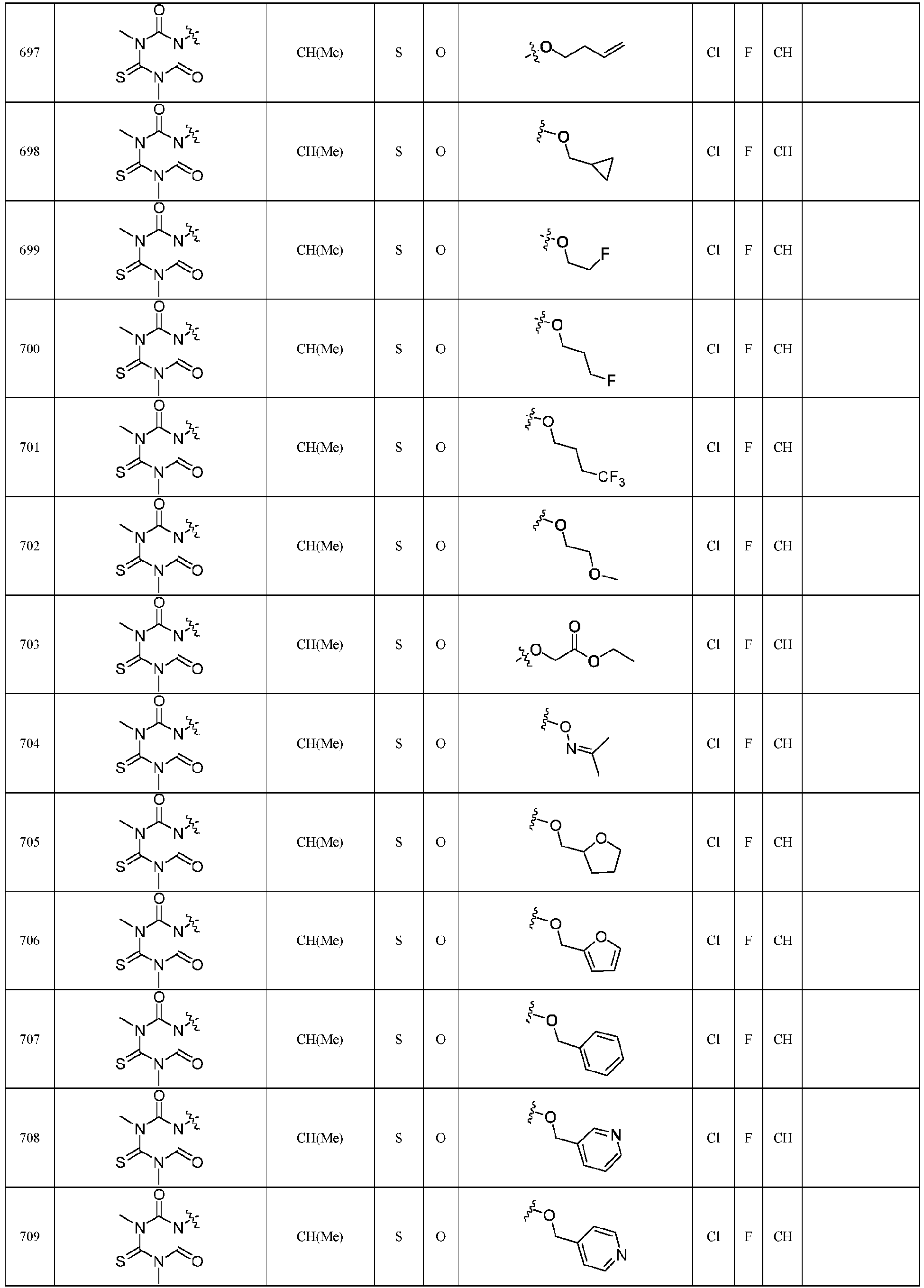

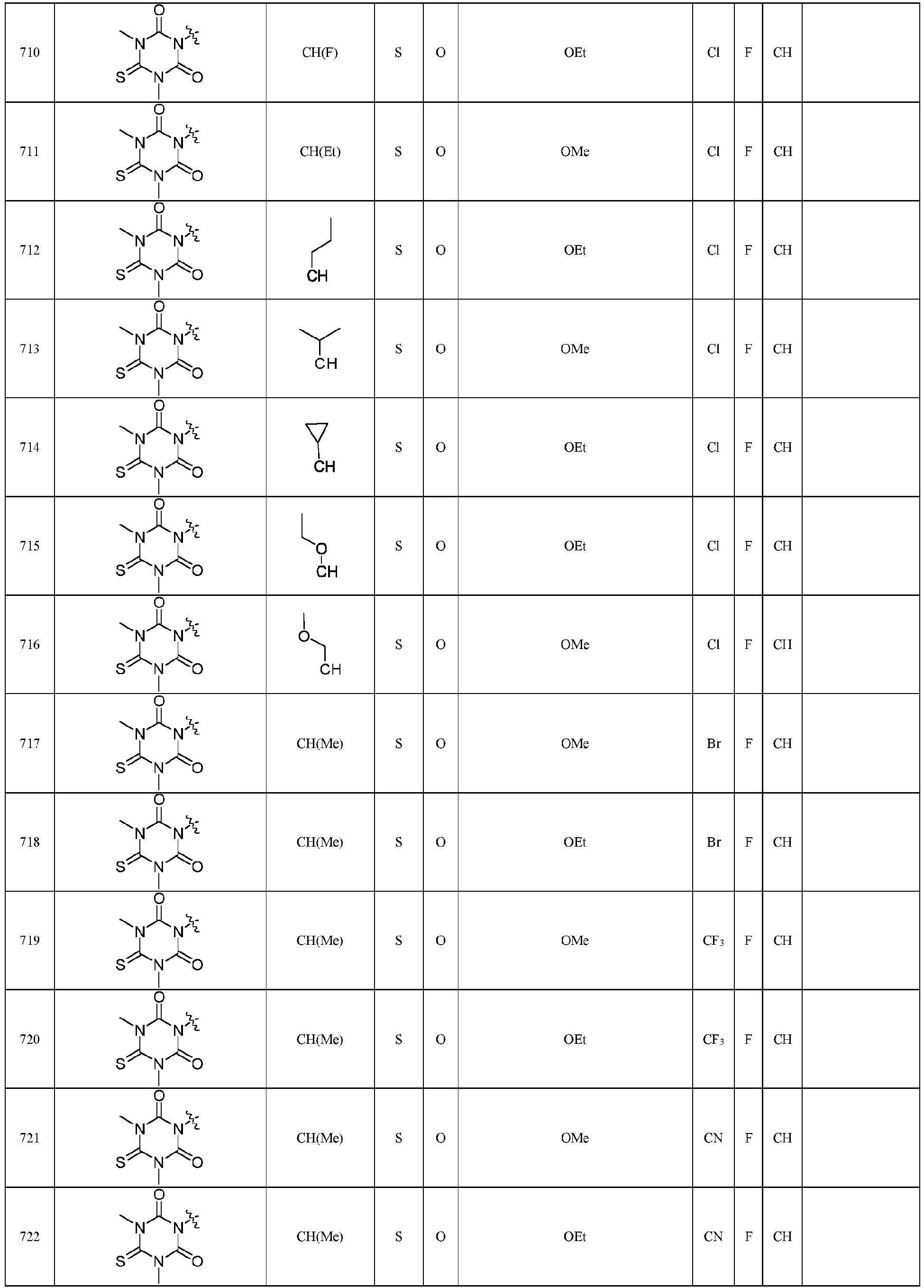

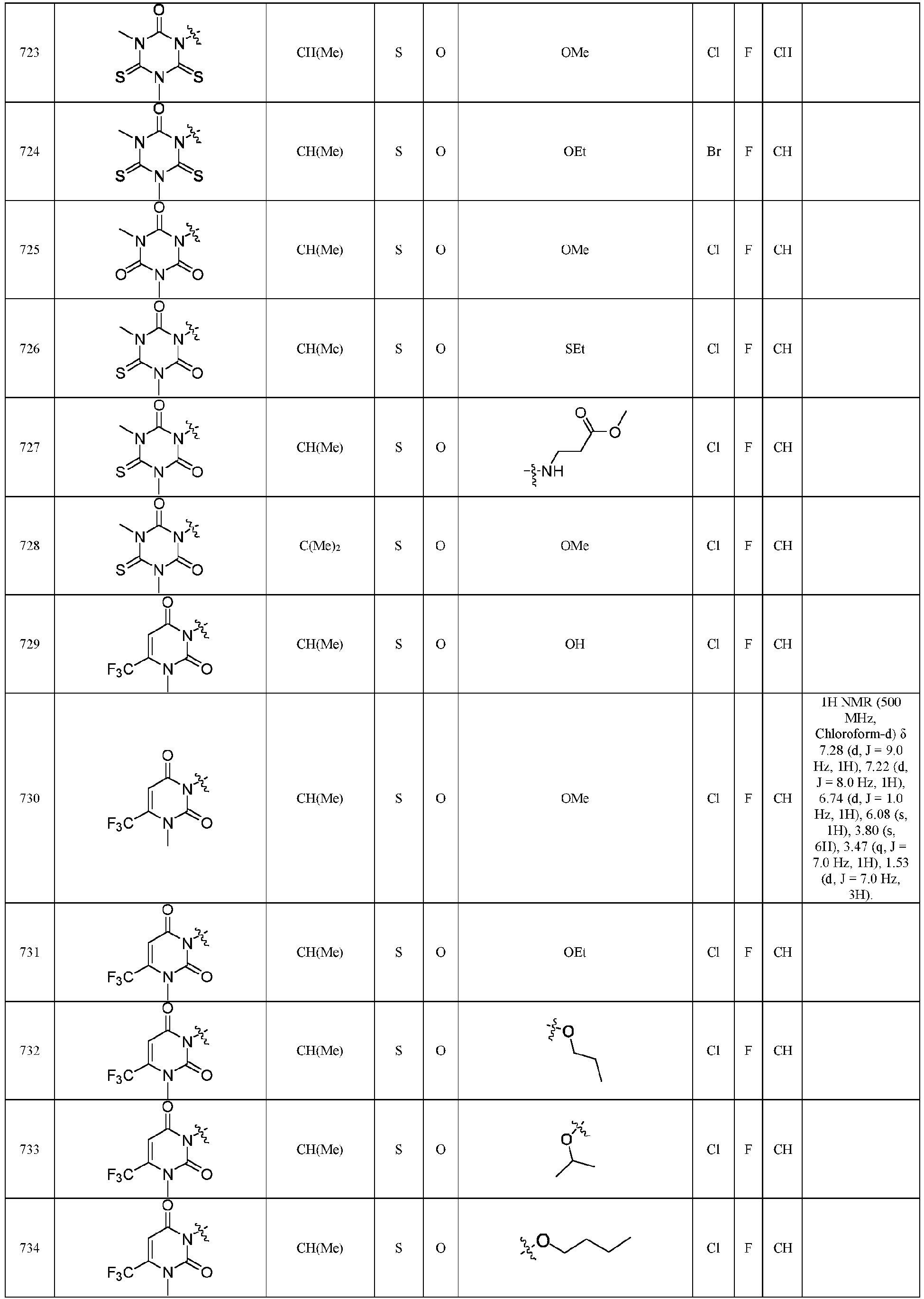

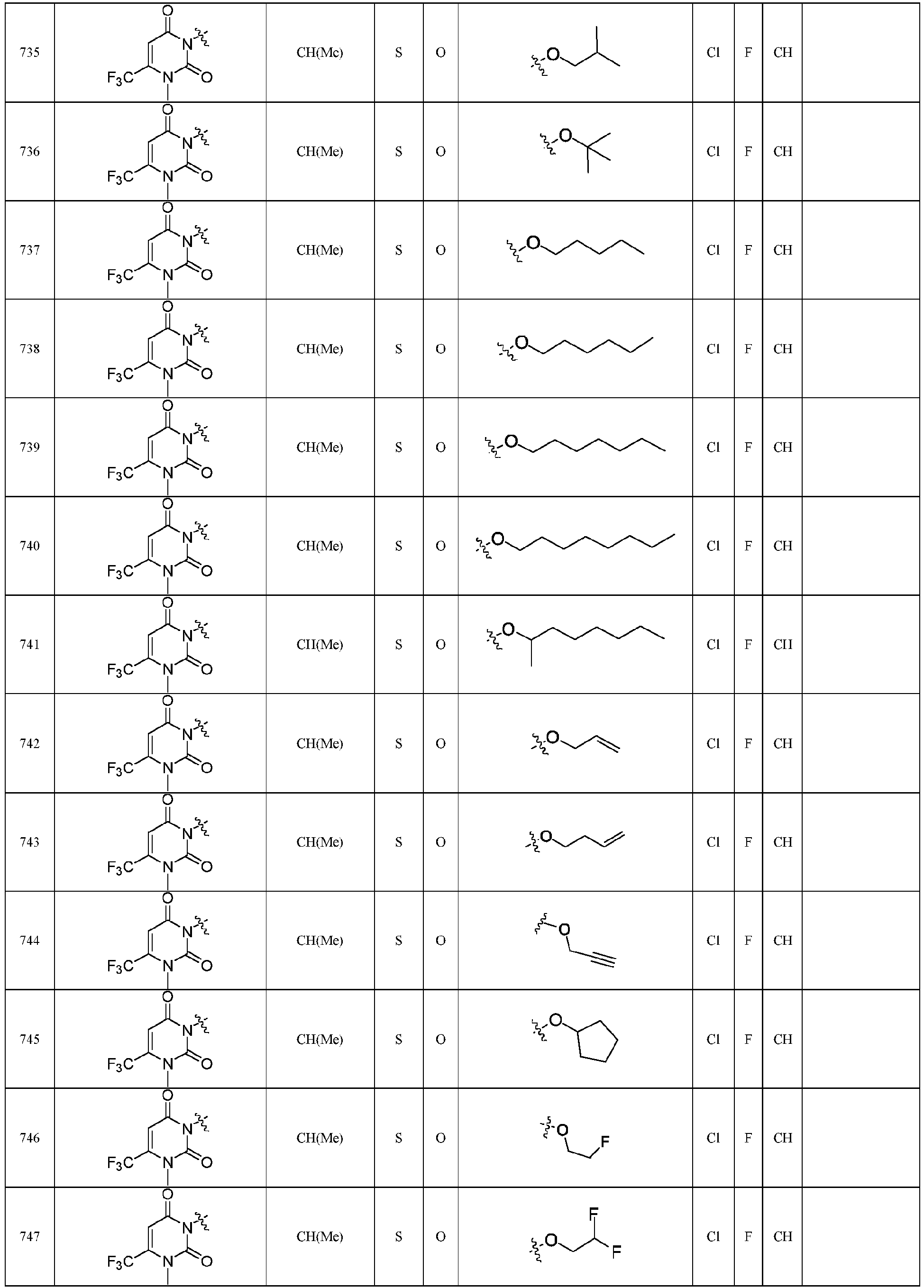

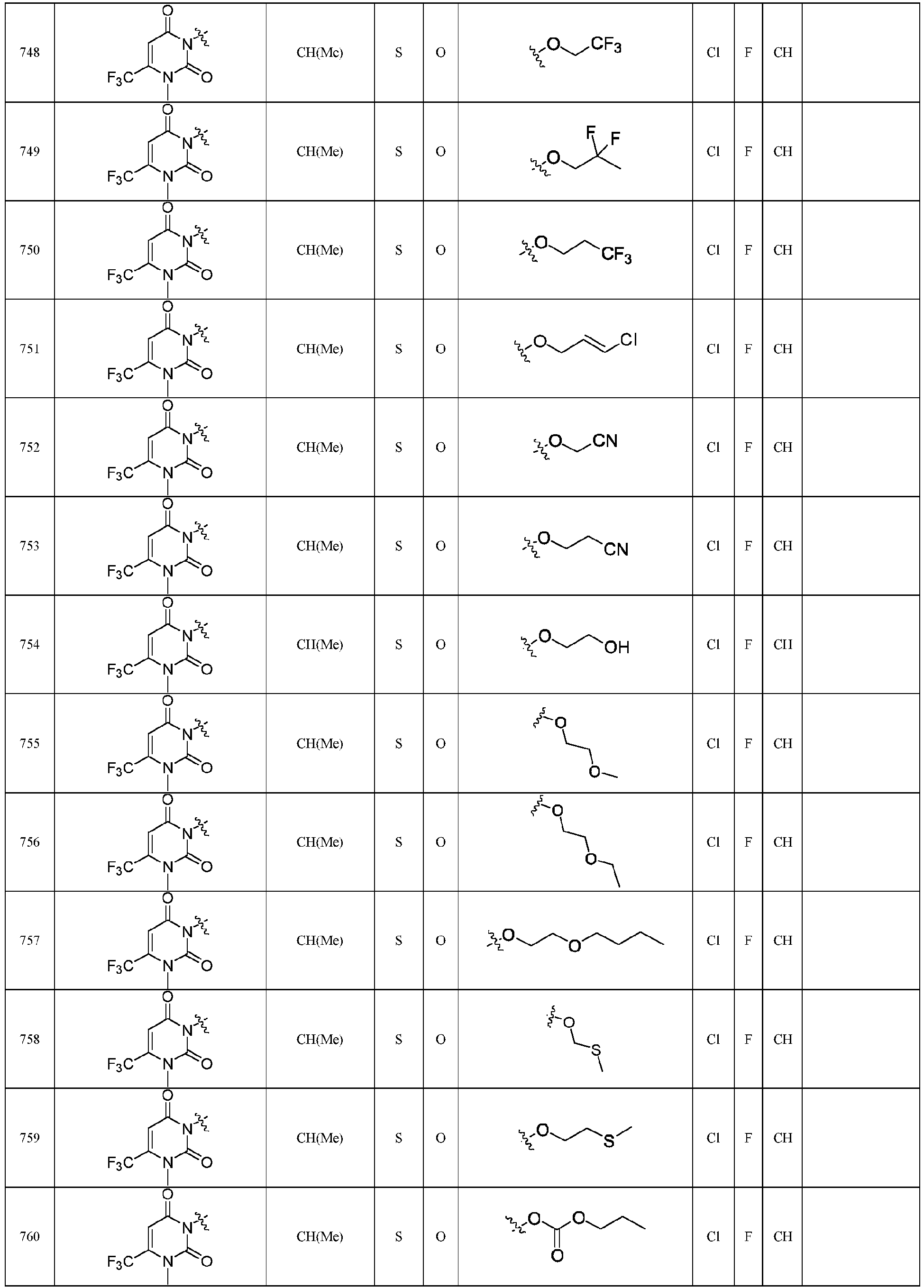

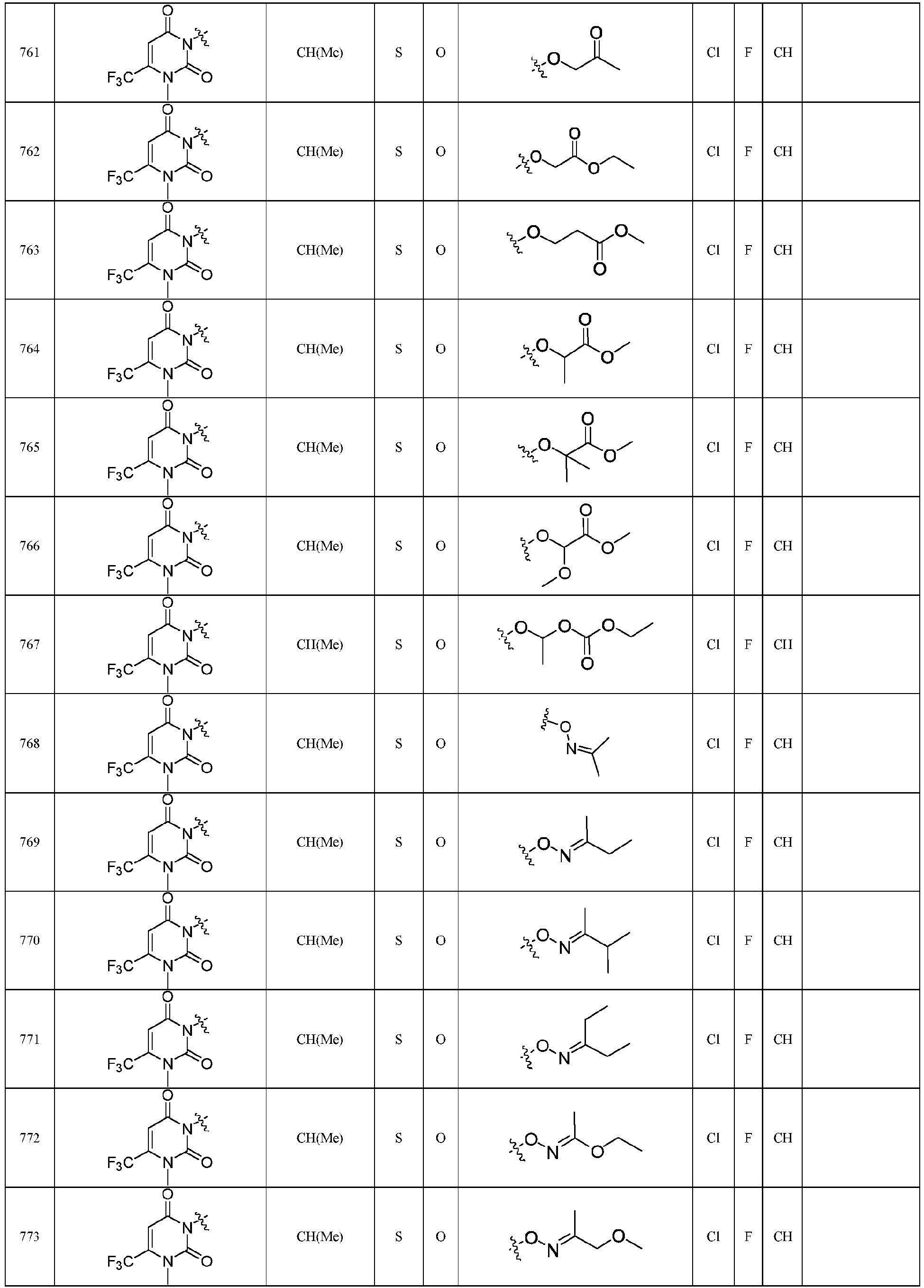

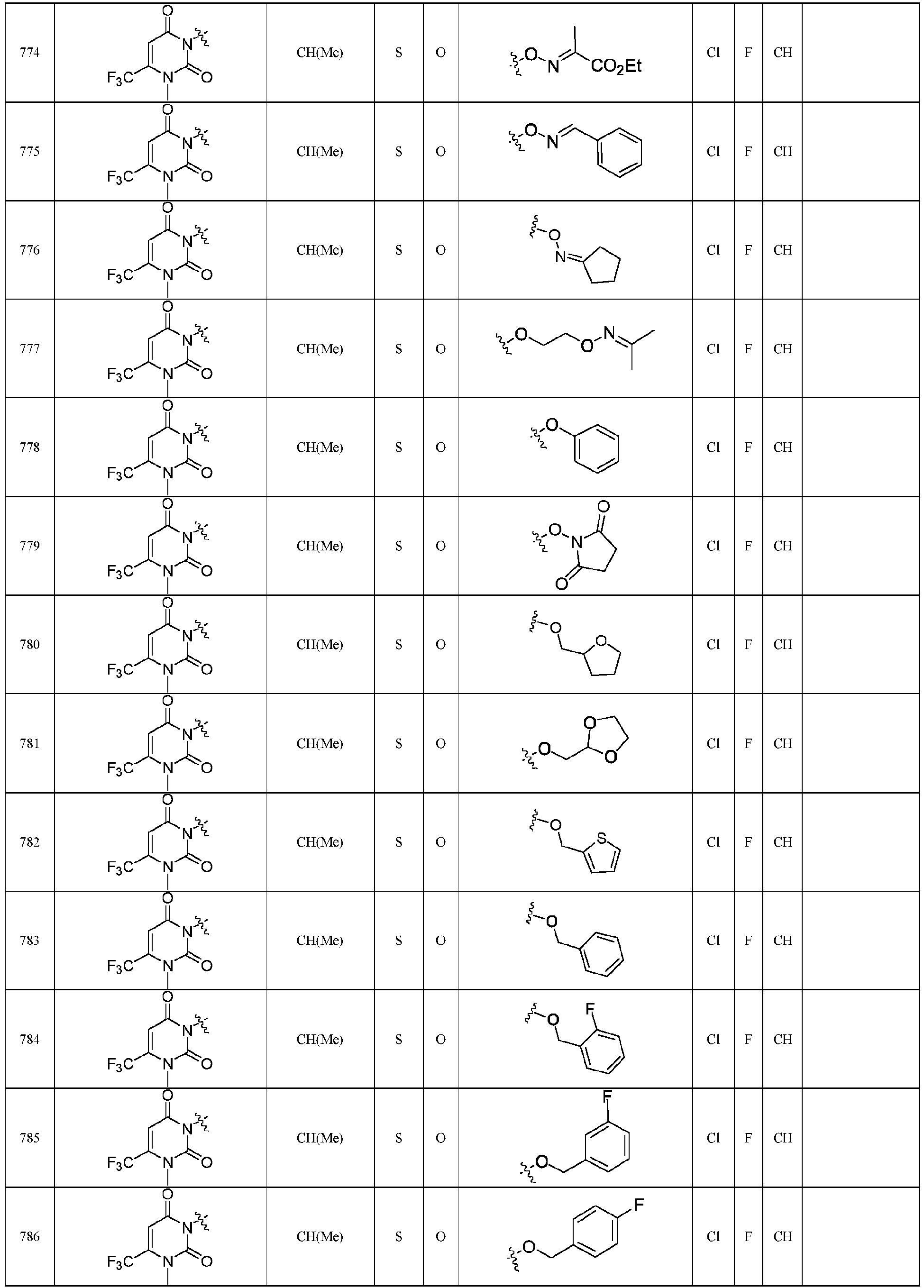

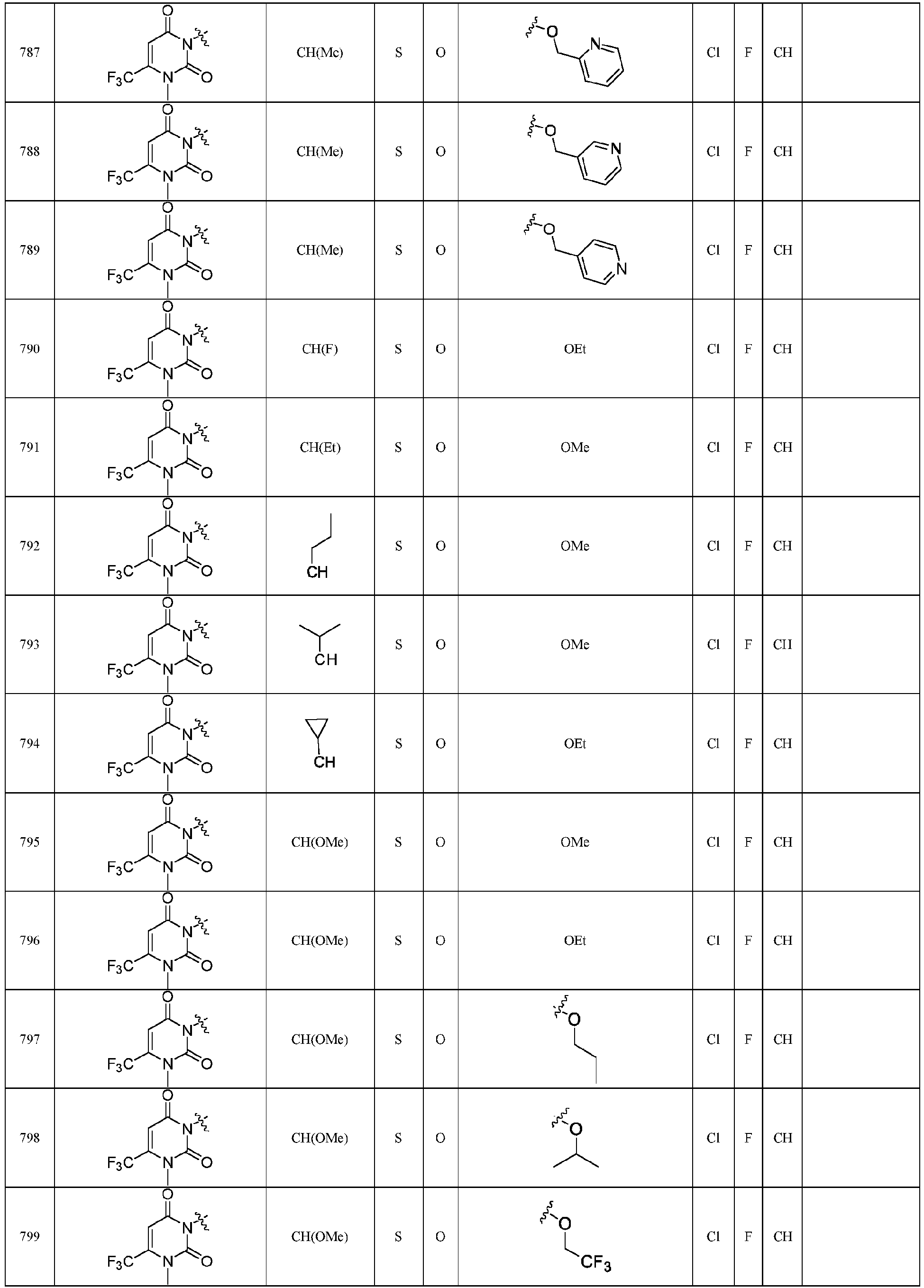

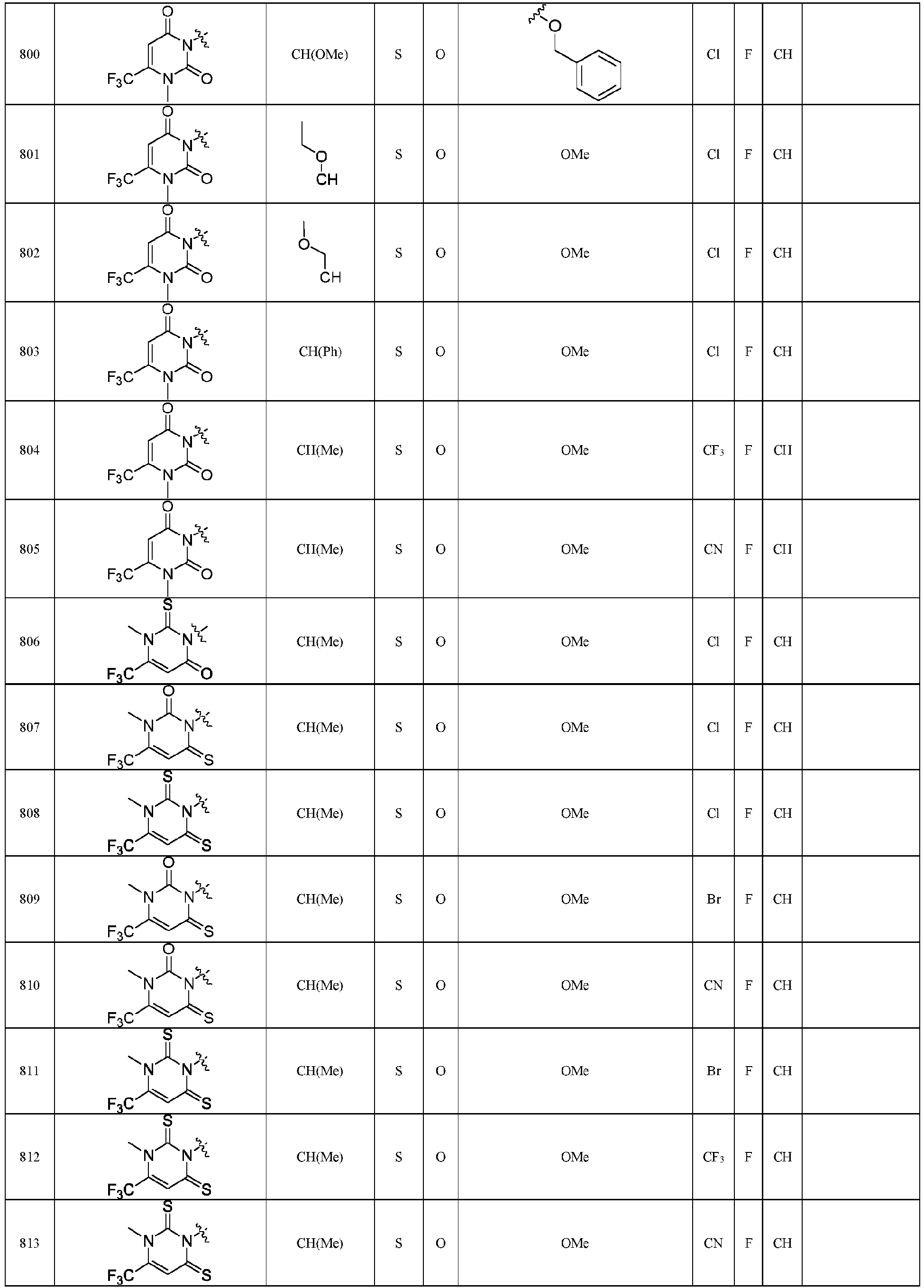

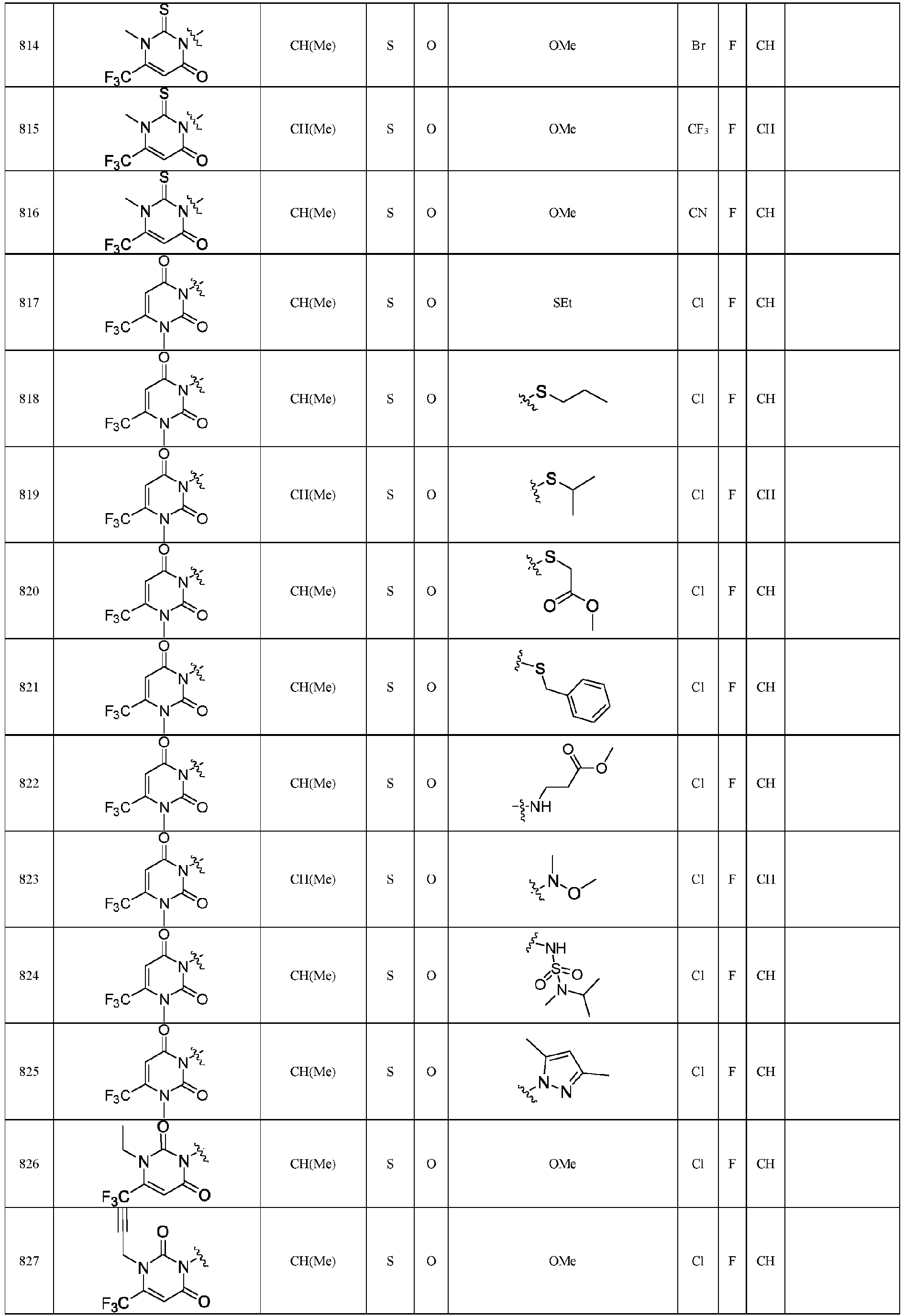

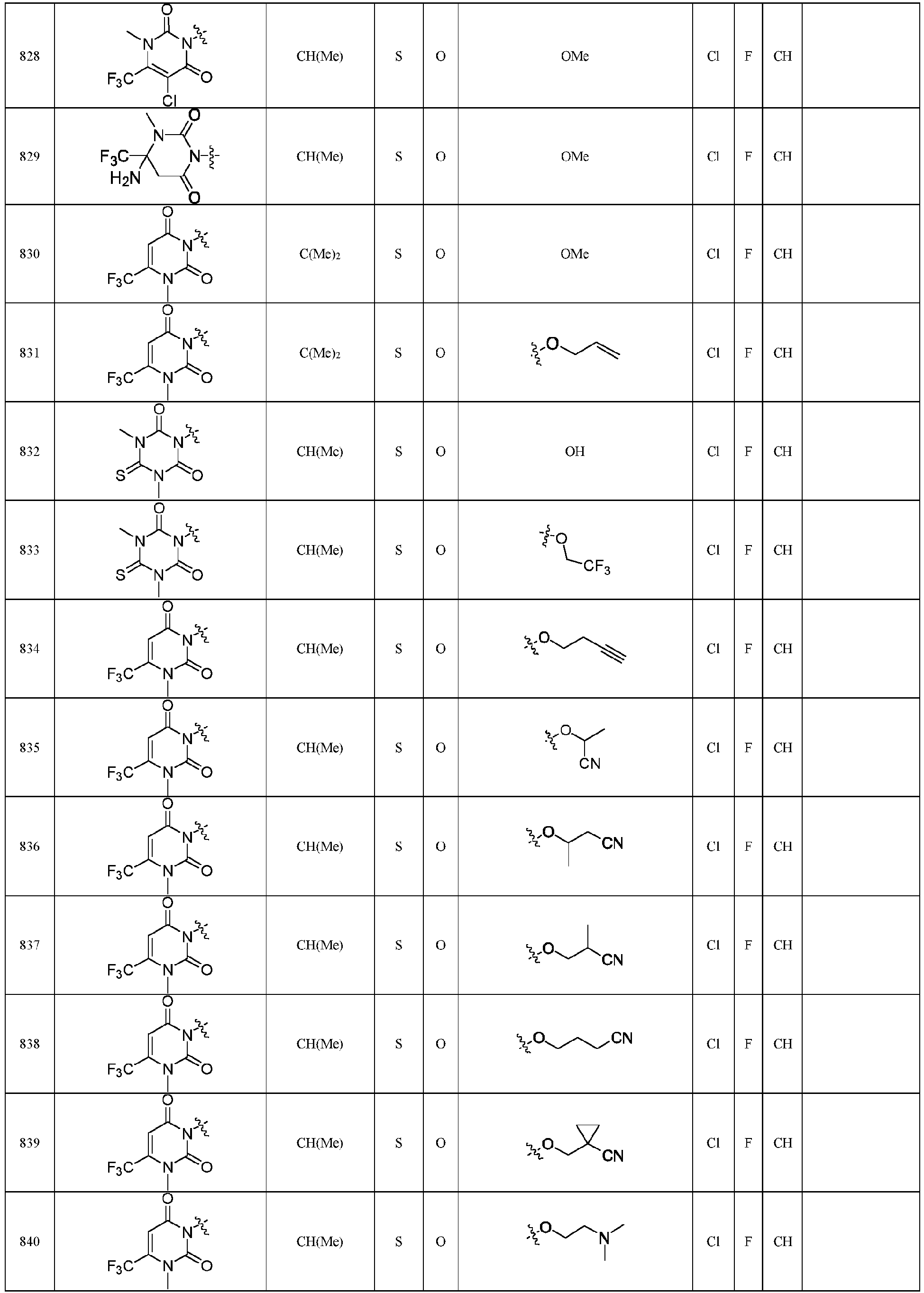

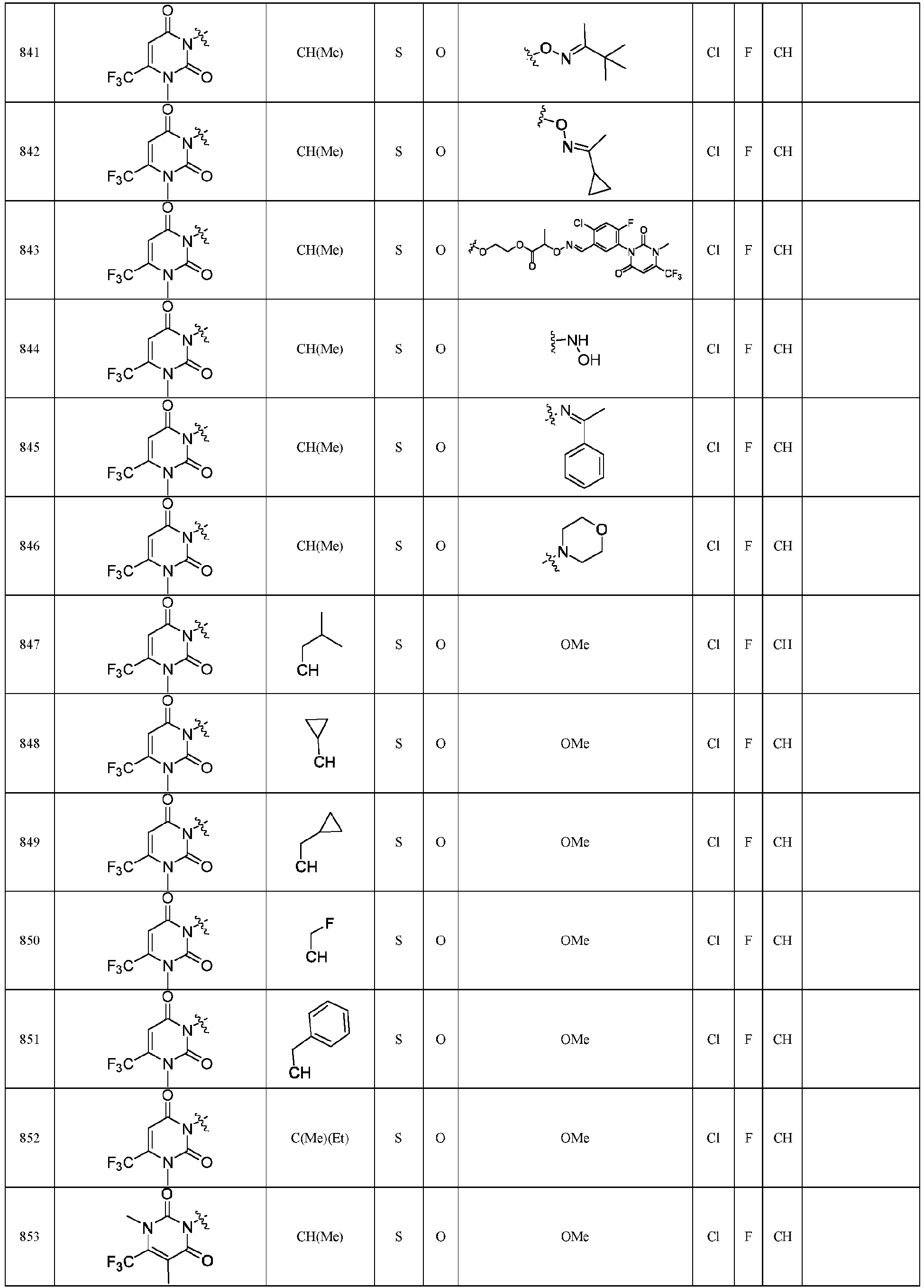

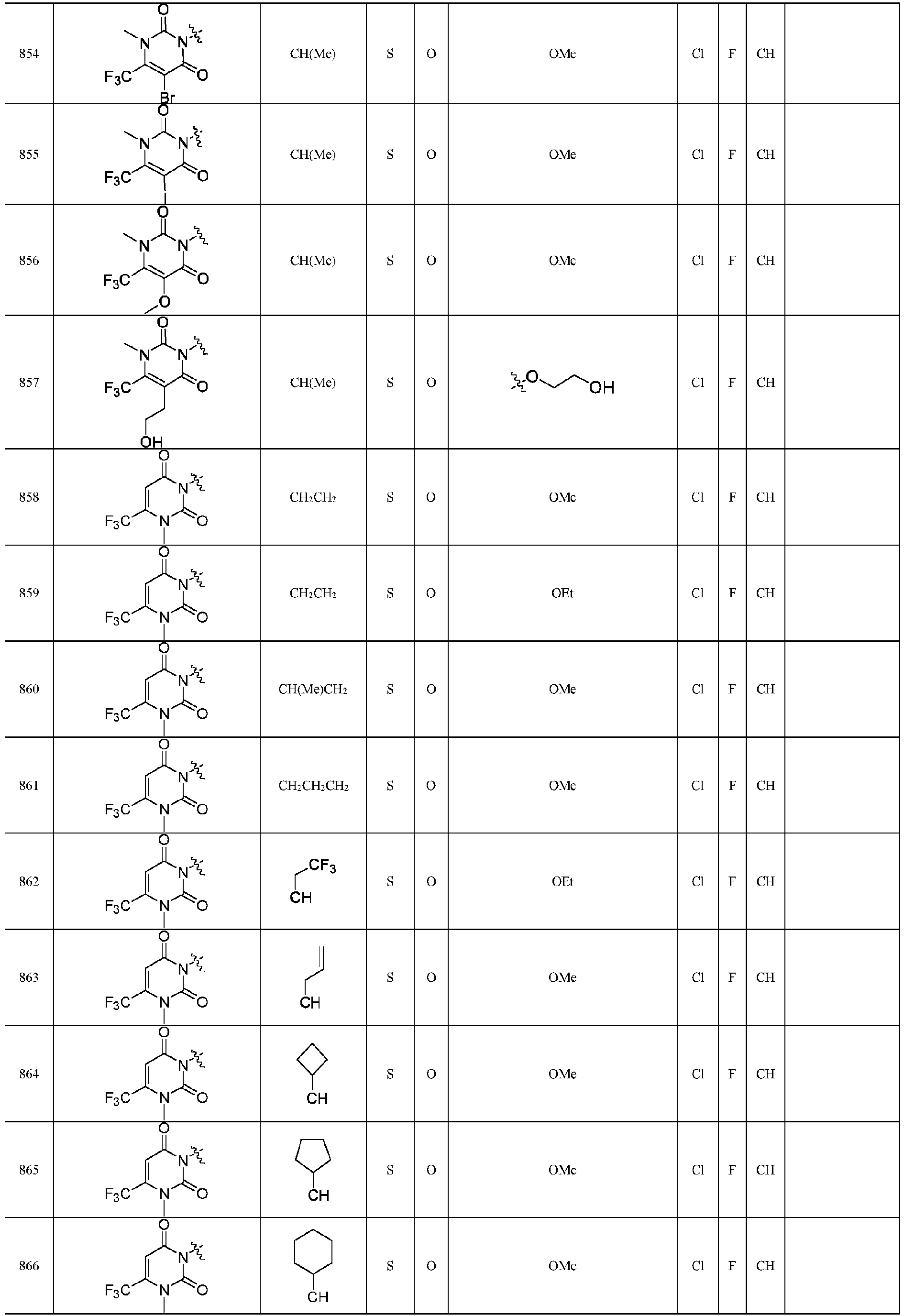

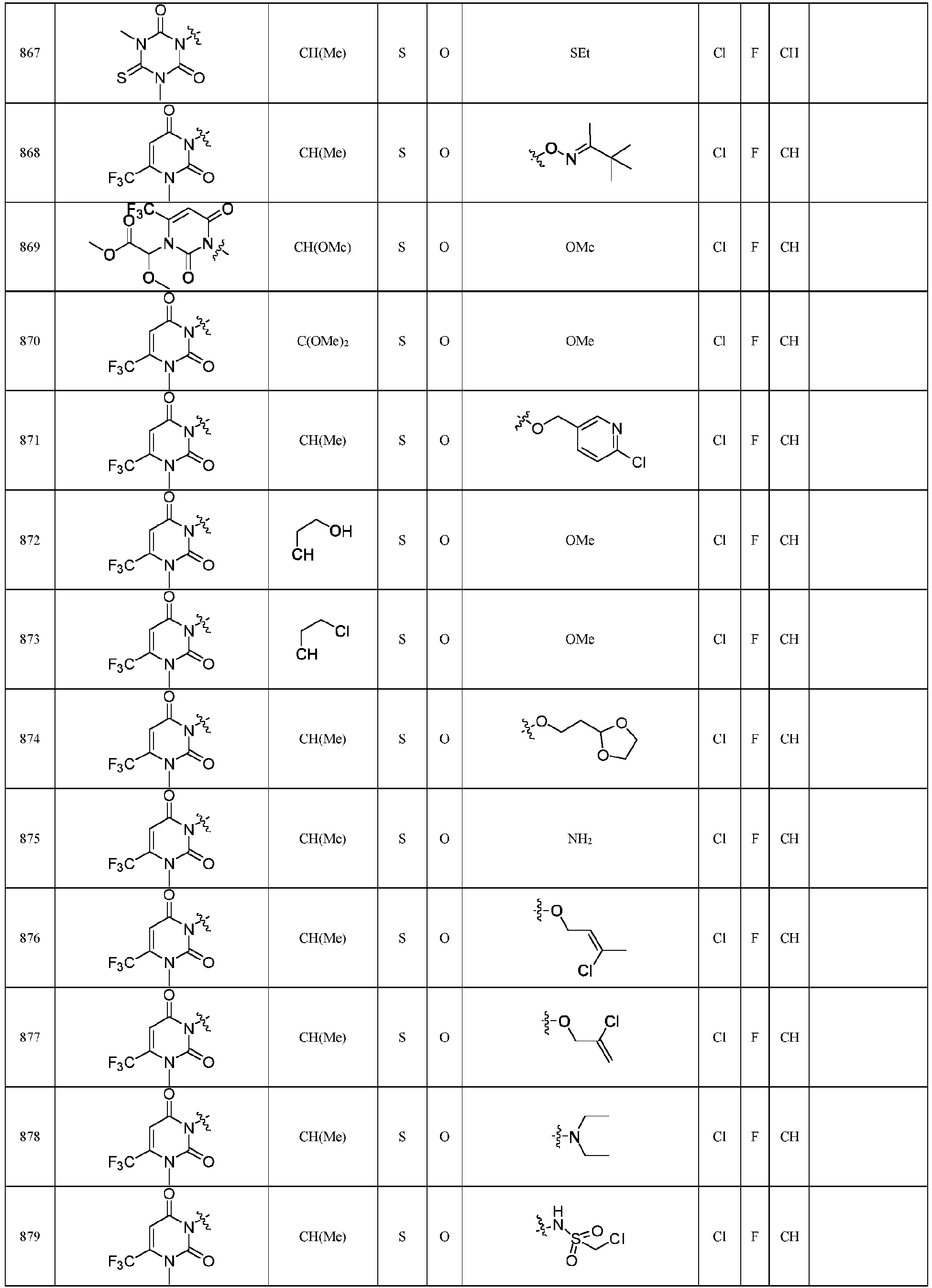

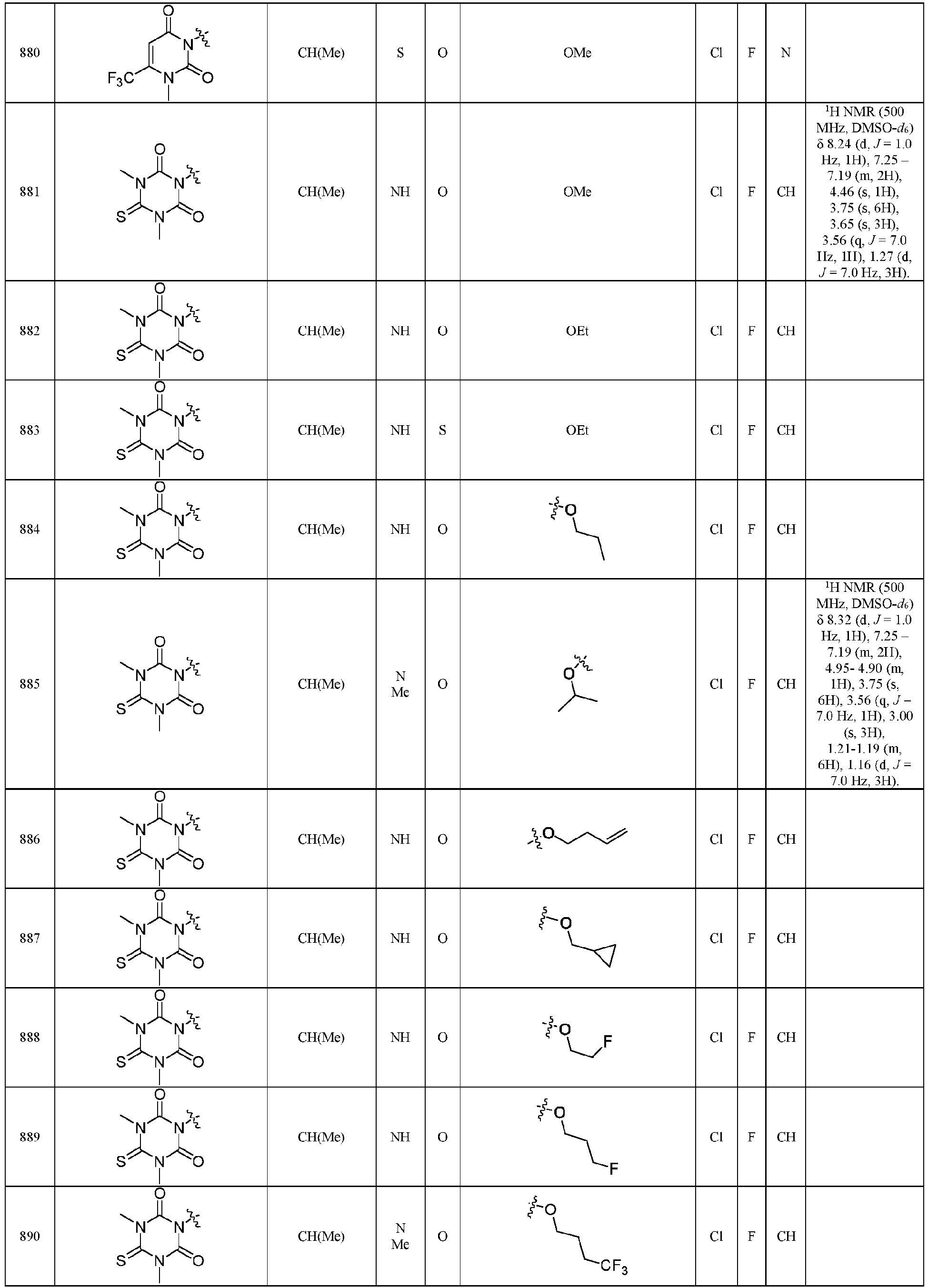

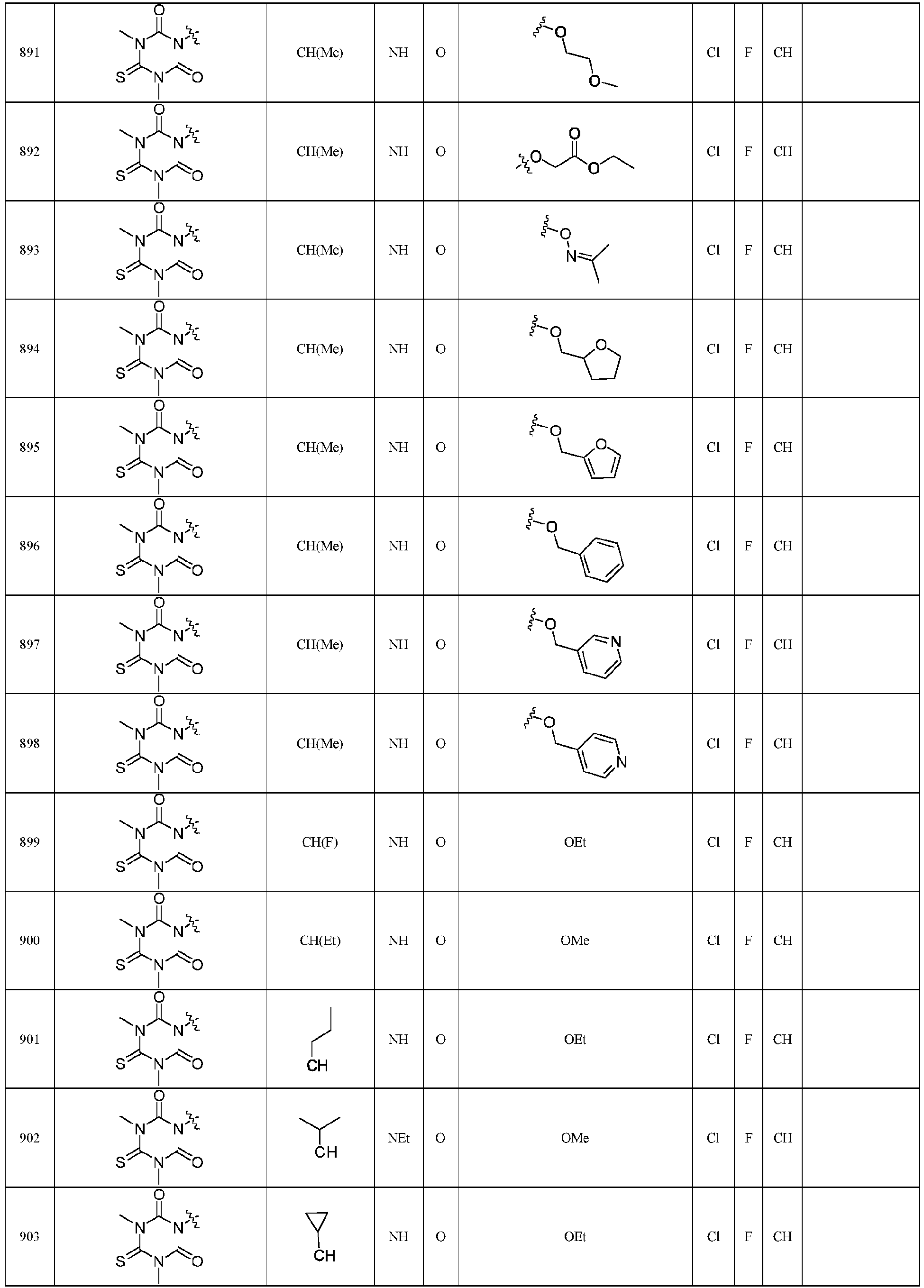

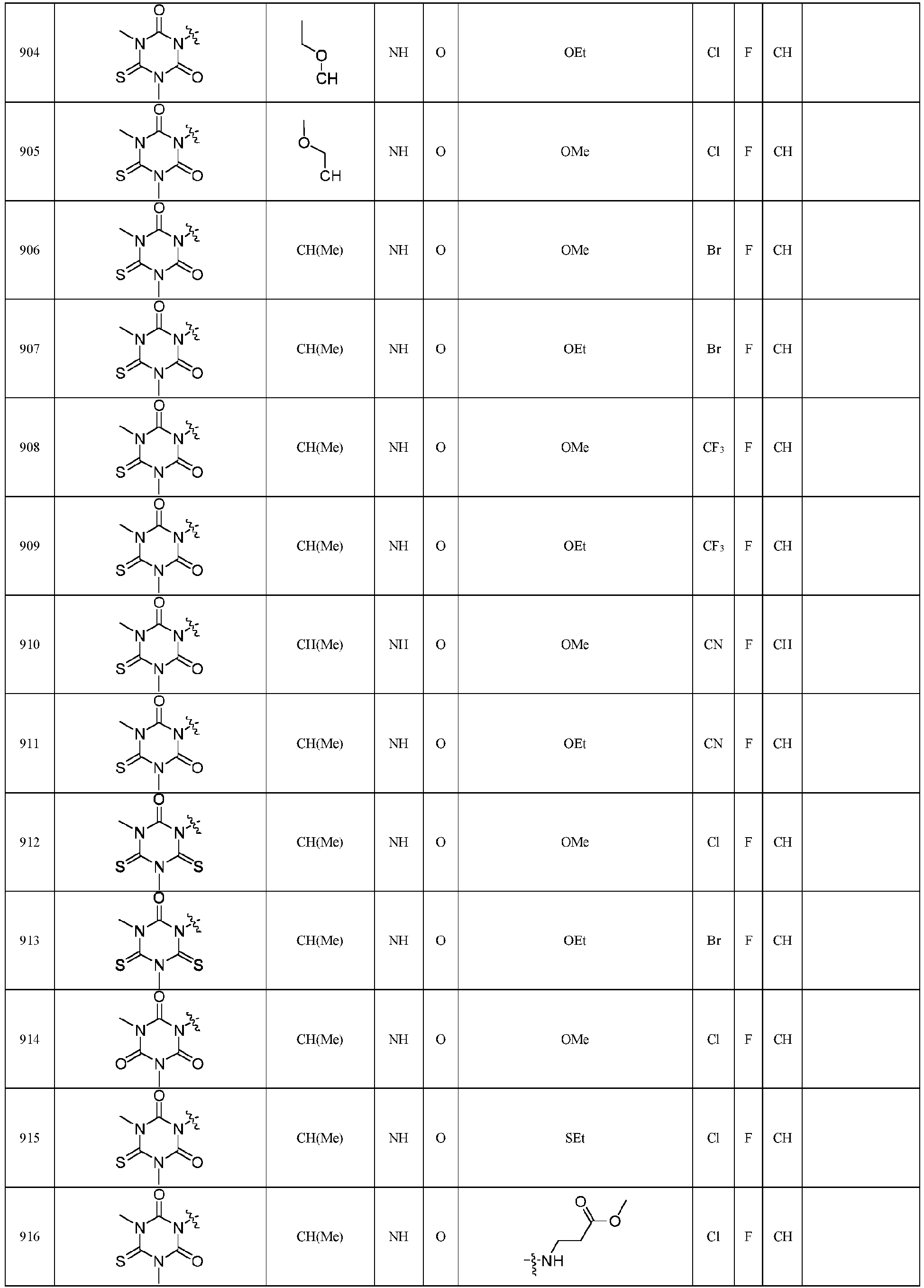

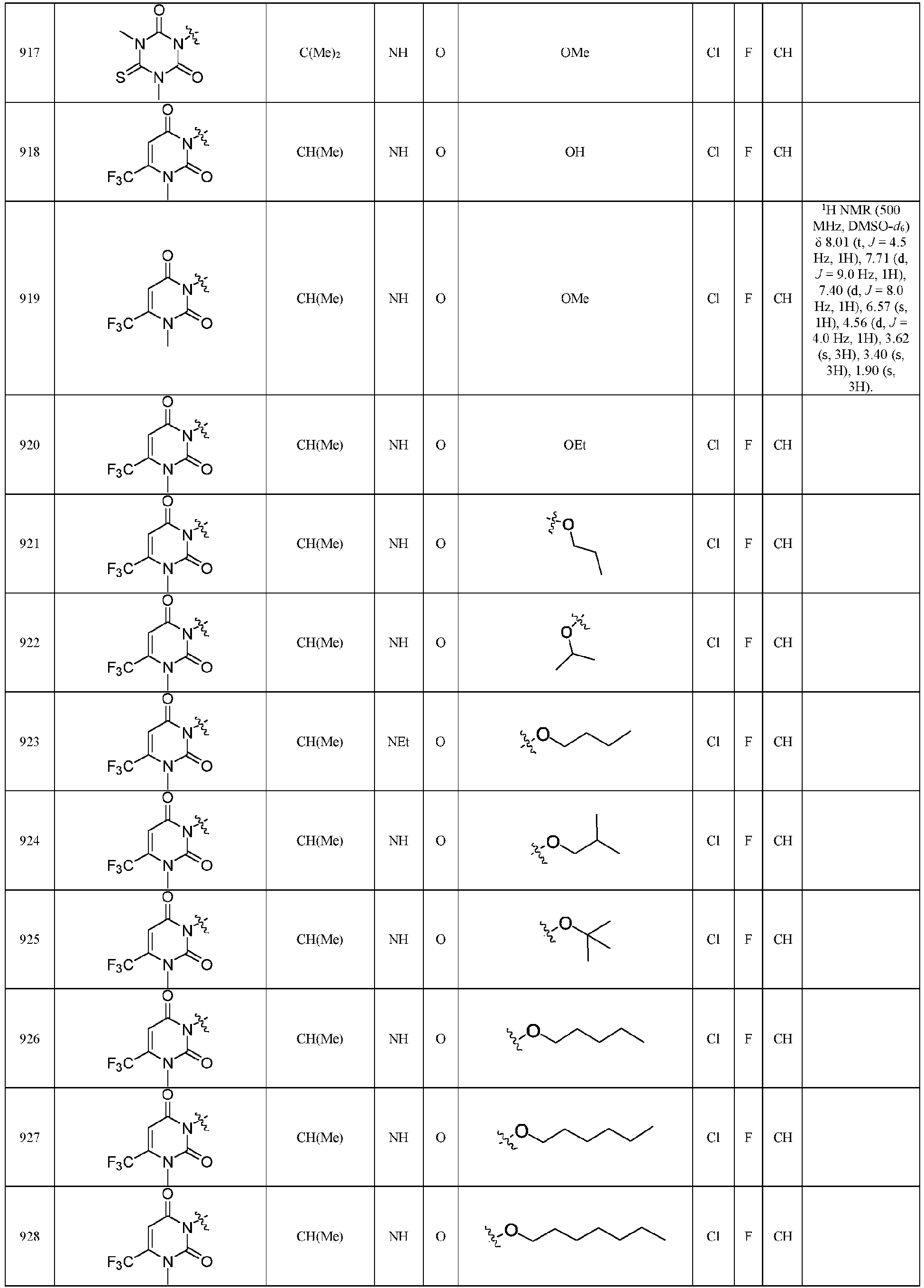

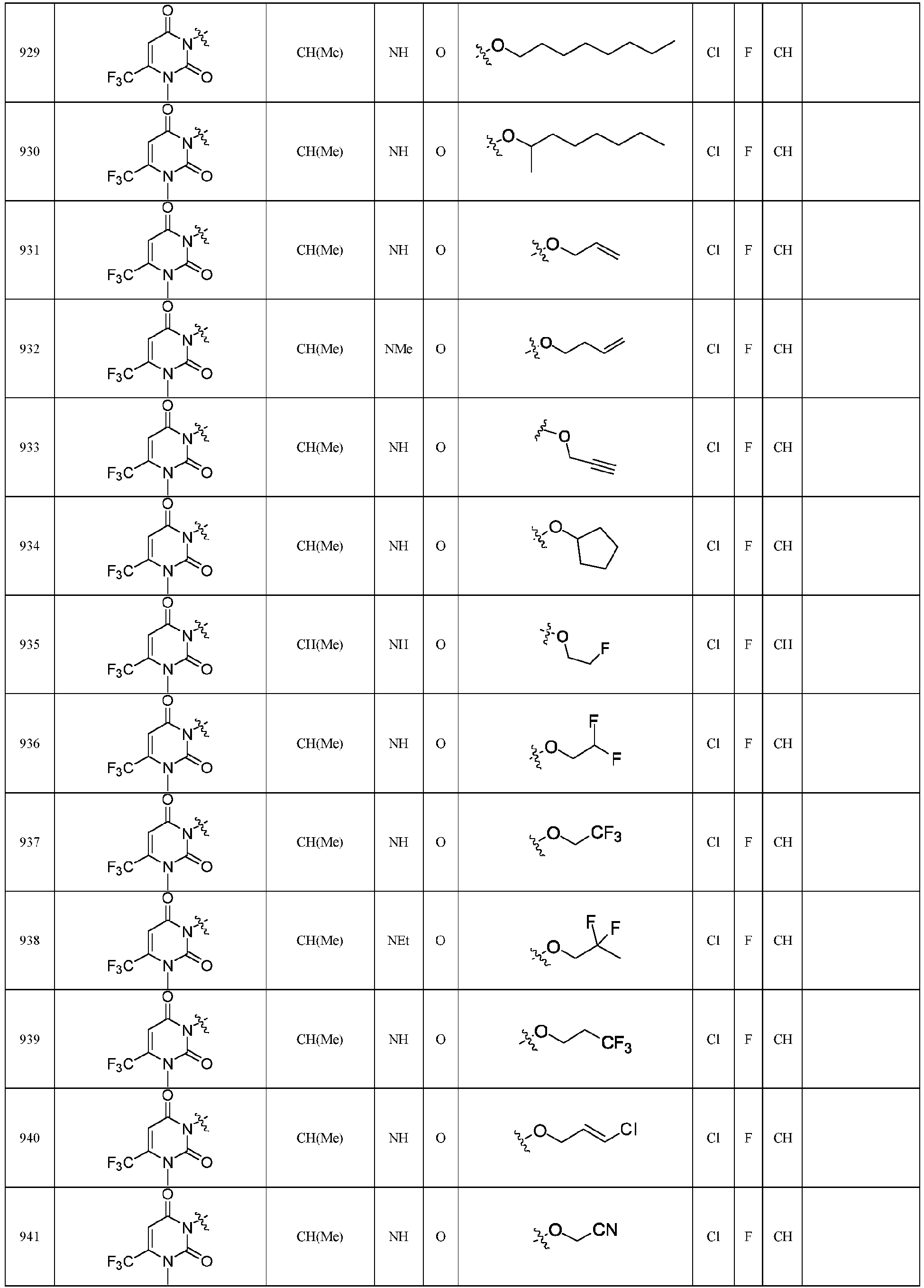

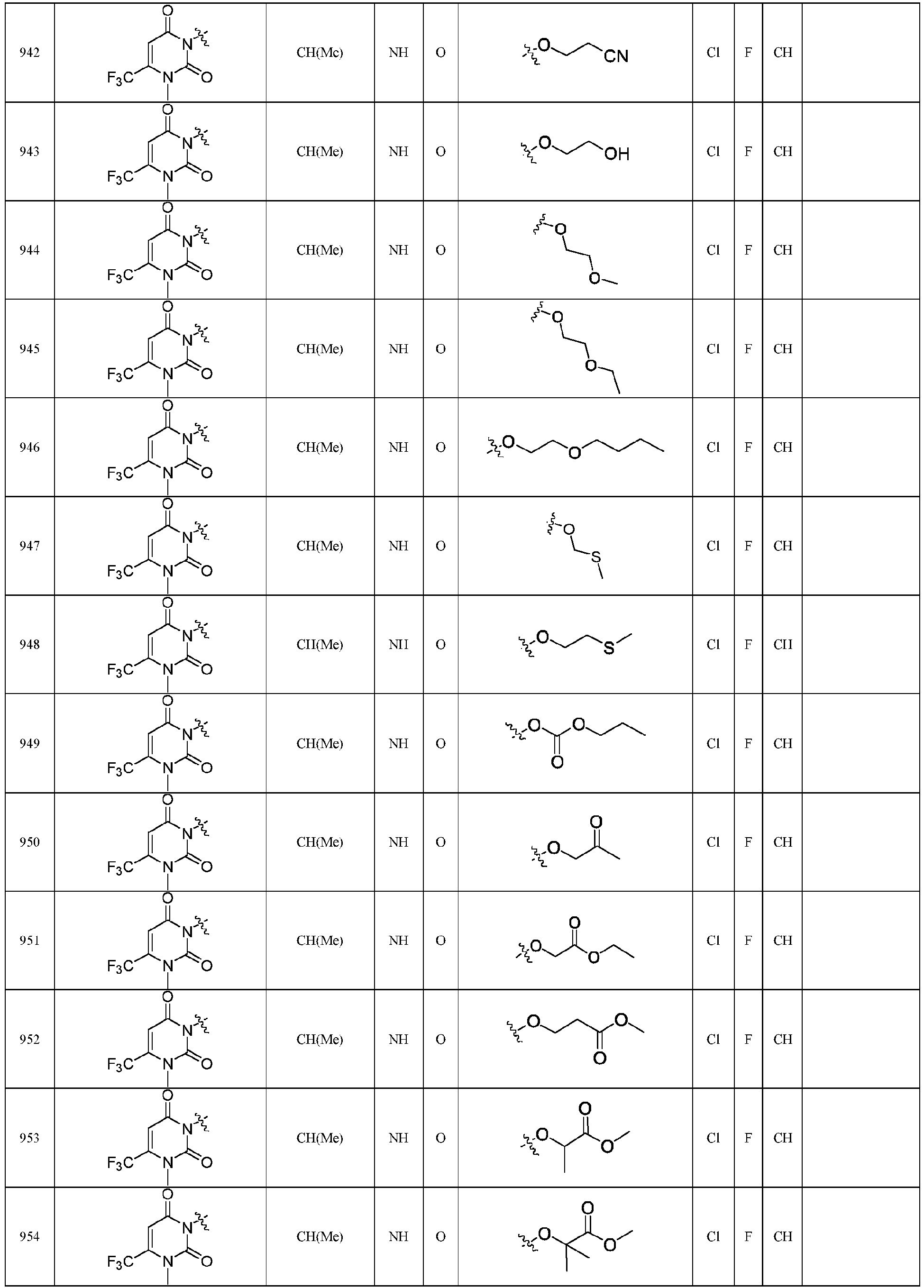

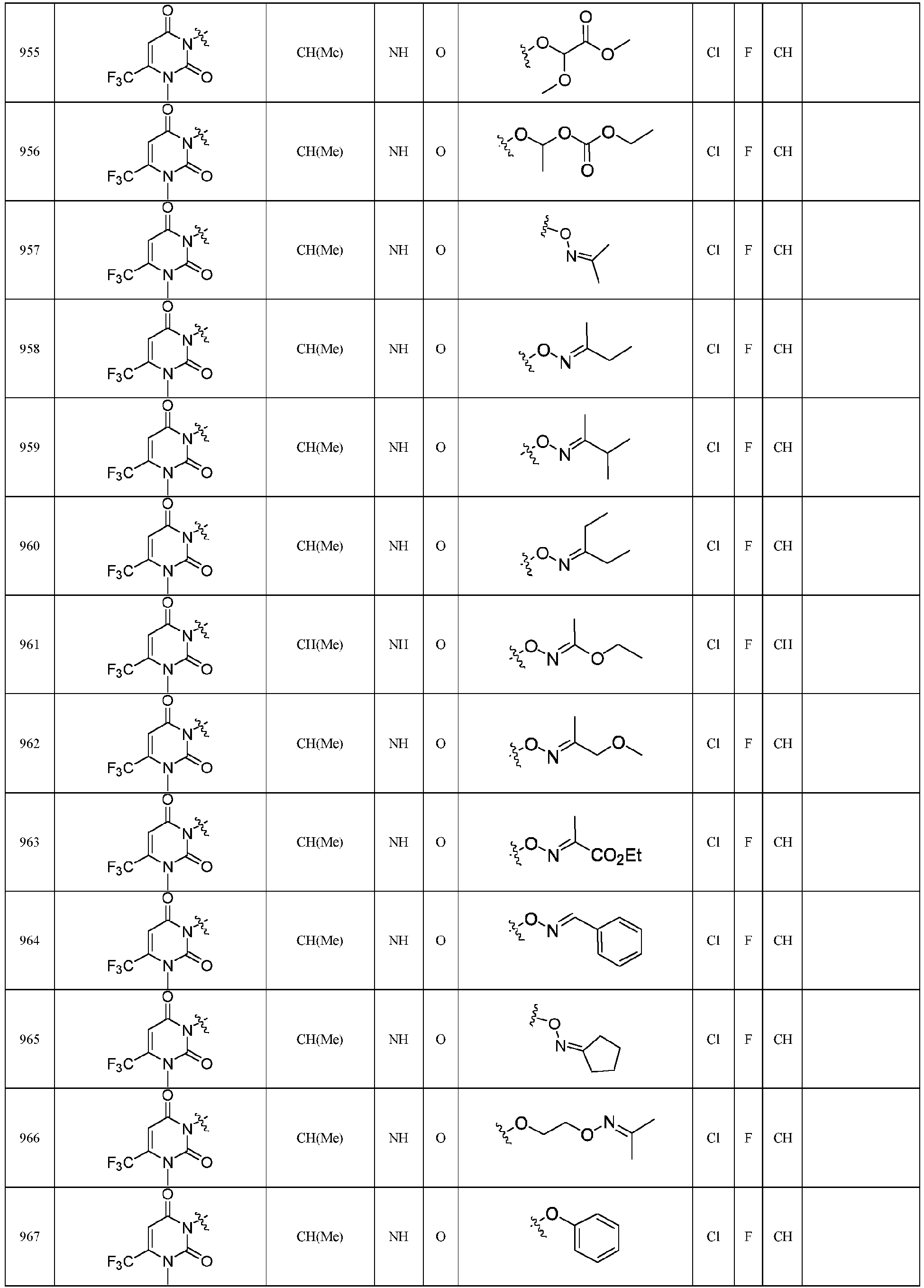

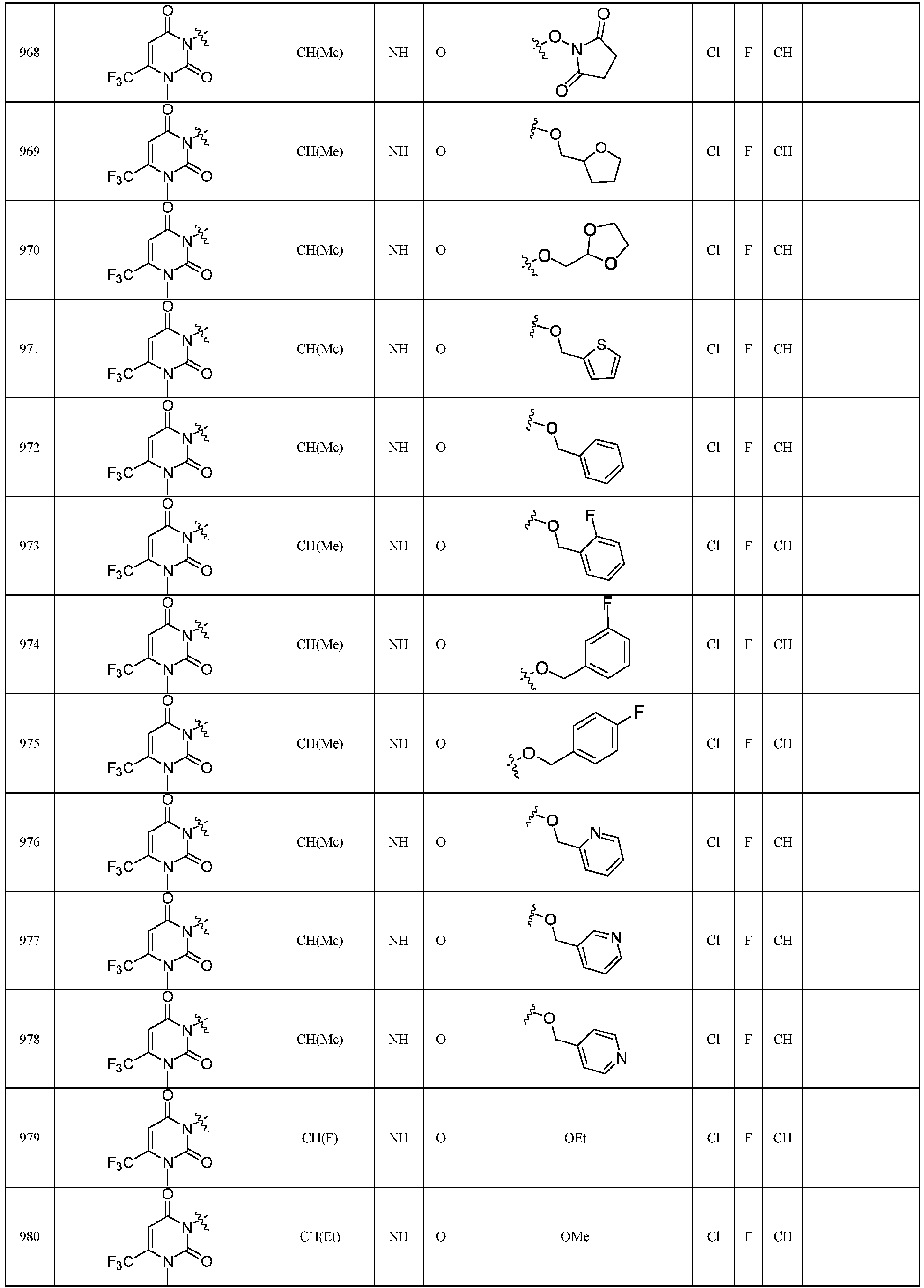

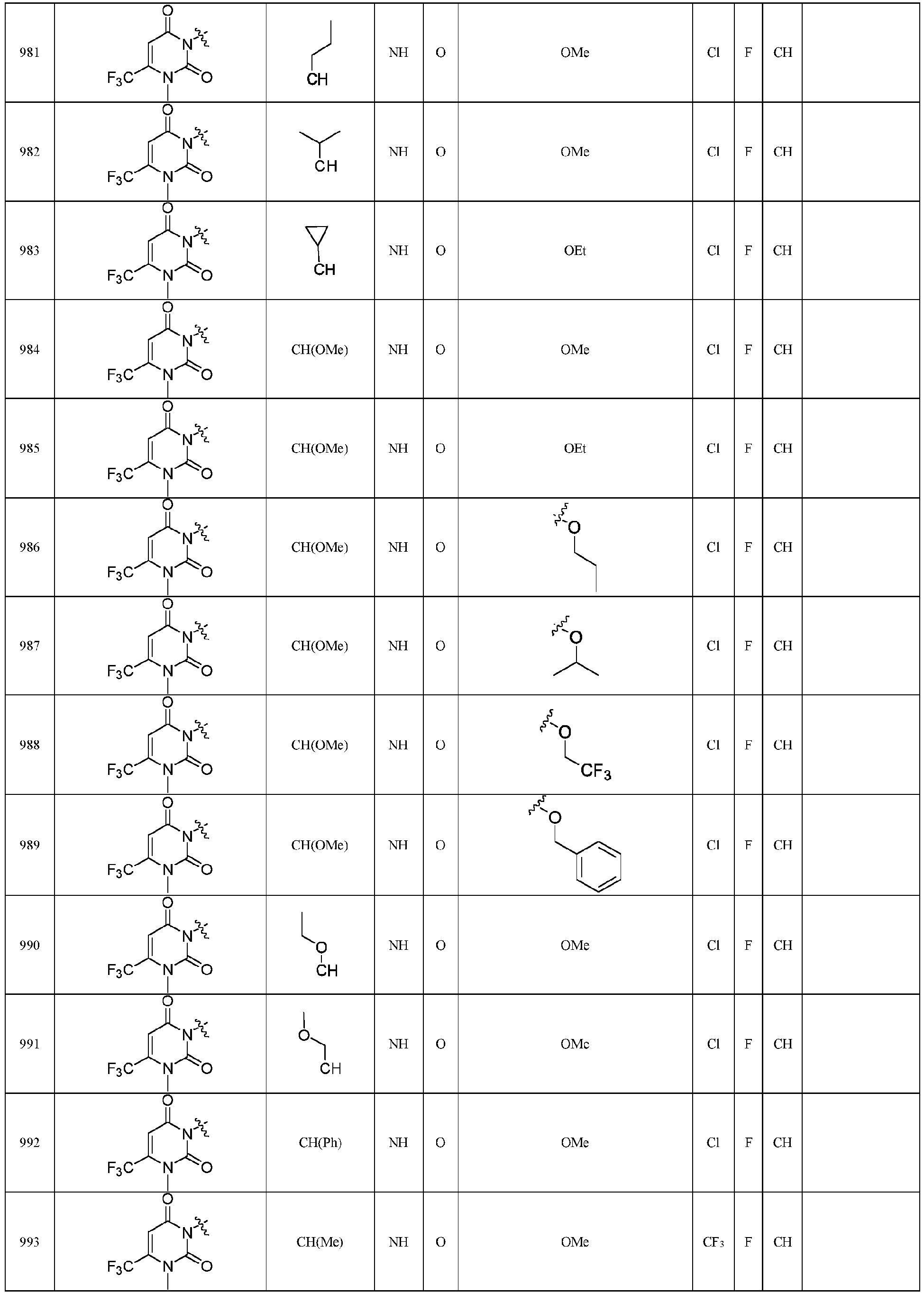

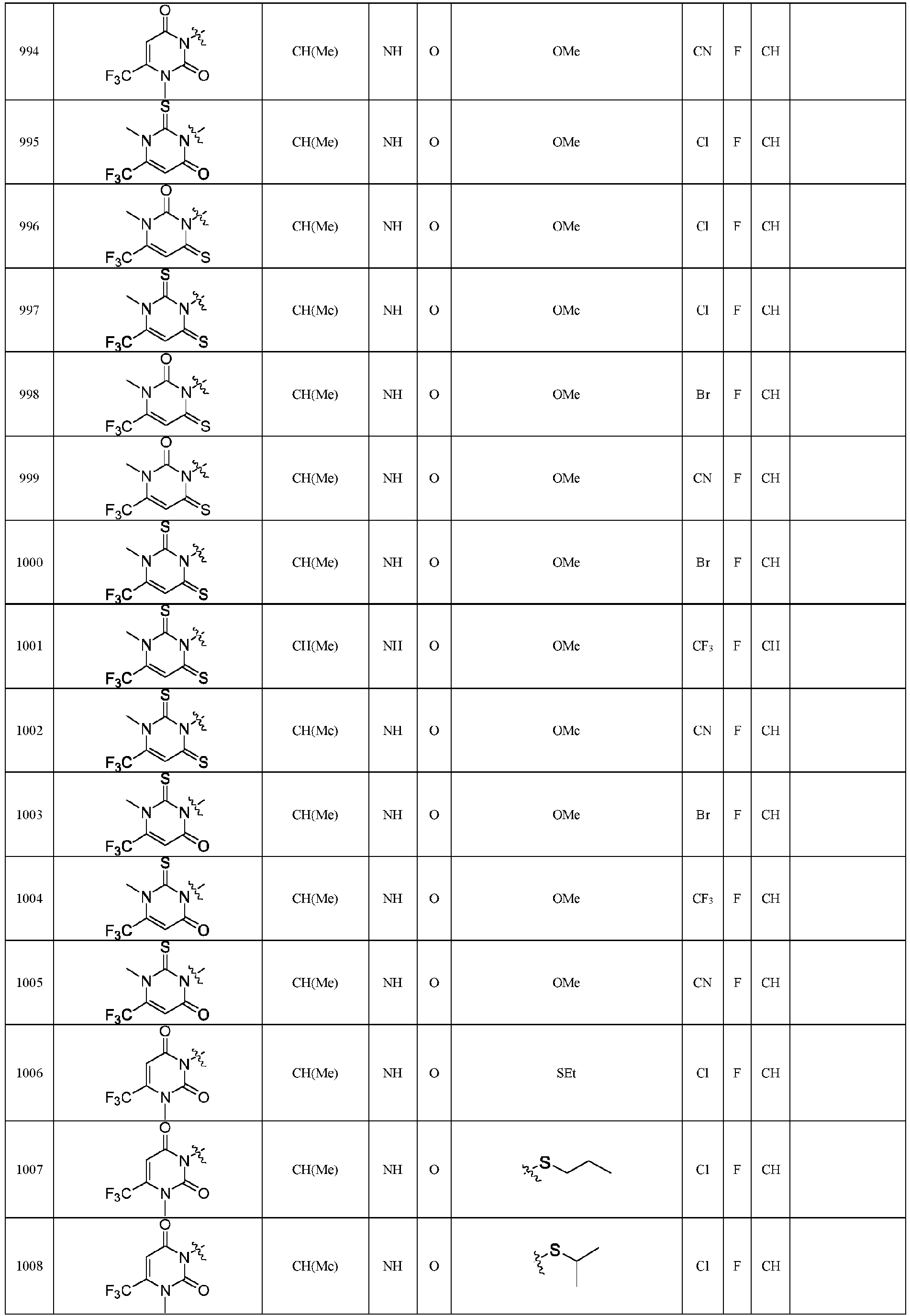

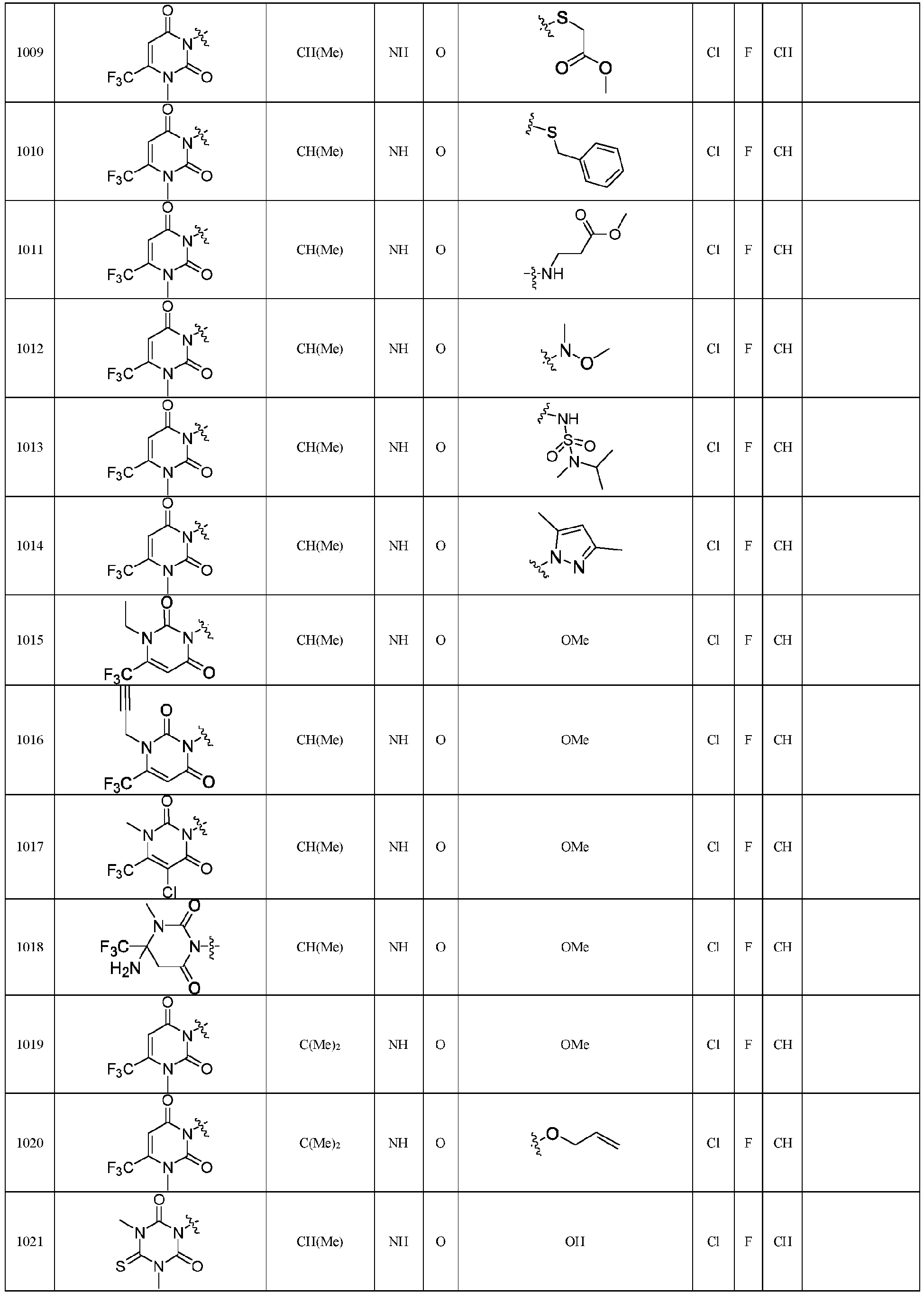

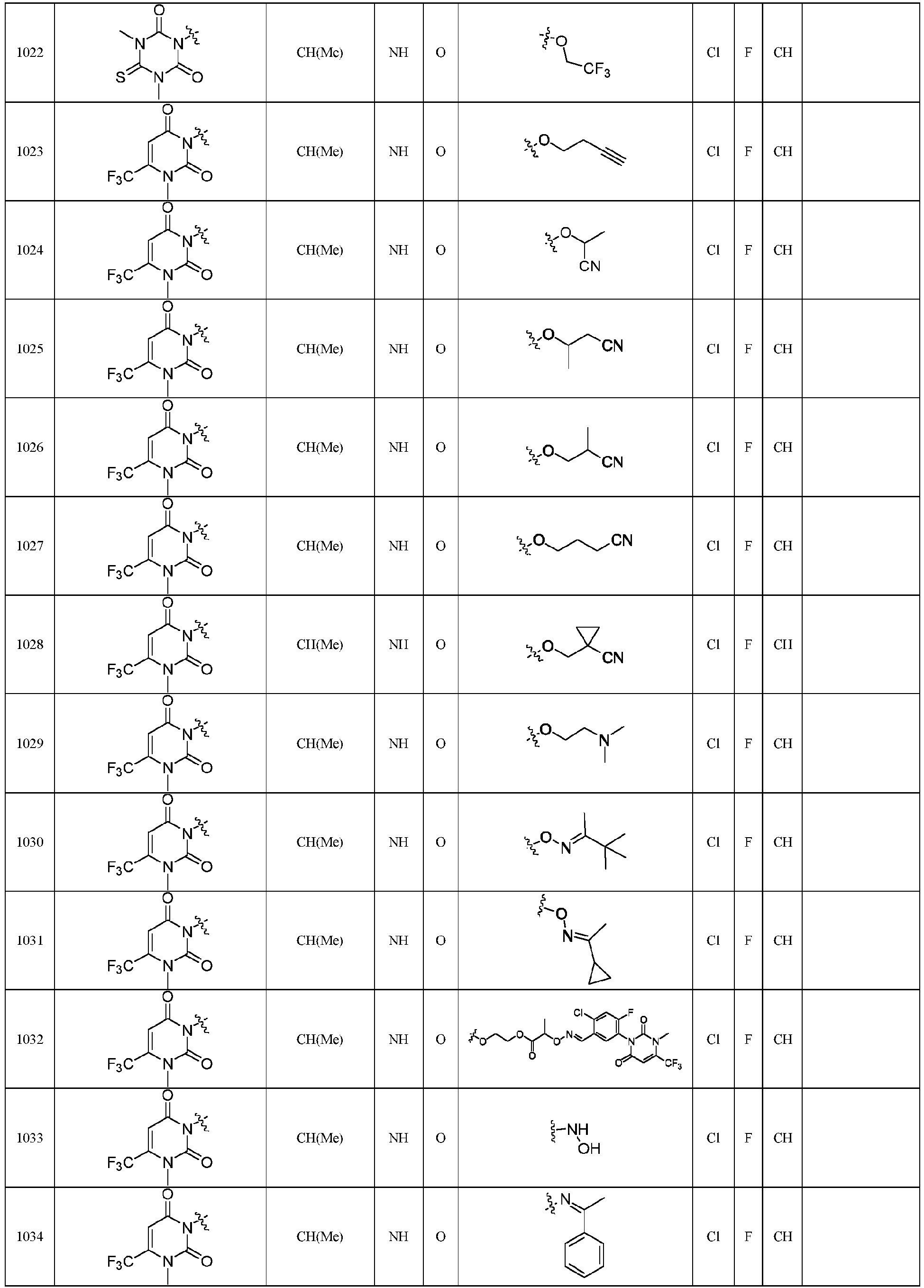

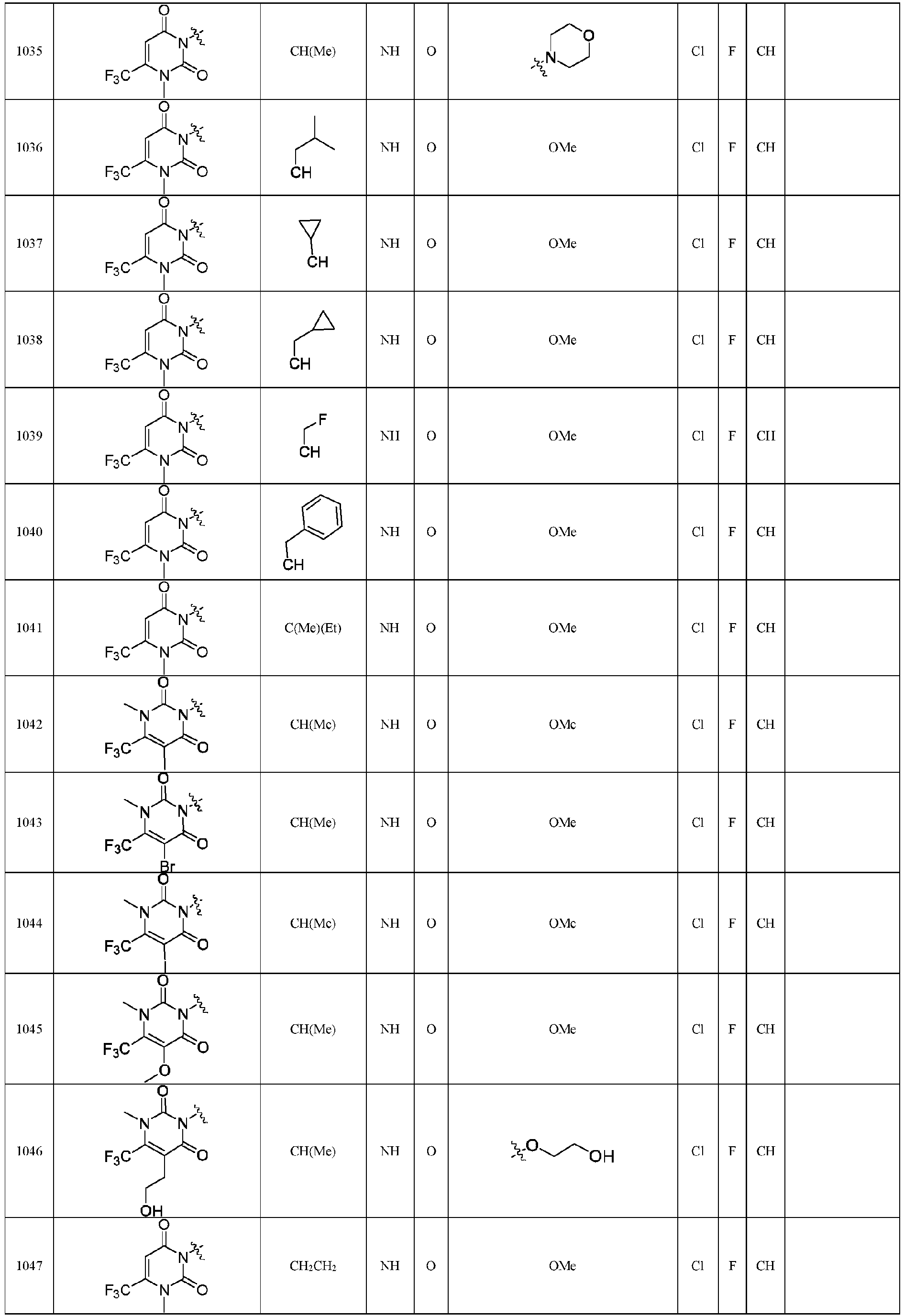

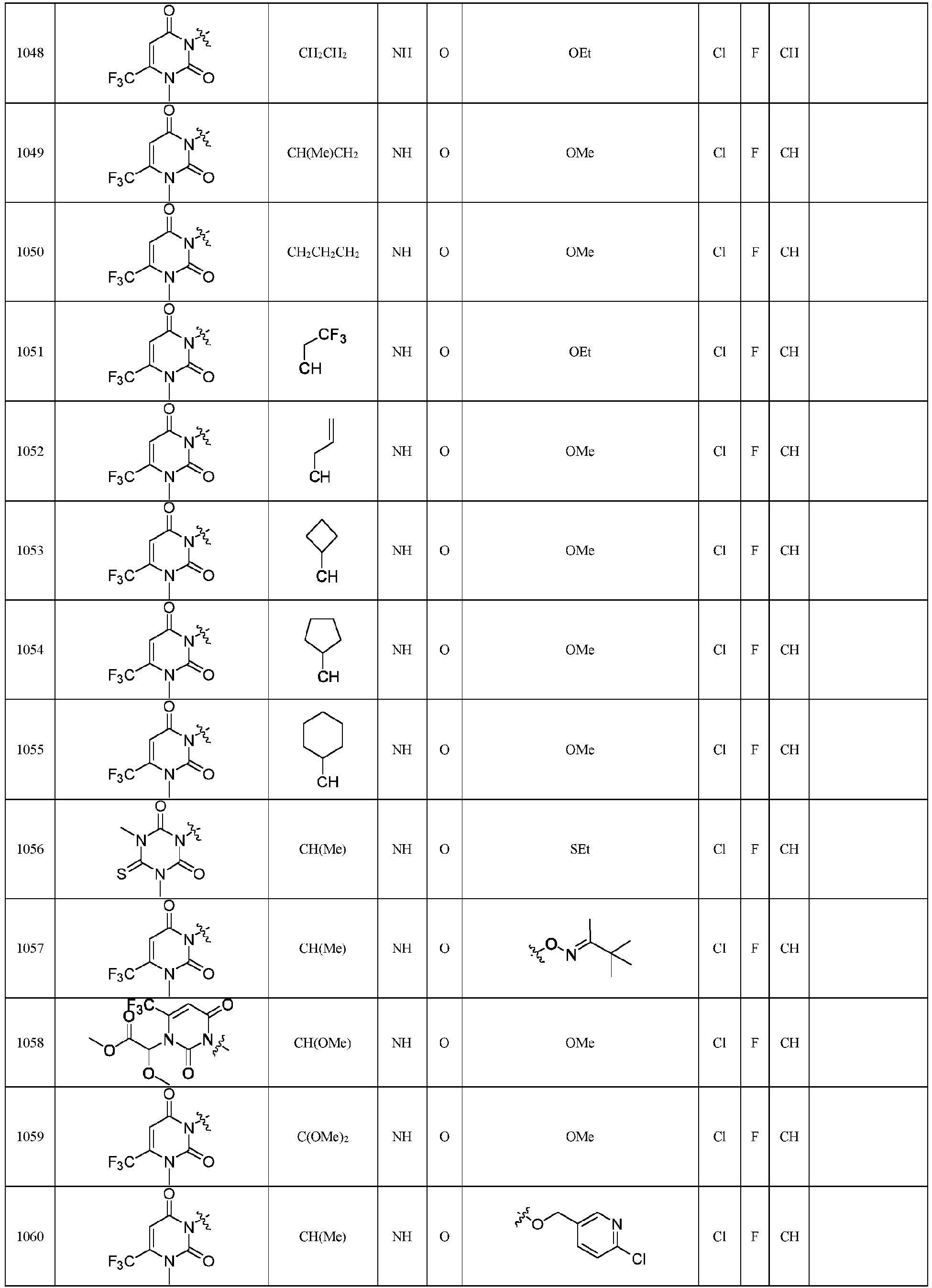

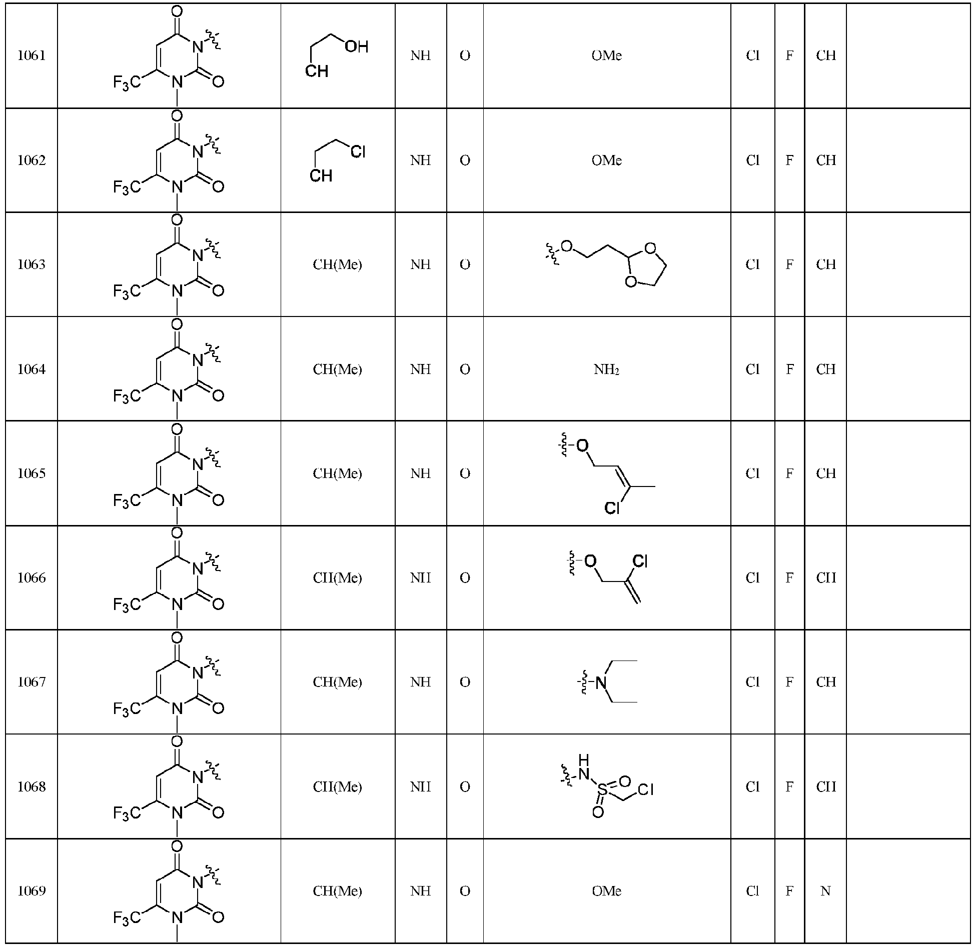

Table A is constructed in the same way as that of Table 1 above, except for replacing the racemate compounds having a chiral center ( 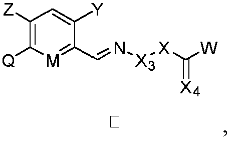 wherein, X represents -C^{∗}X₁X₂-(alkyl)ₙ-, -alkyl-C^{∗}X₁X₂-(alkyl)ₙ-, that is, X₁, X₂ are not the same, the carbon atom at ^{∗} is the chiral center) (that is, compounds 1-188, 193-432, 438-439, 441-469, 471-478, 481-484, 486-493, 495-545, 547-641, 644-669, 672, 674-681, 683-727, 729-829, 832-857, 860, 862-869, 871-916, 918-1018, 1021-1046, 1049, 1051-1058 and 1060-1069) with the corresponding compounds in R configuration and deleting the compounds having no chiral center at the corresponding position, and in Table A, the entries in the column "No." are listed in sequence as "1(R)-188(R), 193(R)-432(R), 438(R)-439(R), 441(R)-469(R), 471(R)-478(R), 481(R)-484(R), 486(R)-493(R), 495(R)-545(R), 547(R)-641(R), 644(R)-669(R), 672(R), 674(R)-681(R), 683(R)-727(R), 729(R)-829(R), 832(R)-857(R), 860(R), 862(R)-869(R), 871(R)-916(R), 918(R)-1018(R), 1021(R)-1046(R), 1049(R), 1051(R)-1058(R) and 1060(R)-1069(R)". For example, "1(R)" corresponds to R configuration of compound "1" in Table 1, "194(R)" corresponds to R configuration of compound "194" in Table 1.

The method for preparing the compound of the invention will be explained in detail in the following program and embodiment. The material is commercial available or prepared through known method reported in the literature or shown in the route. Those skilled in the art should understand that the compound of the invention can also be synthesized by other synthetic route. Although the detailed material and reaction condition in the synthetic route have been explicated in the following text, it is still easy to be replaced by other similar material and condition. Isomer of the compound, for example, that produced with the variation of the preparation method of the present invention is included in the scope of the present invention. In addition, the following preparation method can be further modified according to the disclosures of the present invention by using common chemical method known to those skilled in the art, for example, protection of suitable group in the process of the reaction, etc.

The following method of application can be used to improve further understanding of the preparation method of the present invention. The specific material, class and condition have been determined to be further explication of the present invention, not to be any limit of the reasonable scope thereof. Reagents of the following synthetic compound showed in the table can either be purchased from the market or easily prepared by those skilled in the art.

Examples of representative compounds are as follows, the synthesis methods of other compounds are similar, and will not be described in detail here.

### 1. Synthesis of compound 1

1) 1-1 (10 g, 49.1 mmol, 1.0 eq), Fe powder (8.23 g, 147.4 mmol, 3.0 eq), NH₄Cl (5.26 g, 98.3 mmol, 2.0 eq) and water (50 ml) were added to 500 ml of EtOH solution in sequence. Then, the reaction solution was reacted at 80°C for 1 hour. LCMS test showed the disappearance of raw materials. After filtration, the solution was concentrated to remove ethanol and then extracted with ethyl acetate. The organic phase was washed with saturated brine (100ml^{∗}1), and then concentrated to obtain 1-2 (12 g, crude product) (black solid). 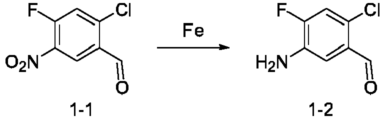
2) 1-2 (12 g, 69.1 mmol, 1.0 eq, crude product) was added to 100 ml of toluene solution. Then, 1-3 (10.8 g, 69.1 mmol, 1.0 eq) was added to the reaction solution at 100°C. After the addition was completed, the reaction solution was reacted at 100°C for 1 hour. LCMS test showed the disappearance of raw materials, and the generation of a product. The reaction solution was concentrated to remove toluene. The resulting crude product was separated by column chromatography to obtain 1-4 (5g) (yellow solid). 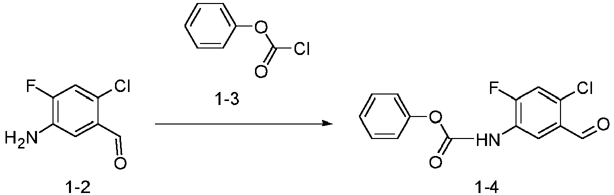
3) 1-5 (3.8 g, 17.0 mmol, 1.0 eq) and AcONa (0.7 g, 8.5 mmol, 0.5 eq) were added to 50 ml of DMF solution. Then, 1-4 (5 g, 17.0 mmol, 1.0 eq) was added to the reaction solution at 60°C. After the addition was completed, the reaction solution was reacted at 60°C for 1 hour. LCMS test showed the disappearance of raw materials, and the occurrence of new peak. After the addition of water (50ml), the reaction solution was extracted with ethyl acetate. The organic phase was washed with saturated brine (100ml^{∗}1), and then concentrated. The resulting crude product was separated by column chromatography to obtain 1-6 (4.0 g, 71.3% yield) (white solid). 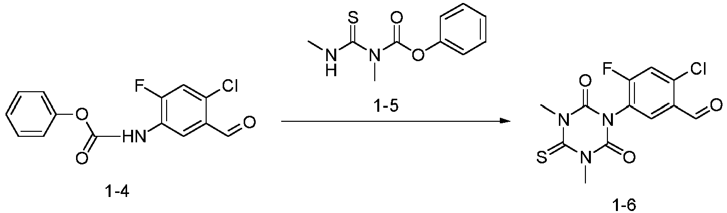
4) 1-6 (4 g, 12.1 mmol, 1.0 eq) was added to 40 ml of EtOH, and then NH₂OHHCl (0.93 g, 13.3 mmol, 1.1 eq) aqueous solution (6 ml) was added dropwise to the reaction solution at 0°C. After the addition was completed, the reaction solution was stirred at 0°C for 2 hours. LCMS test showed that the raw materials were almost consumed, and one major new peak occurred. The reaction solution was concentrated to remove a part of ethanol and then poured into 10 ml of water, causing a solid to precipitate out. After filtration, the filter cake was washed with water and dried to obtain 1-7 (7 g, 71.7% yield) (white solid). 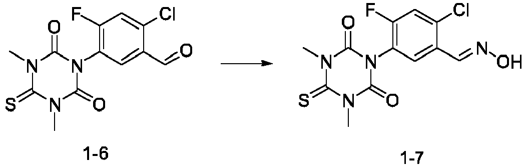
5) 1-7 (0.2 g, 0.58 mmol, 1.0 eq), a (0.14 g, 1.1 mmol, 2 eq) and K₂CO₃ (0.24 g, 1.74 mmol, 3 eq) were added to 5 mL of DMF solution in sequence. Then, the reaction solution was reacted at room temperature for 4 hours. LCMS test showed the disappearance of raw materials, and there were all product peaks. After the addition of water (10ml), the reaction solution was extracted with ethyl acetate. The organic phase was washed with saturated brine (10ml^{∗}1), and then concentrated. The resulting crude product was separated by column chromatography to obtain 1 (0.15 g, 60 % yield) (white solid).

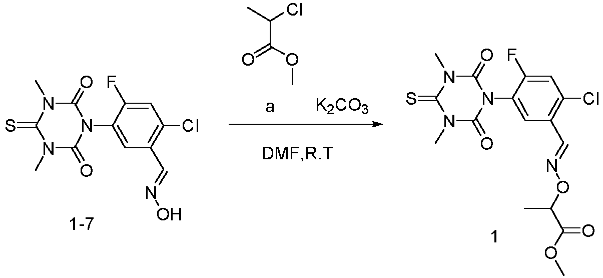

### 2. Synthesis of compound 1(R) configuration

1-7 (0.2 g, 0.58 mmol, 1.0 eq), b (0.14 g, 1.1 mmol, 2 eq), K₂CO₃ (0.24 g, 1.74 mmol, 3 eq) were added to 5 mL of DMF solution in sequence. Then, the reaction solution was reacted at room temperature for 4 hours. LCMS test showed the disappearance of raw materials, and there were all product peaks. After the addition of water (10ml), the reaction solution was extracted with ethyl acetate. The organic phase was washed with saturated brine (10ml^{∗}1), and then concentrated. The resulting crude product was separated by column chromatography to obtain 1(R) (0.15 g, 60% yield, R/S=98/2) (white solid).

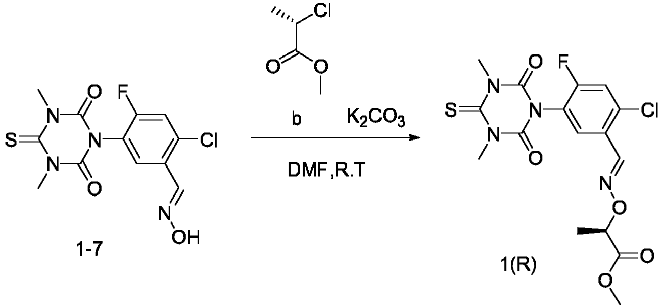

### 3. Synthesis of compound 194(R)

1) 1-1 (20 g, 98.3 mmol, 1.0 eq) was added to 200 ml of EtOH, and then NH₂OHHCl (7.5 g, 108.1 mmol, 1.1 eq) aqueous solution (30 ml) was added dropwise to the reaction solution at 0°C. After the addition was completed, the reaction solution was stirred at 0°C for 3 hours. LCMS test showed that the raw materials were almost consumed and one major new peak occurred. The reaction solution was concentrated to remove a part of ethanol and then poured into 100 ml of water, causing a solid to precipitate out. After filtration, the filter cake was washed with water and dried to obtain 194-1 (20 g, 93% yield) (white solid). 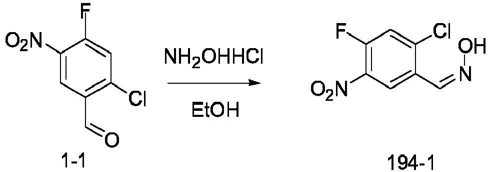
2) 194-1 (5 g, 22.9 mmol, 1.0 eq), Fe powder (3.8 g, 68.6 mmol, 3 eq), NH₄Cl (2.5 g, 45.8 mmol, 2 eq) and water (10ml) were added to 50 ml of EtOH in sequence. Then, the reaction solution was reacted at 80°C for 1 hour. LCMS test showed the occurrence of product peak. The reaction solution was filtered with celite and then concentrated to remove ethanol. After the addition of water (20ml), the reaction solution was extracted with ethyl acetate and then concentrated to obtain a black crude product. The crude product was separated and purified by column chromatography to obtain 194-2 (2 g, 46.4% yield) (gray solid). 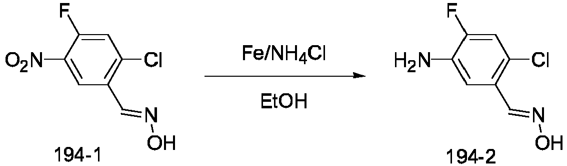
3) 194-2 (1 g, 5.3 mmol, 1.0 eq) and c (1.1 g, 5.3 mmol, 1.0 eq) were added to 20 ml of acetic acid, and the reaction solution was reacted at 110°C for 1 hour. LCMS test showed that the reaction of raw materials was basically completed, and there was one major product peak. The reaction solution was concentrated to remove the solvent. The resulting crude product was separated by column chromatography to obtain 194-3 (1.5 g, 80.5% yield) (white solid). 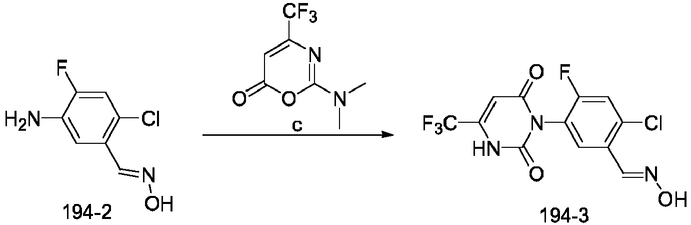
4) 194-3 (0.4 g, 1.1 mmol, 1.5 eq), b (0.18 g, 1.5 mmol, 1.3 eq) and K₂CO₃ (0.2 g, 1.5 mmol, 1.3 eq) were added to 8 ml of DMF in sequence. Then, the reaction solution was reacted at 25°C for 4 hours. LCMS test showed the generation of a product. After the addition of water (10ml), the reaction solution was extracted with ethyl acetate. The organic phase was washed with saturated brine (10ml^{∗}1), and then concentrated. The resulting crude product was separated by column chromatography to obtain 194-4 (0.3 g, 60.2% yield) (white solid). 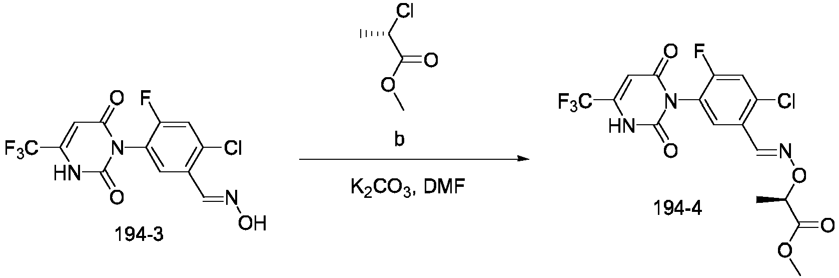
5) 194-4 (0.3 g, 0.69 mmol, 1.0 eq), MeI (0.13 g, 0.9 mmol, 1.3 eq) and K₂CO₃ (0.12 g, 0.9 mmol, 1.3 eq) were added to 6 ml of DMF in sequence. Then, the reaction solution was reacted at 25°C for 2 hours. LCMS test showed the generation of a product. After the addition of water (10ml), the reaction solution was extracted with ethyl acetate. The organic phase was washed with saturated brine (10ml^{∗}1), and then concentrated. The resulting crude product was separated by column chromatography to obtain 194(R) (0.2 g, 64.6% yield, R/S=99/1) (white solid).

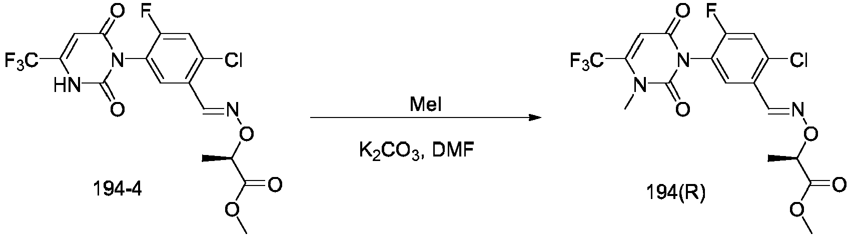

### 4. Synthesis of compound 504

(1) 504-1 (2 g, 1.0 eq), DessMartin (4.8 g, 2 eq) were added to 50 ml of DCM solution. Then, the reaction solution was reacted at room temperature for 2 hours. LCMS test showed the completion of reaction. After the addition of NaHCO₃ aqueous solution (100ml), the reaction solution was extracted with DCM. The organic phase was washed with saturated brine (100ml^{∗}2), and then concentrated. The resulting crude product was separated by column chromatography to obtain 504-2 (1.6 g, 82% yield) (white solid). 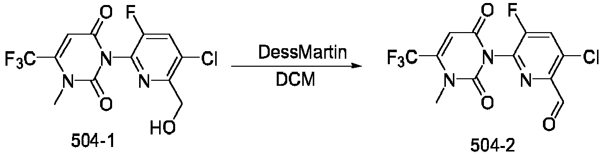
(2) 504-2 (1.6 g, 1.0 eq), water (10 ml), hydroxylamine hydrochloride (0.63 g, 2 eq) were added to 30 ml of ethanol solution. Then, the reaction solution was reacted at room temperature for 2 hours. LCMS test showed the completion of reaction. The reaction solution was concentrated. The resulting crude product was separated by column chromatography to obtain 504-3 (1.1 g, 69% yield) (white oil). 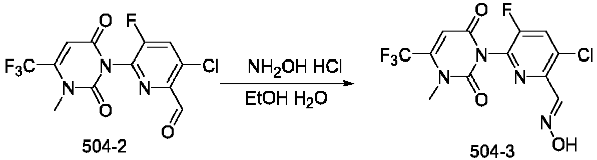
(3) 504-3 (0.3 g, 1.0 eq) and K₂CO₃ (170 mg, 1.5 eq) were added to 10 ml of DMF, then a (150 mg, 1.5 eq) was added to the reaction solution at 25°C, followed by reacting at 25°C for 8 hours. LCMS test showed the generation of a product. After the addition of water (10ml), the reaction solution was extracted with ethyl acetate. The organic phase was washed with saturated brine (20ml^{∗}1), and then concentrated. The resulting crude product was separated by column chromatography to obtain 504 (0.2 g, 54% yield) (white solid).

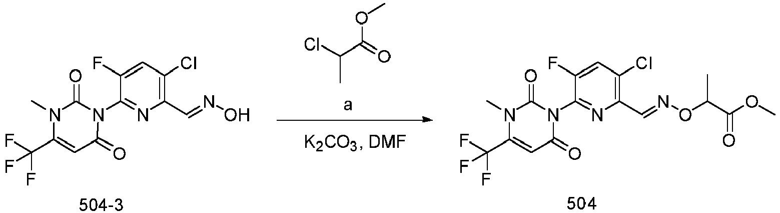

### 5. Synthesis of compound 919

(1) 919-1 (0.3 g, 1.0 eq) and NH₂NH₂H₂O (0.5 g, 85% aqueous solution, 10 eq) were added to 10 ml of THF. Then, the reaction solution was stirred at 60°C for 3 hours. LCMS test showed the generation of a product. The reaction solution was concentrated. The resulting crude product was separated by column chromatography to obtain 919-2 (0.15 g, 48%yield) (white solid). 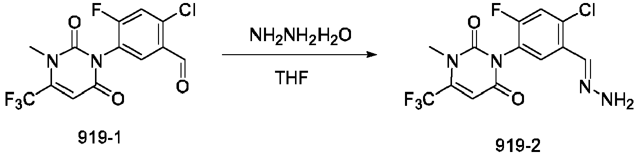
(2) 919-2 (0.15 g, 1.0 eq) and K₂CO₃ (74 mg, 1.3 eq) were added to 6 ml of DMF, then a (55 mg, 1.1 eq) was added to the reaction solution at 25°C, followed by reacting at 25°C for 8 hours. LCMS test showed the generation of a product. After the addition of water (10ml), the reaction solution was extracted with ethyl acetate. The organic phase was washed with saturated brine (20ml^{∗}1), and then concentrated. The resulting crude product was separated by column chromatography to obtain 919 (50 mg, 27% yield) (white solid).

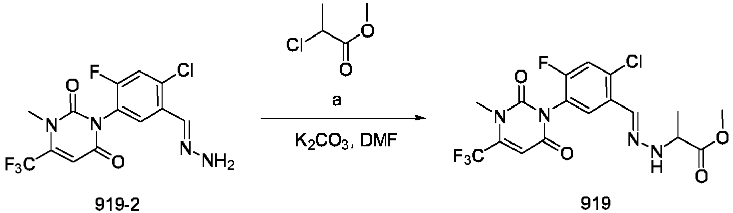

### Biological activity evaluation:

The activity level criteria for plant damage (i.e., growth control rate) are as follows:
Level 5: growth control rate is above 85%;
Level 4: growth control rate is greater than or equal to 60% and less than 85%;
Level 3: growth control rate is greater than or equal to 40% and less than 60%;
Level 2: growth control rate is greater than or equal to 20% and less than 40%;
Level 1: growth control rate is greater than or equal to 5% and less than 20%;
Level 0: growth control rate is less than 5%.

The above growth control rates are fresh weight control rates.

### Experiment on weeding effect in post-emergence stage:

Monocotyledonous and dicotyledonous weed seeds (*Descurainia sophia, Capsella bursa-pastoris, Abutilon theophrasti, Galium aparine, Stellaria media, Lithospermum arvense, rorippa indica, Alopecurus aequalis, Alopecurus japonicus, Eleusine indica, Beckmannia syzigachne, Sclerochloa dura, Conyza Canadensis, Phleum paniculatum, Veronica didyma* Tenore, *Bromus japonicus, Aegilops tauschii, Phalaris arundinacea, Amaranthus retroflexus, Chenopodiaceae, Commelina communis, Sonchus arvensis, Convolvulus arvensis, Cirsium setosum, Bidens tripartita L., Solanum nigrum, Acalypha australis, Digitaria sanguinalis, Echinochloa crusgalli, Setaria viridis, Setaria glauca, Leptochloa chinensis, Monochoria vaginalis, Sagittaria trifolia, Scirpus juncoides, Cyperus rotundus, Cyperus iria, Cyperus difformis*, *Fimbristylis, Portulaca oleracea, Xanthium sibiricum, Pharbitis nil,* etc.) and major crop seeds (wheat, corn, rice, soybean, cotton, oilseed rape, millet, sorghum, potato, sesame, ricinus, etc.) were placed in plastic pots filled with soil, then covered with 0.5-2 cm of soil, allowed to grow in a good greenhouse environment. After 2 weeks of sowing, the test plants were treated in the 2-3 leaf stage. The tested compounds of the present invention were respectively dissolved in acetone, then added with Tween 80 and 1.5 liter/ha of emulsifiable concentrate of methyl oleate as synergist, diluted with a certain amount of water to obtain a solution with a certain concentration, and sprayed with a spray tower onto the plants. After the application, the plants were cultured for 3 weeks in the greenhouse, and then the experimental results of the weeding were counted. The doses of the used compounds were 500, 250, 125, 60, 30, 15, 7.5g/ha, and the averages were obtained by repeating for three times. Representative data are listed in Table 2.

**Table 2. Results on weeding effect in post-emergence stage**

| Compound NO. | *Digitaria sanguinalis* | *Echinochloa crusgalli* | *Setaria viridis* | *Eleusine indica* | *Alopecurus japonicus* | *Abutilon theophrasti* | Dose |
|---|---|---|---|---|---|---|---|
| 1 | 5 | 5 | 5 | 5 | 5 | 5 | 15g/ha |
| 1(R) | 5 | 5 | 5 | 5 | 5 | 5 | 15g/ha |
| 2 | 5 | 5 | 5 | 5 | 5 | 5 | 15g/ha |
| 2(R) | 5 | 5 | 5 | 5 | 5 | 5 | 15g/ha |
| 3 | 5 | 5 | 5 | 5 | 4 | 5 | 15g/ha |
| 3(R) | 5 | 5 | 5 | 5 | 4 | 5 | 15g/ha |
| 4 | 5 | 5 | 5 | 5 | 5 | 5 | 15g/ha |
| 4(R) | 5 | 5 | 5 | 5 | 5 | 5 | 15g/ha |
| 5 | 5 | 5 | 5 | 5 | 5 | 5 | 15g/ha |
| 5(R) | 5 | 5 | 5 | 5 | 5 | 5 | 15g/ha |
| 6 | 5 | 5 | 5 | 5 | 5 | 5 | 15g/ha |
| 6(R) | 5 | 5 | 5 | 5 | 5 | 5 | 15g/ha |
| 9 | 5 | 5 | 5 | 5 | 5 | 5 | 15g/ha |
| 9(R) | 5 | 5 | 5 | 5 | 5 | 5 | 15g/ha |
| 10 | 5 | 5 | 5 | 5 | 5 | 5 | 15g/ha |
| 10(R) | 5 | 5 | 5 | 5 | 5 | 5 | 15g/ha |
| 11 | 5 | 5 | 5 | 5 | 5 | 5 | 15g/ha |
| 11(R) | 5 | 5 | 5 | 5 | 5 | 5 | 15g/ha |
| 12 | 5 | 5 | 5 | 5 | 5 | 5 | 15g/ha |
| 12(R) | 5 | 5 | 5 | 5 | 5 | 5 | 15g/ha |
| 14 | 5 | 5 | 5 | 5 | 4 | 5 | 15g/ha |
| 14(R) | 5 | 5 | 5 | 5 | 4 | 5 | 15g/ha |
| 17 | 5 | 5 | 5 | 5 | 5 | 5 | 15g/ha |
| 17(R) | 5 | 5 | 5 | 5 | 5 | 5 | 15g/ha |
| 20 | 5 | 5 | 5 | 5 | 5 | 5 | 15g/ha |
| 20(R) | 5 | 5 | 5 | 5 | 5 | 5 | 15g/ha |
| 24 | 5 | 5 | 5 | 5 | 5 | 5 | 15g/ha |
| 24(R) | 5 | 5 | 5 | 5 | 5 | 5 | 15g/ha |
| 26 | 5 | 5 | 5 | 5 | 5 | 5 | 15g/ha |
| 26(R) | 5 | 5 | 5 | 5 | 5 | 5 | 15g/ha |
| 42 | 5 | 5 | 5 | 5 | 4 | 5 | 15g/ha |
| 42(R) | 5 | 5 | 5 | 5 | 4 | 5 | 15g/ha |
| 59 | 5 | 5 | 5 | 5 | N | 5 | 15g/ha |
| 59(R) | 5 | 5 | 5 | 5 | 4 | 5 | 15g/ha |
| 60 | 5 | 5 | 5 | 5 | 4 | 5 | 15g/ha |
| 60(R) | 5 | 5 | 5 | 5 | 4 | 5 | 15g/ha |
| 72 | 5 | 5 | 5 | 5 | 4 | 5 | 15g/ha |
| 72(R) | 5 | 5 | 5 | 5 | 5 | 5 | 15g/ha |
| 74 | 4 | 5 | 5 | 4 | N | 5 | 15g/ha |
| 74(R) | 4 | 5 | 5 | 5 | N | 5 | 15g/ha |
| 76 | 5 | 5 | 5 | 5 | 5 | 5 | 15g/ha |
| 76(R) | 5 | 5 | 5 | 5 | 5 | 5 | 15g/ha |
| 80 | 5 | 5 | 5 | 5 | 5 | 5 | 15g/ha |
| 80(R) | 5 | 5 | 5 | 5 | 5 | 5 | 15g/ha |
| 83 | 4 | 5 | 5 | 5 | 4 | 5 | 15g/ha |
| 83(R) | 4 | 5 | 5 | 5 | 4 | 5 | 15g/ha |
| 84 | 4 | 5 | 5 | 5 | 4 | 5 | 15g/ha |
| 84(R) | 4 | 5 | 5 | 5 | 4 | 5 | 15g/ha |
| 85 | 5 | 5 | 5 | 5 | N | 5 | 15g/ha |
| 85(R) | 5 | 5 | 5 | 5 | 4 | 5 | 15g/ha |
| 86 | 3 | 4 | 4 | 5 | 3 | 5 | 15g/ha |
| 86(R) | 3 | 4 | 4 | 5 | 3 | 5 | 15g/ha |
| 87 | 5 | 5 | 5 | 5 | 5 | 5 | 15g/ha |
| 87(R) | 5 | 5 | 5 | 5 | 5 | 5 | 15g/ha |
| 88 | 5 | 5 | 5 | 5 | 5 | 5 | 15g/ha |
| 88(R) | 5 | 5 | 5 | 5 | 5 | 5 | 15g/ha |
| 124 | 5 | 5 | 5 | 5 | 4 | 5 | 15g/ha |
| 124(R) | 5 | 5 | 5 | 5 | 5 | 5 | 15g/ha |
| 161 | 4 | 5 | 5 | 5 | 4 | 5 | 15g/ha |
| 161(R) | 4 | 5 | 5 | 5 | 4 | 5 | 15g/ha |
| 164 | 5 | 5 | 5 | 5 | 5 | 5 | 15g/ha |
| 164(R) | 5 | 5 | 5 | 5 | 5 | 5 | 15g/ha |
| 168 | 5 | 5 | 5 | 5 | 5 | 5 | 15g/ha |
| 168(R) | 5 | 5 | 5 | 5 | 5 | 5 | 15g/ha |
| 183 | 5 | 5 | 5 | 5 | 5 | 5 | 15g/ha |
| 183(R) | 5 | 5 | 5 | 5 | 5 | 5 | 15g/ha |
| 189 | 4 | 4 | 5 | 5 | 4 | 5 | 15g/ha |
| 193 | 5 | 5 | 5 | 5 | 5 | 5 | 15g/ha |
| 193(R) | 5 | 5 | 5 | 5 | 5 | 5 | 15g/ha |
| 194 | 5 | 5 | 5 | 5 | 5 | 5 | 15g/ha |
| 194(R) | 5 | 5 | 5 | 5 | 5 | 5 | 15g/ha |
| 194 | 5 | 5 | 5 | 5 | 4 | 5 | 7.5g/ha |
| 194(R) | 5 | 5 | 5 | 5 | 5 | 5 | 7.5g/ha |
| 196 | 5 | 5 | 5 | 5 | 5 | 5 | 15g/ha |
| 196(R) | 5 | 5 | 5 | 5 | 5 | 5 | 15g/ha |
| 198 | 5 | 5 | 5 | 5 | 5 | 5 | 15g/ha |
| 198(R) | 5 | 5 | 5 | 5 | 5 | 5 | 15g/ha |
| 199 | 5 | 5 | 5 | 5 | 5 | 5 | 15g/ha |
| 199(R) | 5 | 5 | 5 | 5 | 5 | 5 | 15g/ha |
| 200 | 5 | 5 | 5 | 5 | 5 | 5 | 15g/ha |
| 200(R) | 5 | 5 | 5 | 5 | 5 | 5 | 15g/ha |
| 201 | 5 | 5 | 5 | 5 | 5 | 5 | 15g/ha |
| 201(R) | 5 | 5 | 5 | 5 | 5 | 5 | 15g/ha |
| 202 | 5 | 5 | 5 | 5 | 5 | 5 | 15g/ha |
| 202(R) | 5 | 5 | 5 | 5 | 5 | 5 | 15g/ha |
| 203 | 5 | 5 | 5 | 5 | 5 | 5 | 15g/ha |
| 203(R) | 5 | 5 | 5 | 5 | 5 | 5 | 15g/ha |
| 204 | 5 | 5 | 5 | 5 | 5 | 5 | 15g/ha |
| 204(R) | 5 | 5 | 5 | 5 | 5 | 5 | 15g/ha |
| 205 | 5 | 5 | 5 | 5 | 5 | 5 | 15g/ha |
| 205(R) | 5 | 5 | 5 | 5 | 5 | 5 | 15g/ha |
| 206 | 5 | 5 | 5 | 5 | 5 | 5 | 15g/ha |
| 206(R) | 5 | 5 | 5 | 5 | 5 | 5 | 15g/ha |
| 207 | 5 | 5 | 5 | 5 | 5 | 5 | 15g/ha |
| 207(R) | 5 | 5 | 5 | 5 | 5 | 5 | 15g/ha |
| 208 | 5 | 5 | 5 | 5 | 5 | 5 | 15g/ha |
| 208(R) | 5 | 5 | 5 | 5 | 5 | 5 | 15g/ha |
| 209 | 5 | 5 | 5 | 5 | 5 | 5 | 15g/ha |
| 209(R) | 5 | 5 | 5 | 5 | 5 | 5 | 15g/ha |
| 212 | 5 | 5 | 5 | 5 | 5 | 5 | 15g/ha |
| 212(R) | 5 | 5 | 5 | 5 | 5 | 5 | 15g/ha |
| 214 | 5 | 5 | 5 | 5 | 5 | 5 | 15g/ha |
| 214(R) | 5 | 5 | 5 | 5 | 5 | 5 | 15g/ha |
| 216 | 5 | 5 | 5 | 5 | 4 | 5 | 15g/ha |
| 216(R) | 5 | 5 | 5 | 5 | 4 | 5 | 15g/ha |
| 217 | 5 | 5 | 5 | 5 | 5 | 5 | 15g/ha |
| 217(R) | 5 | 5 | 5 | 5 | 5 | 5 | 15g/ha |
| 218 | 5 | 5 | 5 | 5 | 5 | 5 | 15g/ha |
| 218(R) | 5 | 5 | 5 | 5 | 5 | 5 | 15g/ha |
| 220 | 5 | 5 | 5 | 5 | 5 | 5 | 15g/ha |
| 220(R) | 5 | 5 | 5 | 5 | 5 | 5 | 15g/ha |
| 221 | 5 | 5 | 5 | 5 | 4 | 5 | 15g/ha |
| 221(R) | 5 | 5 | 5 | 5 | 5 | 5 | 15g/ha |
| 225 | 5 | 5 | 5 | 5 | 4 | 5 | 15g/ha |
| 225(R) | 5 | 5 | 5 | 5 | 5 | 5 | 15g/ha |
| 226 | 5 | 5 | 5 | 5 | 5 | 5 | 15g/ha |
| 226(R) | 5 | 5 | 5 | 5 | 5 | 5 | 15g/ha |
| 227 | 5 | 5 | 5 | 5 | 5 | 5 | 15g/ha |
| 227(R) | 5 | 5 | 5 | 5 | 5 | 5 | 15g/ha |
| 228 | 5 | 5 | 5 | 5 | 5 | 5 | 15g/ha |
| 228(R) | 5 | 5 | 5 | 5 | 5 | 5 | 15g/ha |
| 230 | 5 | 5 | 5 | 5 | 5 | 5 | 15g/ha |
| 230(R) | 5 | 5 | 5 | 5 | 5 | 5 | 15g/ha |
| 231 | 5 | 5 | 5 | 5 | 5 | 5 | 15g/ha |
| 231(R) | 5 | 5 | 5 | 5 | 5 | 5 | 15g/ha |
| 232 | 5 | 5 | 5 | 5 | 5 | 5 | 15g/ha |
| 232(R) | 5 | 5 | 5 | 5 | 5 | 5 | 15g/ha |
| 233 | 5 | 5 | 5 | 5 | 5 | 5 | 15g/ha |
| 233(R) | 5 | 5 | 5 | 5 | 5 | 5 | 15g/ha |
| 234 | 5 | 5 | 5 | 5 | 5 | 5 | 15g/ha |
| 234(R) | 5 | 5 | 5 | 5 | 5 | 5 | 15g/ha |
| 236 | 5 | 5 | 5 | 5 | 5 | 5 | 60g/ha |
| 236(R) | 5 | 5 | 5 | 5 | 5 | 5 | 60g/ha |
| 238 | 5 | 5 | 5 | 5 | 5 | 5 | 15g/ha |
| 238(R) | 5 | 5 | 5 | 5 | 5 | 5 | 15g/ha |
| 239 | 5 | 5 | 5 | 5 | 5 | 5 | 15g/ha |
| 239(R) | 5 | 5 | 5 | 5 | 5 | 5 | 15g/ha |
| 240 | 5 | 5 | 5 | 5 | 5 | 5 | 30g/ha |
| 240(R) | 5 | 5 | 5 | 5 | 5 | 5 | 30g/ha |
| 241 | 5 | 5 | 5 | 5 | 5 | 5 | 30g/ha |
| 241(R) | 5 | 5 | 5 | 5 | 5 | 5 | 30g/ha |
| 243 | 5 | 5 | 5 | 5 | 5 | 5 | 15g/ha |
| 243(R) | 5 | 5 | 5 | 5 | 5 | 5 | 15g/ha |
| 245 | 5 | 5 | 5 | 5 | 5 | 5 | 30g/ha |
| 245(R) | 5 | 5 | 5 | 5 | 5 | 5 | 30g/ha |
| 246 | 5 | 5 | 5 | 5 | 5 | 5 | 15g/ha |
| 246(R) | 5 | 5 | 5 | 5 | 5 | 5 | 15g/ha |
| 248 | 5 | 5 | 5 | 5 | 5 | 5 | 15g/ha |
| 248(R) | 5 | 5 | 5 | 5 | 5 | 5 | 15g/ha |
| 249 | 5 | 5 | 5 | 5 | 5 | 5 | 15g/ha |
| 249(R) | 5 | 5 | 5 | 5 | 5 | 5 | 15g/ha |
| 250 | 5 | 5 | 5 | 5 | 5 | 5 | 15g/ha |
| 250(R) | 5 | 5 | 5 | 5 | 5 | 5 | 15g/ha |
| 251 | 5 | 5 | 5 | 5 | 5 | 5 | 15g/ha |
| 251(R) | 5 | 5 | 5 | 5 | 5 | 5 | 15g/ha |
| 252 | 5 | 5 | 5 | 5 | 5 | 5 | 15g/ha |
| 252(R) | 5 | 5 | 5 | 5 | 5 | 5 | 15g/ha |
| 253 | 5 | 5 | 5 | 5 | 5 | 5 | 15g/ha |
| 253(R) | 5 | 5 | 5 | 5 | 5 | 5 | 15g/ha |
| 254 | 5 | 5 | 5 | 5 | 5 | 5 | 15g/ha |
| 254(R) | 5 | 5 | 5 | 5 | 5 | 5 | 15g/ha |
| 255 | 5 | 5 | 5 | 5 | 5 | 5 | 30g/ha |
| 255(R) | 5 | 5 | 5 | 5 | 5 | 5 | 30g/ha |
| 258 | 5 | 5 | 5 | 5 | 4 | 5 | 15g/ha |
| 258(R) | 5 | 5 | 5 | 5 | 4 | 5 | 15g/ha |
| 259 | 5 | 5 | 5 | 5 | 5 | 5 | 30g/ha |
| 259(R) | 5 | 5 | 5 | 5 | 5 | 5 | 30g/ha |
| 262 | 5 | 5 | 5 | 5 | 5 | 5 | 15g/ha |
| 262(R) | 5 | 5 | 5 | 5 | 5 | 5 | 15g/ha |
| 263 | 5 | 5 | 5 | 5 | 5 | 5 | 60g/ha |
| 263(R) | 5 | 5 | 5 | 5 | 5 | 5 | 60g/ha |
| 264 | 5 | 5 | 5 | 5 | 5 | 5 | 15g/ha |
| 264(R) | 5 | 5 | 5 | 5 | 5 | 5 | 15g/ha |
| 266 | 5 | 5 | 5 | 5 | 5 | 5 | 30g/ha |
| 266(R) | 5 | 5 | 5 | 5 | 5 | 5 | 30g/ha |
| 268 | 5 | 5 | 5 | 5 | 5 | 5 | 30g/ha |
| 268(R) | 5 | 5 | 5 | 5 | 5 | 5 | 30g/ha |
| 283 | 5 | 5 | 5 | 5 | 5 | 5 | 15g/ha |
| 283(R) | 5 | 5 | 5 | 5 | 5 | 5 | 15g/ha |
| 284 | 5 | 5 | 5 | 5 | 5 | 5 | 15g/ha |
| 284(R) | 5 | 5 | 5 | 5 | 5 | 5 | 15g/ha |
| 285 | 5 | 5 | 5 | 5 | 5 | 5 | 30g/ha |
| 285(R) | 5 | 5 | 5 | 5 | 5 | 5 | 30g/ha |
| 286 | 5 | 5 | 5 | 5 | 5 | 5 | 30g/ha |
| 286(R) | 5 | 5 | 5 | 5 | 5 | 5 | 30g/ha |
| 301 | 5 | 5 | 5 | 5 | 5 | 5 | 15g/ha |
| 301(R) | 5 | 5 | 5 | 5 | 5 | 5 | 15g/ha |
| 302 | 5 | 5 | 5 | 5 | 5 | 5 | 15g/ha |
| 302(R) | 5 | 5 | 5 | 5 | 5 | 5 | 15g/ha |
| 303 | 5 | 5 | 5 | 5 | 5 | 5 | 15g/ha |
| 303(R) | 5 | 5 | 5 | 5 | 5 | 5 | 15g/ha |
| 313 | 5 | 5 | 5 | 5 | 5 | 5 | 15g/ha |
| 313(R) | 5 | 5 | 5 | 5 | 5 | 5 | 15g/ha |
| 315 | 5 | 5 | 5 | 5 | 5 | 5 | 15g/ha |
| 315(R) | 5 | 5 | 5 | 5 | 5 | 5 | 15g/ha |
| 316 | 5 | 5 | 5 | 5 | 5 | 5 | 15g/ha |
| 316(R) | 5 | 5 | 5 | 5 | 5 | 5 | 15g/ha |
| 318 | 5 | 5 | 5 | 5 | 5 | 5 | 15g/ha |
| 318(R) | 5 | 5 | 5 | 5 | 5 | 5 | 15g/ha |
| 319 | 5 | 5 | 5 | 5 | 5 | 5 | 30g/ha |
| 319(R) | 5 | 5 | 5 | 5 | 5 | 5 | 30g/ha |
| 321 | 5 | 5 | 5 | 5 | 5 | 5 | 120g/ha |
| 321(R) | 5 | 5 | 5 | 5 | 5 | 5 | 120g/ha |
| 322 | 5 | 5 | 5 | 5 | 5 | 5 | 120g/ha |
| 322(R) | 5 | 5 | 5 | 5 | 5 | 5 | 120g/ha |
| 331 | 5 | 5 | 5 | 5 | 5 | 5 | 15g/ha |
| 331(R) | 5 | 5 | 5 | 5 | 5 | 5 | 15g/ha |
| 333 | 5 | 5 | 5 | 5 | 5 | 5 | 15g/ha |
| 333(R) | 5 | 5 | 5 | 5 | 5 | 5 | 15g/ha |
| 337 | 5 | 5 | 5 | 5 | 5 | 5 | 15g/ha |
| 337(R) | 5 | 5 | 5 | 5 | 5 | 5 | 15g/ha |
| 342 | 5 | 5 | 5 | 5 | 4 | 5 | 15g/ha |
| 342(R) | 5 | 5 | 5 | 5 | 5 | 5 | 15g/ha |
| 344 | 5 | 5 | 5 | 5 | 5 | 5 | 15g/ha |
| 344(R) | 5 | 5 | 5 | 5 | 5 | 5 | 15g/ha |
| 347 | 5 | 5 | 5 | 5 | 5 | 5 | 30g/ha |
| 347(R) | 5 | 5 | 5 | 5 | 5 | 5 | 30g/ha |
| 349 | 5 | 5 | 5 | 5 | 5 | 5 | 15g/ha |
| 349(R) | 5 | 5 | 5 | 5 | 5 | 5 | 15g/ha |
| 351 | 5 | 5 | 5 | 5 | 5 | 5 | 60g/ha |
| 351(R) | 5 | 5 | 5 | 5 | 5 | 5 | 60g/ha |
| 388 | 5 | 5 | 5 | 5 | 4 | 5 | 15g/ha |
| 388(R) | 5 | 5 | 5 | 5 | 5 | 5 | 15g/ha |
| 390 | 5 | 5 | 5 | 5 | 5 | 5 | 15g/ha |
| 390(R) | 5 | 5 | 5 | 5 | 5 | 5 | 15g/ha |
| 391 | 5 | 5 | 5 | 5 | 5 | 5 | 60g/ha |
| 391(R) | 5 | 5 | 5 | 5 | 5 | 5 | 60g/ha |
| 392 | 5 | 5 | 5 | 5 | 5 | 5 | 60g/ha |
| 392(R) | 5 | 5 | 5 | 5 | 5 | 5 | 60g/ha |
| 393 | 5 | 5 | 5 | 5 | 5 | 5 | 60g/ha |
| 393(R) | 5 | 5 | 5 | 5 | 5 | 5 | 60g/ha |
| 394 | 5 | 5 | 5 | 5 | 5 | 5 | 60g/ha |
| 394(R) | 5 | 5 | 5 | 5 | 5 | 5 | 60g/ha |
| 395 | 5 | 5 | 5 | 5 | 5 | 5 | 60g/ha |
| 395(R) | 5 | 5 | 5 | 5 | 5 | 5 | 60g/ha |
| 396 | 5 | 5 | 5 | 5 | 5 | 5 | 60g/ha |
| 396(R) | 5 | 5 | 5 | 5 | 5 | 5 | 60g/ha |
| 398 | 5 | 5 | 5 | 5 | 5 | 5 | 15g/ha |
| 398(R) | 5 | 5 | 5 | 5 | 5 | 5 | 15g/ha |
| 399 | 5 | 5 | 5 | 5 | 5 | 5 | 15g/ha |
| 399(R) | 5 | 5 | 5 | 5 | 5 | 5 | 15g/ha |
| 400 | 5 | 5 | 5 | 5 | 4 | 5 | 15g/ha |
| 400(R) | 5 | 5 | 5 | 5 | 5 | 5 | 15g/ha |
| 406 | 5 | 5 | 5 | 5 | 4 | 5 | 15g/ha |
| 406(R) | 5 | 5 | 5 | 5 | 5 | 5 | 15g/ha |
| 409 | 5 | 5 | 5 | 5 | 5 | 5 | 30g/ha |
| 409(R) | 5 | 5 | 5 | 5 | 5 | 5 | 30g/ha |
| 416 | 5 | 5 | 5 | 5 | 4 | 5 | 15g/ha |
| 416(R) | 5 | 5 | 5 | 5 | 5 | 5 | 15g/ha |
| 419 | 5 | 5 | 5 | 5 | 5 | 5 | 15g/ha |
| 419(R) | 5 | 5 | 5 | 5 | 5 | 5 | 15g/ha |
| 421 | 5 | 5 | 5 | 5 | 5 | 5 | 15g/ha |
| 421(R) | 5 | 5 | 5 | 5 | 5 | 5 | 15g/ha |
| 424 | 5 | 5 | 5 | 5 | 5 | 5 | 30g/ha |
| 424(R) | 5 | 5 | 5 | 5 | 5 | 5 | 30g/ha |
| 426 | 5 | 5 | 5 | 5 | 5 | 5 | 250g/ha |
| 426(R) | 5 | 5 | 5 | 5 | 5 | 5 | 250g/ha |
| 431 | 5 | 5 | 5 | 5 | 5 | 5 | 15g/ha |
| 431(R) | 5 | 5 | 5 | 5 | 5 | 5 | 15g/ha |
| 432 | 5 | 5 | 5 | 5 | 5 | 5 | 15g/ha |
| 432(R) | 5 | 5 | 5 | 5 | 5 | 5 | 15g/ha |
| 433 | 5 | 5 | 5 | 5 | 5 | 5 | 15g/ha |
| 434 | 5 | 5 | 5 | 5 | 5 | 5 | 30g/ha |
| 438 | 5 | 5 | 5 | 5 | 5 | 5 | 30g/ha |
| 438(R) | 5 | 5 | 5 | 5 | 5 | 5 | 30g/ha |
| 439 | 5 | 5 | 5 | 5 | 5 | 5 | 30g/ha |
| 439(R) | 5 | 5 | 5 | 5 | 5 | 5 | 30g/ha |
| 442 | 5 | 5 | 5 | 5 | 5 | 5 | 15g/ha |
| 442(R) | 5 | 5 | 5 | 5 | 5 | 5 | 15g/ha |
| 443 | 5 | 5 | 5 | 5 | 5 | 5 | 15g/ha |
| 443(R) | 5 | 5 | 5 | 5 | 5 | 5 | 15g/ha |
| 444 | 5 | 5 | 5 | 5 | 5 | 5 | 15g/ha |
| 444(R) | 5 | 5 | 5 | 5 | 5 | 5 | 15g/ha |
| 445 | 5 | 5 | 5 | 5 | 5 | 5 | 15g/ha |
| 445(R) | 5 | 5 | 5 | 5 | 5 | 5 | 15g/ha |
| 446 | 5 | 5 | 5 | 5 | 5 | 5 | 15g/ha |
| 446(R) | 5 | 5 | 5 | 5 | 5 | 5 | 15g/ha |
| 447 | 5 | 5 | 5 | 5 | 5 | 5 | 15g/ha |
| 447(R) | 5 | 5 | 5 | 5 | 5 | 5 | 15g/ha |
| 448 | 5 | 5 | 5 | 5 | 5 | 5 | 15g/ha |
| 448(R) | 5 | 5 | 5 | 5 | 5 | 5 | 15g/ha |
| 449 | 5 | 5 | 5 | 5 | 5 | 5 | 15g/ha |
| 449(R) | 5 | 5 | 5 | 5 | 5 | 5 | 15g/ha |
| 450 | 5 | 5 | 5 | 5 | 5 | 5 | 15g/ha |
| 450(R) | 5 | 5 | 5 | 5 | 5 | 5 | 15g/ha |
| 451 | N | N | N | N | N | 5 | 15g/ha |
| 451(R) | N | N | N | N | N | 5 | 15g/ha |
| 452 | N | N | N | N | N | 5 | 15g/ha |
| 452(R) | N | N | N | N | N | 5 | 15g/ha |
| 453 | 5 | 5 | 5 | 5 | 5 | 5 | 30g/ha |
| 453(R) | 5 | 5 | 5 | 5 | 5 | 5 | 30g/ha |
| 454 | 5 | 5 | 5 | 5 | 5 | 5 | 60g/ha |
| 454(R) | 5 | 5 | 5 | 5 | 5 | 5 | 60g/ha |
| 455 | 5 | 5 | 5 | 5 | 5 | 5 | 15g/ha |
| 455(R) | 5 | 5 | 5 | 5 | 5 | 5 | 15g/ha |
| 456 | 5 | 5 | 5 | 5 | 5 | 5 | 30g/ha |
| 456(R) | 5 | 5 | 5 | 5 | 5 | 5 | 30g/ha |
| 462 | 5 | 5 | 5 | 5 | 5 | 5 | 15g/ha |
| 462(R) | 5 | 5 | 5 | 5 | 5 | 5 | 15g/ha |
| 463 | 5 | 5 | 5 | 5 | 5 | 5 | 15g/ha |
| 463(R) | 5 | 5 | 5 | 5 | 5 | 5 | 15g/ha |
| 469 | 5 | 5 | 5 | 5 | 5 | 5 | 15g/ha |
| 471 | N | N | N | N | N | 5 | 15g/ha |
| 471(R) | N | N | N | N | N | 5 | 15g/ha |
| 473 | 5 | 5 | 5 | 5 | 5 | 5 | 30g/ha |
| 473(R) | 5 | 5 | 5 | 5 | 5 | 5 | 30g/ha |
| 475 | N | N | N | N | N | 5 | 15g/ha |
| 475(R) | N | N | N | N | N | 5 | 15g/ha |
| 476 | N | N | N | N | N | 5 | 15g/ha |
| 476(R) | N | N | N | N | N | 5 | 15g/ha |
| 477 | N | N | N | N | N | 5 | 15g/ha |
| 477(R) | N | N | N | N | N | 5 | 15g/ha |
| 478 | N | N | N | N | N | 5 | 60g/ha |
| 478(R) | N | N | N | N | N | 5 | 60g/ha |
| 479 | N | N | N | N | N | 5 | 15g/ha |
| 480 | N | N | N | N | N | 5 | 15g/ha |
| 481 | N | N | N | N | N | 5 | 15g/ha |
| 481(R) | N | N | N | N | N | 5 | 15g/ha |
| 485 | 5 | 5 | 5 | 5 | 5 | 5 | 15g/ha |
| 486 | 5 | 5 | 5 | 5 | 5 | 5 | 15g/ha |
| 487 | 5 | 5 | 5 | 5 | 5 | 5 | 15g/ha |
| 488 | 5 | 5 | 5 | 5 | 5 | 5 | 15g/ha |
| 489 | 5 | 5 | 5 | 5 | 5 | 5 | 15g/ha |
| 490 | 5 | 5 | 5 | 5 | 5 | 5 | 15g/ha |
| 491 | 5 | 5 | 5 | 5 | 5 | 5 | 15g/ha |
| 491(R) | 5 | 5 | 5 | 5 | 5 | 5 | 15g/ha |
| 493 | 5 | 5 | 5 | 5 | 5 | 5 | 15g/ha |
| 494 | 5 | 5 | 5 | 5 | 5 | 5 | 15g/ha |
| 495 | 5 | 5 | 5 | 5 | 5 | 5 | 15g/ha |
| 495(R) | 5 | 5 | 5 | 5 | 5 | 5 | 15g/ha |
| 496 | 5 | 5 | 5 | 5 | 5 | 5 | 15g/ha |
| 497 | 5 | 5 | 5 | 5 | 5 | 5 | 15g/ha |
| 498 | 5 | 5 | 5 | 5 | 5 | 5 | 15g/ha |
| 498(R) | 5 | 5 | 5 | 5 | 5 | 5 | 15g/ha |
| 499 | 5 | 5 | 5 | 5 | 5 | 5 | 15g/ha |
| 499(R) | 5 | 5 | 5 | 5 | 5 | 5 | 15g/ha |
| 500 | 5 | 5 | 5 | 5 | 5 | 5 | 15g/ha |
| 500(R) | 5 | 5 | 5 | 5 | 5 | 5 | 15g/ha |
| 501 | 5 | 5 | 5 | 5 | 5 | 5 | 15g/ha |
| 501(R) | 5 | 5 | 5 | 5 | 5 | 5 | 15g/ha |
| 502 | 5 | 5 | 5 | 5 | 5 | 5 | 15g/ha |
| 502(R) | 5 | 5 | 5 | 5 | 5 | 5 | 15g/ha |
| 503 | 5 | 5 | 5 | 5 | 5 | 5 | 15g/ha |
| 503(R) | 5 | 5 | 5 | 5 | 5 | 5 | 15g/ha |
| 504 | 5 | 5 | 5 | 5 | 5 | 5 | 15g/ha |
| 504(R) | 5 | 5 | 5 | 5 | 5 | 5 | 15g/ha |
| 511 | 5 | 5 | 5 | 5 | 5 | 5 | 15g/ha |
| 692 | 5 | 5 | 5 | 5 | 5 | 5 | 15g/ha |
| 730 | 5 | 5 | 5 | 5 | 5 | 5 | 15g/ha |
| 730(R) | 5 | 5 | 5 | 5 | 5 | 5 | 15g/ha |
| 881 | 5 | 5 | 5 | 5 | 5 | 5 | 30g/ha |
| 885 | 5 | 5 | 5 | 5 | 5 | 5 | 30g/ha |
| 919 | 5 | 5 | 5 | 5 | 5 | 5 | 30g/ha |
| 919(R) | 5 | 5 | 5 | 5 | 5 | 5 | 30g/ha |
| Control compound A | 1 | 0 | 1 | 1 | 0 | 3 | 15g/ha |
| Control compound B | 2 | 2 | 2 | 1 | 1 | 3 | 15g/ha |
| Control compound C | 2 | 2 | 2 | 1 | 1 | 2 | 15g/ha |
| Control compound D | 3 | 4 | 3 | 3 | 2 | N | 15g/ha |
| Control compound E | 3 | 4 | 4 | 3 | 3 | N | 15g/ha |
| Control compound F | 1 | 0 | 1 | 1 | 0 | 2 | 60g/ha |

Note: N represents untested;
Control compound A: 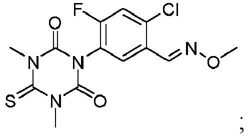
Control compound B: 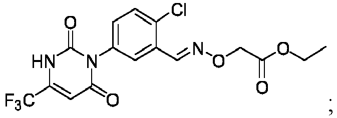
Control compound C: 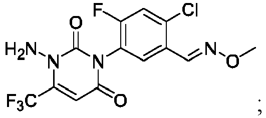
Control compound D: 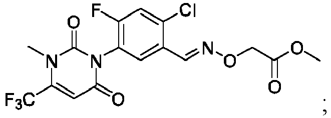
Control compound E: 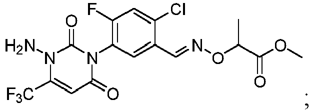
Control compound F:

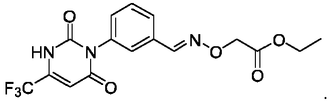

**Table 3. Results of R configuration, S configuration and racemate on weeding effect in post-emergence stage**

| Compound NO. | *Amaranthus retroflexus* | *Echinochloa crusgalli* | *Eleusine indica* | Dose |
|---|---|---|---|---|
| 1(R) | 5 | 4 | 5 | 7.5g/ha |
| 1 | 3 | 3 | 3 | 7.5g/ha |
| 1(S) | 1 | 1 | 1 | 7.5g/ha |
| 194(R) | 5 | 5 | 5 | 7.5g/ha |
| 194 | 5 | 4 | 4 | 7.5g/ha |
| 194(S) | 2 | 1 | 1 | 7.5g/ha |
| Control compound D | 2 | 1 | 2 | 7.5g/ha |

### Experiment on weed effect in pre-emergence stage:

The aforementioned seeds of monocotyledonous and dicotyledonous weeds and main crops were put into a plastic pot loaded with soil and covered with 0.5-2cm soil. The test compounds of the present invention was dissolved with acetone, then added with tween 80, diluted by a certain amount of water to reach a certain concentration, and sprayed immediately after sowing. The obtained seeds were incubated for 4 weeks in the greenhouse after spraying and the test results were observed. It was observed that the herbicide mostly had excellent effect at the application rate of 500,250,125,60,30,15,7.5 g a.i./ha, especially to weeds such as *Echinochloa crusgalli, Digitaria sanguinalis* and *Abutilon theophrasti,* etc.. And many compounds had good selectivity for corn, cotton, wheat, rice, soybean, and peanut etc.. In addition, evaluate the weed control effect with the above activity standard level. Many compounds show excellent activity and selectivity, which are shown in Table 4.

**Table 4. Results on weeding effect in pre -emergence stage**

| Compou nd NO. | *Veron ica didy ma* Tenor e | *Descu rainia sophia* | *Capsel la bursa-pastori* s | *Abutil on theoph rasti* | *Amara nthus retrofl exus* | *Setari a viridis* | Corn | Cotton | So ybe ans | Pe an ut | Dose |
|---|---|---|---|---|---|---|---|---|---|---|---|
| 1(R) | 5 | 5 | 5 | 5 | 5 | 5 | 0 | 0 | 0 | 0 | 30g/ha |
| 2(R) | 5 | 5 | 5 | 5 | 5 | 5 | 0 | 0 | 0 | 0 | 30g/ha |
| 194(R) | 5 | 5 | 5 | 5 | 5 | 5 | 0 | 0 | 0 | 0 | 15g/ha |
| 194(R) | 5 | 5 | 5 | 5 | 5 | 5 | 0 | 0 | 0 | 0 | 30g/ha |
| 194(R) | 5 | 5 | 5 | 5 | 5 | 5 | 0 | 0 | 0 | 0 | 60g/ha |
| 196(R) | 5 | 5 | 5 | 5 | 5 | 5 | 0 | 0 | 0 | 0 | 30g/ha |
| 198(R) | 5 | 5 | 5 | 5 | 5 | 5 | 0 | 0 | 0 | 0 | 30g/ha |
| 199(R) | 5 | 5 | 5 | 5 | 5 | 5 | 0 | 0 | 0 | 0 | 30g/ha |
| 208(R) | 5 | 5 | 5 | 5 | 5 | 5 | 0 | 0 | 0 | 0 | 30g/ha |
| 212(R) | 5 | 5 | 5 | 5 | 5 | 5 | 0 | 0 | 0 | 0 | 30g/ha |
| 216(R) | 5 | 5 | 5 | 5 | 5 | 5 | 0 | 0 | 0 | 0 | 30g/ha |
| 218(R) | 5 | 5 | 5 | 5 | 5 | 5 | 0 | 0 | 0 | 0 | 30g/ha |
| 239(R) | 5 | 5 | 5 | 5 | 5 | 5 | 0 | 0 | 0 | 0 | 30g/ha |
| 246(R) | 5 | 5 | 5 | 5 | 5 | 5 | 0 | 0 | 0 | 0 | 30g/ha |
| 248(R) | 5 | 5 | 5 | 5 | 5 | 5 | 0 | 0 | 0 | 0 | 30g/ha |
| 249(R) | 5 | 5 | 5 | 5 | 5 | 5 | 0 | 0 | 0 | 0 | 30g/ha |
| 253(R) | 5 | 5 | 5 | 5 | 5 | 5 | 0 | 0 | 0 | 0 | 30g/ha |
| 258(R) | 5 | 5 | 5 | 5 | 5 | 5 | 0 | 0 | 0 | 0 | 30g/ha |
| 264(R) | 5 | 5 | 5 | 5 | 5 | 5 | 0 | 0 | 0 | 0 | 30g/ha |
| 283(R) | 5 | 5 | 5 | 5 | 5 | 5 | 0 | 0 | 0 | 0 | 30g/ha |
| 301(R) | 5 | 5 | 5 | 5 | 5 | 5 | 0 | 0 | 0 | 0 | 30g/ha |
| 315(R) | 5 | 5 | 5 | 5 | 5 | 5 | 0 | 0 | 0 | 0 | 30g/ha |
| 333(R) | 5 | 5 | 5 | 5 | 5 | 5 | 0 | 0 | 0 | 0 | 30g/ha |
| 349(R) | 5 | 5 | 5 | 5 | 5 | 5 | 0 | 0 | 0 | 0 | 30g/ha |
| 398(R) | 5 | 5 | 5 | 5 | 5 | 5 | 0 | 0 | 0 | 0 | 30g/ha |
| 421(R) | 5 | 5 | 5 | 5 | 5 | 5 | 0 | 0 | 0 | 0 | 30g/ha |
| 431(R) | 5 | 5 | 5 | 5 | 5 | 5 | 0 | 0 | 0 | 0 | 30g/ha |
| 432(R) | 5 | 5 | 5 | 5 | 5 | 5 | 0 | 0 | 0 | 0 | 30g/ha |
| 433 | 5 | 5 | 5 | 5 | 5 | 5 | 0 | 0 | 0 | 0 | 30g/ha |
| 434 | 5 | 5 | 5 | 5 | 5 | 5 | 0 | 0 | 0 | 0 | 30g/ha |

It is indicated from the experiment of main weeds in wheat and rice fields that the compound of the present invention generally have good weed control efficacy. Above all, it is noted that the compound of the invention have extremely high activity to broad-leaved weeds and cyperaceae weeds, which are resistant to ALS inhibitor, like *Sagittaria trifolia, Scirpus juncoides, Cyperus difformis, Descurainia sophia, Capsella bursa-pastoris, Lithospermum arvense, Galium aparine L.,* and *Cyperus rotundus L.,* etc. , and have excellent commercial value.

### Transplanted rice safety evaluation and weed control effect evaluation in rice field:

Rice field soil was loaded into a 1/1,000,000 ha pot. The seeds of *Echinochloa crusgalli, Scirpus juncoides, Bidens tripartita L., Monochoria vaginalis,* and *Leptochloa chinensis* were sowed and gently covered with soil, then left to stand still in greenhouse in the state of 0.5-1cm of water storage. The tuber of *Sagittaria trifolia* was planted in the next day or 2 days later. It was kept at 3-4cm of water storage thereafter. The weeds were treated by dripping the WP or SC water diluents prepared according to the common preparation method of the compounds of the present invention with pipette homogeneously to achieve specified effective amount when *Echinochloa crusgalli, Scirpus juncoides, Bidens tripartita L., Monochoria vaginalis,* and *Leptochloa chinensis* reached 0.5 leaf stage and *Sagittaria trifolia* reached the time point of primary leaf stage.

In addition, the rice field soil that loaded into the 1/1,000,000 ha pot was leveled to keep water storage at 3-4cm depth. The 5 leaf stage rice (japonica rice) was transplanted at 3cm of transplanting depth the next day. The compound of the present invention was treated by the same way after 5 days of transplantation.

The fertility condition of *Echinochloa crusgalli, Scirpus juncoides, Bidens tripartita L., Monochoria vaginalis, Leptochloa chinensis* and *Sagittaria trifolia* 14 days after the treatment of the compound of the invention and the fertility condition of rice 21 days after the treatment of the compound of the invention respectively with the naked eye. Evaluate the weed control effect with the above activity standard level. Many compounds show excellent activity and selectivity.

**Table 5. Evaluation effect of some compounds**

| Compound NO. | *Leptochloa chinensis* | *Scirpus juncoides* | *Monochoria vaginalis* | Rice | Dose |
|---|---|---|---|---|---|
| 194(R) | 5 | 5 | 5 | 0 | 30 g/ha |
| 196(R) | 5 | 5 | 5 | 0 | 30 g/ha |
| 212(R) | 5 | 5 | 5 | 0 | 30 g/ha |
| 218(R) | 5 | 5 | 5 | 0 | 30 g/ha |
| 421(R) | 5 | 5 | 5 | 0 | 30 g/ha |
| 434 | 5 | 5 | 5 | 0 | 30 g/ha |
| Pyrazosulfuron -ethyl | 2 | 1 | 2 | 1 | 30 g/ha |

| | | | | | |
|---|---|---|---|---|---|
| Note: The seeds of *Echinochloa crusgalli, Scirpus juncoides, Monochoria vaginalis* and *Bidens tripartita L., Sagittaria trifolia* were collected from Heilongjiang Province of China. The tests indicated that the weeds were resistant to the common doses of Pyrazosulfuron-ethyl. | | | | | |

### Composition activity test:

The active ingredient B shuangzuocaotong, huanbifucaotong, benzuofucaotong, and sanzuohuangcaotong was produced by our company, the preparation methods of 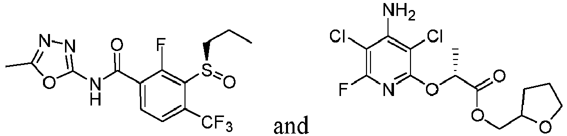 are as follows, and the others were purchased from reagent companies. The technical materials were all dissolved in acetone and diluted with an aqueous solution containing 0.1% emulsifier Tween-80. The dilution is performed as required.
(1) Synthesis of Compound 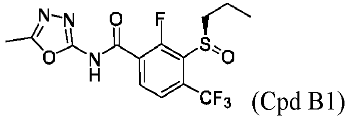
   (1.1) Cpd 1(3 g, 16 mmol, 1.0 eq), NaOH (0.72 g, 18 mmol, 1.1 eq) were added sequentially into 30 ml of DMF, and then Cpd 2 (1.28 g, 16.8 mmol, 1.05 eq) was added dropwise at 0 °C, and the reaction solution was stirred at 0 °C for 1 hour. When LCMS test showed that the reaction of raw materials was basically completed, there was one major new peak. The reaction solution was poured into 30 ml of water, and the mixture was separated, and the aqueous phase was extracted once with 50 ml of ethyl acetate, and the resultant organic phase was washed three times with saturated saline solution (50 ml), dried, evaporated to dryness under reduced pressure and separated by column chromatography to obtain Cpd 3 (3.6 g, 91% yield) (colorless oil). 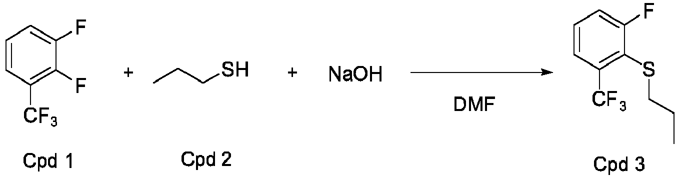
   (1.2) Cpd 3(3.1 g, 13 mmol, 1.0 eq) was added to 30 ml of THF, then n-BuLi (6.42 ml, 2.5 M, 16 mmol, 1.2 eq) was slowly added at -78 °C, then the reaction solution was stirred at -78 °C for 0.5 hour, and slowly fed with CO₂ for 10 minutes, then the reaction solution was slowly warmed to room temperature. The product was detected by LCMS. 20 ml of water was poured into the reaction solution, the mixture was separated, the aqueous phase was extracted once with 30 ml of ethyl acetate, and the resultant aqueous phase was gradually adjusted to pH = 4-5 with concentrated hydrochloric acid, filtered and dried to give Cpd 4(3.2 g, 87% yield) (white solid). 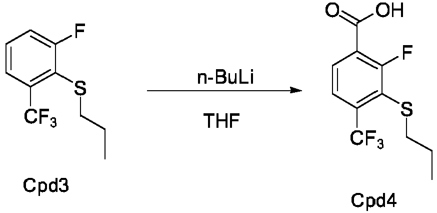
   (1.3) Cpd 4(3.1 g, 11 mmol, 1.0 eq), Cpd 5 (1.66 g, 16.8 mmol, 1.5 eq), DMAP (0.13 g, 1.1 mmol, 0.1 eq) were sequentially added to 30 ml of pyridine. Then, SOCl₂ (2.0 g, 16.8 mmol, 1.5 eq) was slowly added at 0 °C, and the reaction solution was stirred at room temperature for 3 hours. The product was detected by LCMS. Pyridine was removed by concentration, then 30 ml of water was poured into the reaction solution, and the mixture was separated. The aqueous phase was extracted three times with 30 ml of ethyl acetate, and the resultant organic phase was washed three times with saturated saline solution (50 ml), dried, and evaporated to dryness under reduced pressure and separated by column chromatography to to obtain Cpd 6(2.5 g, 63% yield) (white solid). 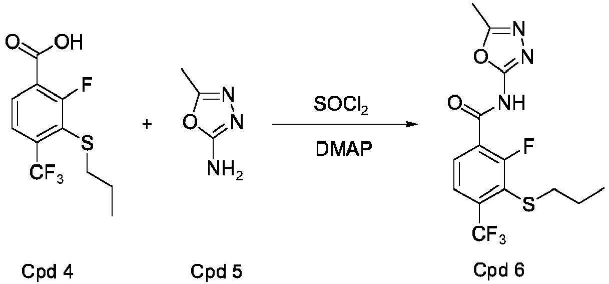
   (1.4) Cpd 6(1 g, 2.8 mmol, 1.0 eq) and m-CPBA (0.54 g, 3.1 mmol, 1.1 eq) were added sequentially in 10 mL of dichloromethane. The reaction solution was then stirred at room temperature for 1 hour. The product was detected by LCMS, and the reaction of raw materials was basically completed. The reaction solution was poured into 10 ml of water, the reaction was quenched with sodium hydrogen sulfite, and the mixture was separated. The aqueous phase was extracted three times with 30 ml of dichloromethane, and the resultant organic phase was washed once with saturated saline solution (30 ml), dried, and evaporated to dryness under reduced pressure, and separated by column chromatography to give Cpd 7(0.85 g, 82% yield) (greyish white solid).
      ¹H NMR (500 MHz, DMSO-*d*₆) 12.57 (s, 1H), 8.07 (dd, J = 8.0, 7.0 Hz, 1H), 7.82 (d, J = 8.0 Hz, 1H), 3.57-3.47 (m, 2H), 2.48 (s, 3H), 1.70-1.52 (m, 2H), 1.08-0.93 (m, 3H). 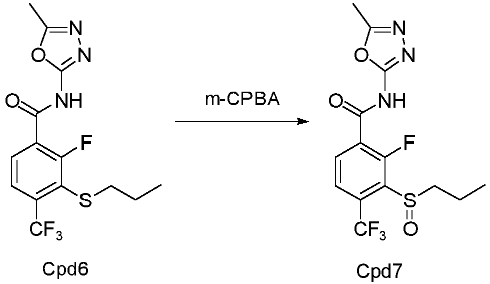
   (1.5) Cpd 7 (0.5 g, 98% purity) was passed through chiral HPLC (Column: CHIRALPAK IG; Column Size: 3 cm × 25 cm, 5 um; Injection: 3.0 ml; Mobile phase: Hex(0.2% FA) : IPA=50:50; Flow rate: 28ml/min; Wavelength: UV 254nm; Temperature: 25 °C; Sample solution: 70mg/ml in EtOH/DCM; Run time= 60 mins) for separation, and then concentrated to obtain Cpd B1 (R-configuration)(0.16 g, Rt=10.51min, 100% ee, purity 98%) in white solids, which were confirmed by single crystal diffraction.

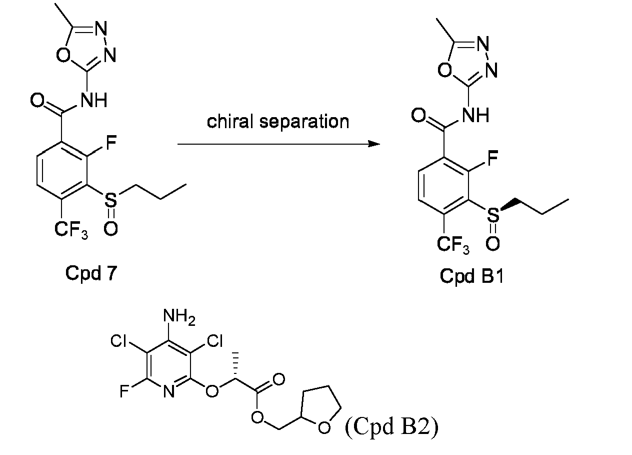

### (2) Synthesis of Compound

(2.1) Cpd a (0.5g, 2.13mmol), Cpd b (313mg, 2.55mmol), a catalytic amount of TBAB (10mg), and DMF (10mL) were added to a round-bottom flask, and stirred at room temperature 15 °C for 24 hr. When there was a small amount of raw materials remained according to LC-MS detection, a further treatment was made. The reaction solution was poured into 50mL of water, and extracted with methyl tert-butyl ether twice (50mL × 2). The organic phase was dried, concentrated, and separated by column chromatography, to obtain Cpd c (300mg, yield 50%), as a white solid.

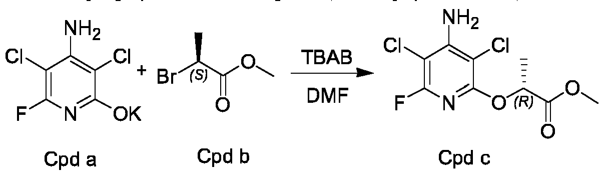

(2.2) Cpd c (0.3g, 1.06mmol), methanol (20mL) were added to a 100 mL single-port flask, lithium hydroxide (44.5mg, 1.06mmol) was dissolved in 2 mL of water, and slowly added dropwise to the single-port flask at room temperature, followed by stirring at room temperature for 12 hr. After completed reaction of the raw materials according to LC-MS detection, the reaction solution was adjusted with 0.5M dilute HCl to pH = 5-6, concentrated, and then extracted with water and ethyl acetate. The organic phase was dried, and concentrated to obtain Cpd d (200mg, yield 70%) as a white solid.

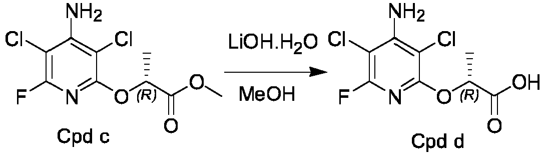

(2.3) Cpd d (200mg, 0.74mmol), Cpd e (75mg, 0.74mmol), DCC (152mg, 0.74mmol), and anhydrous DCM (20mL) were added to a 100 mL round-bottom flask, and reacted at room temperature for 12 hr. After completed reaction of the raw materials according to LC-MS detection, the reaction solution was concentrated, and separated by column chromatography to obtain the Cpd B2 (200mg, yield 77%), as a white solid.

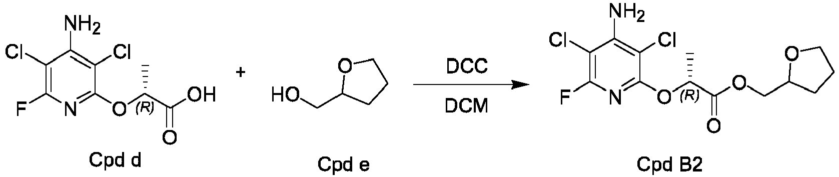

¹H NMR (500 MHz, Chloroform-*d*) δ 5.28 (q, *J* = 7.0 Hz, 1H), 5.15 (s, 2H), 4.27 - 4.07 (m, 3H), 3.91 - 3.73 (m, 2H), 2.04 - 1.82 (m, 3H), 1.66 (d, *J* = 7.0 Hz, 3H), 1.59-1.54 (m, 1H).

### (A)Post-emergence treatment by performing foliage spray:

Weeds were cultivated by a pot culture method. A 180 ×140 mm plastic nutritional bowl contained 4/5 topsoil from the field was placed in an enamel pan, wherein the soil had been air-dried and screened and had an initial moisture content of 20%. Full and uniform weed seeds were selected, soaked in warm water at 25°C for 6 hours, and germinated in a 28°C biochemical incubator (darkness). The weed seeds that had just germinated were evenly placed on the surface of the soil and then covered with 0.5-1 cm soil according to the sizes of seeds.

The culture was carried out in a controllable sunlight greenhouse at 20 to 30°C, in natural light, and relative humidity of 57% to 72%. The soil was loam with an organic matter content of 1.63%, a pH value of 7.1, an alkali-hydrolyzable nitrogen of 84.3 mg/kg, a rapidly available phosphorus of 38.5 mg/kg, and a rapidly available potassium 82.1 mg/kg.

3 pots with 20 weed seeds per pot were treated in one treatment with 4 replications per treatment.

The agents were used for only once in the experiment. In the stage of weeds with 1.5-2 leaves, the weeds were thinned out to maintain 10 weeds per pot and 30 weeds for each treatment, then continued to be cultured to *Conyza Canadensis* 10cm in height, other weeds 3-4 leaves stage and treated.

The well-cultured weeds were evenly placed on a platform with an area of 0.5m², and a solution of agents was sprayed on the stems and leaves thereof by the 3WP-2000-type walking spray tower at a dosage of 450kg/ha and at a spray pressure of 0.3MPa. After all the solution was sprayed, the valve was closed. After 30 seconds, the door of the spray tower was opened, and the nutritional bowl was taken out. Then the valve was opened, and the spray tube was cleaned by spraying 50 ml of water. After the treatment, the weeds were routinely cultured in a greenhouse.

### (B)Soil sealing treatment:

Weeds are cultivated in a controllable sunlight greenhouse at 20 to 30°C, in natural light, and relative humidity of 57% to 72%. The soil was loam with an organic matter content of 1.63%, a pH value of 7.1, an alkali-hydrolyzable nitrogen of 84.3 mg/kg, a rapidly available phosphorus of 38.5 mg/kg, and a rapidly available potassium 82.1 mg/kg. The test soil was placed quantitatively to 3/4 of the pots and then watered from the bottom of the pots to completely wet the soil to saturation. The test weed seeds were germinated, and uniformly and quantitatively sowed on the surface, then covered with 0.5-2 cm soil according to the seed size, and ready-for use 72 hours after sowing.

3 pots with 30 weed seeds per pot were treated in one treatment with 4 replications per treatment.

The well-sowed weeds were evenly placed on a platform with an area of 0.5m², and a solution of agents was sprayed on the soil thereof by the 3WP-2000-type walking spray tower at a dosage of 450kg/ha and at a spray pressure of 0.3MPa. After all the solution was sprayed, the valve was closed. After 30 seconds, the door of the spray tower was opened, and the nutritional bowl was taken out. Then the valve was opened, and the spray tube was cleaned by spraying 50 ml of water.

### (C)Data investigation and statistical analysis:

A method for investigating absolute number was employed, wherein whole seedlings of survival weeds were cut off with a blade along the soil surface, and the fresh weight of the weeds was weighed with an analytical balance. For dead weeds, the fresh weight thereof was zero.

The investigation was performed after 21 days of the treatment for only once.

Theoretical fresh weight inhibition rate of a combination of two active ingredients in each group was calculated by the Gowing method (E0=X+Y-X^{∗}Y/100), and then compared with an actually measured inhibition rate (E), thereby effect of the combination (hereafter referred to as combined effect) on weeds was evaluated: the value of E-E0, which was greater than 10%, corresponded to a synergistic effect, the value of E-E0, which was less than -10%, corresponded to an antagonistic effect, and the value of E-E0, which was from -10% to 10%, corresponded to an additional effect. An optimum ratio of the two active ingredients was determined by the actual control effect, characteristics of herbicides, and balance of a corresponding formula. Wherein, in the formula, X represented the fresh weight inhibition rate of the active ingredient A in a dosage of P, and Y represented the fresh weight inhibition rate of the active ingredient B in a dosage of Q. The statistical results were shown in the table 6.

**Table 6. Actual control effect and combined effect of a combination of A on weeds**

| Components | Weed | Foliage /Soil F/ S | Dose g a.i./ha | Ratio | Contro 1 effect (%) of A applied alone (A) | Contro 1 effect (%) of B applied alone (B) | Actual control effect of A+B (%) E(A+B) | Theoretica 1 control effect of A+B(%) E0(A+B) | E(A+B) -E0(A+ B) |
|---|---|---|---|---|---|---|---|---|---|
| A +topramezo ne | *Echino chloa caudat a Roshev* | F | 1.5+7.5 | 1:5 | 65.9 | 38.5 | 93.5 | 79.0 | 14.5 |
| A +isoxaflutol e | *Echino chloa caudat a Roshev* | F | 1.5+15 | 1:10 | 65.9 | 41.3 | 91.9 | 80.0 | 11.9 |
| A +tembotrio ne | *Echino chloa caudat a Roshev* | F | 1.5+15 | 1:10 | 65.9 | 31.7 | 88.3 | 76.7 | 11.6 |
| A +tefuryltrio ne | *Echino chloa caudat a Roshev* | F | 1.5+30 | 1:20 | 65.9 | 21.5 | 86.6 | 73.2 | 13.4 |
| A +shuangzuo caotong | *Echino chloa caudat a Roshev* | F | 1.5+15 | 1:10 | 65.9 | 35.9 | 94.5 | 78.1 | 16.4 |
| A +huanbifuc aotong | *Echino chloa caudat a Roshev* | F | 1.5+60 | 1:40 | 65.9 | 27.4 | 87.4 | 75.2 | 12.2 |
| A +sanzuohua ngcaotong | *Echino chloa caudat a Roshev* | F | 1.5+30 | 1:20 | 65.9 | 56.7 | 98.4 | 85.2 | 13.2 |
| A +benzuofuc aotong | *Echino chloa caudat a Roshev* | F | 1.5+15 | 1:10 | 65.9 | 25.2 | 89.9 | 74.5 | 15.4 |
| A+CpdB1 | *Echino chloa caudat a Roshev* | F | 1.5+7.5 | 1:5 | 65.9 | 47.8 | 96.2 | 82.2 | 14.0 |
| A +glyphosat e | *Cyperu* s *rotund us* | F | 7.5+300 | 1:40 | 52.4 | 19.4 | 82.4 | 61.6 | 20.8 |
| A +glyphosat e | *Conyza Canad ensis* | F | 45+450 | 1:10 | 75.6 | 51.2 | 100.0 | 88.1 | 11.9 |
| A +glufosinat e ammonium | *Cyperu* s *serotin us* | F | 15+300 | 1:20 | 47.3 | 23.1 | 90.2 | 59.5 | 30.7 |
| A +glufosinat e ammonium | *Conyza Canad ensis* | F | 45+300 | 3:20 | 75.6 | 57.8 | 100.0 | 89.7 | 10.3 |
| A +glufosinat e-P-ammoniu m | *Cyperu* s *serotin us* | F | 15+150 | 1:10 | 47.3 | 21.4 | 85.3 | 58.6 | 26.7 |
| A +paraquat dichloride | *Cyperu* s *difform is* | F | 15+150 | 1:10 | 42.2 | 35.7 | 87.4 | 62.8 | 24.6 |
| A +paraquat dichloride | *Conyza Canad ensis* | F | 45+225 | 1:5 | 75.6 | 48.4 | 100.0 | 87.4 | 12.6 |
| A+diquat dibromide monohydrate | *Cyperu* s *difform is* | F | 15+300 | 1:20 | 42.2 | 18.4 | 79.2 | 52.8 | 26.4 |
| A+diquat dibromide monohydrate | *Conyza Canad ensis* | F | 45+300 | 3:20 | 75.6 | 37.8 | 100.0 | 84.8 | 15.2 |
| A+flurtamon e | *Capsel la bursa-pastori* s | F | 0.75+75 | 1:100 | 48.3 | 56.7 | 95.4 | 77.6 | 17.8 |
| A +difl ufenic an | *Capsel la bursa-pastori* s | F | 0.75+75 | 1:100 | 48.3 | 33.1 | 86.7 | 65.4 | 21.3 |
| A +picolinafe n | *Capsel la bursa-pastori* s | F | 0.75+45 | 1:60 | 48.3 | 44.4 | 91.5 | 71.3 | 20.2 |
| A+clomazon e | *Eleusin e indica* | F | 3+150 | 1:50 | 47.8 | 46.5 | 95.1 | 72.1 | 23.0 |
| A +bixlozone | *Eleusin e indica* | F | 3+180 | 1:60 | 47.8 | 36.7 | 88.8 | 67.0 | 21.8 |
| A +tribenuron -methyl | *Malac hium aquatic um* | F | 7.5+3 | 5:2 | 62.8 | 27.9 | 90.1 | 73.2 | 16.9 |
| A +thifensulf uron methyl | *Malac hium aquatic um* | F | 7.5+4.5 | 5:3 | 62.8 | 30.8 | 87.2 | 74.3 | 12.9 |
| A +pyrazosulf uron-ethyl | *Malac hium aquatic um* | F | 7.5+7.5 | 1:1 | 62.8 | 35.7 | 89.5 | 76.1 | 13.4 |
| A +thiencarba zone-methyl | *Malac hium aquatic um* | F | 7.5+3 | 5:2 | 62.8 | 41.1 | 93.3 | 78.1 | 15.2 |
| A+halosulfur on methyl | *Malac hium aquatic um* | F | 7.5+9 | 5:6 | 62.8 | 36.4 | 94.7 | 76.3 | 18.4 |
| A +rimsulfuro n | *Malac hium aquatic um* | F | 7.5+1.5 | 5:1 | 62.8 | 26.2 | 92.4 | 72.5 | 19.9 |
| A +nicosulfur on | *Malac hium aquatic um* | F | 7.5+3 | 5:2 | 62.8 | 31.4 | 88.5 | 74.5 | 14.0 |
| A +imazamox | *Malac hium aquatic um* | F | 7.5+15 | 1:2 | 62.8 | 27.2 | 91.2 | 72.9 | 18.3 |
| A +clethodim | *Eriochl oa villosa* | F | 0.75+30 | 1:40 | 42.9 | 35.5 | 89.5 | 63.2 | 26.3 |
| A +sethoxydi m | *Eriochl oa villosa* | F | 0.75+45 | 1:60 | 42.9 | 36.7 | 92.3 | 63.9 | 28.4 |
| A +quizalofop -P-methyl | *Eriochl oa villosa* | F | 0.75+15 | 1:20 | 42.9 | 41.7 | 87.3 | 66.7 | 20.6 |
| A +oxyfluorfe n | *Lithosp ermum arvens e* | F | 3+60 | 1:20 | 63.5 | 31.5 | 91.4 | 75.0 | 16.4 |
| A +oxadiazon | *Lithosp ermum arvens e* | F | 3+90 | 1:30 | 63.5 | 24.3 | 92.1 | 72.4 | 19.7 |
| A +oxadiargyl | *Lithosp ermum arvens e* | F | 3+30 | 1:10 | 63.5 | 38.7 | 89.8 | 77.6 | 12.2 |
| A +sulfentraz one | *Lithosp ermum arvens e* | F | 3+90 | 1:30 | 63.5 | 26.5 | 94.1 | 73.2 | 20.9 |
| A +pyraclonil | *Lithosp ermum arvens e* | F | 3+75 | 1:25 | 63.5 | 37.9 | 90.5 | 77.3 | 13.2 |
| A+flumioxaz in | *Lithosp ermum arvens e* | F | 3+7.5 | 2:5 | 63.5 | 29.4 | 92.4 | 74.2 | 18.2 |
| A +saflufenac il | *Lithosp ermum arvens e* | F | 3+0.75 | 4:1 | 63.5 | 39.7 | 98.4 | 78.0 | 20.4 |
| A +carfentraz one-ethyl | *Lithosp ermum arvens e* | F | 3+4.5 | 2:3 | 63.5 | 27.2 | 89.3 | 73.4 | 15.9 |
| A +trifludimo xazin | *Lithosp ermum arvens e* | F | 3+4.5 | 2:3 | 63.5 | 31.8 | 94.8 | 75.1 | 19.7 |
| A+metribuzi n | *Eclipta prostra te* | F | 0.75+15 | 1:20 | 59.2 | 36.3 | 89.3 | 74.0 | 15.3 |
| A+terbuthyla zinc | *Eclipta prostra te* | F | 0.75+150 | 1:200 | 59.2 | 31.7 | 94.1 | 72.1 | 22.0 |
| A +amicarbaz one | *Eclipta prostra te* | F | 0.75+60 | 1:80 | 59.2 | 43.7 | 90.7 | 77.0 | 13.7 |
| A+chlorotolu ron | *Eclipta prostra te* | F | 0.75+225 | 1:300 | 59.2 | 24.5 | 86.2 | 69.2 | 17.0 |
| A +isoproturo n | *Eclipta prostra te* | F | 0.75+225 | 1:300 | 59.2 | 33.9 | 92.5 | 73.0 | 19.5 |
| A +bromacil | *Eclipta prostra te* | F | 0.75+450 | 1:600 | 59.2 | 28.4 | 83.9 | 70.8 | 13.1 |
| A +propanil | *Eclipta prostra te* | F | 0.75+300 | 1:400 | 59.2 | 21.7 | 93.8 | 68.1 | 25.7 |
| A +desmedip ham | *Eclipta prostra te* | F | 0.75+300 | 1:400 | 59.2 | 16.2 | 90.5 | 65.8 | 24.7 |
| A +phenmedi pham | *Eclipta prostra te* | F | 0.75+300 | 1:400 | 59.2 | 20.8 | 86.2 | 67.7 | 18.5 |
| A +bentazone | *Eclipta prostra te* | F | 0.75+150 | 1:200 | 59.2 | 30.2 | 88.4 | 71.5 | 16.9 |
| A +bromoxyn il | *Eclipta prostra te* | F | 0.75+60 | 1:80 | 59.2 | 41.2 | 92.6 | 76.0 | 16.6 |
| A +butralin | *Leptoc hloa chinen sis* | S | 3+180 | 1:60 | 33.2 | 47.4 | 92.3 | 64.9 | 27.4 |
| A +pendimeth alin | *Leptoc hloa chinen sis* | S | 3+150 | 1:50 | 33.2 | 43.2 | 85.8 | 62.1 | 23.7 |
| A +butachlor | *Descur ainia sophia* | S | 3+225 | 1:75 | 40.2 | 42.2 | 92.3 | 65.4 | 26.9 |
| A +pretilachl o r | *Descur ainia sophia* | S | 3+180 | 1:60 | 40.2 | 51.2 | 95.7 | 70.8 | 24.9 |
| A+mefenacet | *Descur ainia sophia* | S | 3+150 | 1:50 | 40.2 | 46.4 | 89.6 | 67.9 | 21.7 |
| A +s-metolac hlor | *Descur ainia sophia* | S | 3+150 | 1:50 | 40.2 | 31.3 | 94.3 | 58.9 | 35.4 |
| A+flufenacet | *Descur ainia sophia* | S | 3+150 | 1:50 | 40.2 | 45.6 | 96.3 | 67.5 | 28.8 |
| A +pyroxasulf one | *Descur ainia sophia* | S | 3+60 | 1:20 | 40.2 | 57.6 | 98.2 | 74.6 | 23.6 |
| A +anilofos | *Descur ainia sophia* | S | 3+75 | 1:25 | 40.2 | 38.3 | 97.3 | 63.1 | 34.2 |
| A +prosulfoca rb | *Echino chloa crusgal li* | S | 15+600 | 1:40 | 51.7 | 32.6 | 86.9 | 67.4 | 19.5 |
| A+ Cpd B2 | *Veronica didyma* Tenore | F | 0.75+90 | 1:120 | 57.3 | 42.1 | 92.6 | 75.3 | 17.3 |
| A+fluroxypyr | *Veronic didyma* Tenore | F | 0.75+60 | 1:80 | 57.3 | 39.3 | 90.3 | 74.1 | 16.2 |
| A +florpyraux ifen benzyl | *Veronic a didyma* Tenore | F | 0.75+15 | 1:20 | 57.3 | 50.3 | 91.5 | 78.8 | 12.7 |
| A +halauxifen -methyl | *Veronic a didyma* Tenore | F | 0.75+3 | 1:4 | 57.3 | 35.6 | 87.6 | 72.5 | 15.1 |
| A+triclopyr | *Veronic didyma* Tenore | F | 0.75+90 | 1:120 | 57.3 | 34.2 | 91.5 | 71.9 | 19.6 |
| A+clopyralid | *Veronic didyma* Tenore | F | 0.75+45 | 1:60 | 57.3 | 28.9 | 83.3 | 69.6 | 13.7 |
| A +picloram | *Veronic didyma* Tenore | F | 0.75+300 | 1:400 | 57.3 | 43.6 | 88.7 | 75.9 | 12.8 |
| A +aminopyra lid | *Veronic a didyma* Tenore | F | 0.75+30 | 1:40 | 57.3 | 32.2 | 90.2 | 71.0 | 19.2 |
| A+dicamba | *Veronic didyma* Tenore | F | 0.75+150 | 1:200 | 57.3 | 29.7 | 87.3 | 70.0 | 17.3 |
| A+2-methyl-4-chlorophen oxyacetic acid | *Veronic a didyma* Tenore | F | 0.75+150 | 1:200 | 57.3 | 39.3 | 90.9 | 74.1 | 16.8 |
| A+2,4-dichlo rophenoxy acetic acid | *Veronic a didyma* Tenore | F | 0.75+150 | 1:200 | 57.3 | 32.6 | 85.2 | 71.2 | 14.0 |
| A +triaziflam | *Amara nthus retrofle* xus | S | 3+30 | 1:10 | 37.3 | 42.1 | 91.3 | 63.7 | 27.6 |
| A +indaziflam | *Amara nthus retrofle* xus | S | 3+15 | 1:5 | 37.3 | 49.3 | 93.3 | 68.2 | 25.1 |
| A +cinmethyli n | *Lolium multifl orum* Lamk. | S | 30+300 | 1:10 | 48.6 | 33.7 | 87.3 | 65.9 | 21.4 |

| | | | | | | | | | |
|---|---|---|---|---|---|---|---|---|---|
| Note: The compound number represented by A is 194 (R). | | | | | | | | | |

In addition, the present invention also provides other specific combinations of component A and component B, to further illustrate the composition of the present invention. The compounds in the column "Component A (Compound No.)" are identified in Table 1. The second column of Table B1 lists specific compounds (for example "topramezone" in the first row) for component B. The remaining rows of Table B1 are similarly constructed.

**Table B1. List of ingredients of the composition**

| Component A (Compound NO.) | Component B |
|---|---|
| 1 | topramezone |
| 1 | isoxaflutole |
| 1 | tembotrione |
| 1 | tefuryltrione |
| 1 | shuangzuocaotong |
| 1 | huanbifucaotong |
| 1 | sanzuohuangcaotong |
| 1 | benzuofucaotong |
| 1 | 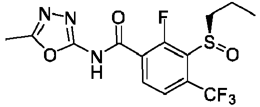 |
| 1 | glyphosate |
| 1 | glufosinate ammonium |
| 1 | glufosinate- P-ammonium |
| 1 | paraquat dichloride |
| 1 | diquat dibromide monohydrate |
| 1 | flurtamone |
| 1 | diflufenican |
| 1 | picolinafen |
| 1 | clomazone |
| 1 | bixlozone |
| 1 | tribenuron-methyl |
| 1 | thifensulfuron methyl |
| 1 | pyrazosulfuron -ethyl |
| 1 | thiencarbazone-methyl |
| 1 | halosulfuron methyl |
| 1 | rimsulfuron |
| 1 | nicosulfuron |
| 1 | imazamox |
| 1 | clethodim |
| 1 | sethoxydim |
| 1 | quizalofop-P-methyl |
| 1 | oxyfluorfen |
| 1 | oxadiazon |
| 1 | oxadiargyl |
| 1 | sulfentrazone |
| 1 | pyraclonil |
| 1 | flumioxazin |
| 1 | saflufenacil |
| 1 | carfentrazone-ethyl |
| 1 | trifludimoxazin |
| 1 | metribuzin |
| 1 | terbuthylazine |
| 1 | amicarbazone |
| 1 | chlorotoluron |
| 1 | isoproturon |
| 1 | bromacil |
| 1 | propanil |
| 1 | desmedipham |
| 1 | phenmedipham |
| 1 | bentazone |
| 1 | bromoxynil |
| 1 | butralin |
| 1 | pendimethalin |
| 1 | butachlor |
| 1 | pretilachlor |
| 1 | mefenacet |
| 1 | s-metolachlor |
| 1 | flufenacet |
| 1 | pyroxasulfone |
| 1 | anilofos |
| 1 | pro sulfoc arb |
| 1 | 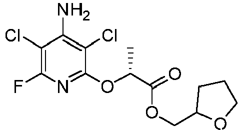 |
| 1 | fluroxypyr |
| 1 | florpyrauxifen benzyl |
| 1 | halauxifen-methyl |
| 1 | triclopyr |
| 1 | clopyralid |
| 1 | picloram |
| 1 | aminopyralid |
| 1 | dicamba |
| 1 | 2-methyl-4-chlorophenoxyacetic acid |
| 1 | 2,4-dichlorophenoxy acetic acid |
| 1 | triaziflam |
| 1 | indaziflam |
| 1 | cinmethylin |

Table B2 is constructed in the same way as that of Table B1 above, except for replacing the entries in the column "Component A (Compound No.)" with the corresponding entries in the column "Component A (Compound No.)" shown below. Therefore, for example, in Table B2, the entries in the column "Component A (Compound No.)" are all expressed as "2" (that is, Compound 2 identified in Table 1), and a mixture of Compound 2 and "topramezone" is specifically listed in the first row under the heading of Table B2. Tables B3 to B552 are similarly constructed.

| Table | "Component A (Compound NO.)" column entry | Table | " Component A (Compound NO.)" column , entry | Table | " Component A (Compound NO.)" column entry | Table | "Component A (Compound NO.)" column entry |
|---|---|---|---|---|---|---|---|
| B2 | 2 | B3 | 3 | B4 | 4 | B5 | 5 |
| B6 | 6 | B7 | 7 | B8 | 8 | B9 | 9 |
| B10 | 10 | B11 | 11 | B12 | 12 | B13 | 13 |
| B14 | 14 | B15 | 15 | B16 | 16 | B17 | 17 |
| B18 | 18 | B19 | 19 | B20 | 20 | B21 | 21 |
| B22 | 22 | B23 | 23 | B24 | 24 | B25 | 25 |
| B26 | 26 | B27 | 27 | B28 | 28 | B29 | 29 |
| B30 | 30 | B31 | 31 | B32 | 32 | B33 | 33 |
| B34 | 34 | B35 | 35 | B36 | 36 | B37 | 37 |
| B38 | 38 | B39 | 39 | B40 | 40 | B41 | 41 |
| B42 | 42 | B43 | 43 | B44 | 44 | B45 | 45 |
| B46 | 46 | B47 | 47 | B48 | 48 | B49 | 49 |
| B50 | 50 | B51 | 51 | B52 | 52 | B53 | 53 |
| B54 | 54 | B55 | 55 | B56 | 56 | B57 | 57 |
| B58 | 58 | B59 | 59 | B60 | 60 | B61 | 61 |
| B62 | 62 | B63 | 63 | B64 | 64 | B65 | 65 |
| B66 | 66 | B67 | 67 | B68 | 68 | B69 | 69 |
| B70 | 70 | B71 | 71 | B72 | 72 | B73 | 73 |
| B74 | 74 | B75 | 75 | B76 | 76 | B77 | 77 |
| B78 | 78 | B79 | 79 | B80 | 80 | B81 | 81 |
| B82 | 82 | B83 | 83 | B84 | 84 | B85 | 85 |
| B86 | 86 | B87 | 87 | B88 | 88 | B89 | 89 |
| B90 | 90 | B91 | 91 | B92 | 92 | B93 | 93 |
| B94 | 94 | B95 | 95 | B96 | 96 | B97 | 97 |
| B98 | 98 | B99 | 99 | B100 | 100 | B101 | 101 |
| B102 | 102 | B103 | 103 | B104 | 104 | B105 | 105 |
| B106 | 106 | B107 | 107 | B108 | 108 | B109 | 109 |
| B110 | 110 | B111 | 111 | B112 | 112 | B113 | 113 |
| B114 | 114 | B115 | 115 | B116 | 116 | B117 | 117 |
| B118 | 118 | B119 | 119 | B120 | 120 | B121 | 121 |
| B122 | 122 | B123 | 123 | B124 | 124 | B125 | 125 |
| B126 | 126 | B127 | 127 | B128 | 128 | B129 | 129 |
| B130 | 130 | B131 | 131 | B132 | 132 | B133 | 133 |
| B134 | 134 | B135 | 135 | B136 | 136 | B137 | 137 |
| B138 | 138 | B139 | 139 | B140 | 140 | B141 | 141 |
| B142 | 142 | B143 | 143 | B144 | 144 | B145 | 145 |
| B146 | 146 | B147 | 147 | B148 | 148 | B149 | 149 |
| B150 | 150 | B151 | 151 | B152 | 152 | B153 | 153 |
| B154 | 154 | B155 | 155 | B156 | 156 | B157 | 157 |
| B158 | 158 | B159 | 159 | B160 | 160 | B161 | 161 |
| B162 | 162 | B163 | 163 | B164 | 164 | B165 | 165 |
| B166 | 166 | B167 | 167 | B168 | 168 | B169 | 169 |
| B170 | 170 | B171 | 171 | B172 | 172 | B173 | 173 |
| B174 | 174 | B175 | 175 | B176 | 176 | B177 | 177 |
| B178 | 178 | B179 | 179 | B180 | 180 | B181 | 181 |
| B182 | 182 | B183 | 183 | B184 | 184 | B185 | 185 |
| B186 | 186 | B187 | 187 | B188 | 188 | B189 | 189 |
| B190 | 190 | B191 | 191 | B192 | 192 | B193 | 193 |
| B194 | 194 | B195 | 195 | B196 | 196 | B197 | 197 |
| B198 | 198 | B199 | 199 | B200 | 200 | B201 | 201 |
| B202 | 202 | B203 | 203 | B204 | 204 | B205 | 205 |
| B206 | 206 | B207 | 207 | B208 | 208 | B209 | 209 |
| B210 | 210 | B211 | 211 | B212 | 212 | B213 | 213 |
| B214 | 214 | B215 | 215 | B216 | 216 | B217 | 217 |
| B218 | 218 | B219 | 219 | B220 | 220 | B221 | 221 |
| B222 | 222 | B223 | 223 | B224 | 224 | B225 | 225 |
| B226 | 226 | B227 | 227 | B228 | 228 | B229 | 229 |
| B230 | 230 | B231 | 231 | B232 | 232 | B233 | 233 |
| B234 | 234 | B235 | 235 | B236 | 236 | B237 | 237 |
| B238 | 238 | B239 | 239 | B240 | 240 | B241 | 241 |
| B242 | 242 | B243 | 243 | B244 | 244 | B245 | 245 |
| B246 | 246 | B247 | 247 | B248 | 248 | B249 | 249 |
| B250 | 250 | B251 | 251 | B252 | 252 | B253 | 253 |
| B254 | 254 | B255 | 255 | B256 | 256 | B257 | 257 |
| B258 | 258 | B259 | 259 | B260 | 260 | B261 | 261 |
| B262 | 262 | B263 | 263 | B264 | 264 | B265 | 265 |
| B266 | 266 | B267 | 267 | B268 | 268 | B269 | 269 |
| B270 | 270 | B271 | 271 | B272 | 272 | B273 | 273 |
| B274 | 274 | B275 | 275 | B276 | 276 | B277 | 277 |
| B278 | 278 | B279 | 279 | B280 | 280 | B281 | 281 |
| B282 | 282 | B283 | 283 | B284 | 284 | B285 | 285 |
| B286 | 286 | B287 | 287 | B288 | 288 | B289 | 289 |
| B290 | 290 | B291 | 291 | B292 | 292 | B293 | 293 |
| B294 | 294 | B295 | 295 | B296 | 296 | B297 | 297 |
| B298 | 298 | B299 | 299 | B300 | 300 | B301 | 301 |
| B302 | 302 | B303 | 303 | B304 | 304 | B305 | 305 |
| B306 | 306 | B307 | 307 | B308 | 308 | B309 | 309 |
| B310 | 310 | B311 | 311 | B312 | 312 | B313 | 313 |
| B314 | 314 | B315 | 315 | B316 | 316 | B317 | 317 |
| B318 | 318 | B319 | 319 | B320 | 320 | B321 | 321 |
| B322 | 322 | B323 | 323 | B324 | 324 | B325 | 325 |
| B326 | 326 | B327 | 327 | B328 | 328 | B329 | 329 |
| B330 | 330 | B331 | 331 | B332 | 332 | B333 | 333 |
| B334 | 334 | B335 | 335 | B336 | 336 | B337 | 337 |
| B338 | 338 | B339 | 339 | B340 | 340 | B341 | 341 |
| B342 | 342 | B343 | 343 | B344 | 344 | B345 | 345 |
| B346 | 346 | B347 | 347 | B348 | 348 | B349 | 349 |
| B350 | 350 | B351 | 351 | B352 | 352 | B353 | 353 |
| B354 | 354 | B355 | 355 | B356 | 356 | B357 | 357 |
| B358 | 358 | B359 | 359 | B360 | 360 | B361 | 361 |
| B362 | 362 | B363 | 363 | B364 | 364 | B365 | 365 |
| B366 | 366 | B367 | 367 | B368 | 368 | B369 | 369 |
| B370 | 370 | B371 | 371 | B372 | 372 | B373 | 373 |
| B374 | 374 | B375 | 375 | B376 | 376 | B377 | 377 |
| B378 | 378 | B379 | 379 | B380 | 380 | B381 | 381 |
| B382 | 382 | B383 | 383 | B384 | 384 | B385 | 385 |
| B386 | 386 | B387 | 387 | B388 | 388 | B389 | 389 |
| B390 | 390 | B391 | 391 | B392 | 392 | B393 | 393 |
| B394 | 394 | B395 | 395 | B396 | 396 | B397 | 397 |
| B398 | 398 | B399 | 399 | B400 | 400 | B401 | 401 |
| B402 | 402 | B403 | 403 | B404 | 404 | B405 | 405 |
| B406 | 406 | B407 | 407 | B408 | 408 | B409 | 409 |
| B410 | 410 | B411 | 411 | B412 | 412 | B413 | 413 |
| B414 | 414 | B415 | 415 | B416 | 416 | B417 | 417 |
| B418 | 418 | B419 | 419 | B420 | 420 | B421 | 421 |
| B422 | 422 | B423 | 423 | B424 | 424 | B425 | 425 |
| B426 | 426 | B427 | 427 | B428 | 428 | B429 | 429 |
| B430 | 430 | B431 | 431 | B432 | 432 | B433 | 433 |
| B434 | 434 | B435 | 435 | B436 | 436 | B437 | 437 |
| B438 | 438 | B439 | 439 | B440 | 440 | B441 | 441 |
| B442 | 442 | B443 | 443 | B444 | 444 | B445 | 445 |
| B446 | 446 | B447 | 447 | B448 | 448 | B449 | 449 |
| B450 | 450 | B451 | 451 | B452 | 452 | B453 | 453 |
| B454 | 454 | B455 | 455 | B456 | 456 | B457 | 457 |
| B458 | 458 | B459 | 459 | B460 | 460 | B461 | 461 |
| B462 | 462 | B463 | 463 | B464 | 464 | B465 | 465 |
| B466 | 466 | B467 | 467 | B468 | 468 | B469 | 469 |
| B470 | 470 | B471 | 471 | B472 | 472 | B473 | 473 |
| B474 | 474 | B475 | 475 | B476 | 476 | B477 | 477 |
| B478 | 478 | B479 | 479 | B480 | 480 | B481 | 481 |
| B482 | 482 | B483 | 483 | B484 | 484 | B485 | 485 |
| B486 | 486 | B487 | 487 | B488 | 488 | B489 | 489 |
| B490 | 490 | B491 | 491 | B492 | 492 | B493 | 493 |
| B494 | 494 | B495 | 495 | B496 | 496 | B497 | 497 |
| B498 | 498 | B499 | 499 | B500 | 500 | B501 | 501 |
| B502 | 502 | B503 | 503 | B504 | 504 | B505 | 505 |
| B506 | 506 | B507 | 507 | B508 | 508 | B509 | 509 |
| B510 | 510 | B511 | 511 | B512 | 512 | B513 | 513 |
| B514 | 514 | B515 | 515 | B516 | 516 | B517 | 517 |
| B518 | 518 | B519 | 519 | B520 | 520 | B521 | 521 |
| B522 | 522 | B523 | 523 | B524 | 524 | B525 | 525 |
| B526 | 526 | B527 | 527 | B528 | 528 | B529 | 529 |
| B530 | 530 | B531 | 531 | B532 | 532 | B533 | 533 |
| B534 | 534 | B535 | 535 | B536 | 536 | B537 | 537 |
| B538 | 538 | B539 | 539 | B540 | 540 | B541 | 541 |
| B542 | 542 | B543 | 543 | B544 | 544 | B545 | 545 |
| B546 | 546 | B547 | 547 | B548 | 692 | B549 | 730 |
| B550 | 881 | B551 | 885 | B552 | 919 | | |

Table C1 is constructed in the same way as that of Table B1 above, except for replacing the entries in the column "Component A (Compound No.)" with the corresponding entries in the column "Component A (Compound No.)" shown below. Therefore, for example, in Table C1, the entries in the column "Component A (Compound No.)" are all expressed as "1(R)" (that is, the R configuration of Compound 1 identified in Table A), and a mixture of Compound 1(R) and "topramezone" is specifically listed in the first row under the heading of Table C1. Tables C2 to C535 are similarly constructed.

| Table | "Component A (Compound NO.)" column entry | Table | "Component A (Compound NO.)" column entry | Table | "Component A (Compound NO.)" column entry | Table | "Component A (Compound NO.)" column entry |
|---|---|---|---|---|---|---|---|
| C2 | 2(R) | C3 | 3(R) | C4 | 4(R) | C5 | 5(R) |
| C6 | 6(R) | C7 | 7(R) | C8 | 8(R) | C9 | 9(R) |
| C10 | 10(R) | C11 | 11(R) | C12 | 12(R) | C13 | 13(R) |
| C14 | 14(R) | C15 | 15(R) | C16 | 16(R) | C17 | 17(R) |
| C18 | 18(R) | C19 | 19(R) | C20 | 20(R) | C21 | 21(R) |
| C22 | 22(R) | C23 | 23(R) | C24 | 24(R) | C25 | 25(R) |
| C26 | 26(R) | C27 | 27(R) | C28 | 28(R) | C29 | 29(R) |
| C30 | 30(R) | C31 | 31(R) | C32 | 32(R) | C33 | 33(R) |
| C34 | 34(R) | C35 | 35(R) | C36 | 36(R) | C37 | 37(R) |
| C38 | 38(R) | C39 | 39(R) | C40 | 40(R) | C41 | 41(R) |
| C42 | 42(R) | C43 | 43(R) | C44 | 44(R) | C45 | 45(R) |
| C46 | 46(R) | C47 | 47(R) | C48 | 48(R) | C49 | 49(R) |
| C50 | 50(R) | C51 | 51(R) | C52 | 52(R) | C53 | 53(R) |
| C54 | 54(R) | C55 | 55(R) | C56 | 56(R) | C57 | 57(R) |
| C58 | 58(R) | C59 | 59(R) | C60 | 60(R) | C61 | 61(R) |
| C62 | 62(R) | C63 | 63(R) | C64 | 64(R) | C65 | 65(R) |
| C66 | 66(R) | C67 | 67(R) | C68 | 68(R) | C69 | 69(R) |
| C70 | 70(R) | C71 | 71(R) | C72 | 72(R) | C73 | 73(R) |
| C74 | 74(R) | C75 | 75(R) | C76 | 76(R) | C77 | 77(R) |
| C78 | 78(R) | C79 | 79(R) | C80 | 80(R) | C81 | 81(R) |
| C82 | 82(R) | C83 | 83(R) | C84 | 84(R) | C85 | 85(R) |
| C86 | 86(R) | C87 | 87(R) | C88 | 88(R) | C89 | 89(R) |
| C90 | 90(R) | C91 | 91(R) | C92 | 92(R) | C93 | 93(R) |
| C94 | 94(R) | C95 | 95(R) | C96 | 96(R) | C97 | 97(R) |
| C98 | 98(R) | C99 | 99(R) | C100 | 100(R) | C101 | 101(R) |
| C102 | 102(R) | C103 | 103(R) | C104 | 104(R) | C105 | 105(R) |
| C106 | 106(R) | C107 | 107(R) | C108 | 108(R) | C109 | 109(R) |
| C110 | 110(R) | C111 | 111(R) | C112 | 112(R) | C113 | 113(R) |
| C114 | 114(R) | C115 | 115(R) | C116 | 116(R) | C117 | 117(R) |
| C118 | 118(R) | C119 | 119(R) | C120 | 120(R) | C121 | 121(R) |
| C122 | 122(R) | C123 | 123(R) | C124 | 124(R) | C125 | 125(R) |
| C126 | 126(R) | C127 | 127(R) | C128 | 128(R) | C129 | 129(R) |
| C130 | 130(R) | C131 | 131(R) | C132 | 132(R) | C133 | 133(R) |
| C134 | 134(R) | C135 | 135(R) | C136 | 136(R) | C137 | 137(R) |
| C138 | 138(R) | C139 | 139(R) | C140 | 140(R) | C141 | 141(R) |
| C142 | 142(R) | C143 | 143(R) | C144 | 144(R) | C145 | 145(R) |
| C146 | 146(R) | C147 | 147(R) | C148 | 148(R) | C149 | 149(R) |
| C150 | 150(R) | C151 | 151(R) | C152 | 152(R) | C153 | 153(R) |
| C154 | 154(R) | C155 | 155(R) | C156 | 156(R) | C157 | 157(R) |
| C158 | 158(R) | C159 | 159(R) | C160 | 160(R) | C161 | 161(R) |
| C162 | 162(R) | C163 | 163(R) | C164 | 164(R) | C165 | 165(R) |
| C166 | 166(R) | C167 | 167(R) | C168 | 168(R) | C169 | 169(R) |
| C170 | 170(R) | C171 | 171(R) | C172 | 172(R) | C173 | 173(R) |
| C174 | 174(R) | C175 | 175(R) | C176 | 176(R) | C177 | 177(R) |
| C178 | 178(R) | C179 | 179(R) | C180 | 180(R) | C181 | 181(R) |
| C182 | 182(R) | C183 | 183(R) | C184 | 184(R) | C185 | 185(R) |
| C186 | 186(R) | C187 | 187(R) | C188 | 188(R) | C189 | 193(R) |
| C190 | 547(R) | C191 | 195(R) | C192 | 196(R) | C193 | 197(R) |
| C194 | 198(R) | C195 | 199(R) | C196 | 200(R) | C197 | 201(R) |
| C198 | 202(R) | C199 | 203(R) | C200 | 204(R) | C201 | 205(R) |
| C202 | 206(R) | C203 | 207(R) | C204 | 208(R) | C205 | 209(R) |
| C206 | 210(R) | C207 | 211(R) | C208 | 212(R) | C209 | 213(R) |
| C210 | 214(R) | C211 | 215(R) | C212 | 216(R) | C213 | 217(R) |
| C214 | 218(R) | C215 | 219(R) | C216 | 220(R) | C217 | 221(R) |
| C218 | 222(R) | C219 | 223(R) | C220 | 224(R) | C221 | 225(R) |
| C222 | 226(R) | C223 | 227(R) | C224 | 228(R) | C225 | 229(R) |
| C226 | 230(R) | C227 | 231(R) | C228 | 232(R) | C229 | 233(R) |
| C230 | 234(R) | C231 | 235(R) | C232 | 236(R) | C233 | 237(R) |
| C234 | 238(R) | C235 | 239(R) | C236 | 240(R) | C237 | 241(R) |
| C238 | 242(R) | C239 | 243(R) | C240 | 244(R) | C241 | 245(R) |
| C242 | 246(R) | C243 | 247(R) | C244 | 248(R) | C245 | 249(R) |
| C246 | 250(R) | C247 | 251(R) | C248 | 252(R) | C249 | 253(R) |
| C250 | 254(R) | C251 | 255(R) | C252 | 256(R) | C253 | 257(R) |
| C254 | 258(R) | C255 | 259(R) | C256 | 260(R) | C257 | 261(R) |
| C258 | 262(R) | C259 | 263(R) | C260 | 264(R) | C261 | 265(R) |
| C262 | 266(R) | C263 | 267(R) | C264 | 268(R) | C265 | 269(R) |
| C266 | 270(R) | C267 | 271(R) | C268 | 272(R) | C269 | 273(R) |
| C270 | 274(R) | C271 | 275(R) | C272 | 276(R) | C273 | 277(R) |
| C274 | 278(R) | C275 | 279(R) | C276 | 280(R) | C277 | 281(R) |
| C278 | 282(R) | C279 | 283(R) | C280 | 284(R) | C281 | 285(R) |
| C282 | 286(R) | C283 | 287(R) | C284 | 288(R) | C285 | 289(R) |
| C286 | 290(R) | C287 | 291(R) | C288 | 292(R) | C289 | 293(R) |
| C290 | 294(R) | C291 | 295(R) | C292 | 296(R) | C293 | 297(R) |
| C294 | 298(R) | C295 | 299(R) | C296 | 300(R) | C297 | 301(R) |
| C298 | 302(R) | C299 | 303(R) | C300 | 304(R) | C301 | 305(R) |
| C302 | 306(R) | C303 | 307(R) | C304 | 308(R) | C305 | 309(R) |
| C306 | 310(R) | C307 | 311(R) | C308 | 312(R) | C309 | 313(R) |
| C310 | 314(R) | C311 | 315(R) | C312 | 316(R) | C313 | 317(R) |
| C314 | 318(R) | C315 | 319(R) | C316 | 320(R) | C317 | 321(R) |
| C318 | 322(R) | C319 | 323(R) | C320 | 324(R) | C321 | 325(R) |
| C322 | 326(R) | C323 | 327(R) | C324 | 328(R) | C325 | 329(R) |
| C326 | 330(R) | C327 | 331(R) | C328 | 332(R) | C329 | 333(R) |
| C330 | 334(R) | C331 | 335(R) | C332 | 336(R) | C333 | 337(R) |
| C334 | 338(R) | C335 | 339(R) | C336 | 340(R) | C337 | 341(R) |
| C338 | 342(R) | C339 | 343(R) | C340 | 344(R) | C341 | 345(R) |
| C342 | 346(R) | C343 | 347(R) | C344 | 348(R) | C345 | 349(R) |
| C346 | 350(R) | C347 | 351(R) | C348 | 352(R) | C349 | 353(R) |
| C350 | 354(R) | C351 | 355(R) | C352 | 356(R) | C353 | 357(R) |
| C354 | 358(R) | C355 | 359(R) | C356 | 360(R) | C357 | 361(R) |
| C358 | 362(R) | C359 | 363(R) | C360 | 364(R) | C361 | 365(R) |
| C362 | 366(R) | C363 | 367(R) | C364 | 368(R) | C365 | 369(R) |
| C366 | 370(R) | C367 | 371(R) | C368 | 372(R) | C369 | 373(R) |
| C370 | 374(R) | C371 | 375(R) | C372 | 376(R) | C373 | 377(R) |
| C374 | 378(R) | C375 | 379(R) | C376 | 380(R) | C377 | 381(R) |
| C378 | 382(R) | C379 | 383(R) | C380 | 384(R) | C381 | 385(R) |
| C382 | 386(R) | C383 | 387(R) | C384 | 388(R) | C385 | 389(R) |
| C386 | 390(R) | C387 | 391(R) | C388 | 392(R) | C389 | 393(R) |
| C390 | 394(R) | C391 | 395(R) | C392 | 396(R) | C393 | 397(R) |
| C394 | 398(R) | C395 | 399(R) | C396 | 400(R) | C397 | 401(R) |
| C398 | 402(R) | C399 | 403(R) | C400 | 404(R) | C401 | 405(R) |
| C402 | 406(R) | C403 | 407(R) | C404 | 408(R) | C405 | 409(R) |
| C406 | 410(R) | C407 | 411(R) | C408 | 412(R) | C409 | 413(R) |
| C410 | 414(R) | C411 | 415(R) | C412 | 416(R) | C413 | 417(R) |
| C414 | 418(R) | C415 | 419(R) | C416 | 420(R) | C417 | 421(R) |
| C418 | 422(R) | C419 | 423(R) | C420 | 424(R) | C421 | 425(R) |
| C422 | 426(R) | C423 | 427(R) | C424 | 428(R) | C425 | 429(R) |
| C426 | 430(R) | C427 | 431(R) | C428 | 432(R) | C429 | 438(R) |
| C430 | 439(R) | C431 | 503(R) | C432 | 441(R) | C433 | 442(R) |
| C434 | 443(R) | C435 | 444(R) | C436 | 445(R) | C437 | 446(R) |
| C438 | 447(R) | C439 | 448(R) | C440 | 449(R) | C441 | 450(R) |
| C442 | 451(R) | C443 | 452(R) | C444 | 453(R) | C445 | 454(R) |
| C446 | 455(R) | C447 | 456(R) | C448 | 457(R) | C449 | 458(R) |
| C450 | 459(R) | C451 | 460(R) | C452 | 461(R) | C453 | 462(R) |
| C454 | 463(R) | C455 | 464(R) | C456 | 465(R) | C457 | 466(R) |
| C458 | 467(R) | C459 | 468(R) | C460 | 469(R) | C461 | 508(R) |
| C462 | 471(R) | C463 | 472(R) | C464 | 473(R) | C465 | 474(R) |
| C466 | 475(R) | C467 | 476(R) | C468 | 477(R) | C469 | 478(R) |
| C470 | 533(R) | C471 | 542(R) | C472 | 481(R) | C473 | 482(R) |
| C474 | 483(R) | C475 | 484(R) | C476 | 543(R) | C477 | 486(R) |
| C478 | 487(R) | C479 | 488(R) | C480 | 489(R) | C481 | 490(R) |
| C482 | 491(R) | C483 | 492(R) | C484 | 493(R) | C485 | 692(R) |
| C486 | 495(R) | C487 | 496(R) | C488 | 497(R) | C489 | 498(R) |
| C490 | 499(R) | C491 | 500(R) | C492 | 501(R) | C493 | 502(R) |
| C494 | 504(R) | C495 | 505(R) | C496 | 506(R) | C497 | 507(R) |
| C498 | 509(R) | C499 | 510(R) | C500 | 511(R) | C501 | 512(R) |
| C502 | 513(R) | C503 | 514(R) | C504 | 515(R) | C505 | 516(R) |
| C506 | 517(R) | C507 | 518(R) | C508 | 519(R) | C509 | 520(R) |
| C510 | 521(R) | C511 | 522(R) | C512 | 523(R) | C513 | 524(R) |
| C514 | 525(R) | C515 | 526(R) | C516 | 527(R) | C517 | 528(R) |
| C518 | 529(R) | C519 | 530(R) | C520 | 531(R) | C521 | 532(R) |
| C522 | 534(R) | C523 | 535(R) | C524 | 536(R) | C525 | 537(R) |
| C526 | 538(R) | C527 | 539(R) | C528 | 540(R) | C529 | 541(R) |
| C530 | 544(R) | C531 | 545(R) | C532 | 730(R) | C1 | 1(R) |
| C533 | 881(R) | C534 | 885(R) | C535 | 919(R) | | |

At the same time, it is found after several tests that the compounds and compositions of the present invention have good selectivity to many gramineae grasses such as zoysia japonica, bermuda grass, tall fescue, bluegrass, ryegrass and seashore paspalum etc, and are able to control many important grass weeds and broad-leaved weeds. The compounds also show excellent selectivity and commercial value in the tests on sugarcane, soybean, cotton, oil sunflower, potato, orchards and vegetables in different herbicide application methods.

## Claims

1. A carboxylic acid derivative-substituted iminoaryl compound, represented by general formula I' :
Q represents 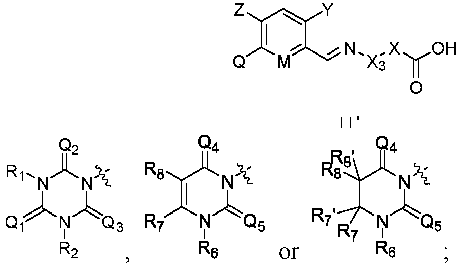
Y represents halogen, haloalkyl or cyano;
Z represents halogen;
M represents CH or N;
X represents -CX₁X₂-(alkyl)ₙ-, -alkyl-CX₁X₂-(alkyl)ₙ- or -(CH₂)ᵣ-;
X₁, X₂ each independently represent H, halogen, cyano, amino, nitro, formyl, cyanoalkyl, hydroxyalkyl, alkyl, alkenyl, alkynyl, cycloalkyl, cycloalkylalkyl, alkoxy, alkylthio, alkylamino, haloalkoxy, haloalkylthio, alkyl carbonyl, alkoxy carbonyl, alkoxyalkyl, haloalkoxyalkyl, alkylaminoalkyl, aryl, heterocyclyl, arylalkyl or heterocyclic alkyl, wherein, the "alkyl", "alkenyl" and "alkynyl" are each independently unsubstituted or substituted by halogen, the "cycloalkyl", "cycloalkylalkyl", "aryl", "heterocyclyl", "arylalkyl" and "heterocyclic alkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring; and X₁, X₂ are not hydrogen at the same time;
X₃ represents O, S, NH or N-alkyl;
Q₁, Q₂, Q₃, Q₄, Q₅ each independently represent O or S;
R₁, R₂ each independently represent H, cyano, alkyl, alkenyl, alkynyl, formyl alkyl, cyanoalkyl, amino, aminoalkyl, amino carbonyl, amino carbonylalkyl, aminosulfonyl, cycloalkyl, cycloalkylalkyl, cycloalkenyl, cycloalkenyl alkyl, heterocyclyl, heterocyclic alkyl, aryl, arylalkyl, R₄R₅N-(CO)-NR₃-, 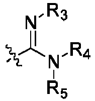 , R₃-S(O)ₘ-(alkyl)ₙ-, R₃-O-(alkyl)ₙ-, R₃-(CO)-(alkyl)ₙ-, R₃-O-(alkyl)ₙ-(CO)-, R₃-(CO)-O-(alkyl)ₙ-, R3-S-(CO)-(alkyl)n-, R₃-O-(CO)-alkyl- or R₃-O-(CO)-O-alkyl-, wherein,
the "alkyl", "alkenyl" and "alkynyl" are each independently unsubstituted or substituted by halogen,
the "amino", "aminoalkyl", "amino carbonyl", "amino carbonylalkyl" and "aminosulfonyl" are each independently unsubstituted or substituted by one or two groups selected from -R₁₁, -OR₁₁, -(CO)R₁₁, -(CO)OR₁₁, -alkyl-(CO)OR₁₁, -(SO₂)R₁₁, -(SO₂)OR₁₁, -alkyl-(SO₂)R₁₁, -(CO)N(R₁₂)₂ and -(SO₂)N(R₁₂)₂,
the "cycloalkyl", "cycloalkylalkyl", "cycloalkenyl", "cycloalkenyl alkyl", "heterocyclyl", "heterocyclic alkyl", "aryl" and "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
R₆ represents alkyl, alkenyl, alkynyl or cyano, wherein, the "alkyl", "alkenyl" and "alkynyl" are each independently unsubstituted or substituted by at least one group selected from halogen, alkoxy and alkoxy carbonyl;
R₇, R₇', R₈, R₈' each independently represent H, alkyl, halogen, haloalkyl, amino, hydroxyalkyl or alkoxy;
R₃, R₄, R₅ each independently represent H, alkyl, alkenyl, alkynyl, cycloalkyl, cycloalkylalkyl, cycloalkenyl, cycloalkenylalkyl, heterocyclyl, heterocyclic alkyl, aryl or arylalkyl, wherein, the "alkyl", "alkenyl" and "alkynyl" are each independently unsubstituted or substituted by halogen, the "cycloalkyl", "cycloalkylalkyl", "cycloalkenyl", "cycloalkenylalkyl", "heterocyclyl", "heterocyclic alkyl", "aryl" and "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
R₁₁ independently represents alkyl, alkenyl, alkynyl, cycloalkyl, cycloalkylalkyl, cycloalkenyl, cycloalkenylalkyl, phenyl, benzyl, wherein, the "alkyl", "alkenyl" and "alkynyl" are each independently unsubstituted or substituted by halogen, the "phenyl" and "benzyl" are each independently unsubstituted or substituted by at least one group selected from halogen, cyano, nitro, alkyl, haloalkyl, alkoxy carbonyl, alkylthio, alkylsulfonyl, alkoxy and haloalkoxy;
R₁₂ independently represents H, alkyl, alkenyl, alkynyl, alkoxy, alkylsulfonyl, cycloalkyl, cycloalkylalkyl, cycloalkenyl or cycloalkenylalkyl, or N(R₁₂)₂ in -(CO)N(R₁₂)₂ or -(SO₂)N(R₁₂)₂ each independently represents unsubstituted or substituted heterocyclyl with nitrogen atom at 1-position;
R₁₃ independently represents H, alkyl, haloalkyl, phenyl or phenyl substituted by at least one group selected from halogen, cyano, nitro, alkyl, haloalkyl, alkoxy carbonyl, alkylthio, alkylsulfonyl, alkoxy and haloalkoxy;
r represents an integer of 2 or more; m represents 0, 1 or 2; n independently represents 0 or 1;
the derivative means that the carboxylic acid functional group in the general formula is changed into any ester, acylhydrazide, imidate, thioimidate, amidine, amide, orthoester, acyl cyanide, acyl halide, thioester, thionoester, dithiolester, nitrile or any other carboxylic acid derivative.

2. The carboxylic acid derivative-substituted iminoaryl compound according to claim 1, which is **characterized in that** the compound is represented by general formula I: 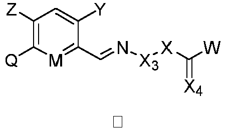
wherein, W represents OX₅, SX₅ or N(X₅)₂;
X₃, X₄ each independently represent O, S, NH or N-alkyl;
X₅ represents H, alkyl, alkenyl, alkynyl, cycloalkyl, cycloalkenyl, heterocyclyl, aryl, 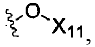 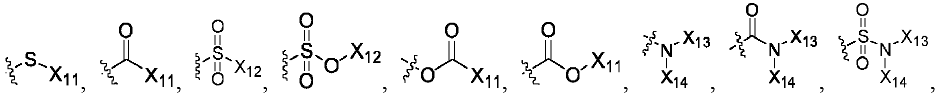 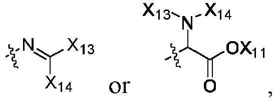 wherein, the "alkyl", "alkenyl" and "alkynyl" are each independently unsubstituted or substituted by at least one group selected from halogen, cyano, nitro, cycloalkyl, cycloalkenyl, heterocyclyl, aryl, 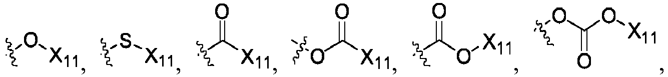 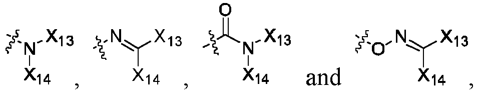 the "cycloalkyl", "cycloalkenyl", "heterocyclyl" and "aryl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
or N(X₅)₂ represents 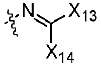 or unsubstituted or substituted heterocyclyl with nitrogen atom at 1-position;
X₁₁ independently represents H, alkyl, alkenyl, alkynyl, haloalkyl, haloalkenyl, haloalkynyl, cycloalkyl, cycloalkylalkyl, cycloalkenyl, cycloalkenyl alkyl, heterocyclyl, heterocyclic alkyl, aryl, arylalkyl or 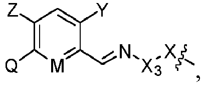 wherein, the "cycloalkyl", "cycloalkylalkyl", "cycloalkenyl", "cycloalkenyl alkyl", "heterocyclyl", "heterocyclic alkyl", "aryl" and "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
X₁₂ independently represents alkyl, alkenyl, alkynyl, haloalkyl, haloalkenyl, haloalkynyl, cycloalkyl, cycloalkylalkyl, cycloalkenyl, cycloalkenyl alkyl, heterocyclyl, heterocyclic alkyl, aryl or arylalkyl, wherein, the "cycloalkyl", "cycloalkylalkyl", "cycloalkenyl", "cycloalkenyl alkyl", "heterocyclyl", "heterocyclic alkyl", "aryl" and "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring;
X₁₃, X₁₄ each independently represent H, halogen, cyano, alkoxy, alkoxyalkyl, alkyl carbonyl, alkoxy carbonyl, alkylsulfonyl, alkyl, alkenyl, alkynyl, cycloalkyl, cycloalkylalkyl, cycloalkenyl, cycloalkenyl alkyl, aryl, arylalkyl, heterocyclyl or heterocyclic alkyl, or C, X₁₃, X₁₄, taken together, form unsubstituted or substituted cyclic structure, or N, X₁₃, X₁₄, taken together, form unsubstituted or substituted heterocyclyl with nitrogen atom at 1-position, wherein, the "alkyl", "alkenyl" and "alkynyl" are each independently unsubstituted or substituted by halogen, the "cycloalkyl", "cycloalkylalkyl", "cycloalkenyl", "cycloalkenyl alkyl", "aryl", "arylalkyl", "heterocyclyl" and "heterocyclic alkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -OR₁₃, -SR₁₃, -(CO)OR₁₃, -(SO₂)R₁₃, -N(R₁₃)₂ and -O-alkyl-(CO)OR₁₃, or two adjacent carbon atoms on the ring together with unsubstituted or halogen-substituted -OCH₂CH₂- or -OCH₂O- form a fused ring.

3. The carboxylic acid derivative-substituted iminoaryl compound according to claim 1 or 2, which is **characterized in that**,
Y represents halogen, halo C1-C8 alkyl or cyano;
X represents -CX₁X₂-(C1-C8 alkyl)ₙ-, -(C1-C8 alkyl)-CX₁X₂-(C1-C8 alkyl)ₙ- or -(CH₂)ᵣ-;
X₁, X₂ each independently represent H, halogen, cyano, amino, nitro, formyl, cyano C1-C8 alkyl, hydroxy C1-C8 alkyl, C1-C8 alkyl, C2-C8 alkenyl, C2-C8 alkynyl, C3-C8 cycloalkyl, C3-C8 cycloalkyl C1-C8 alkyl, C1-C8 alkoxy, C1-C8 alkylthio, C1-C8 alkylamino, halo C1-C8 alkoxy, halo C1-C8 alkylthio, C1-C8 alkyl carbonyl, C1-C8 alkoxy carbonyl, C1-C8 alkoxy C1-C8 alkyl, halo C1-C8 alkoxy C1-C8 alkyl, C1-C8 alkylamino C1-C8 alkyl, aryl, heterocyclyl, aryl C1-C8 alkyl or heterocyclyl C1-C8 alkyl, wherein, the "C1-C8 alkyl", "C2-C8 alkenyl" and "C2-C8 alkynyl" are each independently unsubstituted or substituted by halogen, the "C3-C8 cycloalkyl", "C3-C8 cycloalkyl C1-C8 alkyl", "aryl", "heterocyclyl", "aryl C1-C8 alkyl" and "heterocyclyl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring; and X₁, X₂ are not hydrogen at the same time;
R₁, R₂ each independently represent H, cyano, C1-C8 alkyl, C2-C8 alkenyl, C2-C8 alkynyl, formyl C1-C8 alkyl, cyano C1-C8 alkyl, amino, amino C1-C8 alkyl, amino carbonyl, amino carbonyl C1-C8 alkyl, aminosulfonyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl, aryl C1-C8 alkyl, R₄R₅N-(CO)-NR₃-, 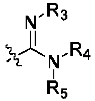 , R₃-S(O)ₘ-(C1-C8 alkyl)ₙ-, R₃-O-(C1-C8 alkyl)ₙ-, R₃-(CO)-(C1-C8 alkyl)n-, R₃-O-(C1-C8 alkyl)n-(CO)-, R₃-(CO)-O-(C1-C8 alkyl)n-, R₃-S-(CO)-(C1-C8 alkyl)n-, R₃-O-(CO)-(C1-C8 alkyl)- or R₃-O-(CO)-O-(C1-C8 alkyl)-, wherein,
the "C1-C8 alkyl", "C2-C8 alkenyl" and "C2-C8 alkynyl" are each independently unsubstituted or substituted by halogen,
the "amino", "amino C1-C8 alkyl", "amino carbonyl", "amino carbonyl C1-C8 alkyl" and "aminosulfonyl" are each independently unsubstituted or substituted by one or two groups selected from -R₁₁, -OR₁₁, -(CO)R₁₁, -(CO)OR₁₁, -(C1-C8 alkyl)-(CO)OR₁₁, -(SO₂)R₁₁, -(SO₂)OR₁₁, -(C1-C8 alkyl)-(SO₂)R₁₁, -(CO)N(R₁₂)₂ and -(SO₂)N(R₁₂)₂,
the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" and "aryl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
R₆ represents C1-C8 alkyl, C2-C8 alkenyl, C2-C8 alkynyl or cyano, wherein, the "C1-C8 alkyl", "C2-C8 alkenyl" and "C2-C8 alkynyl" are each independently unsubstituted or substituted by at least one group selected from halogen, C1-C8 alkoxy and C1-C8 alkoxy carbonyl;
R₇, R₇', R₈, R₈' each independently represent H, C1-C8 alkyl, halogen, halo C1-C8 alkyl, amino, hydroxy C1-C8 alkyl or C1-C8 alkoxy;
R₃, R₄, R₅ each independently represent H, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl or aryl C1-C8 alkyl, wherein, the "C1-C8 alkyl", "C2-C8 alkenyl" and "C2-C8 alkynyl" are each independently unsubstituted or substituted by halogen, the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" and "aryl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
R₁₁ independently represents C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, phenyl, benzyl, wherein, the "C1-C8 alkyl", "C2-C8 alkenyl" and "C2-C8 alkynyl" are each independently unsubstituted or substituted by halogen, the "phenyl" and "benzyl" are each independently unsubstituted or substituted by at least one group selected from halogen, cyano, nitro, C1-C8 alkyl, halo C1-C8 alkyl, C1-C8 alkoxy carbonyl, C1-C8 alkylthio, C1-C8 alkylsulfonyl, C1-C8 alkoxy and halo C1-C8 alkoxy;
R₁₂ independently represents H, C1-C8 alkyl, C2-C8 alkenyl, C2-C8 alkynyl, C1-C8 alkoxy, C1-C8 alkylsulfonyl, C3-C8 cycloalkyl, C3-C8 cycloalkyl C1-C8 alkyl, C3-C8 cycloalkenyl or C3-C8 cycloalkenyl C1-C8 alkyl, or N(R₁₂)₂ in -(CO)N(R₁₂)₂ or -(SO₂)N(R₁₂)₂ independently represents heterocyclyl 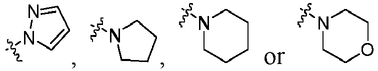 with nitrogen atom at 1-position that is unsubstituted or substituted by at least one group selected from oxo and C1-C8 alkyl;
R₁₃ independently represents H, C1-C8 alkyl, halo C1-C8 alkyl, phenyl or phenyl substituted by at least one group selected from halogen, cyano, nitro, C1-C8 alkyl, halo C1-C8 alkyl, C1-C8 alkoxy carbonyl, C1-C8 alkylthio, C1-C8 alkylsulfonyl, C1-C8 alkoxy and halo C1-C8 alkoxy;
r represents 2, 3, 4, 5 or 6;
preferably, when the general formula is I, X₃, X₄ each independently represent O, S, NH or N-(C1-C8)alkyl;
X₅ represents H, C1-C8 alkyl, C2-C8 alkenyl, C2-C8 alkynyl, C3-C8 cycloalkyl, C3-C8 cycloalkenyl, heterocyclyl, aryl, 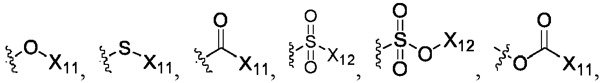 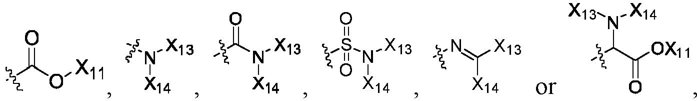 wherein, the "C1-C8 alkyl", "C2-C8 alkenyl" and "C2-C8 alkynyl" are each independently unsubstituted or substituted by at least one group selected from halogen, cyano, nitro, C3-C8 cycloalkyl, C3-C8 cycloalkenyl, heterocyclyl, aryl, 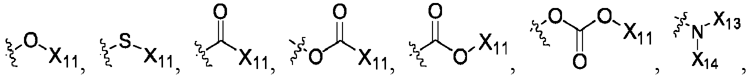 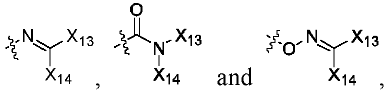 the "C3-C8 cycloalkyl", "C3-C8 cycloalkenyl", "heterocyclyl" and "aryl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
or N(X₅)₂ represents 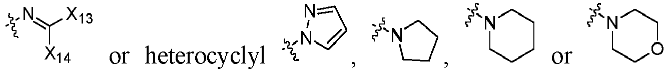 with nitrogen atom at 1-position that is unsubstituted or substituted by at least one group selected from oxo and C1-C8 alkyl;
X₁₁ independently represents H, C1-C8 alkyl, C2-C8 alkenyl, C2-C8 alkynyl, halo C1-C8 alkyl, halo C2-C8 alkenyl, halo C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl, aryl C1-C8 alkyl or 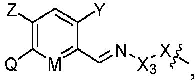 wherein, the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" and "aryl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
X₁₂ independently represents C1-C8 alkyl, C2-C8 alkenyl, C2-C8 alkynyl, halo C1-C8 alkyl, halo C2-C8 alkenyl, halo C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl or aryl C1-C8 alkyl, wherein, the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" and "aryl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring;
X₁₃, X₁₄ each independently represent H, halogen, cyano, C1-C8 alkoxy, C1-C8 alkoxy C1-C8 alkyl, C1-C8 alkyl carbonyl, C1-C8 alkoxy carbonyl, C1-C8 alkylsulfonyl, C1-C8 alkyl, C2-C8 alkenyl, C2-C8 alkynyl, C3-C8 cycloalkyl, C3-C8 cycloalkylalkyl, C3-C8 cycloalkenyl, C3-C8 cycloalkenyl C1-C8 alkyl, aryl, aryl C1-C8 alkyl, heterocyclyl or heterocyclyl C1-C8 alkyl, or C, X₁₃, X₁₄, taken together, form 5~8 membered carbocyclyl or oxygen, sulfur or nitrogen-containing heterocyclyl, or N, X₁₃, X₁₄, taken together, form heterocyclyl with nitrogen atom at 1-position, wherein, the "C1-C8 alkyl", "C2-C8 alkenyl" and "C2-C8 alkynyl" are each independently unsubstituted or substituted by halogen, the "C3-C8 cycloalkyl", "C3-C8 cycloalkyl C1-C8 alkyl", "C3-C8 cycloalkenyl", "C3-C8 cycloalkenyl C1-C8 alkyl", "aryl", "aryl C1-C8 alkyl", "heterocyclyl" and "heterocyclyl C1-C8 alkyl" are each independently unsubstituted or substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -OR₁₃, -SR₁₃, -(CO)OR₁₃, -(SO₂)R₁₃, -N(R₁₃)₂ and -O-(C1-C8 alkyl)-(CO)OR₁₃, or two adjacent carbon atoms on the ring together with unsubstituted or halogen-substituted -OCH₂CH₂- or -OCH₂O- form a fused ring, the "5~8 membered carbocyclyl or oxygen, sulfur or nitrogen-containing heterocyclyl" is unsubstituted or substituted by 1-4 groups selected from C1-C8 alkyl, C1-C8 alkoxy carbonyl and benzyl, or together with aryl or heterocyclyl forms a fused ring, the "heterocyclyl with nitrogen atom at 1-position"is unsubstituted or substituted by at least one group selected from oxo and C1-C8 alkyl.

4. The carboxylic acid derivative-substituted iminoaryl compound according to claim 2 or 3, which is **characterized in that**,
Y represents halogen, halo C1-C6 alkyl or cyano;
X represents -CX₁X₂-(C1-C6 alkyl)ₙ-, -(C1-C6 alkyl)-CX₁X₂-(C1-C6 alkyl)n- or -(CH₂)r-;
X₁, X₂ each independently represent H, halogen, cyano, amino, nitro, formyl, cyano C1-C6 alkyl, hydroxy C1-C6 alkyl, C1-C6 alkyl, C2-C6 alkenyl, C2-C6 alkynyl, C3-C6 cycloalkyl, C3-C6 cycloalkyl C1-C6 alkyl, C1-C6 alkoxy, C1-C6 alkylthio, C1-C6 alkylamino, halo C1-C6 alkoxy, halo C1-C6 alkylthio, C1-C6 alkyl carbonyl, C1-C6 alkoxy carbonyl, C1-C6 alkoxy C1-C6 alkyl, halo C1-C6 alkoxy C1-C6 alkyl, C1-C6 alkylamino C1-C6 alkyl, aryl, heterocyclyl, aryl C1-C6 alkyl or heterocyclyl C1-C6 alkyl, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C6 alkyl", "aryl", "heterocyclyl", "aryl C1-C6 alkyl" and "heterocyclyl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring; and X₁, X₂ are not hydrogen at the same time;
R₁, R₂ each independently represent H, cyano, C1-C6 alkyl, C2-C6 alkenyl, C2-C6 alkynyl, formyl C1-C6 alkyl, cyano C1-C6 alkyl, amino, amino C1-C6 alkyl, amino carbonyl, amino carbonyl C1-C6 alkyl, aminosulfonyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl, aryl C1-C6 alkyl, R₄R₅N-(CO)-NR₃-, 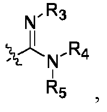 R₃-S(O)ₘ-(C1-C6 alkyl)ₙ-, R₃-O-(C1-C6 alkyl)ₙ-, R₃-(CO)-(C1-C6 alkyl)n-, R₃-O-(C1-C6 alkyl)n-(CO)-, R₃-(CO)-O-(C1-C6 alkyl)n-, R₃-S-(CO)-(C1-C6 alkyl)n-, R₃-O-(CO)-(C1-C6 alkyl)- or R₃-O-(CO)-O-(C1-C6 alkyl)-, wherein,
the "C1-C6 alkyl", "C2-C6 alkenyl" and "C2-C6 alkynyl" are each independently unsubstituted or substituted by halogen,
the "amino", "amino C1-C6 alkyl", "amino carbonyl", "amino carbonyl C1-C6 alkyl" and "aminosulfonyl" are each independently unsubstituted or substituted by one or two groups selected from -R₁₁, -OR₁₁, -(CO)R₁₁, -(CO)OR₁₁, -(C1-C6 alkyl)-(CO)OR₁₁, -(SO₂)R₁₁, -(SO₂)OR₁₁, -(C1-C6 alkyl)-(SO₂)R₁₁, -(CO)N(R₁₂)₂ and -(SO₂)N(R₁₂)₂,
the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" and "aryl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
R₆ represents C1-C6 alkyl, C2-C6 alkenyl, C2-C6 alkynyl or cyano, wherein, the "C1-C6 alkyl", "C2-C6 alkenyl" and "C2-C6 alkynyl" are each independently unsubstituted or substituted by 1, 2 or 3 groups selected from halogen, C1-C6 alkoxy and C1-C6 alkoxy carbonyl;
R₇, R₇', R₈, R₈' each independently represent H, C1-C6 alkyl, halogen, halo C1-C6 alkyl, amino, hydroxy C1-C6 alkyl or C1-C6 alkoxy;
R₃, R₄, R₅ each independently represent H, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl or aryl C1-C6 alkyl, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" and "aryl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
R₁₁ independently represents C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, phenyl, benzyl, wherein, the "C1-C6 alkyl", "C2-C6 alkenyl" and "C2-C6 alkynyl" are each independently unsubstituted or substituted by halogen, the "phenyl" and "benzyl"are each independently unsubstituted or substituted by 1, 2 or 3 groups selected from halogen, cyano, nitro, C1-C6 alkyl, halo C1-C6 alkyl, C1-C6 alkoxy carbonyl, C1-C6 alkylthio, C1-C6 alkylsulfonyl, C1-C6 alkoxy and halo C1-C6 alkoxy;
R₁₂ independently represents H, C1-C6 alkyl, C2-C6 alkenyl, C2-C6 alkynyl, C1-C6 alkoxy, C1-C6 alkylsulfonyl, C3-C6 cycloalkyl, C3-C6 cycloalkyl C1-C6 alkyl, C3-C6 cycloalkenyl or C3-C6 cycloalkenyl C1-C6 alkyl, or N(R₁₂)₂ in -(CO)N(R₁₂)₂ or -(SO₂)N(R₁₂)₂ independently represents heterocyclyl 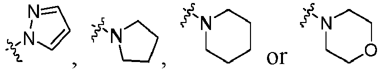 with nitrogen atom at 1-position that is unsubstituted or substituted by 1, 2 or 3 groups selected from oxo and C1-C6 alkyl;
R₁₃ independently represents H, C1-C6 alkyl, halo C1-C6 alkyl, phenyl or phenyl substituted by 1, 2 or 3 groups selected from halogen, cyano, nitro, C1-C6 alkyl, halo C1-C6 alkyl, C1-C6 alkoxy carbonyl, C1-C6 alkylthio, C1-C6 alkylsulfonyl, C1-C6 alkoxy and halo C1-C6 alkoxy;
preferably, when the general formula is I, X₃, X₄ each independently represent O, S, NH or N-(C1-C6)alkyl;
X₅ represents H, C1-C6 alkyl, C2-C6 alkenyl, C2-C6 alkynyl, C3-C6 cycloalkyl, C3-C6 cycloalkenyl, heterocyclyl, aryl, 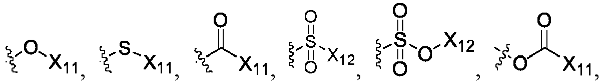 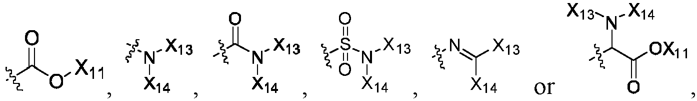 wherein, the "C1-C6 alkyl", "C2-C6 alkenyl" and "C2-C6 alkynyl" are each independently unsubstituted or substituted by at least one group selected from halogen, cyano, nitro, C3-C6 cycloalkyl, C3-C6 cycloalkenyl, heterocyclyl, aryl, 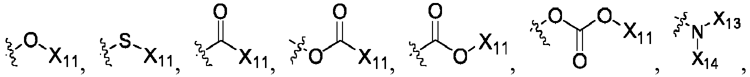 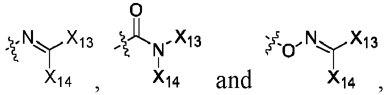 the "C3-C6 cycloalkyl", "C3-C6 cycloalkenyl", "heterocyclyl" and "aryl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
or N(X₅)₂ represents 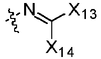 or heterocyclyl 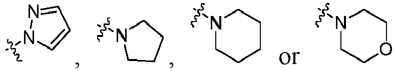 with nitrogen atom at 1-position that is unsubstituted or substituted by 1, 2 or 3 groups selected from oxo and C1-C6 alkyl;
X₁₁ independently represents H, C1-C6 alkyl, C2-C6 alkenyl, C2-C6 alkynyl, halo C1-C6 alkyl, halo C2-C6 alkenyl, halo C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl, aryl C1-C6 alkyl or 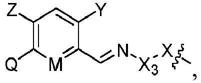 wherein, the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" and "aryl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
X₁₂ independently represents C1-C6 alkyl, C2-C6 alkenyl, C2-C6 alkynyl, halo C1-C6 alkyl, halo C2-C6 alkenyl, halo C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl or aryl C1-C6 alkyl, wherein, the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" and "aryl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring;
X₁₃, X₁₄ each independently represent H, halogen, cyano, C1-C6 alkoxy, C1-C6 alkoxy C1-C6 alkyl, C1-C6 alkyl carbonyl, C1-C6 alkoxy carbonyl, C1-C6 alkylsulfonyl, C1-C6 alkyl, C2-C6 alkenyl, C2-C6 alkynyl, C3-C6 cycloalkyl, C3-C6 cycloalkylalkyl, C3-C6 cycloalkenyl, C3-C6 cycloalkenyl C1-C6 alkyl, aryl, aryl C1-C6 alkyl, heterocyclyl or heterocyclyl C1-C6 alkyl, or C, X₁₃, X₁₄, taken together, form 5~8 membered carbocyclyl or oxygen, sulfur or nitrogen-containing heterocyclyl, or N, X₁₃, X₁₄, taken together, form heterocyclyl 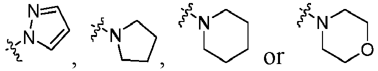 with nitrogen atom at 1-position, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C6 alkyl", "C3-C6 cycloalkenyl", "C3-C6 cycloalkenyl C1-C6 alkyl", "aryl", "aryl C1-C6 alkyl", "heterocyclyl" and "heterocyclyl C1-C6 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C6 alkyl)-(CO)OR₁₃, or two adjacent carbon atoms on the ring together with unsubstituted or halogen-substituted -OCH₂CH₂- or -OCH₂O- form a fused ring, the "5~8 membered carbocyclyl or oxygen, sulfur or nitrogen-containing heterocyclyl" is unsubstituted or substituted by 1, 2 or 3 groups selected from C1-C6 alkyl, C1-C6 alkoxy carbonyl and benzyl, or together with aryl or heterocyclyl forms a fused ring, the 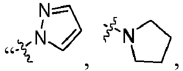 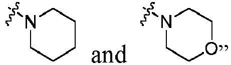 are unsubstituted or substituted by 1, 2 or 3 groups selected from oxo and C1-C6 alkyl.

5. The carboxylic acid derivative-substituted iminoaryl compound according to any of claims 1 to 4, which is **characterized in that**,
X represents -CX₁X₂-(C1-C3 alkyl)ₙ-, -(C1-C3 alkyl)-CX₁X₂-(C1-C3 alkyl)n- or -(CH₂)r-;
X₁, X₂ each independently represent H, halogen, cyano, amino, nitro, formyl, cyano C1-C3 alkyl, hydroxy C1-C3 alkyl, C1-C6 alkyl, C2-C6 alkenyl, C2-C6 alkynyl, C3-C6 cycloalkyl, C3-C6 cycloalkyl C1-C3 alkyl, C1-C6 alkoxy, C1-C6 alkylthio, C1-C6 alkylamino, halo C1-C6 alkoxy, halo C1-C6 alkylthio, C1-C6 alkyl carbonyl, C1-C6 alkoxy carbonyl, C1-C6 alkoxy C1-C3 alkyl, halo C1-C6 alkoxy C1-C3 alkyl, C1-C6 alkylamino C1-C3 alkyl, aryl, heterocyclyl, aryl C1-C3 alkyl or heterocyclyl C1-C3 alkyl, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C3 alkyl", "aryl", "heterocyclyl", "aryl C1-C3 alkyl" and "heterocyclyl C1-C3 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring; and X₁, X₂ are not hydrogen at the same time;
preferably, when the general formula is I, X₅ represents H, C1-C6 alkyl, C2-C6 alkenyl, C2-C6 alkynyl, C3-C6 cycloalkyl, C3-C6 cycloalkenyl, heterocyclyl, aryl, 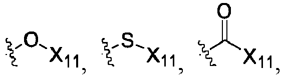 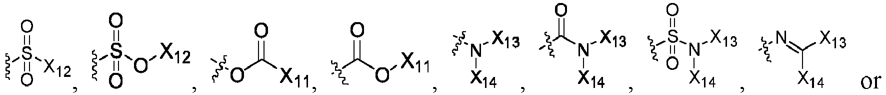 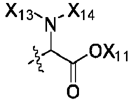 , wherein, the "C1-C6 alkyl", "C2-C6 alkenyl" and "C2-C6 alkynyl" are each independently unsubstituted or substituted by 1, 2 or 3 groups selected from halogen, cyano, nitro, C3-C6 cycloalkyl, C3-C6 cycloalkenyl, heterocyclyl, aryl, 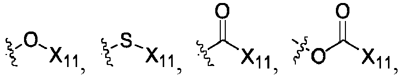 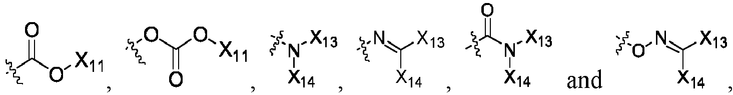 the "C3-C6 cycloalkyl", "C3-C6 cycloalkenyl", "heterocyclyl" and "aryl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring;
or N(X₅)₂ represents 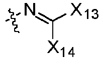 or heterocyclyl 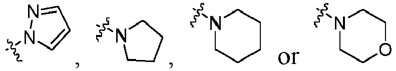 with nitrogen atom at 1-position that is unsubstituted or substituted by 1, 2 or 3 groups selected from oxo and C1-C6 alkyl;
X₁₁ independently represents H, C1-C6 alkyl, C2-C6 alkenyl, C2-C6 alkynyl, halo C1-C6 alkyl, halo C2-C6 alkenyl, halo C2-C6 alkynyl, C3-C6 cycloalkyl, C3-C6 cycloalkyl C1-C3 alkyl, C3-C6 cycloalkenyl, C3-C6 cycloalkenyl C1-C3 alkyl, heterocyclyl, heterocyclyl C1-C3 alkyl, aryl, aryl C1-C3 alkyl or 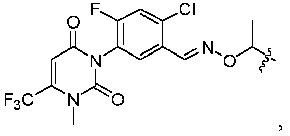 wherein, the "C3-C6 cycloalkyl", "C3-C6 cycloalkyl C1-C3 alkyl", "C3-C6 cycloalkenyl", "C3-C6 cycloalkenyl C1-C3 alkyl", "heterocyclyl", "heterocyclyl C1-C3 alkyl", "aryl" and "aryl C1-C3 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring;
X₁₂ independently represents C1-C6 alkyl, C2-C6 alkenyl, C2-C6 alkynyl, halo C1-C6 alkyl, halo C2-C6 alkenyl, halo C2-C6 alkynyl, C3-C6 cycloalkyl, C3-C6 cycloalkyl C1-C3 alkyl, C3-C6 cycloalkenyl, C3-C6 cycloalkenyl C1-C3 alkyl, heterocyclyl, heterocyclyl C1-C3 alkyl, aryl or aryl C1-C3 alkyl, wherein, the "C3-C6 cycloalkyl", "C3-C6 cycloalkyl C1-C3 alkyl", "C3-C6 cycloalkenyl", "C3-C6 cycloalkenyl C1-C3 alkyl", "heterocyclyl", "heterocyclyl C1-C3 alkyl", "aryl" and "aryl C1-C3 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring;
X₁₃, X₁₄ each independently represent H, halogen, cyano, C1-C6 alkoxy, C1-C6 alkoxy C1-C3 alkyl, C1-C6 alkyl carbonyl, C1-C6 alkoxy carbonyl, C1-C6 alkylsulfonyl, C1-C6 alkyl, C2-C6 alkenyl, C2-C6 alkynyl, C3-C6 cycloalkyl, C3-C6 cycloalkylalkyl, C3-C6 cycloalkenyl, C3-C6 cycloalkenyl C1-C3 alkyl, aryl, aryl C1-C3 alkyl, heterocyclyl or heterocyclyl C1-C3 alkyl, or C, X₁₃, X₁₄, taken together, form 5~8 membered saturated carbocyclyl, 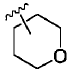 or 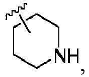 or N, X₁₃, X₁₄, taken together, form heterocyclyl 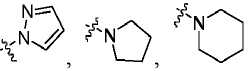 or 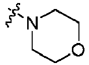 with nitrogen atom at 1-position, wherein, the "C1-C6 alkyl", "C2-C6 alkenyl" and "C2-C6 alkynyl" are each independently unsubstituted or substituted by halogen, the "C3-C6 cycloalkyl", "C3-C6 cycloalkyl C1-C3 alkyl", "C3-C6 cycloalkenyl", "C3-C6 cycloalkenyl C1-C3 alkyl", "aryl", "aryl C1-C3 alkyl", "heterocyclyl" and "heterocyclyl C1-C3 alkyl" are each independently unsubstituted or substituted by 1, 2 or 3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -OR₁₃, -SR₁₃, -(CO)OR₁₃, -(SO₂)R₁₃, -N(R₁₃)₂ and -O-(C1-C3 alkyl)-(CO)OR₁₃, or two adjacent carbon atoms on the ring together with unsubstituted or halogen-substituted -OCH₂CH₂- or -OCH₂O- form a fused ring, the "5~8 membered saturated carbocyclyl, 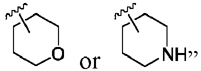 is unsubstituted or substituted by 1, 2 or 3 groups selected from C1-C6 alkyl, C1-C6 alkoxy carbonyl and benzyl, or together with phenyl or thienyl forms a fused ring, the ' 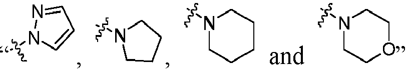 are unsubstituted or substituted by 1, 2 or 3 groups selected from oxo and C1-C6 alkyl;
more preferably, Q represents

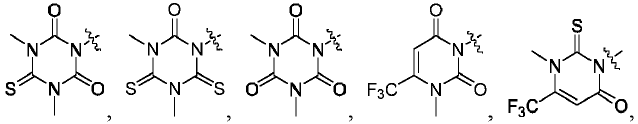

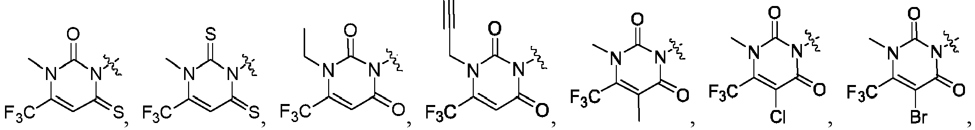

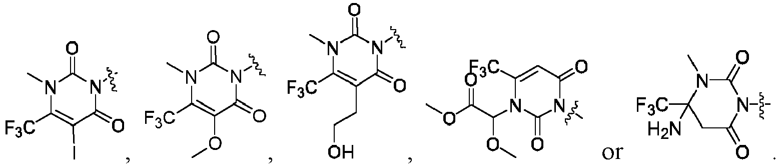

6. The carboxylic acid derivative-substituted iminoaryl compound according to any of claims 1 to 5, which is **characterized in that**, when the carbon atom connected to X₁ and X₂ in the general formula is a chiral center, it is in R configuration, and based on the content of stereoisomers having R and S configurations at this position, it has a stereochemical purity of 60-100% (R), preferably 70-100% (R), more preferably 80-100% (R), still more preferably 90-100% (R), further more preferably 95-100% (R); or preferably, it is any one selected from Table 1 and Table A.

7. A method for preparing the carboxylic acid derivative-substituted iminoaryl compound according to any of claims 1 to 6, which comprises the following steps:
subjecting a compound represented by general formula II and a compound represented by general formula III' to an elimination reaction to obtain a compound represented by general formula I', with the chemical reaction equation shown as follows: 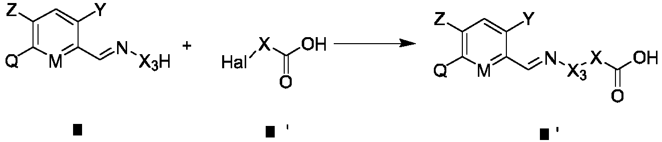
or, subjecting a compound represented by general formula II and a compound represented by general formula III to an elimination reaction to obtain a compound represented by general formula I, with the chemical reaction equation shown as follows: 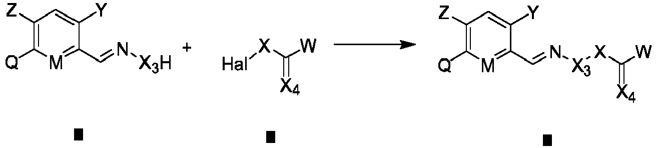
wherein, Hal represents halogen, other substituents Q, M, W, Y, Z, X, X₃ and X₄ are as defined in any of claims 1 to 6;
preferably, the reaction is carried out in the presence of a base and a solvent; more preferably, the base is at least one selected from inorganic bases and organic bases; more preferably, the solvent is at least one selected from DMF, methanol, ethanol, acetonitrile, dichloroethane, DMSO, Dioxane, dichloromethane and ethyl acetate.

8. A herbicidal composition, which is **characterized in that**, the composition comprises (i) at least one of the carboxylic acid derivative-substituted iminoaryl compounds according to any of claims 1 to 6 in a herbicidally effective amount; preferably, the composition further comprises (ii) one or more other herbicides in a herbicidally effective amount and/or safeners; more preferably, the composition further comprises (iii) a formulation auxiliary accepted in agricultural chemistry; still more preferably, the other herbicide is one or more selected from the following compounds and acids, salts and esters thereof:
(1) HPPD inhibitor selected from: topramezone, isoxaflutole, tembotrione, tefuryltrione, shuangzuocaotong, huanbifucaotong, sanzuohuangcaotong, benzuofucaotong and 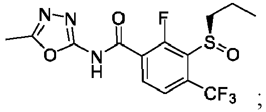
(2) PDS inhibitor selected from: flurtamone, diflufenican and picolinafen;
(3) DOXP inhibitor selected from: clomazone and bixlozone;
(4) ALS inhibitor selected from: tribenuron-methyl, thifensulfuron methyl, pyrazosulfuron-ethyl, thiencarbazone-methyl, halosulfuron methyl, rimsulfuron, nicosulfuron and imazamox;
(5) ACCase inhibitor selected from: clethodim, sethoxydim and quizalofop-P-methyl;
(6) PPO inhibitor selected from: oxyfluorfen, oxadiazon, oxadiargyl, sulfentrazone, pyraclonil, flumioxazin, saflufenacil, carfentrazone-ethyl and trifludimoxazin;
(7) PSII inhibitor selected from: metribuzin, terbuthylazine, amicarbazone, chlorotoluron, isoproturon, bromacil, propanil, desmedipham, phenmedipham, bentazone and bromoxynil;
(8) inhibitor of microtubule assembly selected from: butralin and pendimethalin;
(9) VLCFA inhibitor selected from: butachlor, pretilachlor, mefenacet, s-metolachlor, flufenacet, pyroxasulfone and anilofos;
(10) lipid synthesis inhibitor (non-acetyl-CoA carboxylase): prosulfocarb;
(11) Synthetic hormones selected from: 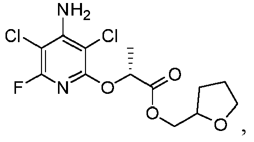 fluroxypyr, florpyrauxifen benzyl, halauxifen-methyl, triclopyr, clopyralid, picloram, aminopyralid, dicamba, 2-methyl-4-chlorophenoxyacetic acid and 2,4-dichlorophenoxy acetic acid;
(12) EPSPS inhibitor: glyphosate;
(13) GS inhibitor selected from: glufosinate ammonium and glufosinate-P-ammonium;
(14) PSI inhibitor selected from: paraquat dichloride and diquat dibromide monohydrate;
(15) Cellulose synthesis inhibitor selected from: triaziflam and indaziflam;
(16) other herbicides: cinmethylin.

9. A method for controlling an undesirable plant, **characterized in that** it comprises applying at least one of the carboxylic acid derivative-substituted iminoaryl compounds according to any of claims 1 to 6 or the herbicidal composition according to claim 8 in a herbicidally effective amount on a plant or in its area or to soil or water to control the emergence or growth of undesirable plant; preferably, the undesirable plant includes herbicide-resistant or tolerant weed species.

10. Use of at least one of the carboxylic acid derivative-substituted iminoaryl compounds according to any of claims 1 to 6 or the herbicidal composition according to claim 8 for controlling a undesirable plant; preferably, the carboxylic acid derivative-substituted iminoaryl compound is used to control a weed in a useful crop, the useful crop is a genetically modified crop or a crop treated by genome editing technique, the weed includes herbicide-resistant or tolerant weed species.
